(19) Europäisches Patentamt
European Patent Office
Office européen des brevets

(11) EP 4 163 283 A1

(12) **EUROPEAN PATENT APPLICATION**
published in accordance with Art. 153(4) EPC

(43) Date of publication:
**12.04.2023 Bulletin 2023/15**

(21) Application number: **21818029.7**

(22) Date of filing: **03.06.2021**

(51) International Patent Classification (IPC):
***C07D 487/04*** *(2006.01)* ***C07D 519/00*** *(2006.01)*
***A61K 31/519*** *(2006.01)* ***A61P 35/00*** *(2006.01)*
***A61P 35/02*** *(2006.01)* ***A61P 29/00*** *(2006.01)*
***A61P 37/02*** *(2006.01)* ***A61P 37/00*** *(2006.01)*

(52) Cooperative Patent Classification (CPC):
**A61K 31/519; A61K 31/529; A61P 9/10; A61P 19/02; A61P 29/00; A61P 35/00; A61P 35/02; A61P 37/00; A61P 37/02; C07D 487/04; C07D 498/22; C07D 519/00**

(86) International application number:
**PCT/CN2021/098124**

(87) International publication number:
**WO 2021/244609 (09.12.2021 Gazette 2021/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.06.2020 CN 202010498602**
**28.08.2020 CN 202010882652**
**24.03.2021 CN 202110311256**

(71) Applicant: **Scinnohub Pharmaceutical Co., Ltd**
**High-tech Development Zone**
**Chengdu, Sichuan 610041 (CN)**

(72) Inventors:
- **WANG, Jing**
  **Chengdu, Sichuan 610064 (CN)**
- **ZHAO, Shuchun**
  **Chengdu, Sichuan 610064 (CN)**
- **ZENG, Shaomei**
  **Chengdu, Sichuan 610064 (CN)**
- **WANG, Zao**
  **Chengdu, Sichuan 610064 (CN)**
- **WEI, Xuezhen**
  **Chengdu, Sichuan 610064 (CN)**
- **HUANG, Tingting**
  **Chengdu, Sichuan 610064 (CN)**
- **SHAO, Tao**
  **Chengdu, Sichuan 610064 (CN)**
- **ZHANG, Xiaodong**
  **Chengdu, Sichuan 610064 (CN)**
- **TANG, Jun**
  **Chengdu, Sichuan 610064 (CN)**

(74) Representative: **Nederlandsch Octrooibureau**
**P.O. Box 29720**
**2502 LS The Hague (NL)**

(54) **COMPOUND HAVING MACROCYCLIC STRUCTURE AND USE THEREOF**

(57) The present invention relates to a compound of formula (I) in which L', A, $X^1$, $X^2$, $X^3$, $R^1$, $R^2$, $R^3$, $R^4$, $R^5$, $R^6$, $R^9$, $R^{10}$, m, and n are as defined in the description, or a stereoisomer, a pharmaceutically acceptable salt, a solvate, or a tautomer thereof, and a use thereof as an RET, SRC and/or BTK kinase inhibitor in the treatment of diseases such as cancers.

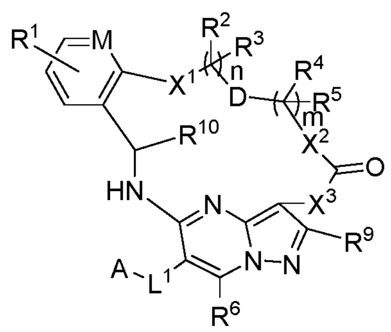

(I)

EP 4 163 283 A1

## Description

### Technical field

The present invention relates to a compound and a stereoisomer, a pharmaceutically acceptable salt, a solvate or a tautomer thereof, and a use thereof as inhibitors of kinases such as RET, BTK. More specifically, the present invention provides a novel compound and a stereoisomer thereof, as inhibitors of RET, SRC and/or BTK, as well as use thereof for the treatment of diseases mediated by RET, SRC, and/or BTK.

### Background

RET (rearranged during transfection) is a proto-oncogene located on chromosome 10. The RET protein, encoded by gene *RET,* is a receptor tyrosine kinase (RTK) on the cell membrane, as a member of the cadherin superfamily. Gene *RET* plays an important role in the development of the renal and intestinal nervous system during the embryonic stage and is critical for the homeostasis of a variety of tissues including neurons, neuroendocrine, hematopoietic tissues, and male germ cells. While the classical activation of RTK requires ligand-receptor interactions, the activation of RET requires an interaction between a ligand thereof (Glial cell derived neurotrophic factor family ligand, GFLS) and an accessory receptor (GFLS family receptor-a). The resulting GFLs-GFRα complex binds to the RET extracellular domain, which leads to phosphorylation of the intracellular tyrosine kinase domain, recruitment of relevant adapter proteins, activation of signaling cascades such as cell proliferation, and then activation of several pathways. The associated signaling pathways include MAPK, PI3K, JAK-STAT, PKA, PKC, etc.

There are two main mechanisms for the carcinogenic activation of RET, one is that the rearrangement of chromosomes produces a new fusion protein, usually relating to fusion of the kinase domain of RET and the protein containing the self-dimerization domain; and the other is the point mutation of *RET* gene. The mutated *RET* gene may encode a RET protein with abnormal activity, which can cause abnormal signaling and various effects, including cell growth, survival, invasion, metastasis, etc. Persistent signaling can cause excessive cell proliferation and induce a variety of cancers.

RET rearrangements are present in 1%-2% of patients with NSCLC and 5%-10% of patients with papillary thyroid cancer, and RET point mutations are present in 60% of patients with medullary thyroid cancer. The most common fusion types of RET are KIF5B-RET and CCDC6-RET, followed by NCOA4-RET and TRIM33-RET, and ZNF477P-RET, ERCC1-RET, HTR4-RET and CLIP1-RET are also reported.

Abnormal RET expression and/or activity has been demonstrated in different cancers and gastrointestinal disorders such as irritable bowel syndrome (IBS).

Currently, most of the anti-RET drugs are multikinase inhibitors, such as Vandetanib (primarily used for the treatment of symptomatic or progressive medullary thyroid cancers that are unresectable, locally advanced or metastatic) and Sorafenib (useful for liver cancer, kidney cancer, as well as locally recurrent or metastatic, progressive, radioiodine-refractory differentiated thyroid cancer). The drugs with a broad anti-cancer spectrum may have toxic side effects. For example, the most common adverse reactions (>20%) associated with Vandetanib were diarrhea, rashes, acne, nausea, hypertension, headache, fatigue, loss of appetite, and abdominal pain (Package Insert of Vandetanib, FDA); the most common adverse events of sorafenib were rashes (38%), diarrhea (37%), hand-foot skin reactions (35%), and fatigue (33%) (Package Insert of Sorafenib, FDA). The selective RET inhibitors, Selpercatinib and Pralsetinib, have been approved for use in thyroid cancer and non-small cell lung cancers (Package Inserts of Selpercatinib and Prasteinib, FDA). Given that not all patients with RET rearrangements/mutations respond to these drugs, it is necessary to develop potent inhibitors having high activity, less side effects, and high specificity, as well as targeting RET mutations and rearrangements.

The literatures on RET inhibitors have been successively published. For example, WO2019/126121 disclosed macrocyclic compounds as RET kinase inhibitors. The specific description of this patent is not considered as part of the present invention.

After antigens bind to B cell receptor (BCR) in the plasma membrane, the PLCG2 is phosphorylated at several specific sites and then downstream signaling is initiated through calcium mobilization, which in turn activates members of the protein kinase C (PKC) family. Phosphorylation of PLCG2 is closely associated with the adaptor B-cell linker protein BLNK, and BTK serves as a platform for several signaling proteins, and is involved in cytokine receptor signaling pathways. Moreover, BTK, as part of the Toll-like receptor (TLR) pathway, plays an important role in the function of innate immune cells and adaptive immunity. B-cell receptor (BCR)-dependent induction of BTK activates signaling pathways, mainly by pooling transcription factor NF-κB and nuclear factor of activated T-cells (NFAT). Both conditions are mediated by protein kinase C (PKC).

BTK kinases are involved in the transduction of a variety of important signals *in vivo* and their activation has important effects on multiple cellular processes. BTK blockade can lead to severe immunodeficiency and then affect B

cell maturation. During immune responses, BTK modulates B cell proliferation and apoptosis by mediating B cell activation and then inducing gene expression. Overexpression of BTK in normal human monocytes will promote the production of TNF-$\alpha$, whereas abnormal BTK gene expression decreases the production of TNF-$\alpha$, which allows BTK activation and thereby induces the production of proinflammatory cytokines by macrophages. The structure and activation mechanisms of BTK [4] make it become a target for a wide range of diseases, such as B cell malignancies, asthma, rheumatoid arthritis, and systemic lupus erythematosus.

The *c-Src* gene is the first proto-oncogene discovered. Non-receptor tyrosine kinases of Src family are present in almost all posterior cells, regulate the response of cells to external stimuli by regulating a variety of growth factors, cytokines, adhesion, and antigen receptors. The kinases of SRC family includes SRC, LCK, HCK, FYN, YES, FGR, BLK, LYN, and FRK. The kinases of Src family are typical modular signal transducer proteins, and contain conserved domains, including a myristoylated N-terminal fragment, followed by SH3, SH2 and tyrosine kinase domains, and a short C-terminal fragment. The protein tyrosine kinases (SFKs) of Src family, as an important class of non-receptor tyrosine kinases, play important roles in cell growth, differentiation, metastasis, and survival. Currently, the approved drugs targeting SFKs, such as Dasatinib, Bosutinib, Vandetanib, and Ponatinib, are all multikinase inhibitors.

Btk is a key molecule in B cell antigen receptor (BCR)-coupled signaling, and its activity is regulated by Lyn and Syk. Furthermore, studies show that kinases of Src family function in the upstream of Btk and are activated through a non phosphorylation-mediated mechanism (Ronen Gabizon, J. Med. Chem. 2020, 63, 5100-51011). BTK inhibitors inhibit B-cell lymphoma by suppressing cell proliferation, chemotaxis, and adhesion, and are mainly used for B-cell malignancies such as mantle cell lymphoma (MCL), chronic lymphocytic leukemia (CLL), and Waldenstrom macroglobulinemia (WM) (IMBRUCA, Summary Review, FDA@Drugs). The action mechanism of BTK inhibitors is binding to Cys-481 in the BTK (active) site to prevent BTK activation. At present, due to the presence of patients resistant to BTK C481S, there is an urgent need to develop effective drugs for resistant patients (Lian Xu, Blood, 2017, May 4; 129(18): 2519-2525)。

Furthermore, TRK inhibition has unique on-target side effects, including dizziness, weight gain, ataxia, sensory abnormalities, and withdrawal pain when treatment is discontinued or terminated. Compounds of interest in the present invention have a low TRK inhibitory effect, thereby can mitigate the associated side effects.

New compounds that can be used to prevent and/or treat RET, SRC, and/or BTK-mediated diseases such as cancer, autoimmune diseases are needed.

## Content of the invention

In one aspect, the present invention provides a compound of formula (I) or a stereoisomer, a pharmaceutically acceptable salt, a solvate or a tautomer thereof:

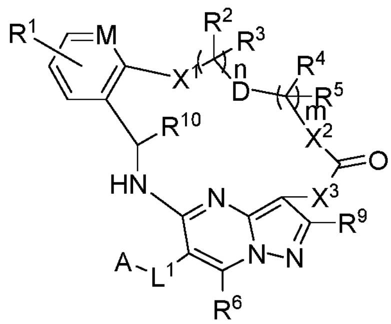

(I)

$L^1$ is selected from absence, O, S, NH, N($C_{1-6}$alkyl), $(CH_2)_n$NH, C(O), hydrogen;

A is selected from absence, substituted or unsubstituted 3- to 10-membered cycloalkyl, substituted or unsubstituted 3- to 10-membered heterocycloalkyl, substituted or unsubstituted 6- to 8-membered monoaryl, substituted or unsubstituted 5- to 10-membered monoheteroaryl and substituted or unsubstituted 8- to 10-membered fused heteroaryl; when said groups are substituted, the substituent(s) is or is selected from oxo, halo, amino, hydroxyl, cyano, $C_{1-6}$alkyl, $C_{3-6}$ cycloalkyl, $C_{3-6}$ heterocycloalkyl, oxo$C_{3-6}$ heterocycloalkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-(CH_2)_qC(O)NHR^a$, $-C(O)NR^aR^b$, halo$C_{1-6}$alkyl, hydroxyl$C_{1-6}$alkyl and amino$C_{1-6}$alkyl; wherein the heteroatom(s) in said heterocycloalkyl, monoheteroaryl, fused heteroaryl is N, O or S; when $L^1$ is hydrogen, A is absent;

alternatively, the substituent on said cycloalkyl, heterocycloalkyl, aryl, heteroaryl, fused heteroaryl, and the adjacent

atom in the ring are linked to form a ring;

M is selected from N and CH;

each of $X^1$, $X^2$, and $X^3$ is independently selected from absence, O, S, $S(O)_2$, NH, an $N(C_{1-6}alkyl)$;

D is selected from absence, substituted or unsubstituted 3- to 10-membered cycloalkyl, or 3- to 10-membered heterocycloalkyl, wherein the heteroatom(s) is N, O, S; when said groups are substituted, the substituent(s) is or is selected from oxo, halo, amino, hydroxyl, cyano, $C_{1-6}$alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-6}$alkyl, hydroxyl$C_{1-6}$alkyl and amino$C_{1-6}$alkyl;

$R^1$, $R^2$, $R^3$, $R^4$ and $R^5$ are each independently selected from hydrogen, halo, amino, hydroxyl, cyano, $C_{1-6}$alkyl, $C_{1-6}$ alkoxyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-6}$alkyl, hydroxyl$C_{1-6}$ alkyl and amino$C_{1-6}$alkyl;

alternatively, $R^2$ and $R^3$, together with the carbon atom to which they are attached, form a 3- to 7-membered cycloalkyl or 3- to 7-membered heterocycloalkyl, and said cycloalkyl or heterocycloalkyl may be substituted by halo, amino, hydroxyl, cyano, $C_{1-6}$alkyl, $C_{1-6}$ alkoxyl, di($C_{1-6}$alkyl)amino, halo$C_{1-6}$alkyl, hydroxyl$C_{1-6}$alkyl and amino$C_{1-6}$alkyl;

alternatively, $R^4$ and $R^5$, together with the carbon atom to which they are attached, form a 3- to 7-membered cycloalkyl or 3- to 7-membered heterocycloalkyl, and said cycloalkyl or heterocycloalkyl may be substituted by halo, amino, hydroxyl, cyano, $C_{1-6}$alkyl, $C_{1-6}$ alkoxyl, di($C_{1-6}$alkyl)amino, halo$C_{1-6}$alkyl, hydroxyl$C_{1-6}$alkyl and amino$C_{1-6}$alkyl;

alternatively, $R^4$ and $R^5$, together with the carbon atom to which they are attached and $X^2$, form the following structure:

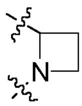
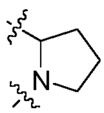
or
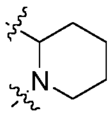
;

$R^6$ is selected from hydrogen, amino, hydroxyl, halo, cyano, substituted or unsubstituted 3- to 10-membered cycloalkyl, 3- to 10-membered heterocycloalkyl, 6- to 8-membered monoaryl, 5- to 10-membered monoheteroaryl and 8- to 10-membered fused heteroaryl, $-NR^7R^8$, $-NHR^7$, $-(CH_2)_qNR^aR^b$, $-NHC(O)OR^7$, $-NHC(O)NHR^a$, $-NHC(O)R^7$, $-OR^7$, $-OC(O)OR^7$, $-OC(O)R^7$, $-C(O)R^7$, $-C(O)NHR^7$, and $-C(O)NR^7R^8$; when said groups are substituted, the substituent(s) is or is selected from oxo, halo, amino, hydroxyl, cyano, $C_{1-6}$alkyl, 5- to 6-membered aryloxy, 5- to 6-membered heteroaryloxy, $-NH(CH_2)_qR^a$, $-N(CH_2)_qR^aR^b$, $-NC(O)OR^a$, $-NC(O)R^a$, $-OR^a$, $-OC(O)OR^a$, $-OC(O)R^a$, $-C(O)NHR^a$, $-C(O)NR^aR^b$, halo$C_{1-6}$alkyl, hydroxyl$C_{1-6}$ alkyl and amino$C_{1-6}$alkyl; alternatively, the substituent on said cycloalkyl, heterocycloalkyl, aryl, heteroaryl, fused heteroaryl, and the adjacent atom in the ring are linked to form a ring;

$R^7$ and $R^8$ are selected from $C_{1-4}$ alkyl, halo$C_{1-4}$ alkyl, hydroxyl$C_{1-4}$ alkyl, amino$C_{1-4}$ alkyl, halo$C_{1-4}$ alkyl, substituted or unsubstituted 3- to 10-membered cycloalkyl, 3- to 10-membered heterocycloalkyl, 6-to 8-membered monoaryl, 5- to 10-membered monoheteroaryl, 8- to 10-membered fused heteroaryl; the substituent(s) is oxo, halo, amino, hydroxyl, cyano, $C_{1-6}$alkyl, 5- to 6-membered aryloxy, 5- to 6-membered heteroaryloxy, $-NH(CH_2)_qR^a$, $-N(CH_2)_qR^aR^b$, $-NC(O)OR^a$, $-NC(O)R^a$, $-OR^a$, $-OC(O)OR^a$, $-OC(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl; alternatively, the substituent on said cycloalkyl, heterocycloalkyl, aryl, heteroaryl, and fused heteroaryl and the adjacent atom in the ring are linked to form a ring;

$R^9$ and $R^{10}$ are independently selected from hydrogen, $C_{1-6}$alkyl, halo$C_{1-6}$alkyl, halogen and cyano; preferably, said $C_{1-6}$alkyl is selected from methyl, ethyl, propyl, isopropyl, n-butyl, and isobutyl;

$R^a$ and $R^b$ are independently selected from $C_{1-4}$ alkyl, $C_{1-4}$ alkyl, $C_{5-6}$ aryl, $C_{5-6}$ heteroaryl, and $C_{1-4}$ alkylsulfonyl, in which said $C_{1-4}$ alkyl, $C_{5-6}$ aryl, and $C_{5-6}$ heteroaryl can be substituted by halo, amino, $C_{3-6}$ cycloalkyl, $C_{3-6}$ heterocycloalkyl, wherein the heteroatom(s) is N, O, or S;

m, n, and q are each independently selected from 0, 1, 2, 3, and 4;

$L^1$ and $R^6$ are not both hydrogen.

In an embodiment, the present invention provides the compound of above general formula (I) or a stereoisomer, a pharmaceutically acceptable salt, a solvate or a tautomer thereof, wherein

$L^1$ is selected from absence, O, S, NH, $N(C_{1-6}alkyl)$, $(CH_2)_nNH$, C(O);

A is selected from substituted or unsubstituted 3- to 6-membered cycloalkyl, substituted or unsubstituted 3- to 6-membered heterocycloalkyl, substituted or unsubstituted 6- to 8-membered monoaryl, substituted or unsubstituted 5- to 7-membered monoheteroaryl and substituted or unsubstituted 8- to 10-membered fused heteroaryl; when said groups are substituted, the substituent(s) is selected from oxo, halo, amino, hydroxyl, cyano, $C_{1-6}$alkyl, $C_{3-6}$ cycloalkyl,

$C_{3-6}$ heterocycloalkyl, oxo$C_{3-6}$ heterocycloalkyl, 5- to 6-membered aryloxy, 5- to 6-membered heteroaryloxy, -$NR^aR^b$, -$NHR^a$, -$(CH_2)_qNR^aR^b$, -NHC(O)$OR^a$, -NHC(O)$NHR^a$, -NHC(O)$R^a$, -$OR^a$, -OC(O)$OR^a$, -OC(O)$R^a$, -C(O)$R^a$, -C(O)$NHR^a$, -$(CH_2)_q$C(O)$NHR^a$, -C(O)$NR^aR^b$, halo$C_{1-6}$alkyl, hydroxyl$C_{1-6}$ alkyl and amino$C_{1-6}$alkyl; wherein the heteroatom(s) in said heterocycloalkyl, monoheteroaryl, fused heteroaryl is N, O or S;

$R^a$ and $R^b$ are independently selected from $C_{1-4}$ alkyl, phenyl, $C_{1-4}$ alkylsulfonyl, in which said $C_{1-4}$ alkyl can be substituted by halo, amino, and $C_{3-6}$ cycloalkyl;

$X^1$ is selected from absence, O, S, and $S(O)_2$; $X^2$is selected from NH and N($C_{1-6}$alkyl); and $X^3$ is selected from absence or NH;

D is selected from absence, substituted or unsubstituted 3- to 6-membered cycloalkyl or 3- to 6-membered heterocycloalkyl, and the heteroatom(s) in said cycloalkyl is N, O; the substituent(s) is halo, amino, hydroxyl, $C_{1-4}$ alkyl and halo$C_{1-4}$ alkyl;

$R^1$ is halo, amino, $C_{1-4}$ alkyl or halo$C_{1-4}$ alkyl;

$R^2$, $R^3$, $R^4$, and $R^5$ are each independently selected from hydrogen, $C_{1-6}$alkyl or halo$C_{1-6}$alkyl;

$R^6$ is selected from hydrogen, amino, -$NR^7R^8$ or -$NHR^7$;

$R^7$ and $R^8$ are selected from $C_{1-4}$ alkyl and halo$C_{1-4}$ alkyl;

$R^9$ is selected from hydrogen, $C_{1-6}$alkyl, halo$C_{1-6}$alkyl, halogen and cyano; preferably, said $C_{1-6}$alkyl is selected from methyl, ethyl, propyl, isopropyl, n-butyl, and isobutyl;

$R^{10}$ is selected from hydrogen, $C_{1-6}$alkyl; preferably, said $C_{1-6}$alkyl is selected from methyl, ethyl, propyl, isopropyl, n-butyl, and isobutyl;

m is selected from 0, 1 or 2;

n is selected from 0, 1 or 2.

q is selected from 0, 1 or 2.

In an embodiment, the present invention provides the compound of above general formula (I) or a stereoisomer, a pharmaceutically acceptable salt, a solvate or a tautomer thereof, wherein $L^1$ is selected from absence, O, S, NH, N($C_{1-6}$alkyl), $(CH_2)_n$NH, C(O); preferably, $L^1$ is absent or NH; more preferably, $L^1$ is absent.

In an embodiment, the present invention provides the compound of above general formula (I) or a stereoisomer, a pharmaceutically acceptable salt, a solvate or a tautomer thereof, wherein A is selected from absence, substituted or unsubstituted 3- to 10-membered cycloalkyl, substituted or unsubstituted 3-to 10-membered heterocycloalkyl, substituted or unsubstituted 6- to 8-membered monoaryl, substituted or unsubstituted 5- to 10-membered monoheteroaryl, and substituted or unsubstituted 8- to 10-membered fused heteroaryl; when said groups are substituted, the substituent(s) is or is selected from oxo, halo, amino, hydroxyl, cyano, $C_{1-6}$alkyl, $C_{3-6}$ cycloalkyl, $C_{3-6}$ heterocycloalkyl, oxo$C_{3-6}$ heterocycloalkyl, 5- to 6-membered aryloxy, 5- to 6-membered heteroaryloxy, -$NR^aR^b$, -$NHR^a$, -$(CH_2)_qNR^aR^b$, -NHC(O)$OR^a$, -NHC(O)$NHR^a$, -NHC(O)$R^a$, -$OR^a$, -OC(O)$OR^a$, -OC(O)$R^a$, -C(O)$R^a$, -C(O)$NHR^a$, C(O)$NR^aR^b$, halo$C_{1-6}$alkyl, hydroxyl$C_{1-6}$ alkyl and amino$C_{1-6}$alkyl; wherein the heteroatom(s) is N, O or S.

In an embodiment, the present invention provides the compound of above general formula (I) or a stereoisomer, a pharmaceutically acceptable salt, a solvate or a tautomer thereof, wherein A is selected from substituted or unsubstituted 3- to 6-membered cycloalkyl, substituted or unsubstituted 3- to 6-membered heterocycloalkyl, substituted or unsubstituted 6- to 8-membered monoaryl, substituted or unsubstituted 5- to 7-membered monoheteroaryl, and substituted or unsubstituted 8- to 10-membered fused heteroaryl; when said groups are substituted, the substituent(s) is selected from oxo, halo, amino, hydroxyl, cyano, $C_{1-6}$alkyl, $C_{3-6}$ cycloalkyl, $C_{3-6}$ heterocycloalkyl, oxo$C_{3-6}$ heterocycloalkyl, 5- to 6-membered aryloxy, 5- to 6-membered heteroaryloxy, -$NR^aR^b$, -$NHR^a$, -$(CH_2)_qNR^aR^b$, -NHC(O)$OR^a$, -NHC(O)$NHR^a$, -NHC(O)$R^a$, -$OR^a$, -OC(O)$OR^a$, -OC(O)$R^a$, -C(O)$R^a$, -C(O)$NHR^a$, -$(CH_2)_q$C(O)$NHR^a$, -C(O)$NR^aR^b$, halo$C_{1-6}$alkyl, hydroxyl$C_{1-6}$ alkyl and amino$C_{1-6}$alkyl; wherein the heteroatom(s) in said heterocycloalkyl, monoheteroaryl, fused heteroaryl is N, O or S;

preferably, A is selected from substituted or unsubstituted 3- to 6-membered cycloalkyl, substituted or unsubstituted 3- to 6-membered heterocycloalkyl, substituted or unsubstituted phenyl, and substituted or unsubstituted 5- to 6-membered monoheteroaryl; when said groups are substituted, the substituent(s) is selected from halo, amino, hydroxyl, cyano, $C_{1-6}$alkyl, $C_{3-6}$ cycloalkyl, $C_{3-6}$ heterocycloalkyl, oxo$C_{3-6}$ heterocycloalkyl, 5- to 6-membered aryloxy, 5- to 6-membered heteroaryloxy, -$NR^aR^b$, -$NHR^a$, -$(CH_2)_qNR^aR^b$, -NHC(O)$OR^a$, -NHC(O)$NHR^a$, -NHC(O)$R^a$, -$OR^a$, -OC(O)$OR^a$, -OC(O)$R^a$, -C(O)$R^a$, -C(O)$NHR^a$, -$(CH_2)_q$C(O)$NHR^a$, -C(O)$NR^aR^b$, halo$C_{1-6}$alkyl, hydroxyl$C_{1-6}$ alkyl and amino$C_{1-6}$alkyl; wherein the heteroatom(s) in said heterocycloalkyl, monoheteroaryl, fused heteroaryl is N, O or S;

$R^a$ and $R^b$ are independently selected from $C_{1-4}$ alkyl, phenyl, and $C_{1-4}$ alkylsulfonyl, wherein said $C_{1-4}$ alkyl may be substituted by halo, amino, and $C_{3-6}$ cycloalkyl;

or

more preferably, A is selected from substituted or unsubstituted following groups: phenyl, pyridyl, cyclopentyl, cyclohexyl, cyclobutyl, cyclopropyl,

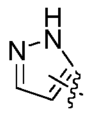 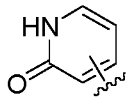 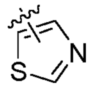 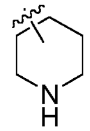 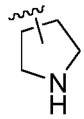 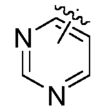

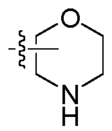 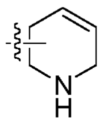 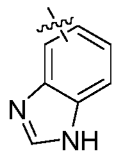 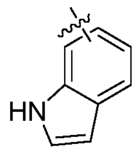 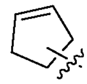 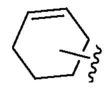

when said groups are substituted, the substituent(s) is selected from oxo, halo, amino, hydroxyl, cyano, $C_{1-4}$ alkyl, substituted or unsubstituted 5- to 6-membered aryloxy, 5- to 6-membered heteroaryloxy, $-NR^aR^b$, $-NHR^a$, $-(CH_2)_qN-R^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl, and amino$C_{1-4}$ alkyl; the heteroatom(s) in said heteroaryl is N, O, or S;

$R^a$ and $R^b$ are independently selected from $C_{1-4}$ alkyl; preferably, $R^a$ and $R^b$ are independently selected from methyl, ethyl, propyl, isopropyl, n-butyl, isobutyl, and tert-butyl;

q is selected from 0, 1, and 2;

or

more preferably, A is selected from substituted or unsubstituted following groups: phenyl, cyclopentenyl, cyclohexenyl and pyrazolyl; preferably, phenyl;

when said groups are substituted, the substituent(s) is selected from halo, amino, $C_{1-4}$ alkyl, phenoxy, $-NR^aR^b$, $-NHR^a$, $-NHC(O)OR^a$, $-NHC(O)NHR^a$, $-OR^a$, $-C(O)NHR^a$, and $-(CH_2)_qC(O)NHR^a$;

$R^a$ and $R^b$ are independently selected from $C_{1-4}$ alkyl;

q is selected from 1 and 2.

In an embodiment, the present invention provides the compound of above general formula (I) or a stereoisomer, a pharmaceutically acceptable salt, a solvate or a tautomer thereof, wherein when group A is a substituted group, it is substituted by 1, 2 or 3 substituents; preferably, A is selected from phenyl or cyclohexenyl, which is mono-substituted by said substituents at o-, m-, or p-position, or disubstituted at m- or p-position.

In an embodiment, the present invention provides the compound of above general formula (I) or a stereoisomer, a pharmaceutically acceptable salt, a solvate or a tautomer thereof, wherein M is N.

In an embodiment, the present invention provides the compound of above general formula (I) or a stereoisomer, a pharmaceutically acceptable salt, a solvate or a tautomer thereof, wherein $X^1$ is selected from absence, O, S, and $S(O)_2$; $X^2$ is selected from NH and $N(C_{1-6}alkyl)$; $X^3$ is selected from absence or NH;

preferably, $X^1$ is O; $X^2$ is NH; $X^3$ is absent.

In an embodiment, the present invention provides the compound of above general formula (I) or a stereoisomer, a pharmaceutically acceptable salt, a solvate or a tautomer thereof, wherein D is selected from absence, substituted or unsubstituted 3- to 6-membered cycloalkyl or 3- to 6-membered heterocycloalkyl, and the heteroatom(s) in said heterocycloalkyl is N, O; the substituent(s) is halo, amino, hydroxyl, $C_{1-4}$ alkyl and halo$C_{1-4}$ alkyl; preferably, D is selected from cyclopropyl, cyclobutyl, cyclopentyl, oxacyclopropyl, oxocyclopropyl, oxocyclobutyl, oxocyclopentyl, aziridinyl, azetidinyl or pyrrolidinyl;

preferably, D is absent.

In an embodiment, the present invention provides the compound of above general formula (I) or a stereoisomer, a pharmaceutically acceptable salt, a solvate or a tautomer thereof, wherein $R^1$ locates at m-position of M, and preferably, $R^1$ is halo, amino, $C_{1-4}$ alkyl or halo$C_{1-4}$ alkyl; preferably, halogen, and more preferably, F or Cl.

In an embodiment, the present invention provides the compound of above general formula (I) or a stereoisomer, a pharmaceutically acceptable salt, a solvate or a tautomer thereof, wherein $R^2$, $R^3$, $R^4$, and $R^5$ are each independently selected from hydrogen, $C_{1-6}$alkyl or halo$C_{1-6}$alkyl;

preferably, $R^2$ is selected from hydrogen, $C_{1-6}$alkyl; and $R^3$, $R^4$, and $R^5$ are hydrogen;

In an embodiment, the present invention provides the compound of above general formula (I) or a stereoisomer, a pharmaceutically acceptable salt, a solvate or a tautomer thereof, wherein $R^4$, and $R^5$, together with the carbon to which they are attached and $X^2$, form the following structure:

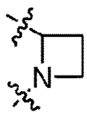 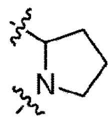 or 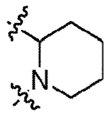,

preferably,

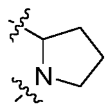.

In an embodiment, the present invention provides the compound of above general formula (I) or a stereoisomer, a pharmaceutically acceptable salt, a solvate or a tautomer thereof, wherein $R^6$ is selected from hydrogen, amino, $-NR^7R^8$ or $-NHR^7$; preferably, $R^6$ is selected from hydrogen and amino;

$R^7$ and $R^8$ are selected from $C_{1-4}$ alkyl and halo$C_{1-4}$ alkyl.

In an embodiment, the present invention provides the compound of above general formula (I) or a stereoisomer, a pharmaceutically acceptable salt, a solvate or a tautomer thereof, wherein $R^9$ is selected from hydrogen, $C_{1-6}$alkyl, halo$C_{1-6}$alkyl, halogen and cyano; $R^{10}$ is selected from hydrogen and $C_{1-6}$alkyl;

preferably, $R^9$ is hydrogen; $R^{10}$ is selected from hydrogen and $C_{1-6}$alkyl; more preferably, $R^9$ and $R^{10}$ are both hydrogen.

In an embodiment, the present invention provides the compound of above general formula (I) or a stereoisomer, a pharmaceutically acceptable salt, a solvate or a tautomer thereof, wherein m is selected from 0, 1 or 2, and is preferably 1; n is selected from 0, 1 or 2, and is preferably 1.

In an embodiment, the present invention provides the compound of above general formula (I) or a stereoisomer, a pharmaceutically acceptable salt, a solvate or a tautomer thereof, wherein

$L^1$ is absent;
A is selected from substituted or unsubstituted following groups: phenyl, cyclopentenyl, cyclohexenyl and pyrazolyl; when said groups are substituted, they are substituted by 1 or 2 substituents selected from halo, amino, $C_{1-4}$ alkyl, phenoxy, $-NR^aR^b$, $-NHR^a$, $-NHC(O)OR^a$, $-NHC(O)NHR^a$, $-OR^a$, $-C(O)NHR^a$, and $-(CH_2)_qC(O)NHR^a$;
$R^a$ and $R^b$ are independently selected from $C_{1-4}$ alkyl;
q is selected from 1 and 2;
M is N;
$X^1$ is O; $X^2$ is NH; $X^3$ is absent;
D is absent;
$R^1$ is halogen;
$R^2$ is selected from hydrogen, $C_{1-6}$alkyl; and $R^3$, $R^4$ and $R^5$ are hydrogen;
$R^6$ is selected from hydrogen and amino;
$R^9$ is hydrogen;
$R^{10}$ is selected from hydrogen and $C_{1-6}$alkyl;
m is 1, and n is 1.

In an embodiment, the present invention provides the compound of formula (I) having the structure of formula (Ia) or a stereoisomer, a pharmaceutically acceptable salt, a solvate or a tautomer thereof,

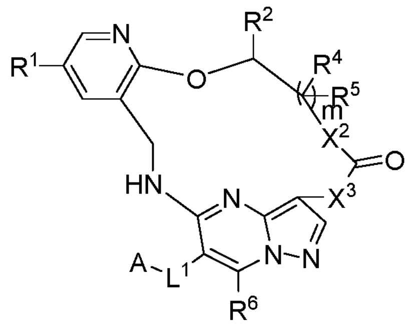

(Ia)

wherein $L^1$, A, $X^2$, $X^3$, $R^1$, $R^2$, $R^4$, $R^5$, $R^6$, and m are as defined in any embodiment of above formula (I).

In an embodiment, the present invention provides the compound of formula (I) having the structure of formula (Ib) or a stereoisomer, a pharmaceutically acceptable salt, a solvate or a tautomer thereof, wherein

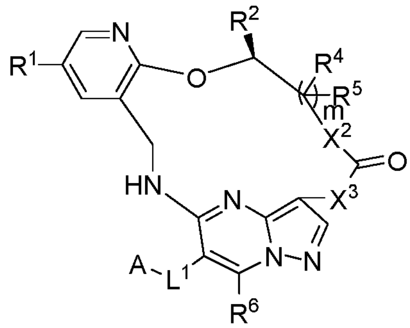

(Ib)

wherein $R^2$ is $C_{1-6}$alkyl, and preferably methyl;
$L^1$, A, $X^2$, $X^3$, $R^1$, $R^4$, $R^5$, $R^6$, m are as defined in any embodiment of above formula (I).

In another aspect, the present invention provides the compound of formula (I') or a stereoisomer thereof,

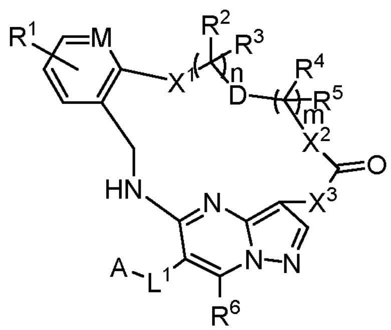

(I')

wherein

$L^1$ is selected from absence, O, S, NH, N($C_{1-6}$alkyl), $(CH_2)_n$NH, and hydrogen;
A is selected from absence, substituted or unsubstituted 3- to 10-membered cycloalkyl, 3- to 10-membered heterocycloalkyl, 6- to 8-membered monoaryl, 5- to 10-membered monoheteroaryl and 8-to 10-membered fused heteroaryl; the substituent(s) is oxo, halo, amino, hydroxyl, cyano, $C_{1-6}$alkyl, 5- to 6-membered aryloxy, 5- to 6-membered

heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-6}$alkyl, hydroxyl$C_{1-6}$ alkyl and amino$C_{1-6}$alkyl; the heteroatom(s) is N, O, or S;

when $L^1$ is a hydrogen, A is absent;

alternatively, the substituent on said cycloalkyl, heterocycloalkyl, aryl, heteroaryl, and fused heteroaryl, and the adjacent atom in the ring are linked to form a ring;

M is selected from N and CH;

$X^1$, $X^2$, and $X^3$ are dependently selected from absence, O, S, $S(O)_2$, NH;

D is selected from absence, substituted or unsubstituted 3- to 10-membered cycloalkyl or 3- to 10-membered heterocycloalkyl, the heteroatom(s) is N, O, S; the substituent(s) is oxo, halo, amino, hydroxyl, cyano, $C_{1-6}$alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-6}$alkyl, hydroxyl$C_{1-6}$alkyl and amino$C_{1-6}$alkyl;

$R^1$, $R^2$, $R^3$, $R^4$, and $R^5$ are each independently selected from hydrogen, halo, amino, hydroxyl, cyano, $C_{1-6}$alkyl, $C_{1-6}$ alkoxyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-6}$alkyl, hydroxyl$C_{1-6}$ alkyl and amino$C_{1-6}$alkyl;

alternatively, $R^2$ and $R^3$, together with the carbon to which they are attached, form a 3- to 7-membered cycloalkyl or 3- to 7-membered heterocycloalkyl, and said cycloalkyl or heterocycloalkyl may be substituted by halo, amino, hydroxyl, cyano, $C_{1-6}$alkyl, $C_{1-6}$ alkoxyl, di($C_{1-6}$alkyl)amino, halo$C_{1-6}$alkyl, hydroxyl$C_{1-6}$alkyl and amino$C_{1-6}$alkyl;

alternatively, $R^4$ and $R^5$, together with the carbon to which they are attached, form a 3- to 7-membered cycloalkyl or 3- to 7-membered heterocycloalkyl, and said cycloalkyl or heterocycloalkyl may be substituted by halo, amino, hydroxyl, cyano, $C_{1-6}$alkyl, $C_{1-6}$ alkoxyl, di($C_{1-6}$alkyl)amino, halo$C_{1-6}$alkyl, hydroxyl$C_{1-6}$alkyl and amino$C_{1-6}$alkyl;

$R^6$ is selected from hydrogen, amino, hydroxyl, halo, cyano, substituted or unsubstituted 3- to 10-membered cycloalkyl, 3- to 10-membered heterocycloalkyl, 6- to 8-membered monoaryl, 5- to 10-membered monoheteroaryl, and 8- to 10-membered fused heteroaryl, $-NR^7R^8$, $-NHR^7$, $-(CH_2)_qNR^aR^b$, $-NHC(O)OR^7$, $-NHC(O)NHR^a$, $-NHC(O)R^7$, $-OR^7$, $-OC(O)OR^7$, $-OC(O)R^7$, $-C(O)R^7$, $-C(O)NHR^7$, and $-C(O)NR^7R^8$; the substituent(s) is oxo, halo, amino, hydroxyl, cyano, $C_{1-6}$alkyl, 5- to 6-membered aryloxy, 5- to 6-membered heteroaryloxy, $-NH(CH_2)_qR^a$, $-N(CH_2)_qR^aR^b$, $-NC(O)OR^a$, $-NC(O)R^a$, $-OR^a$, $-OC(O)OR^a$, $-OC(O)R^a$, $-C(O)NHR^a$, $-C(O)NR^aR^b$, halo$C_{1-6}$alkyl, hydroxyl$C_{1-6}$ alkyl and amino$C_{1-6}$alkyl; alternatively, the substituent on said cycloalkyl, heterocycloalkyl, aryl, heteroaryl, and fused heteroaryl, and the adjacent atom in the ring are linked to form a ring;

$R^7$ and $R^8$ are selected from $C_{1-4}$ alkyl, halo$C_{1-4}$ alkyl, hydroxyl$C_{1-4}$ alkyl, amino$C_{1-4}$ alkyl, halo$C_{1-4}$ alkyl, substituted or unsubstituted 3- to 10-membered cycloalkyl, 3- to 10-membered heterocycloalkyl, 6- to 8-membered monoaryl, 5- to 10-membered monoheteroaryl, and 8- to 10-membered fused heteroaryl; the substituent(s) is oxo, halo, amino, hydroxyl, cyano, $C_{1-6}$alkyl, 5- to 6-membered aryloxy, 5-to 6-membered heteroaryloxy, $-NH(CH_2)_qR^a$, $-N(CH_2)_qR^aR^b$, $-NC(O)OR^a$, $-NC(O)R^a$, $-OR^a$, $-OC(O)OR^a$, $-OC(O)R^a$, $-C(O)NHR^a$, $-C(O)NR^aR^b$, halo$C_{1-4}$alkyl, hydroxyl$C_{1-4}$ alkyl and amino$C_{1-4}$ alkyl; alternatively, the substituent on said cycloalkyl, heterocycloalkyl, aryl, heteroaryl, and fused heteroaryl, and the adjacent atom in the ring are linked to form a ring;

$R^a$ and $R^b$ are independently selected from $C_{1-4}$ alkyl;

m, n, and q are each independently selected from 0, 1, 2, 3, 4;

$L^1$ and $R^6$ are not both hydrogen.

Further, the present invention provides the compound of formula (I') or a stereoisomer thereof, wherein:

$L^1$ is selected from absence, O, NH, N($C_{1-6}$alkyl), $(CH_2)_nNH$;

A is selected from substituted or unsubstituted 3- to 6-membered cycloalkyl, 3- to 6-membered heterocycloalkyl, 6- to 8-membered monoaryl, 5- to 7-membered monoheteroaryl; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl; the heteroatom(s) is N, O, S;

alternatively, the substituent on said cycloalkyl, heterocycloalkyl, aryl, heteroaryl, and fused heteroaryl, and the adjacent atom in the ring are linked to form a ring;

M is selected from N;

$X^1$, $X^2$, and $X^3$ are independently selected from O, NH or absence;

D is selected from absence, substituted or unsubstituted 3- to 6-membered cycloalkyl or 3- to 6-membered heterocycloalkyl, and said heteroatom is N, O, S; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl,

hydroxyl$C_{1-4}$ alkyl and amino$C_{1-4}$ alkyl;

$R^1$, $R^2$, $R^3$, $R^4$, and $R^5$ are each independently selected from hydrogen, halo, amino, hydroxyl, cyano, $C_{1-4}$ alkyl, $C_{1-4}$ alkoxyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl;

alternatively, $R^2$ and $R^3$, together with the carbon to which they are attached, form 3- to 7-membered cycloalkyl or 3- to 7-membered heterocycloalkyl, and said cycloalkyl or heterocycloalkyl may be substituted by halo, amino, $C_{1-4}$ alkyl, $C_{1-4}$ alkoxyl, di($C_{1-4}$ alkyl)amino, halo$C_{1-4}$ alkyl, hydroxyl$C_{1-4}$ alkyl and amino$C_{1-4}$ alkyl;

alternatively, $R^4$ and $R^5$, together with the carbon to which they are attached, form a 3- to 7-membered cycloalkyl or heterocycloalkyl, and said cycloalkyl or heterocycloalkyl may be substituted by halo, amino, $C_{1-4}$ alkyl, $C_{1-4}$ alkoxyl, di($C_{1-4}$ alkyl)amino, halo$C_{1-4}$ alkyl, hydroxyl$C_{1-4}$ alkyl and amino$C_{1-4}$ alkyl;

$R^6$ is selected from hydrogen, amino, hydroxyl, halo, cyano, substituted or unsubstituted 3- to 10-membered cycloalkyl, 3- to 10-membered heterocycloalkyl, 6- to 8-membered monoaryl, 5- to 10-membered monoheteroaryl and 8- to 10-membered fused heteroaryl, $-NR^7R^8$, $-NHR^7$, $-(CH_2)_qNR^aR^b$, $-NHC(O)OR^7$, $-NHC(O)NHR^a$, $-NHC(O)R^7$, $-OR^7$, $-OC(O)OR^7$, $-OC(O)R^7$, $-C(O)R^7$, $-C(O)NHR^7$, $-C(O)NR^7R^8$; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl; alternatively, the substituent(s) on said cycloalkyl, heterocycloalkyl, aryl, heteroaryl, and fused heteroaryl, and the adjacent atom in the ring are linked to form a ring;

$R^7$ and $R^8$ are selected from $C_{1-4}$ alkyl, halo$C_{1-4}$ alkyl, hydroxyl$C_{1-4}$ alkyl, amino$C_{1-4}$ alkyl, halo$C_{1-4}$ alkyl, substituted or unsubstituted 3- to 10-membered cycloalkyl, 3- to 10-membered heterocycloalkyl, 6-to 8-membered monoaryl, 5- to 10-membered monoheteroaryl, 8- to 10-membered fused heteroaryl; and the substituent(s) is oxo, halo, amino, hydroxyl, cyano, $C_{1-4}$ alkyl, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl; alternatively, the substituent(s) on said cycloalkyl, heterocycloalkyl, aryl, heteroaryl, and fused heteroaryl, and the adjacent atom in the ring are linked to form a ring;

$R^a$ and $R^b$ are independently selected from $C_{1-4}$ alkyl;

m, n, and q are each independently selected from 0, 1, and 2.

Further, the present invention provides the compound of formula (I') or a stereoisomer thereof, wherein:

$R^1$ is halo, amino, $C_{1-4}$ alkyl, and halo$C_{1-4}$ alkyl;

$R^2$, $R^3$, $R^4$, $R^5$are each independently selected from hydrogen, halo, amino, $C_{1-4}$ alkyl, $C_{1-4}$ alkoxyl, di($C_{1-4}$ alkyl)amino, halo$C_{1-4}$ alkyl, hydroxyl$C_{1-4}$ alkyl and amino$C_{1-4}$ alkyl.

Further, the present invention provides the compound of formula (I') or a stereoisomer thereof, which has the structure of following formula (II):

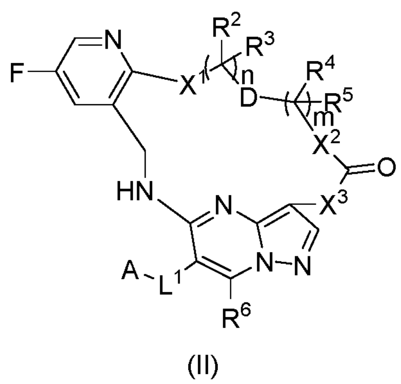

(II)

$L^1$ is selected from absence, O, NH, N($C_{1-6}$alkyl), and $(CH_2)_nNH$;

A is selected from substituted or unsubstituted 3- to 6-membered cycloalkyl, 3- to 6-membered heterocycloalkyl, 6- to 8-membered monoaryl, and 5- to 7-membered monoheteroaryl; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$,

halo$C_{1-4}$ alkyl, hydroxyl$C_{1-4}$ alkyl and amino$C_{1-4}$ alkyl; the heteroatom(s) is N, O, S;

alternatively, the substituent on said cycloalkyl, heterocycloalkyl, aryl, heteroaryl, and fused heteroaryl, and the adjacent atom in the ring are linked to form a ring;

$X^1$ is selected from absence or O; $X^2$ is selected from absence or NH; $X^3$ is selected from absence or NH;

D is selected from absence, substituted or unsubstituted 3- to 6-membered cycloalkyl or 3- to 6-membered heterocycloalkyl, and said heteroatom is N, O, S; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl;

$R^1$ is halo, amino, $C_{1-4}$ alkyl, and halo$C_{1-4}$ alkyl;

$R^2$, $R^3$, $R^4$, and $R^5$ are each independently selected from hydrogen, halo, amino, $C_{1-4}$ alkyl, $C_{1-4}$ alkoxyl, di($C_{1-4}$ alkyl)amino, halo$C_{1-4}$ alkyl, hydroxyl$C_{1-4}$ alkyl and amino$C_{1-4}$ alkyl;

$R^6$ is selected from hydrogen, amino, hydroxyl, halo, cyano, substituted or unsubstituted 3- to 6-membered cycloalkyl, 3- to 6-membered heterocycloalkyl, 6- to 8-membered monoaryl, 5- to 7-membered monoaryl, $-NR^7R^8$, $-NHR^7$, $-(CH_2)_qNR^aR^b$, $-NHC(O)OR^7$, $-NHC(O)NHR^a$, $-NHC(O)R^7$, $-OR^7$, $-OC(O)OR^7$, $-OC(O)R^7$, $-C(O)R^7$, $-C(O)NHR^7$, $-C(O)NR^7R^8$; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl;

alternatively, the substituent on said cycloalkyl, heterocycloalkyl, aryl, heteroaryl, and fused heteroaryl, and the adjacent atom in the ring are linked to form a ring;

$R^7$ and $R^8$ are selected from $C_{1-4}$ alkyl, halo$C_{1-4}$ alkyl, hydroxyl$C_{1-4}$ alkyl, amino$C_{1-4}$ alkyl, halo$C_{1-4}$ alkyl, substituted or unsubstituted 3- to 10-membered cycloalkyl, 3- to 10-membered heterocycloalkyl, 6-to 8-membered monoaryl, 5- to 10-membered monoheteroaryl, 8- to 10-membered fused heteroaryl; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl;

alternatively, the substituent on said cycloalkyl, heterocycloalkyl, aryl, heteroaryl, and fused heteroaryl, and the adjacent atom in the ring are linked to form a ring;

$R^a$ and $R^b$ are dependently selected from $C_{1-4}$ alkyl;

m, n, and q are selected from 0, 1, and 2.

Further, the compound of formula (I') according to the present invention has the structure of following formula (II):

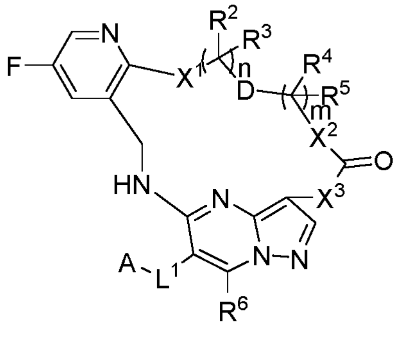

(II)

$L^1$ is selected from absence, O, NH, N($C_{1-6}$alkyl), and $(CH_2)_nNH$;

A is selected from substituted or unsubstituted 3- to 6-membered cycloalkyl, 3- to 6-membered heterocycloalkyl, 6- to 8-membered monoaryl, and 5- to 7-membered monoheteroaryl; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl; the heteroatom(s) is N, O, S;

alternatively, the substituent on said cycloalkyl, heterocycloalkyl, aryl, heteroaryl, and fused heteroaryl, and the adjacent atom in the ring are linked to form a ring;

$X^1$ is selected from absence or O; $X^2$ is selected from absence or NH; $X^3$ is selected from absence or NH;

D is selected from absence, substituted or unsubstituted 3- to 6-membered cycloalkyl or 3- to 6-membered heterocycloalkyl, and the heteroatom(s) is N, O, S; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$,

-NHC(O)NHR$^a$, -NHC(O)R$^a$, -OR$^a$, -OC(O)OR$^a$, -OC(O)R$^a$, -C(O)R$^a$, -C(O)NHR$^a$, -C(O)NR$^a$R$^b$, halo$C_{1-4}$ alkyl, hydroxyl$C_{1-4}$ alkyl and amino$C_{1-4}$ alkyl;

$R^1$ is halo, amino, $C_{1-4}$ alkyl, and halo$C_{1-4}$ alkyl;

$R^2$, $R^3$, $R^4$, and $R^5$ are each independently selected from hydrogen, halo, amino, $C_{1-4}$ alkyl, $C_{1-4}$ alkoxyl, di($C_{1-4}$ alkyl)amino, halo$C_{1-4}$ alkyl, hydroxyl$C_{1-4}$ alkyl and amino$C_{1-4}$ alkyl;

$R^6$ is selected from hydrogen, amino, hydroxyl, halo, cyano, substituted or unsubstituted 3- to 6-membered cycloalkyl, 3- to 6-membered heterocycloalkyl, 6- to 8-membered monoaryl, 5- to 7-membered monoaryl, -NR$^7$R$^8$, -NHR$^7$, -$(CH_2)_q$NR$^a$R$^b$, -NHC(O)OR$^7$, -NHC(O)NHR$^a$, -NHC(O)R$^7$, -OR$^7$, -OC(O)OR$^7$, -OC(O)R$^7$, -C(O)R$^7$, -C(O)NHR$^7$, -C(O)NR$^7$R$^8$; the substituent(s) is oxo, halo, amino, hydroxyl, cyano, $C_{1-4}$ alkyl, 5- to 6-membered aryloxy, 5- to 6-membered heteroaryloxy, -NR$^a$R$^b$, -NHR$^a$, -$(CH_2)_q$NR$^a$R$^b$, -NHC(O)OR$^a$, -NHC(O)NHR$^a$, -NHC(O)R$^a$, -OR$^a$, -OC(O)OR$^a$, -OC(O)R$^a$, -C(O)R$^a$, -C(O)NHR$^a$, -C(O)NR$^a$R$^b$, halo$C_{1-4}$ alkyl, hydroxyl$C_{1-4}$ alkyl and amino$C_{1-4}$ alkyl;

alternatively, the substituent on said cycloalkyl, heterocycloalkyl, aryl, heteroaryl, and fused heteroaryl, and the adjacent atom in the ring are linked to form a ring;

$R^7$ and $R^8$ are selected from $C_{1-4}$ alkyl, halo$C_{1-4}$ alkyl, hydroxyl$C_{1-4}$ alkyl, amino$C_{1-4}$ alkyl, halo$C_{1-4}$ alkyl, substituted or unsubstituted 3- to 10-membered cycloalkyl, 3- to 10-membered heterocycloalkyl, 6-to 8-membered monoaryl, 5- to 10-membered monoheteroaryl, and 8- to 10-membered fused heteroaryl; the substituent(s) is oxo, halo, amino, hydroxyl, cyano, $C_{1-4}$ alkyl, 5- to 6-membered aryloxy, 5- to 6-membered heteroaryloxy, -NR$^a$R$^b$, -NHR$^a$, -$(CH_2)_q$N-R$^a$R$^b$, -NHC(O)OR$^a$, -NHC(O)NHR$^a$, -NHC(O)R$^a$, -OR$^a$, -OC(O)OR$^a$, -OC(O)R$^a$, -C(O)R$^a$, -C(O)NHR$^a$, -C(O)NR$^a$R$^b$, halo$C_{1-4}$ alkyl, hydroxyl$C_{1-4}$ alkyl and amino$C_{1-4}$ alkyl;

alternatively, the substituent on said cycloalkyl, heterocycloalkyl, aryl, heteroaryl, and fused heteroaryl, and the adjacent atom in the ring are linked to form a ring;

$R^a$ and $R^b$ are independently selected from $C_{1-4}$ alkyl;

m, n, and q are selected from 0, 1, and 2.

Further, the compound of formula (I') according to the present invention or a stereoisomer thereof has the structure of following formula (III):

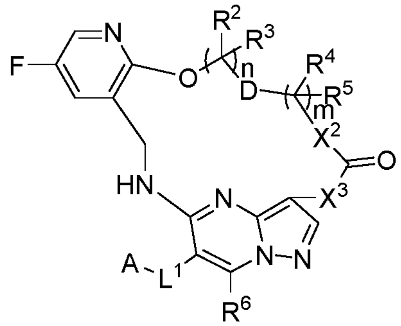

(III)

wherein each variable is defined as above.

Further, the present invention provides the compound of formula (III) or a stereoisomer thereof, characterized in that:

A is selected from substituted or unsubstituted 3- to 6-membered cycloalkyl, 3- to 6-membered heterocycloalkyl, 5- to 6-membered aryl or heteroaryl; the substituent(s) is oxo, halo, amino, hydroxyl, cyano, $C_{1-4}$ alkyl, substituted or unsubstituted 5- to 6-membered aryloxy, 5- to 6-membered heteroaryloxy, -NR$^a$R$^b$, -NHR$^a$, -$(CH_2)_q$NR$^a$R$^b$, -NHC(O)OR$^a$, -NHC(O)NHR$^a$, -NHC(O)R$^a$, -OR$^a$, -OC(O)OR$^a$, -OC(O)R$^a$, -C(O)R$^a$, -C(O)NHR$^a$, -C(O)NR$^a$R$^b$, halo$C_{1-4}$ alkyl, hydroxyl$C_{1-4}$ alkyl and amino$C_{1-4}$ alkyl; the heteroatom(s) is N, O, S; preferably, said halo$C_{1-4}$ alkyl is $CF_3$;

alternatively, the substituent on said cycloalkyl, heterocycloalkyl, aryl, heteroaryl, and fused heteroaryl, and the adjacent atom in the ring are linked to form a ring;

$R^a$ and $R^b$ are independently selected from $C_{1-4}$ alkyl; preferably, $R^a$ and $R^b$ are independently selected from methyl, ethyl, propyl, isopropyl, n-butyl, isobutyl, and tert-butyl.

Further, the present invention provides compound of above formula (I') or a stereoisomer thereof, characterized in that it has the structure of following formula (IV):

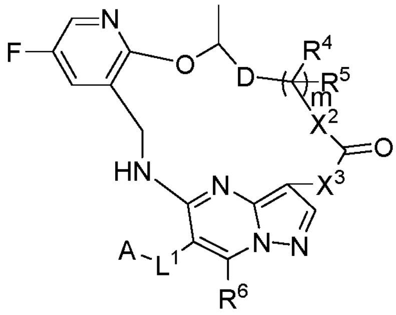

(IV)

$L^1$ is selected from absence, O, S, NH, N($C_{1-6}$alkyl), ($CH_2$) $_n$NH, C(O), and hydrogen;
A is selected from substituted or unsubstituted 3- to 6-membered cycloalkyl, 3- to 6-membered heterocycloalkyl, 5- to 6-membered aryl or heteroaryl; the substituent(s) is oxo, halo, amino, hydroxyl, cyano, $C_{1-4}$ alkyl, substituted or unsubstituted 5- to 6-membered aryloxy, 5- to 6-membered heteroaryloxy, $C_{3-6}$ cycloalkyl, $C_{3-6}$ heterocycloalkyl, oxo$C_{3-6}$ heterocycloalkyl, -$NR^aR^b$, -$NHR^a$, -$(CH_2)_qNR^aR^b$, -NHC(O)$OR^a$, -NHC(O)$NHR^a$, -NHC(O)$R^a$, -$OR^a$, -OC(O)$OR^a$, -OC(O)$R^a$, -C(O)$R^a$, -C(O)$NHR^a$, -C(O)$NR^aR^b$, halo$C_{1-4}$ alkyl, hydroxyl$C_{1-4}$ alkyl and amino$C_{1-4}$ alkyl; the heteroatom(s) is N, O, S;
alternatively, the substituent on said cycloalkyl, heterocycloalkyl, aryl, heteroaryl, and fused heteroaryl, and the adjacent atom in the ring are linked to form a ring;
$R^4$ and $R^5$ are selected from hydrogen or $C_{1-4}$ alkyl;
$X^2$ and $X^3$ are each independently selected from absence or NH;
D is selected from absence, substituted or unsubstituted 3- to 6-membered cycloalkyl or 3- to 6-membered heterocycloalkyl, and the heteroatom(s) is N, O; the substituent(s) is halo, amino, hydroxyl, $C_{1-4}$ alkyl and halo$C_{1-4}$ alkyl; preferably, D is selected from cyclopropyl, cyclobutyl, cyclopentyl, oxacyclopropyl, oxocyclopropyl, oxocyclobutyl, oxocyclopentyl, aziridinyl, azetidinyl, and pyrrolidinyl; aziridinyl
$R^6$ is selected from hydrogen, amino, hydroxyl, halo, cyano, substituted or unsubstituted 3- to 10-membered cycloalkyl, 3- to 10-membered heterocycloalkyl, 6- to 8-membered monoaryl, 5- to 10-membered monoheteroaryl and 8- to 10-membered fused heteroaryl, -$NR^7R^8$, -$NHR^7$, -$(CH_2)_qNR^aR^b$, -NHC(O)$OR^7$, -NHC(O)$NHR^a$, -NHC(O)$R^7$, -$OR^7$, -OC(O)$OR^7$, -OC(O)$R^7$, -C(O)$R^7$, -C(O)$NHR^7$, and -C(O)$NR^7R^8$; the substituent(s) is oxo, halo, amino, hydroxyl, cyano, $C_{1-4}$ alkyl, 5- to 6-membered aryloxy, 5- to 6-membered heteroaryloxy, -$NR^aR^b$, -$NHR^a$, -$(CH_2)_qNR^aR^b$, -NHC(O)$OR^a$, -NHC(O)$NHR^a$, -NHC(O)$R^a$, -$OR^a$, -OC(O)$OR^a$, -OC(O)$R^a$, -C(O)$R^a$, -C(O)$NHR^a$, -C(O)$NR^aR^b$, halo$C_{1-4}$ alkyl, hydroxyl$C_{1-4}$ alkyl and amino$C_{1-4}$ alkyl;
alternatively, the substituent on said cycloalkyl, heterocycloalkyl, aryl, heteroaryl, and fused heteroaryl, and the adjacent atom in the ring are linked to form a ring;
$R^7$ and $R^8$ are selected from $C_{1-4}$ alkyl, halo$C_{1-4}$ alkyl, hydroxyl$C_{1-4}$ alkyl, amino$C_{1-4}$ alkyl, halo$C_{1-4}$ alkyl, substituted or unsubstituted 3- to 10-membered cycloalkyl, 3- to 10-membered heterocycloalkyl, 6- to 8-membered monoaryl, 5- to 10-membered monoheteroaryl, and 8- to 10-membered fused heteroaryl; the substituent(s) is oxo, halo, amino, hydroxyl, cyano, $C_{1-4}$ alkyl, 5- to 6-membered aryloxy, 5- to 6-membered heteroaryloxy, -$NR^aR^b$, -$NHR^a$, -$(CH_2)_qNR^aR^b$, -NHC(O)$OR^a$, -NHC(O)$NHR^a$, -NHC(O)$R^a$, -$OR^a$, -OC(O)$OR^a$, -OC(O)$R^a$, -C(O)$R^a$, -C(O)$NHR^a$, -C(O)$NR^aR^b$, halo$C_{1-4}$ alkyl, hydroxyl$C_{1-4}$ alkyl and amino$C_{1-4}$ alkyl; alternatively, the substituent on said cycloalkyl, heterocycloalkyl, aryl, heteroaryl, and fused heteroaryl, and the adjacent atom in the ring are linked to form a ring;
$R^a$ and $R^b$ are independently selected from $C_{1-4}$ alkyl, $C_{1-4}$ alkyl, $C_{5-6}$aryl, $C_{5-6}$heteroaryl, and $C_{1-4}$ alkylsulfonyl, wherein said $C_{1-4}$ alkyl, $C_{5-6}$ aryl, and $C_{5-6}$ heteroaryl may be substituted by halo, amino, $C_{3-6}$cycloalkyl, and $C_{3-6}$ heterocycloalkyl, wherein the heteroatom(s) is N, O, S;
m and q are selected from 0, 1, and 2;

Further, the present invention provides the above compounds or stereoisomers, in which $R^a$ and $R^b$ are independently selected from methyl, ethyl, propyl, butyl, isopropyl, tert-butyl, cyclopropyl, methylcyclopropyl, cyclobutyl, and pyridinyl.

Further, the present invention provides the above compounds or stereoisomers, characterized in that they have the structure of formula (IV):

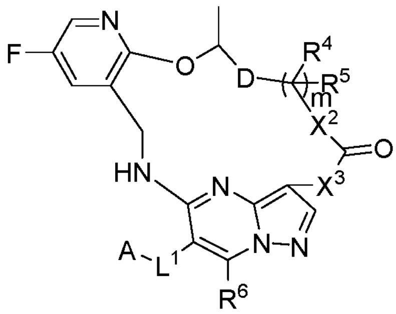

(IV)

$L^1$ is selected from absence, O, NH, N($C_{1-6}$alkyl), ($CH_2$) $_n$NH;
A is selected from substituted or unsubstituted 3- to 6-membered cycloalkyl, 3- to 6-membered heterocycloalkyl, 5- to 6-membered aryl or heteroaryl; the substituent(s) is oxo, halo, amino, hydroxyl, cyano, $C_{1-4}$ alkyl, substituted or unsubstituted 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl; the heteroatom(s) is N, O, S;
alternatively, the substituent on said cycloalkyl, heterocycloalkyl, aryl, heteroaryl, and fused heteroaryl, and the adjacent atom in the ring are linked to form a ring;
$R^4$ and $R^5$ are selected from hydrogen or $C_{1-4}$ alkyl;
$X^2$ and $X^3$ are each independently selected from absence or NH;
D is selected from absence, substituted or unsubstituted 3- to 6-membered cycloalkyl or 3- to 6-membered heterocycloalkyl, and the heteroatom(s) is N, O; the substituent(s) is halo, amino, hydroxyl, $C_{1-4}$ alkyl, and halo$C_{1-4}$ alkyl; preferably, D is selected from cyclopropyl, cyclobutyl, cyclopentyl, oxacyclopropyl, oxocyclopropyl, oxocyclobutyl, oxocyclopentyl, aziridinyl, azetidinyl, and pyrrolidinyl;
$R^6$ is selected from hydrogen, amino, hydroxyl, halo, cyano, substituted or unsubstituted 3- to 10-membered cycloalkyl, 3- to 10-membered heterocycloalkyl, 6- to 8-membered monoaryl, 5- to 10-membered monoheteroaryl and 8- to 10-membered fused heteroaryl, $-NR^7R^8$, $-NHR^7$, $-(CH_2)_qNR^aR^b$, $-NHC(O)OR^7$, $-NHC(O)NHR^a$, $-NHC(O)R^7$, $-OR^7$, $-OC(O)OR^7$, $-OC(O)R^7$, $-C(O)R^7$, $-C(O)NHR^7$, $-C(O)NR^7R^8$; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl;
alternatively, the substituent on said cycloalkyl, heterocycloalkyl, aryl, heteroaryl, and fused heteroaryl, and the adjacent atom in the ring are linked to form a ring;
$R^7$ and $R^8$ are selected from $C_{1-4}$ alkyl, halo$C_{1-4}$ alkyl, hydroxyl$C_{1-4}$ alkyl, amino$C_{1-4}$ alkyl, halo$C_{1-4}$ alkyl, substituted or unsubstituted 3- to 10-membered cycloalkyl, 3- to 10-membered heterocycloalkyl, 6- to 8-membered monoaryl, 5- to 10-membered monoheteroaryl, and 8- to 10-membered fused heteroaryl; the substituent(s) is oxo, halo, amino, hydroxyl, cyano, $C_{1-4}$ alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl; alternatively, the substituent on said cycloalkyl, heterocycloalkyl, aryl, heteroaryl, and fused heteroaryl, and the adjacent atom in the ring are linked to form a ring;
$R^a$ and $R^b$ are independently selected from $C_{1-4}$ alkyl; preferably, $R^a$ and $R^b$ are independently selected from methyl, ethyl, propyl, isopropyl, n-butyl, isobutyl, and tert-butyl.
m and q are selected from 0, 1, and 2;
preferably, the present invention provides the above compounds or stereoisomer thereof, characterized in that:

D is absent;
$R^4$ and $R^5$ are each independently hydrogen;
$X^2$ is NH;
$X^3$ is absent;
$R^6$ is hydrogen;
m is 1.

Preferably, the present invention provides the above compounds or stereoisomer thereof, characterized in that: $L^1$ is absent, and $R^6$ is amino.

Preferably, the present invention provides the above compounds or stereoisomer thereof, characterized in that A is substituted or unsubstituted benzene ring, pyridine ring, cyclopentyl, cyclohexyl, cyclobutyl, cyclopropyl,

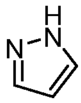, 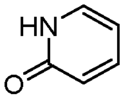, 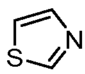, 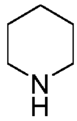, 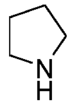, 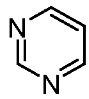, 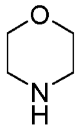,

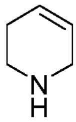, 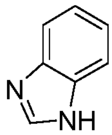, 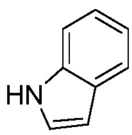, 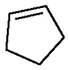, 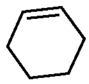;

preferably, A is selected from substituted or unsubstituted benzene ring;

the substituent(s) is oxo, halo, amino, hydroxyl, cyano, $C_{1-4}$ alkyl, substituted or unsubstituted 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl, and amino$C_{1-4}$ alkyl; the heteroatom(s) is N, O, S;

$R^a$ and $R^b$ are independently selected from $C_{1-4}$ alkyl; preferably, $R^a$ and $R^b$ are independently selected from methyl, ethyl, propyl, isopropyl, n-butyl, isobutyl, and tert-butyl;

q is selected from 0, 1, and 2.

Preferably, the present invention provides the above compounds or stereoisomer thereof, characterized in that A is substituted or unsubstituted benzene ring, pyridine ring, cyclopentyl, cyclohexyl, cyclobutyl, cyclopropyl,

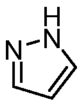, 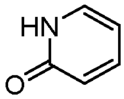, 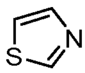, 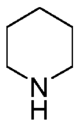, 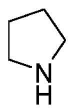, 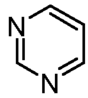, 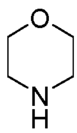, 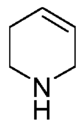;

preferably, A is selected from substituted or unsubstituted benzene ring;

the substituent(s) is oxo, halo, amino, hydroxyl, cyano, $C_{1-4}$ alkyl, substituted or unsubstituted 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl; the heteroatom(s) is N, O, S;

$R^a$ and $R^b$ are independently selected from $C_{1-4}$ alkyl; preferably, $R^a$ and $R^b$ are independently selected from methyl, ethyl, propyl, isopropyl, n-butyl, isobutyl, and tert-butyl;

q is selected from 0, 1, and 2.

When the preceding embodiments are further defined by the following embodiments, only part of technical features may be further defined. It should be understood that the undefined technical features in the following embodiments have the same definitions as defined in the previous embodiments.

Preferably, the present invention provides the following compounds or stereoisomer thereof:

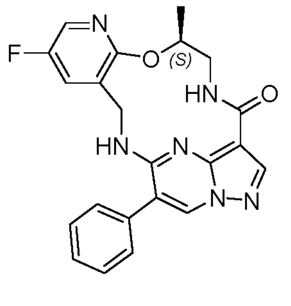

1

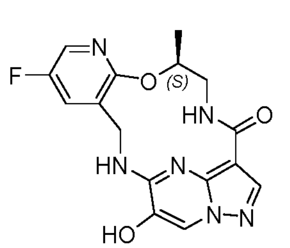

2

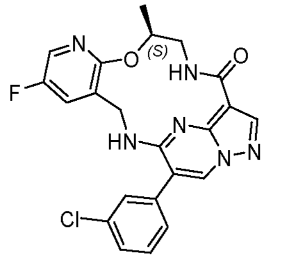

3

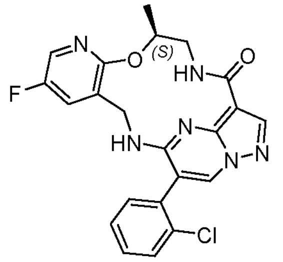

4

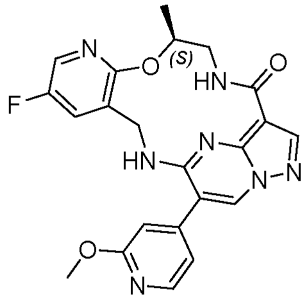

5

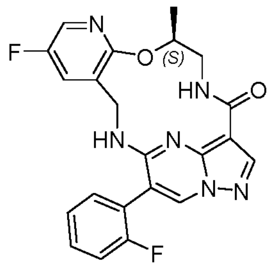

6

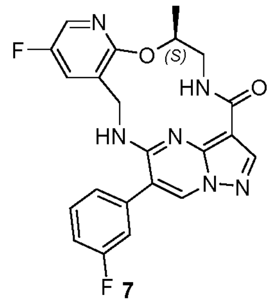

7

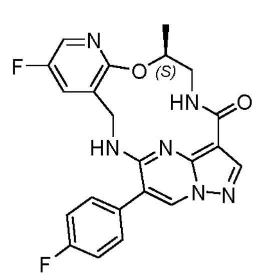

8

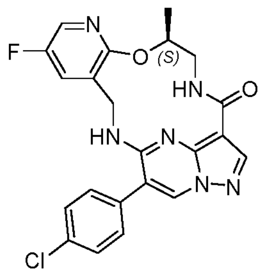

9

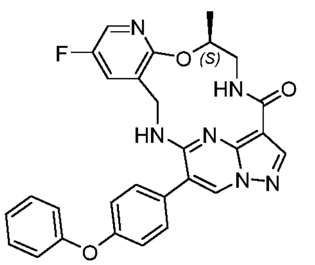

10

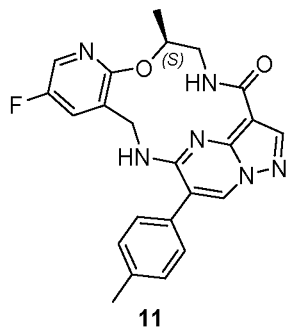

11

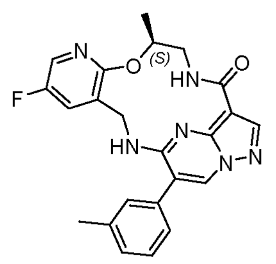

12

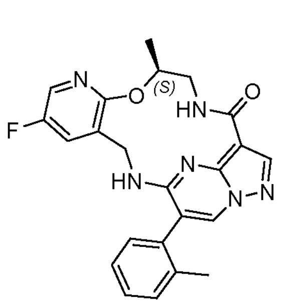

13

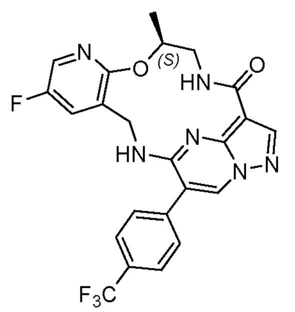

14

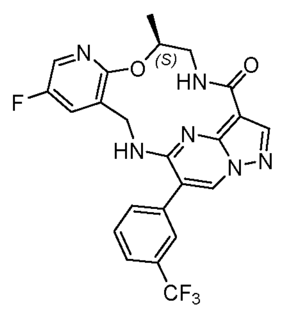

15

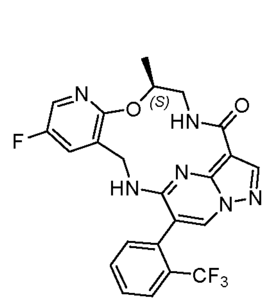

16

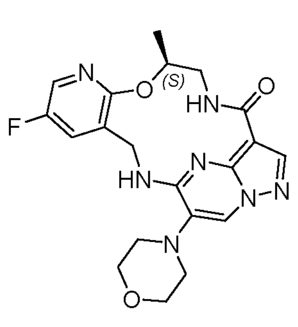

17

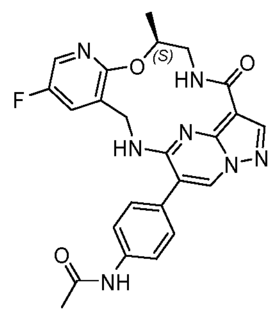

18

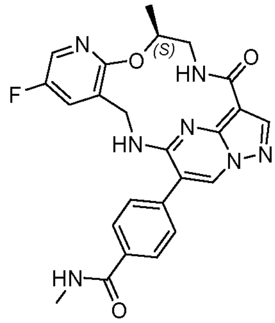

19

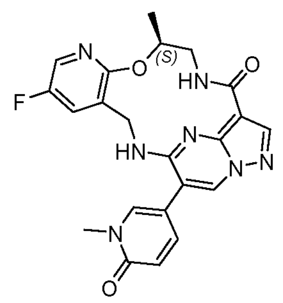

20

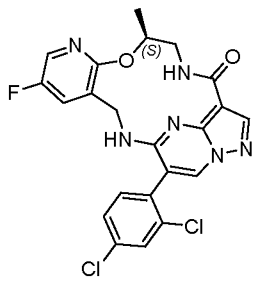

21

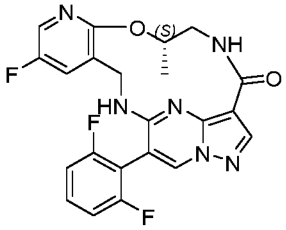

22

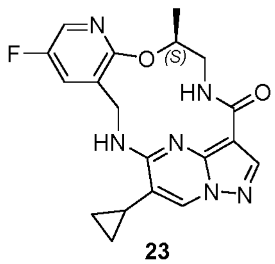

23

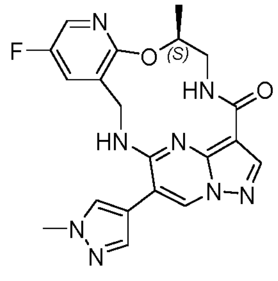

24

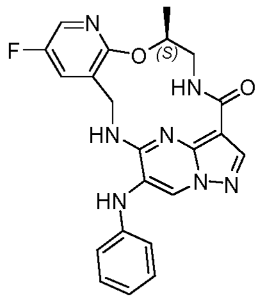

25

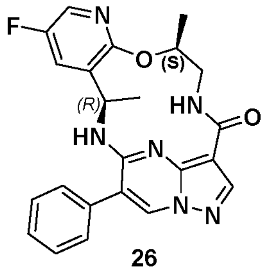

26

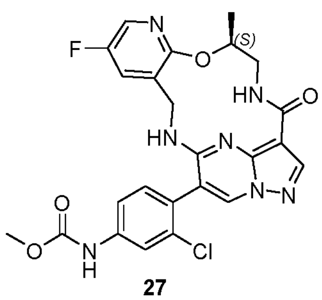

27

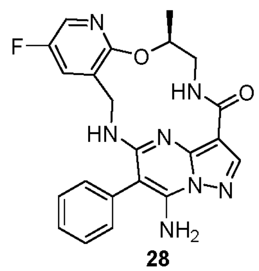

28

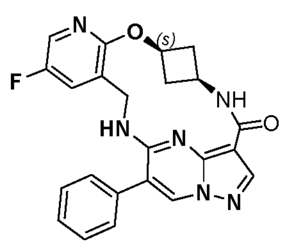

29

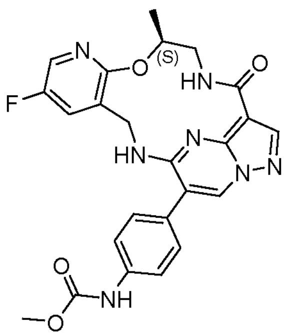

30

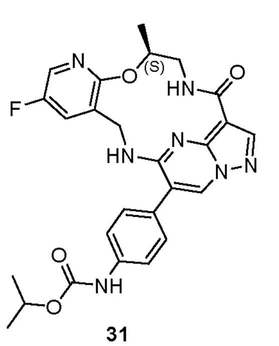

31

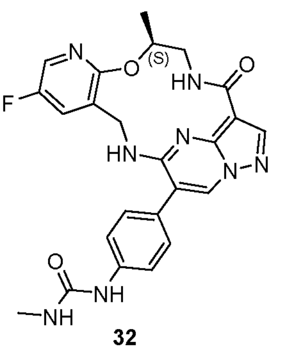

32

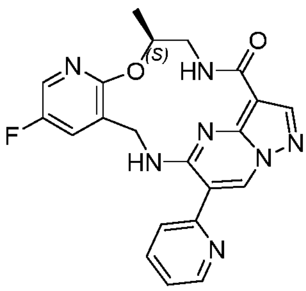

**33**

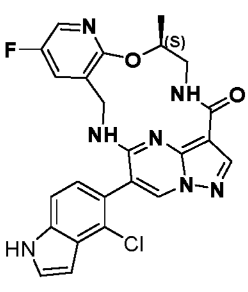

**34**

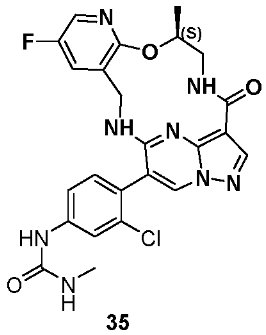

**35**

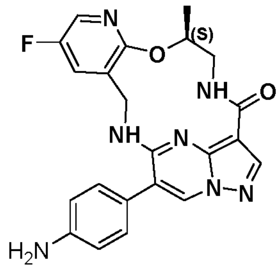

**36**

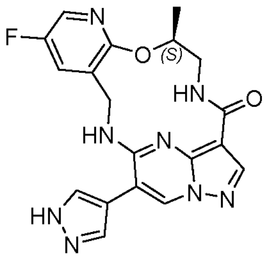

**37**

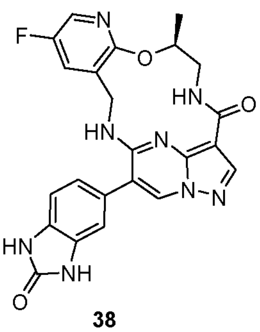

**38**

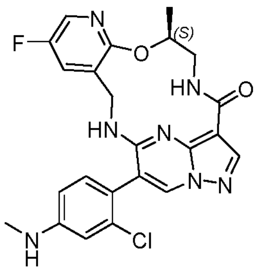

**39**

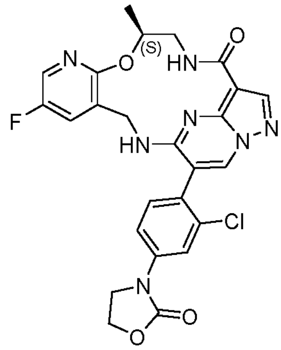

**40**

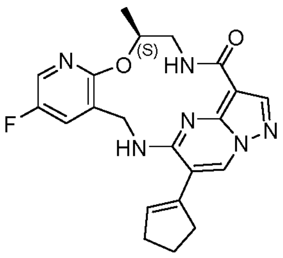

**41**

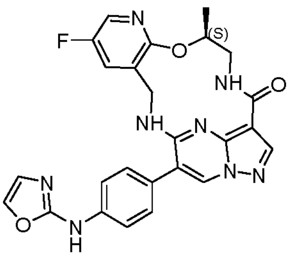

**42**

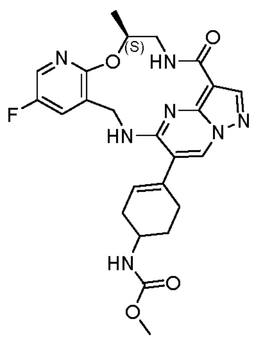

**43**

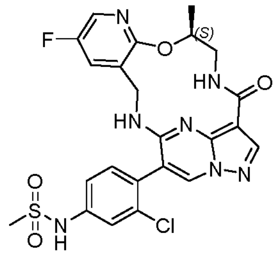

**44**

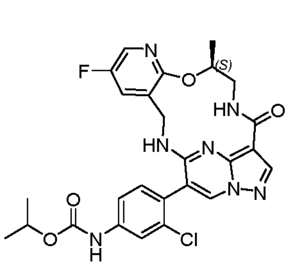

**45**

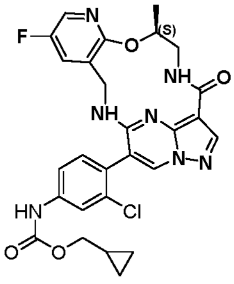

**46**

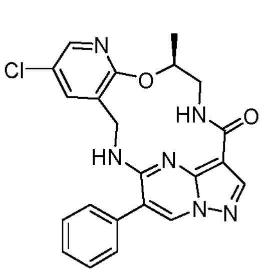

**47**

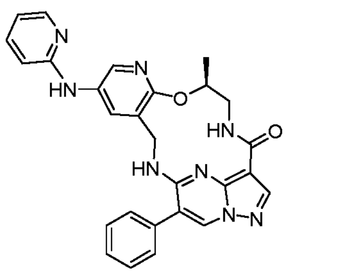

**48**

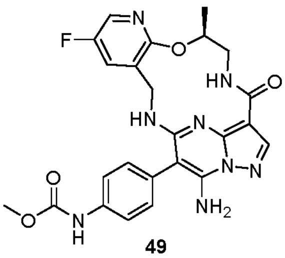
49

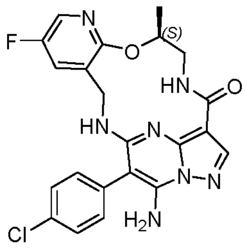
50

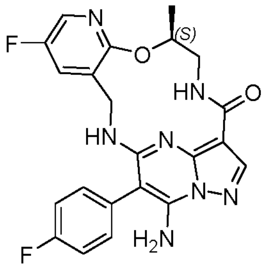
51

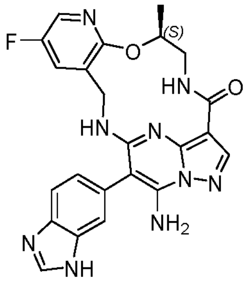
52

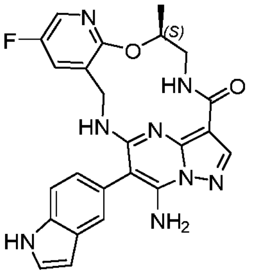
53

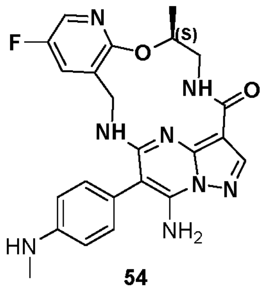
54

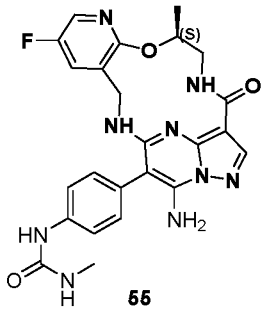
55

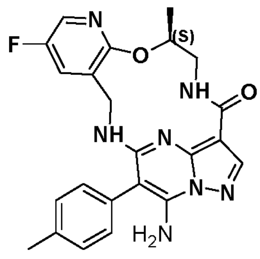
56

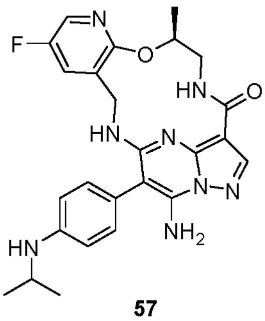
57

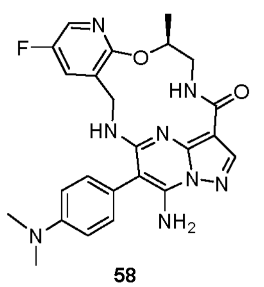
58

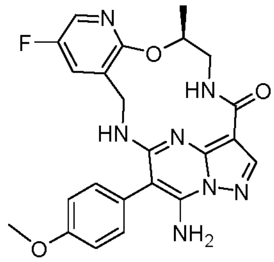
59

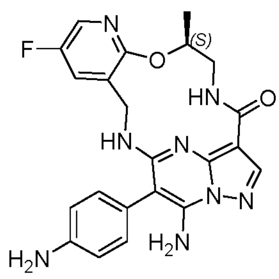
60

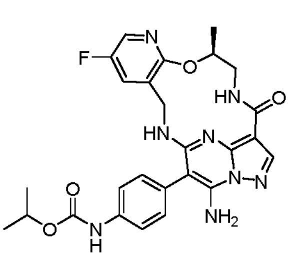
61

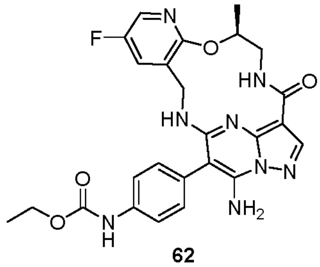
62

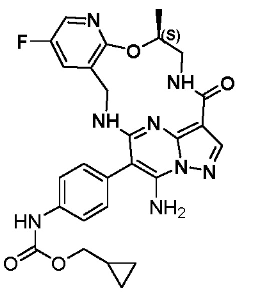
63

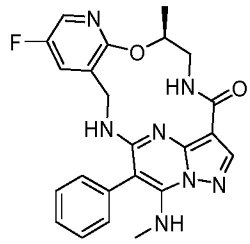
64

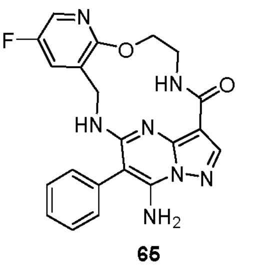
65

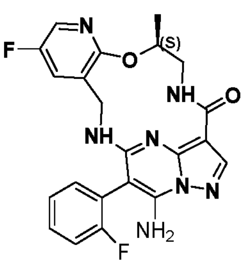
66

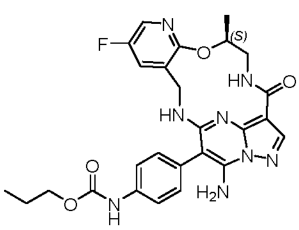
67

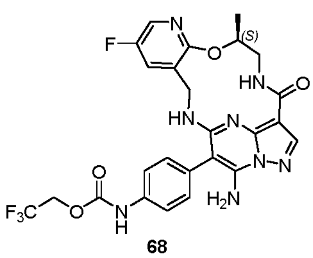
68

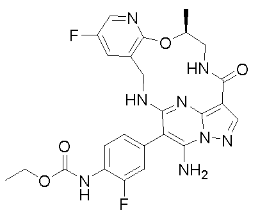
69

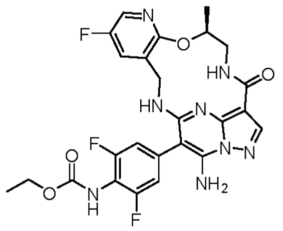
70

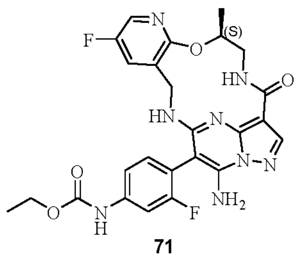
71

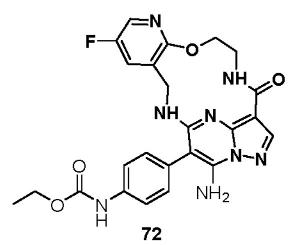
72

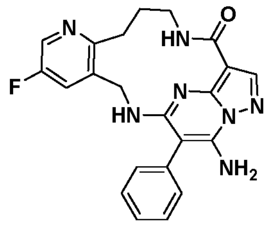
73

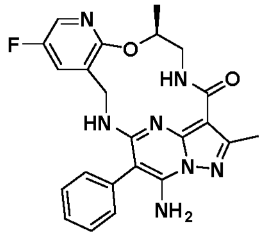
74

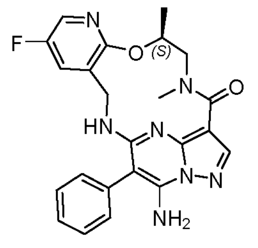
75

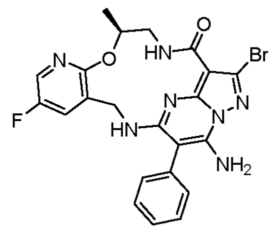
76

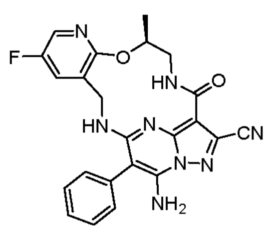
77

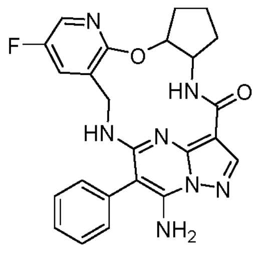
78

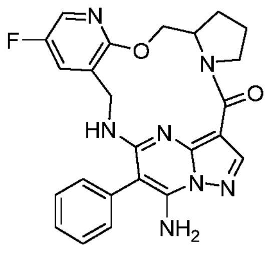
79

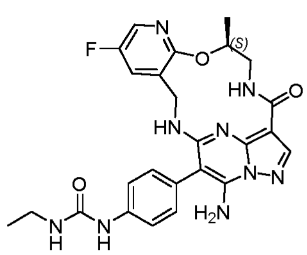
80

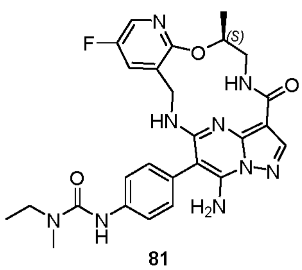
81

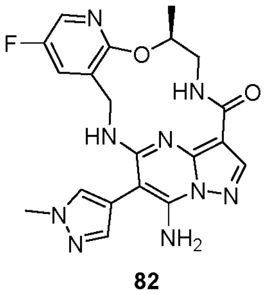
82

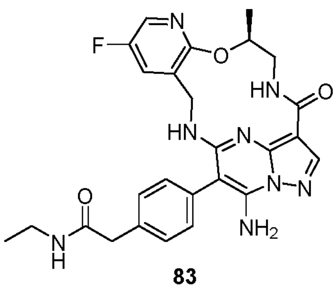
83

The present invention also relates to the pharmaceutically acceptable salts, solvates, or tautomers of compounds of the general formulae or specific compounds mentioned above.

In another aspect of the present invention, the present invention provides a pharmaceutical composition containing the compound of the present invention and a pharmaceutically acceptable excipient. In one specific aspect, the present invention provides the pharmaceutical composition of the present invention for the prevention or treatment of diseases which are caused by RET genes, RET kinases, or dysregulated expression or activity or levels of either of them, such as cancers, BTK-mediated diseases and/or SRC-mediated diseases. In one specific aspect, the pharmaceutical composition may additionally contain other active component(s) suitable for use in combination with the compound of the present invention.

In another aspect of the present invention, the present invention provides a pharmaceutical combination (or pharmaceutical combination product) containing the compound of the present invention and an additional active agent.

The present invention also provides the use of the compounds or stereoisomers, pharmaceutically acceptable salts, solvates, or tautomers as described above in the manufacture of a medicament for the treatment of diseases or disorders, wherein said diseases or disorders are selected from cancers.

Further, cancers described in the present invention are lung cancer, papillary thyroid cancer, medullary thyroid cancer, differentiated thyroid cancer, recurrent thyroid cancer, refractory differentiated thyroid cancer, type 2A or type

2B multiple endocrine neoplasia (MEN2A or MEN2B respectively), pheochromocytoma, parathyroid hyperplasia, breast cancer, colorectal cancer, papillary renal cell cancer, gastrointestinal mucosal ganglioneuroma, and cervical cancer.

Further, the cancers mentioned in the present invention are associated with the following dysregulations: caners caused by dysregulated expression or activity or level of RET gene, RET kinase, or either of them. Preferably, said caners are medullary thyroid cancer (MTC), non-small cell lung cancer (NSCLC), as well as metastatic solid tumors and advanced solid tumors with RET gene mutations/fusion.

The present invention provides the use of the compounds or stereoisomers in the manufacture of a medicament for the treatment of BTK-mediated diseases.

Further, BTK-mediated diseases described in the present invention are selected from cancers or autoimmune diseases. Preferably, said cancers are selected from one or more of diffuse large B-cell lymphoma, mantle cell lymphoma, chronic lymphocytic lymphoma, extranodal marginal zone B-cell lymphoma, B-cell chronic lymphocytic leukemia, B-cell prolymphocytic leukemia, mature B-cell acute lymphoblastic leukemia, 17p-deficient chronic lymphoblastic leukemia, Waldenstrom macroglobulinemia, lymphoplasmacytic lymphoma, splenic marginal zone lymphoma, plasma cell myeloma, plasmacytoma, intranodal marginal zone B-cell lymphoma, mantle cell lymphoma, intravascular large B cell lymphoma, and primary exudative lymphoma; said autoimmune diseases are selected from one or more of systemic lupus erythematosus, rheumatoid arthritis, and multiple sclerosis.

Further, the present invention provides the use of the compound or stereoisomer of the present invention in the manufacture of a medicament for the treatment of SRC-mediated diseases; preferably, said SRC-mediated diseases are selected from cancers; more preferably, said cancers are selected from one or more of triple-negative breast cancer (TNBC), non-small cell lung cancer, pancreatic cancer, colorectal cancer, and prostate cancer.

In another aspect of the present invention, the present invention provides a method for the prevention or treatment of diseases caused by RET genes, RET kinases, or dysregulated expression or activity or levels of either of them, such as cancers, BTK-mediated diseases and/or SRC-mediated diseases, in subjects, such as mammals, especially humans, comprising administration of an effective amount of the compound according to the present invention or pharmaceutical composition containing the same as described herein.

**Detailed description of the invention**

Unless stated otherwise, the following terms used in the description and claim have the following meanings.

"Alkyl" refers to an aliphatic hydrocarbon group, and means a saturated hydrocarbon group. The alkyl moiety may be either a straight-chain alkyl or a branched-chain alkyl, for example, $C_{1-6}$alkyl, $C_{1-4}$ alkyl or $C_{1-3}$ alkyl. $C_{1-6}$alkyl denotes an alkyl having 1 to 6 carbons, such as the alkyl having 1 carbon, 2 carbons, 3 carbons, 4 carbons, 5 carbons, and 6 carbons. Non-restrictive examples of alkyl include methyl, ethyl, n-propyl, isopropyl, n-butyl, isobutyl, sec-butyl, tert-butyl, n-pentyl, neo-pentyl, n-hexyl, and so on. Said alkyl can be unsubstituted or substituted by one or more substituents, and the substituents include but are not limited to alkyl, alkoxyl, cyano, hydroxyl, carbonyl, carboxyl, aryl, heteroaryl, amino, halo, sulfonyl, sulfinyl, phosphono, etc.

"Ring" refers to any covalently closed structure, including, for example, a carbon ring (e.g., aryl or cycloalkyl), heterocycle (e.g., heteroaryl or heterocycloalkyl), aromatic ring (e.g., aryl or heteroaryl), and non-aromatic ring (e.g., cycloalkyl or heterocycloalkyl). The rings may be optionally substituted, or may be monocyclic or polycyclic. A typical polycycle generally comprises two rings or three rings. The ring of the present application typically contains 1-20 ring atoms, such as 1 ring atom, 2 ring atoms, 3 ring atoms, 4 ring atoms, 5 ring atoms, 6 ring atoms, 7 ring atoms, 8 ring atoms, 9 ring atoms, 10 ring atoms, 11 ring atoms, 12 ring atoms, 13 ring atoms, 14 ring atoms, 15 ring atoms, 16 ring atoms, 17 ring atoms, 18 ring atoms, 19 ring atoms, or 20 ring atoms.

"Membered" is the number of skeleton atoms consisting of a ring. The typical 5-membered rings include, for example, cyclopentyl, pyrrole, imidazole, thiazole, furan and thiophene, etc.; the typical 6-membered rings include, for example, cyclohexyl, pyridine, pyrane, pyrazine, thiapyran, pyridazine, pyrimidine, and benzene, etc.. Among these, the ring in which the skeleton atoms include a heteroatom are called heterocycle; the aromatic groups containing heteroatoms are heteroaryls; and the non-aromatic groups containing heteroatom(s) are heterocycloalkyl, which contains heterocyclalkyl.

"Heteroatom" refers to atoms other than carbon or hydrogen. One or more of heteroatoms in the heterocycle of the present application may be independently selected from O, S, N, Si, and P, but not limited to those.

"Aryl" refers to a monocyclic or fused polycyclic (that is, sharing a pair of adjacent carbons) moiety having a conjugated $\pi$ electronic system and 6 to 14 carbon atoms (6-14-membered), and preferably having 6 to 10 atoms, such as phenyl and naphthyl. Phenyl is more preferable.

The term "heteroaryl" refers to a heteroaromatic system containing 1 to 4 (such as 1, 2, 3 or 4) heteroatoms and 5 to 14 (such as 5, 6, 7, 8, 9, 10, 11, 12, 13, and 14) ring atoms, in which the heteroatom is selected from O, S, and N. Heteroaryl is preferably 5-10-membered, and contains 1 to 3 heteroatoms; more preferably, is 5- or 6-membered and contains 1 to 2 heteroatoms; preferably, for example, imidazolyl, furanyl, thiophenyl, thiazolyl, pyrazolyl, oxazolyl, pyrrolyl,

tetrazolyl, pyridyl, pyrimidinyl, thiadiazolyl, pyrazinyl, etc., and preferably imidazolyl, thiazolyl, pyrazolyl or pyrimidinyl, thiazolyl; more preferably pyrazolyl, such as 1*H*-pyrazol-4-yl, or thiazolyl. Said heteroaryl ring may be fused to aryl, heterocycloalkyl, cycloalkyl rings or other heteroaryl, and thus form fused heteroaryl. Fused heteroaryl is preferably 8-10-membered fused heteroaryl, including but not limited to: indolyl such as 1H-indol-5-yl, 2-oxo-2,3-dihydro-1H-benzo[d]imidazolyl such as 2-oxo-2,3-dihydro-1H-benzo[d]imidazol-5-yl or 1H-benzo[d]imidazolyl such as 1H-benzo[d]imidazol-6-yl.

"Cycloalkyl" refers to a saturated or partially unsaturated (containing one or more double bonds, but no ring has fully-conjugated $\pi$ electronic system) cyclic hydrocarbon substituent containing 1 to 3 rings, which includes monocyclocalkyl, bicyclocalkyl, and tricycloalkyl, that contains 3 to 20 ring-forming carbons, preferably 3 to 10 carbons (i.e., 3- to 10-membered cycloalkyl, also known as $C_3$-$C_{10}$ cycloalkyl), for example 3 to 8 carbons, 3 to 7 carbons, 3 to 6 carbons, and 5 to 6 carbons. Preferably, cyclocalkyl is selected from monocyclocalkyl derived from the following rings:

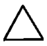 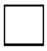 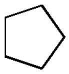 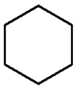 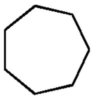 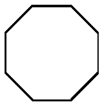

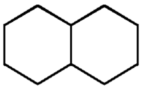 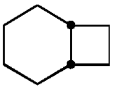 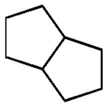 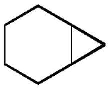

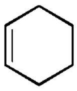 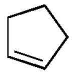

preferably cyclopropyl, cyclobutyl, cyclopentyl, cyclohexyl, cycloheptyl, cyclooctyl, cyclopentenyl or cyclohexenyl.

It should be understood that, according to the structure or the context, when cycloalkyl is connected with two groups, for example, when group D is cycloalkyl, the cycloalkyl is a bivalent group, that is, has two connection sites. At this time, it can also be called cycloalkylene. Examples of preferred cycloalkylene include, but are not limited to, monocyclic structures, such as cyclopropylene, cyclobutylene, cyclopentylene (such as cyclopentan-1,2-diyl, cyclopentan-1,3-diyl), cyclohexylene (such as cyclohexan-1,2-diyl, cyclohexan-1,3-diyl, cyclohexan-1,4-diyl), cycloheptylene or cyclooctylene, etc.

"Heterocycloalkyl" and "cyclic heteroalkyl" can be used interchangeably, and refer to saturated non-aromatic single ring, fused ring, bridged ring and spiro ring containing one or more (e.g., 1, 2, 3 or 4) heteroatoms, wherein the heteroatom may be N, O, S or $SO_2$

(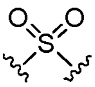),

and preferably N, O and/or S. Heterocycloalkyl may be 3- to 10-membered monocyclic or bicyclic or tricyclic group (e.g., 3-membered, 4-membered, 5-membered, 6-membered, 7-membered, 8-membered, 9-membered, or 10-membered, that is, containing 3, 4, 5, 6, 7, 8, 9, or 10 ring atoms). Typical heterocycloalkyl includes, but is not limited to monovalent groups derived from the following rings:

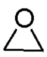 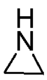 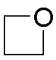 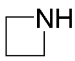 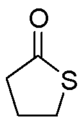 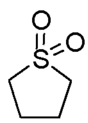 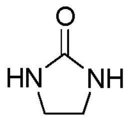 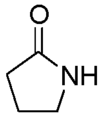 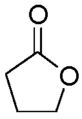

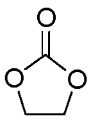 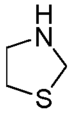 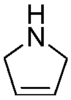 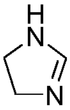 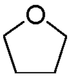 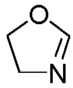 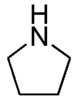 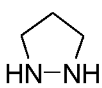 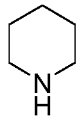

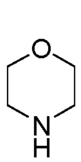 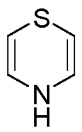 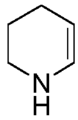 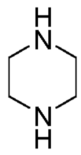 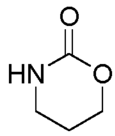 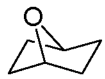 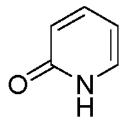

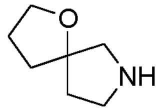 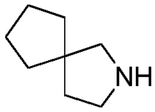 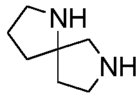 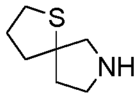

These heterocycloalkyl can also be expressed in a commonly understood structural form, such as

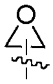 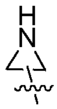 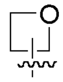 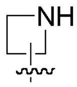 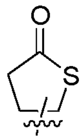 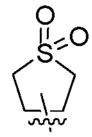 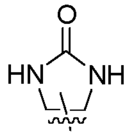 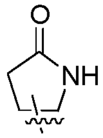 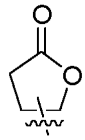

It should be understood that, according to the structure or the context, when heterocycloalkyl is connected with two groups, the heterocycloalkyl is a bivalent group, that is, having two connection sites. At this time, it may also be called heterocycloalkylene. Examples of heterocycloalkylene include, but are not limited to the bivalent groups derived from above groups, such as

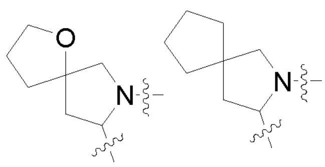 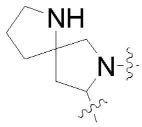 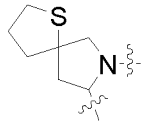

"Oxo" means a hydrogen on a carbon is substituted by =O.
"halogen" or "halo" refers to fluorine, chlorine, bromine, or iodine.

"Haloalkyl" means that at least one hydrogen in alkyl is substituted by a halogen, such as $CF_3$.

"Substituted" refers to one or more hydrogens in the group, preferably up to 5 hydrogens (such as 1, 2, 3, 4, and 5 hydrogens), and more preferably 1 to 3 hydrogens can be independently substituted from each other by a corresponding number of substituents. Obviously, substituents are only in their possible chemical positions. Those skilled in the art can determine (through experiment or theory) possible or impossible substitutions without undue effort. For example, amino or hydroxyl groups with a free hydrogen may be unstable when it binds to a carbon atom of unsaturated (e.g. olefinic) bonds.

"Inhibitor" refers to the agent that can reduce enzyme activity.

"Optionally" means that the event or environment described subsequently may or may not occur, and the description includes the occasion where the event or environment occurs or does not occur.

The term "substituted or unsubstituted" used herein refers to any group is mono-substituted or multi-substituted by a specified substituent to the extent that such mono-substitution or multi-substitution (including multi-substitution in the same part) is chemically permissible. Each substituent may locate at any available position in the group, and may be connected by any available atom in the substituent. "Any available position" refers to any position in the group that may be obtained chemically by a method known in the art or taught herein, and does not produce excessively unstable molecules. When there are two or more substituents in any group, each substituent is defined independently from any other substituent, and so the substituent may be the same or different.

"Stereoisomer" described in the present invention means that, when the compound of the present invention contains one or more asymmetric centers, it may exist in the form of a racemate and a racemic mixture, a single enantiomer, a diastereomer mixture and a single diastereomer. The compound of the present invention may have an asymmetric center and thus lead to the existence of two optical isomers. The scope of the present invention includes all possible optical isomers and their mixtures. If the compound of the present invention contains an olefinic double bond, the scope of the present invention includes cis-isomers and trans-isomers, unless specified otherwise. The compounds

of the present invention may exist in the form of tautomers (one of the functional group isomers), which have different hydrogen connection sites due to the shift of one or more double bonds. For example, a ketone and its enol form are ketone-enol tautomers. Each tautomer and a mixture thereof are within the scope of the present invention. For all compounds, enantiomers, diastereomers, racemates, mesomers, cis- and trans-isomers, tautomers, geometric isomers, epimers and mixtures thereof are all within the scope of the present invention.

The term "compound of the present invention" as used herein is intended to cover the compounds of general formula (I) as defined herein or any preferred or specific embodiments thereof (including compounds of formula (I'), (Ia), (Ib), (II), (III), (IV), etc., as well as compounds in examples), and stereoisomers, pharmaceutically acceptable salts, tautomers, or solvates thereof.

The term "pharmaceutically acceptable" as used herein refers to molecular entities and compositions that are approved or may be approved by the corresponding authorities of various countries or listed in the generally recognized pharmacopoeia for animals and more specifically for humans, or that will not produce adverse, allergic or other unwanted reactions when administered in appropriate amounts to animals, such as humans.

As used herein, the term "pharmaceutically acceptable salt" means a salt of a compound of the present invention that is pharmaceutically acceptable and has the desired pharmacological activity of the parent compound. Specifically, such salts are non-toxic and may be inorganic acid addition salts or organic acid addition salts and alkali addition salts.

As used herein, the term "subject" includes human or non-human animals. Exemplary human subjects include human subjects with diseases (such as those described herein) (referred to as patients) or normal subjects. In the present invention, "non-human animals" include all vertebrates, such as non-mammals (such as birds, amphibians, reptiles) and mammals, such as non-human primates, domestic animals and/or domesticated animals (such as sheep, dogs, cats, cows, pigs, etc.).

"Pharmaceutical composition" described in the present invention refers to a composition comprising one or more compounds of formula (I) or stereoisomers, tautomers, pharmaceutically acceptable salts or solvates thereof, as well as carriers or excipients commonly recognized in the art for delivering bioactive compounds to organisms (such as humans).

As used herein, the term "pharmaceutical combination" means compounds of the present invention may be used in combination with other active agents for the object of the present invention. Said other active agents may be one or more additional compounds of the present invention, or may be a second or additional (e.g., a third) compound that is compatible with the compound of the present invention (that is, they may not adversely affect each other) or has a complementary activity. This type of active agents is appropriately combined in an amount to achieve the desired object. Said other active agents may be administrated together with the compound of the present invention in a single pharmaceutical composition, or said other active agents and the compound of the present invention may be administrated separately in different discrete units. When administrated separately, administrations may be carried out simultaneously or sequentially. Said sequential administration may be performed closely or distantly in time.

It should be understood that the compound structure, group etc. of the present invention conform to the chemical valence rules. The connecting bonds of some groups or structures are omitted when writing. For example, in some cases, it is recorded that M in formula I is selected from N. Based on the structure of the general formula, it is known that M is =N-. Whether "M is selected from N" or "M is selected from =N-" may be understood by those skilled in the art. In addition, $X^1$ in formula I is selected from NH. Based on the structure of the general formula, it is known that $X^1$ is -NH-. Other groups may be similarly understood and interpreted.

Obviously, based on the above content of the present invention, according to the common technical knowledge and the conventional means in the field, without department from above basic technical spirits, other various modifications, alternations or changes may also be made.

## Advantageous effects of the invention

The present invention provides a class of compounds with the structural characteristics of general formula (I). By studies, it was found that such compounds may effectively inhibit the activity of kinases (wild type or mutant type) such as RET, SRC and/or BTK, thereby preventing or treating kinases (such as RET, SRC and/or BTK) related diseases.

The compounds of the present invention have the following advantageous effects:

High RET, SRC, and/or BTK kinase inhibitory activity; the assay of the preferable compounds according to the present invention indicated the $IC_{50}$ values are in the range of 0.1 nM to 1 $\mu$M), and preferably in the range of 0.1 nM to 0.1 $\mu$M); and/or

high activity for mutant RET, SRC and/or BTK, thereby they may be used to treat related diseases with drug resistance due to mutations; and/or

high kinase selectivity, thereby having reduced side effects. For example, the compound of the present invention has low TRK inhibitory effect, and so it may reduce the related side effects.

Based on the above advantageous effects of the compound according to the present invention, the present invention also provides technical solutions in the following aspects.

**Pharmaceutical composition and its administration**

The pharmaceutical composition of the present invention may be prepared via the techniques well-known by those skilled in the art, such as those published in Remington's Pharmaceutical Sciences, the $20^{th}$ edition. For example, the above pharmaceutical composition of the present inventions may be prepared by mixing the compounds of the present invention with one or more of pharmaceutically acceptable excipients. The preparation may further involve the step of mixing of one or more of other active components with the compound of the present invention and one or more of pharmaceutically acceptable excipients.

The selection of excipients included in a particular composition will depend on a number of factors, such as the mode of administration and the form of the composition provided. Suitable pharmaceutically acceptable excipients are well known by those skilled in the art and are described in such as Ansel, Howard C., et al., Ansel's Pharmaceutical Dosage Forms and Drug Delivery Systems. Philadelphia: Lippincott, Williams & Wilkins, 2004, including, for example, adjuvants, diluents (such as glucose, lactose or mannitol), carriers, pH regulators, buffers, sweeteners, fillers, stabilizers, surfactants, wetting agents, lubricants, emulsions, suspending agents, preservatives, antioxidants, opacifiers, glidants, processing agents, colorants, aromatizing agents, flavoring agents, and other known additives.

The pharmaceutical composition of the present invention may be administrated in a standard way. For example, appropriate administration ways include by oral, intravenous, rectal, parenteral, local, percutaneous, eye, nose, cheek or lung (inhalation) administration, wherein parenteral infusion includes intramuscular, intravenous, arterial, intraperitoneal or subcutaneous administration. For these purposes, the compounds of the present invention may be prepared in the form of tablets, capsules, syrups, powders, granules, aqueous or oily solutions or suspensions, (lipid) emulsions, dispersible powders, suppositories, ointments, creams, drops, aerosols, dry powder preparations and sterile injectable aqueous or oily solutions or suspensions by methods known in the art.

The preventive or therapeutic dose of the compound of the present invention will vary according to a series of factors, including the subjects to be treated, the severity of the diseases or disorders, the rate of administration, the disposal of the compound, and the judgment of the physician. In general, the effective dose is about 0.0001-5000 mg/kg body weight per day, for example, about 0.01-1000 mg/kg/day (single or multiple administrations). For a 70 kg person, this will be about 0.007 mg/day to about 7000 mg/day, for example, about 0.7 mg/day to about 1500 mg/day in total. According to the administration mode, the content or amount of the compound of the present invention in the pharmaceutical composition may be about 0.01 mg-1000 mg, suitably 0.1-500 mg, preferably 0.5-300 mg, more preferably 1-150 mg, particularly preferably 1-50 mg, such as 1.5 mg, 2 mg, 4 mg, 10 mg, 25 mg, etc.; accordingly, the pharmaceutical composition of the present invention will comprise compounds of the present invention in an amount of 0.05-99% w/w (weight percent), such as 0.05-80% w/w, such as 0.10-70% w/w, such as 0.10-50% w/w, and all weight percents are based on the total composition. It should be understood that it may be necessary to use doses beyond these limits in some cases.

By following specific examples, the above content of the present invention is further illustrated. However, it should not be construed that the scope of above subject of the present invention is only limited to following examples. The techniques realized based on above content of the present invention are all within the scope of the present invention.

It should be understood that, when there is any discrepancy between a chemical name and chemical structure for the compound of the present invention, in general, the structure prevails, unless it may be determined from the context that the compound name is correct.

**Examples**

In order to further illustrate the present invention, the active compound provided in the present invention, which is used to inhibit RET, as well as the preparation method and uses thereof, are described in detail below in combination with examples.

The following abbreviations have the following meanings:

$(Boc)_2O$ represents tert-butyl dicarbonate;
$Cs_2CO_3$ represents cesium carbonate;
DMAP represents 4-dimethylaminopyridine;
$K_2CO_3$ represents potassium carbonate;
NaHCOs represents sodium bicarbonate;
DMF represents N,N-dimethylformamide;
DMSO represents dimethyl sulfoxide;

DCM represents dichloromethane;
EtOH represents ethanol;
THF represents tetrahydrofuran;
TEA represents triethylamine;
DIPEA represents N,N-diisopropylethylamine;
DIAD represents diisopropyl azodiformate;
$PPh_3$ represents triphenylphosphine;
LiOH represents lithium hydroxide;
HCl represents hydrogen chloride;
$POCl_3$ represents phosphorus oxychloride;
$PCl_5$ represents phosphorus pentachloride;
FDPP represents pentafluorophenyl diphenylphosphate;
Xphos Pd G3 represents methanesulfonato(2-dicyclohexylphosphino-2',4',6'-tri-isopropyl-1,1'-biphenyl)(2'-amino-1,1'-biphenyl-2-yl)palladium (II);
Xphos Pd G2 represents chloro(2-dicyclohexylphosphino-2',4',6'-triisopropyl-1,1'-biphenyl) [2-(2'-amino-1,1'-biphenyl)]palladium (II);
$K_3PO_4$ represents anhydrous potassium phosphate;
$Pd_2(dba)_3$ represents tris(dibenzylideneacetone)dipalladium(0);
$Pd(PPh_3)_4$ represents tetrakis(triphenylphosphine)palladium;
t-BuXphos represents 2-di-tert-butylphosphino-2',4',6'-triisopropyl-1,1'-biphenyl;
Xphos represents 2-dicyclohexylphosphino-2,4,6-triisopropylbiphenyl.

The present patent also provides synthesis methods of the above compounds. The synthesis methods of the present invention are mainly based on the preparation method reported in the chemical literature, or the related synthesis is carried out with commercially available chemical reagents as starting material.

**Method 1:**

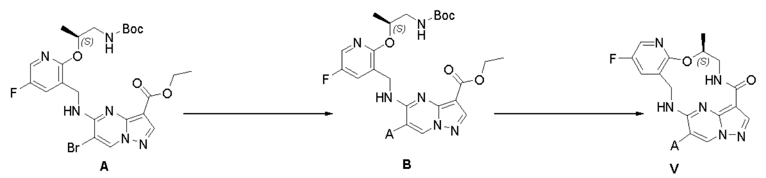

Step 1:

(1) When the substituent A in **compound B** is hydroxyl, the final product **V** may be obtained only via step 2;
(2) When **A** in **compound B** is aryl or heteroaryl, **compound A** reacts with boronic acid or boronate in the presence of **Xphos Pd G3, Xphos,** and $\mathbf{K_3PO_4}$ at **80** °C, to obtain **compound B;**
(3) When the substituent A in **compound B** is arylamine, heteroarylamine, **compound A** reacts with corresponding amines in the presence of **$\mathbf{Pd_2(dba)_3}$ t-BuXphos, $\mathbf{K_2CO_3}$ and tert-butyl alcohol** at **80 °C**, to obtain **compound B;**
(4) When the substituent A in **compound B** is aliphatic amine, **compound A** reacts with corresponding amines in the presence of $Cs_2CO_3$ at 130 °C under microwave, to obtain **compound B;**

Step 2:

**Compound B** is dissolved in the mixed solvent of methanol and tetrahydrofuran, to which is then added an aqueous solution of LiOH, and the mixture is subjected to hydrolysis reaction at 60 °C. After completion of the reaction, the pH value of the reaction solution is adjusted to 2-3 with 2 N hydrochloric acid, and then the organic phase is extracted three times with DCM. The organic phases are combined, dried over anhydrous sodium sulfate, filtered, and concentrated to dryness. Then, the solution of hydrochloric acid in 1,4-dioxane is added to remove the protective group. The reaction system is concentrated to dry, and then DCM and DMF are added, followed by successive addition of DIPEA and FDPP, to carry out the ring closing reaction and obtain compound **V.**

**Example 1:** The synthetic steps for the preparation of (S,$1^3$E,$1^4$E)-$4^5$-fluoro-6-methyl-$1^6$-phenyl-5-oxa-2,8-diaza-1(5,3)-pyrazolo[1,5-a]pyrimidina-4(3,2)-pyridinacyclononaphan-9-one (**compound 1**) were shown as follows:

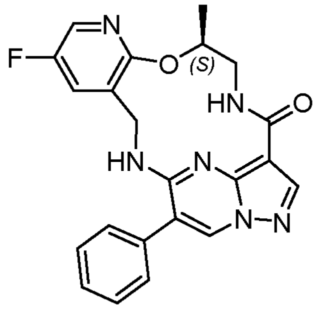

**1**

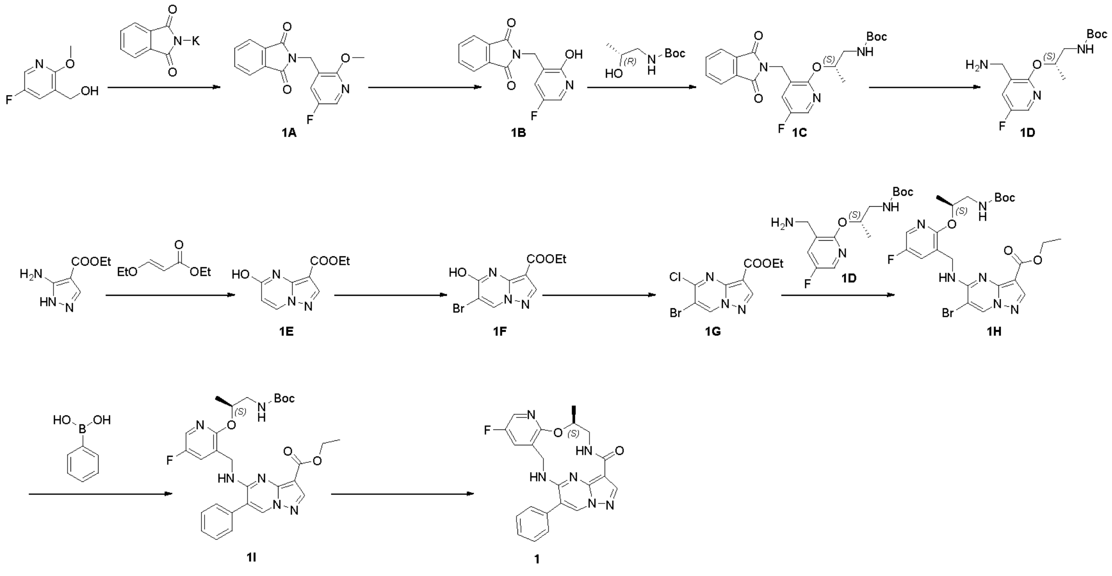

**Step** 1: Preparation of 2-((5-fluoro-2-methoxypyridin-3-yl)methyl)isoindolin-1,3-dione (**compound 1A**)

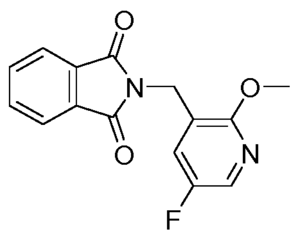

**1A**

Under the protection of argon, (5-fluoro-2-methoxypyridin-3-yl)methanol (3.14 g, 20 mmol) and triethylamine (2.83 g, 28 mmol) were dissolved in DCM (100 mL) in a 250 mL single neck bottle, to which was added methylsulfonyl chloride (2.4 g, 21 mmol) dropwise at 0 °C, and then the reaction system was stirred at room temperature for 2 h. After that, water was added to quench the reaction, and then the resultant mixture was extracted with ethyl acetate. The organic layer was successively washed with water and saturated NaCl solution, dried over anhydrous sodium sulfate, , filtered, and then concentrated. The residue was dissolved in DMF (50 mL), and then potassium phthalimide (5.55 g,

30 mmol) was added at 0 °C. Then, the mixture was stirred overnight at room temperature, and the reaction was quenched by adding water, then extracted with ethyl acetate. The organic layer was washed with saturated NaCl solution, dried over anhydrous sodium sulfate, filtered, and then concentrated to obtain **compound 1A.**
MS (ESI) m/z 287.0 $(M+H)^+$

**Step 2:** Preparation of 2-((5-fluoro-2-hydroxypyridin-3-yl)methyl)isoindolin-1 ,3-dione **(compound** 1B)

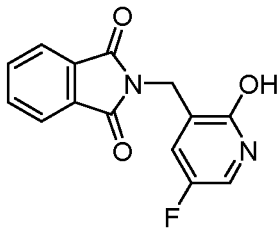

**1B**

2-((5-Fluoro-2-methoxypyridin-3-yl)methyl)isoindolin-1 ,3-dione (2 g, 6.99 mmol) was dissolved in 30 mL of ethanol, to which was added 4 N solution of HCl in dioxane (50 mL, 209.7 mmol), and then the system was stirred at 80 °C overnight. After the reaction was complete, the reaction solution was concentrated, and a small amount of water was added to the residue. The resultant mixture was filtered, and the resultant solid was washed with water, and dried *in vacuum,* **compound 1B** was finally obtained. MS (ESI) *m*/*z* 273.1 $(M+H)^+$.

**Step 3:** Preparation of tert-Butyl (S)-(2-((3-(((1,3-dioxoisoindol-2-yl)methyl)-5-fluoropyridin-2-yl) oxy)propyl)carbamate (**compound 1C**)

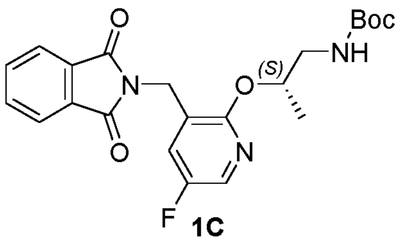

**1C**

Under the protection of argon, 2-((5-fluoro-2-hydroxypyridin-3-yl)methyl) isoindolin-1,3-dione (600 mg, 2.21 mmol), tert-butyl (R)-(2-hydroxypropyl)carbamate (772 mg, 4.41 mmol), and $PPh_3$ (1.16 g, 4.41 mmol) were dissolved in DCM (30 mL), and then DIAD (891 mg, 4.41 mmol) was added dropwise at 0 °C. The mixture was allowed to react at room temperature for 2 h. After completion of the reaction, the reaction solution was concentrated to dryness, and the residue was purified by column chromatography to obtain **compound 1C.**
MS (ESI) *m*/*z* 430.2 $(M+H)^+$.

**Step 4:** Preparation of tert-butyl (S)-(2-((3-(aminomethyl)-5-fluoropyridin-2-yl)oxy)propyl)carbamate (**compound 1D**)

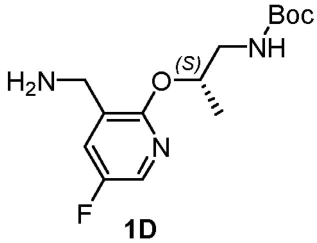

**1D**

tert-Butyl (S)-(2-((3-((1,3-dioxoisoindol-2-yl)methyl)-5-fluoropyridin-2-yl)oxy)propyl)carbamate (0.9 g, 2.10 mmol) obtained in previous step was dissolved in 50 mL of ethanol, to which was then added hydrazine hydrate (0.262 g, 4.20 mmol, 80%), and the mixture was stirred at 80 °C for 2 h. After completion of the reaction, the reaction mixture was filtered, and the filtrate was collected and concentrated. The crude product was purified by Flash C-18 column to provide **compound 1D.**
MS (ESI) m/z 300.2 $(M+H)^+$.

**Step 5:** Preparation of ethyl 5-hydroxypyrazolo[1 ,5-a]pyrimidin-3-carboxylate(**compound 1E**)

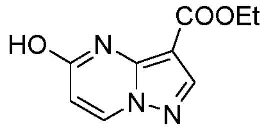

**1E**

Ethyl 5-amino-1H-pyrazol-4-carboxylate(10 g, 64.4 mmol) was dissolved in DMF (200 mL), to which was added $K_3PO_4$ (20.5 g, 98.2 mmol) at room temperature, and after the reaction mixture was stirred for 0.5 h, ethyl (E)-3-ethoxyacrylate (11.1 g, 77.3 mmol) was added. Then, the reaction system was heated to 110 °C and stirred for 16 h. After completion of the reaction, the reaction mixture was poured into 500 mL of water and then extracted three times with dichloromethane. The organic phases were combined, and concentrated under reduced pressure. The crude product was separated and purified by column chromatography to obtain **compound 1E.**
MS (ESI) m/z 208.2 $(M+H)^+$

**Step 6:** Preparation of ethyl 5-hydroxy-6-bromopyrazolo[1,5-a]pyrimidin-3-carboxylate(**compound 1F**)

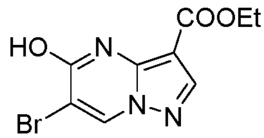

**1F**

Ethyl 5-Hydroxypyrazolo[1,5-a]pyrimidin-3-carboxylate(10.0 g, 47.8 mmol) was dissolved in acetic acid (100 mL), to which was slowly added bromine (7.64 g, 47.8 mmol) dropwise. After addition, the reaction mixture was heated to 180 °C and allowed to react at this temperature for 6 h, then cooled to room temperature. The reaction mixture was slowly poured into ice water for quenching. After the resultant solution was stirred for half an hour, white solids were produced, and then filtered, washed three times with water to finally obtain **compound 1F.**
MS (ESI) m/z 287.1 $(M+H)^+$

**Step 7:** Preparation of ethyl 5-chloro-6-bromopyrazolo[1 ,5-a]pyrimidin-3-carboxylate(**compound 1G**)

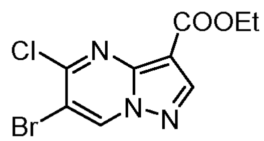

**1G**

Ethyl 5-hydroxy-6-bromopyrazolo[1 ,5-a]pyrimidin-3-carboxylate(2.86 g, 10.0 mmol) was dissolved in $POCl_3$ (50 mL), to which was then added $PCl_5$ (2.5 g, 12.0 mmol), and the reaction system was stirred for 16 h at 110 °C. The

reaction mixture system was concentrated under reduced pressure, and **compound** 1G was obtained by isolation and purification over column chromatography.
MS (ESI) m/z 305.6 $(M+H)^+$

**Step 8:** Preparation of ethyl (S)-6-bromo-5-(((2-((1-((tert-butyloxycarbonyl)amino)propan-2-yl)oxy)-5-fluoropyridin-3-yl)methyl)amino)pyrazolo[1,5-a]pyrimidin-3-carboxylate(**compound 1H**)

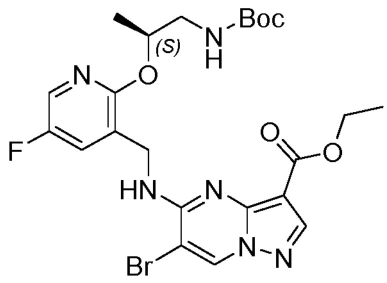

**1H**

Ethyl 5-chloro-6-bromopyrazolo[1,5-a]pyrimidin-3-carboxylate(6.1g, 20.0 mmol) was dissolved in ethanol (50 mL), to which was then added tert-butyl (S)-(2-((3-aminomethyl)-5-fluoropyridin-2-yl-oxy)propyl)carbamate (6.0 g, 20.0 mmol) and DIPEA (5.1 g, 40.0 mmol). Then, the reaction system was heated to 60 °C, stirred for 3 h, and concentrated to remove ethanol. To the residue, was then added water. Then, the resultant mixture was extracted three times with DCM. The combined organic phase was dried over anhydrous sodium sulfate, filtered, and concentrated under reduced pressure. The crude product was separated and purified by chromatographic column to obtain **compound 1H.**
MS (ESI) m/z 568.4 $(M+H)^+$

**Step 9:** Preparation of ethyl (S)-6-phenyl-5-(((2-((1-((tert-butyloxycarbonyl)amino)propan-2-yl)oxy)-5-fluoropyridin-3-yl)methyl)amino)pyrazolo[1,5-a]pyrimidin-3-carboxylate(**compound 1I**)

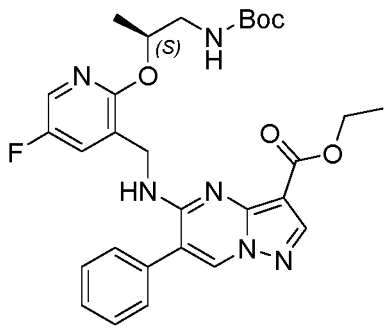

**1I**

Under the protection of argon, ethyl (S)-6-bromo-5-(((2-((1-((tert-butyloxycarbonyl)amino) propan-2-yl)oxy)-5-fluoropyridin-3-yl)methyl)amino)pyrazolo[1,5-a]pyrimidin-3-carboxylate(567 mg, 1.0mmol), phenylboronic acid (147 mg, 1.2 mmol), Xphos Pd G3 (90 mg, 0.1 mmol), Xphos (112 mg, 0.2 mmol), and $K_3PO_4$ (636 mg, 3.0 mmol) were dissolved in 16.0 mL of 1,4-dioxane/water (3:1), and then the reaction system was heated to 80 °C and stirred for 3h. The reaction mixture was poured into water, and the aqueous phase was extracted with ethyl acetate. The organic phases were combined, dried over anhydrous sodium sulfate, filtered, and concentrated. The residue was separated and purified over chromatographic column to provide **compound 1I.**
MS (ESI) m/z 565.6 $(M+H)^+$

**Step 10:** Preparation of (S,$1^3$E,$1^4$E)-$4^5$-fluoro-6-methyl-$1^6$-phenyl-5-oxa-2,8-diaza-1(5,3)-pyrazolo[1,5-a]pyrimidina-4(3,2)-pyridinacyclononaphan-9-one (**compound 1**)

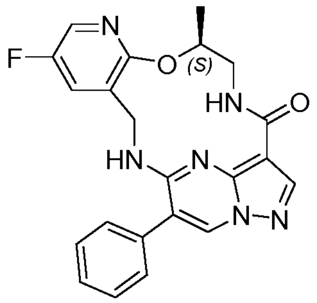

**1**

Ethyl (S)-6-phenyl-5-(((2-((1-((tert-butyloxycarbonyl)amino)propan-2-yl)oxy)-5-fluoropyridin-3-yl)methyl)amino)pyrazolo[1,5-a]pyrimidin-3-carboxylate(338 mg, 0.60mmol) was dissolved in a mixed solution of methanol and THF, to which was then added aqueous solution of LiOH (288 mg, 12.09 mmol). After addition, the reaction system was heated to 60 °C and stirred for 16 h. Then, the reaction system was cooled to 0 °C, and the pH was adjusted to 2-3 with 2N hydrochloric acid. The resultant mixture was extracted three times with DCM. The organic phases were combined, dried over anhydrous sodium sulfate, filtered, and concentrated to dryness. Then, a solution of hydrochloric acid in 1,4-dioxane was added, and the resultant mixture was stirred at room temperature for 1 h. The reaction system was concentrated to dryness, to which were then added DCM and DMF, followed by successive addition of DIPEA (15.78 g, 122.08 mmol) and FDPP (2.95 g, 7.69 mmol). After addition, the reaction mixture was stirred for 16 h at room temperature. The reaction system was concentrated to dryness under reduced pressure, and the residue was separated by high pressure preparative separation to obtain **compound 1** of Example 1.

MS (ESI) m/z 419.4 $(M+H)^+$
$^1$H NMR (400 MHz, DMSO-$d_6$) δ 9.70 - 9.54 (m, 1H), 8.61 (s, 1H), 8.09 (d, *J* = 11.3 Hz, 2H), 8.03 (d, *J* = 3.0 Hz, 1H), 7.78 (dd, *J* = 9.1, 3.1 Hz, 1H), 7.62 - 7.50 (m, 5H), 5.18 -4.83 (m, 2H), 4.17 - 3.92 (m, 2H), 3.14 (m, 1H), 1.47 (d, *J* = 6.1 Hz, 3H).

**Example 2:** The synthetic step of (S,$1^3$*E*,$1^4$*E*)-$4^5$-fluoro-6-methyl-$1^6$-hydroxy-5-oxa-2,8-diaza-1(5,3)-pyrazolo[1,5-a]pyrimidina-4(3,2)-pyridinacyclononaphan-9-one (**compound 2**) was shown in the following.

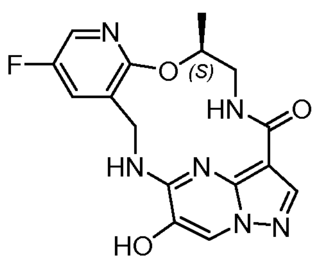

**2**

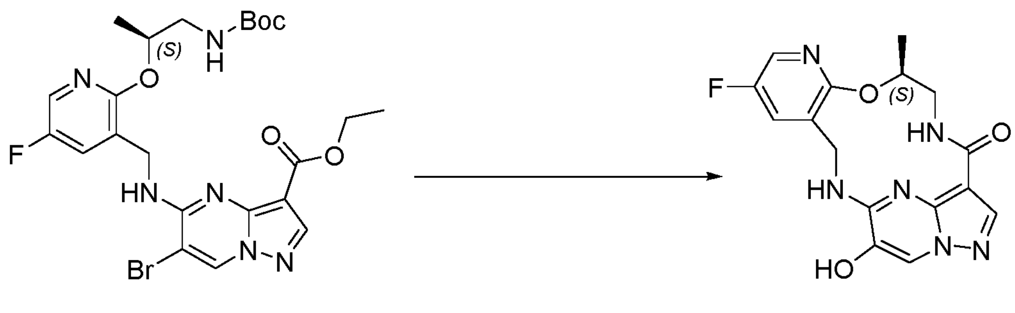

1H 2

**Step 1:** Preparation of (S,$1^3$*E*,$1^4$*E*)-$4^5$-fluoro-6-methyl-$1^6$-hydroxy-5-oxa-2,8-diaza-1(5,3)-pyrazolo[1,5-a]pyrimidina-4(3,2)-pyridinacyclononaphan-9-one (**compound 2**)

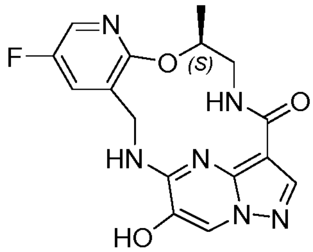

2

Ethyl (S)-6-*o*-trifluoromethylphenyl-5-((((2-((1-(((tert-butyloxycarbonyl)amino)propan-2-yl) oxy)-5-fluoropyridin-3-3-yl)methyl)pyrazolo[1,5-a]pyrimidin-3-carboxylate (340 mg, 0.60 mmol) was dissolved in a mixed solution of methanol and THF, to which was then added LiOH (288 mg, 12.09 mmol) aqueous solution. After addition, the reaction system was heated to 60 °C and stirred for 16 h. Then, the reaction system was cooled to 0 °C, and the pH was adjusted to 2-3 with 2N hydrochloric acid. The resultant mixture was extracted three times with DCM. The organic phases were combined, dried over anhydrous sodium sulfate, filtered, and concentrated to dryness. Then, the solution of hydrochloric acid in 1,4-dioxane was added, and then the resultant mixture was stirred at room temperature for 1 h, then evaporated under reduced pressure to remove the solvent, to which were then added DCM and DMF, followed by successive addition of DIPEA (15.78 g, 122.08 mmol) and FDPP (2.95 g, 7.69 mmol). After addition, the mixture was stirred for 16 h at room temperature. The reaction system was concentrated to dryness under reduced pressure, and the residue was separated by high pressure preparative separation to obtain **compound 2** of Example 2.

MS (ESI) m/z 359.3 $(M+H)^+$.

$^1$H NMR (400 MHz, DMSO-$d_6$) δ 9.83 - 9.68 (m, 1H), 8.65 (t, *J* = 6.2 Hz, 1H), 8.10 - 7.98 (m, 2H), 7.93 - 7.76 (m, 2H), 5.10 - 4.89 (m, 2H), 4.14 - 3.92 (m, 2H), 3.40 (dd, *J* = 10.7, 8.8 Hz, 1H), 3.11 (m, 1H), 1.45 (d, *J* = 6.1 Hz, 3H).

**Example 3:** The synthetic steps for the preparation of (S,$1^3$E,$1^4$E)-$1^6$-(3-chlorophenyl)-$4^5$-fluoro-6-methyl-5-oxa-2,8-diaza-1(5,3)-pyrazolo[1,5-a]pyrimidina-4(3, 2)-pyridinacyclononaphan-9-one were shown as follows:

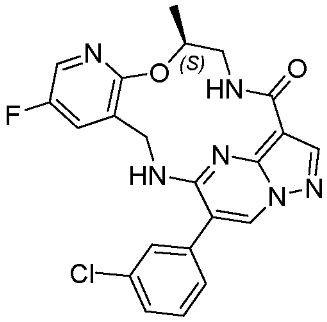

**3**

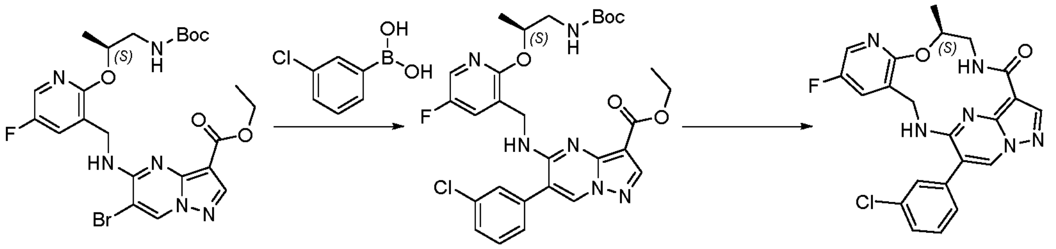

**1H** **3A** 3

**Step 1** . Preparation of ethyl (S)-5-((((2-((1-(((tert-butyloxycarbonyl)amino)propan-2-yl)oxy)-5-fluoropyridin-3-yl)methyl)amino)-6-(3-chlorophenyl)pyrazolo[1,5-a]pyrimidin-3-carboxylate(**compound 3A**)

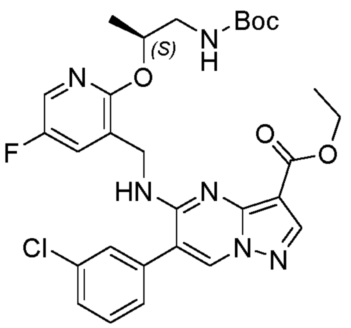

**3A**

Under the protection of argon, ethyl (S)-6-bromo-5-((((2-((1-(((tert-butyloxycarbonyl)amino)propan-2-yl)oxy)-5-fluoropyridin-3-yl)methyl)pyrazolo[1,5-a]pyrimidin-3-carboxylate(100 mg, 0.18 mmol), (3-chlorophenyl)boronic acid (35.8 mg, 0.23 mmol), Xphos Pd G3 (15 mg, 0.018 mmol), Xphos (17 mg, 0.035 mmol), $K_3PO_4$ (93.7 mg, 0.44 mmol), 1,4-dioxane (2 mL), and $H_2O$ (0.6 mL) were added into a reaction flask, and the above mixture was allowed to react for 3 h at 90 °C, then cooled to room temperature. The reaction was quenched with water, and then extracted three times with ethyl acetate. The organic phases were combined, dried over anhydrous sodium sulfate, filtered, and concentrated. The crude product was purified by column chromatography to obtain **compound 3A.**
MS (ESI) m/z 600.0 $(M+H)^+$

**Step 2:** Preparation of (S,$1^3$E,$1^4$E)-$1^6$-(3-chlorophenyl)-$4^5$-fluoro-6-methyl-5-oxa-2,8-diaza-1(5,3)-pyrazolo[1,5-a]pyrimidina-4(3,2)-pyridinacyclononaphan-9-one (**compound 3**)

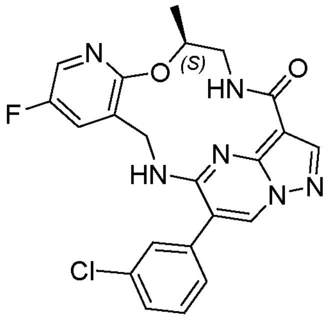

3

Ethyl (S)-5-((((2-((1-(((tert-butyloxycarbonyl)amino)propan-2-yl)oxy)-5-fluoropyridin-3-yl) methyl)amino)-6-(3-chlorophenyl)pyrazolo[1,5-a]pyrimidin-3-carboxylate(85 mg, 0.14 mmol), LiOH (68 mg, 2.84 mmol), methanol (3 mL), THF (1 mL), and water (1 mL) were added into a reaction flask, and the above mixture was allowed to react overnight at 70 °C, then naturally cooled to room temperature. The pH was adjusted to 4-5 with 2 M hydrochloric acid, and then the reaction mixture was extracted three times with DCM. The organic phase was dried over anhydrous sodium sulfate, filtered and concentrated. To the obtained residue, was added the solution of HCl in 1,4-dioxane (4.0 M, 50 mL), and the resultant mixture was stirred for 1 h at room temperature. Then, the solvent was removed by rotatory evaporation, and the residue was further dried by sucking with oil pump. To the residue, were added DMF (30 mL) and DCM (60 mL), followed by successive addition of DIPEA (220 mg, 1.71 mmol) and FDPP (98.6 mg, 0.26 mmol) under stirring. The mixture was stirred at room temperature for 16 h. Then, 2M $Na_2CO_3$ solution (30 mL) was added for quenching the reaction, and the resultant mixture was extracted three times with DCM. The organic phase was dried over anhydrous sodium sulfate, filtered, and concentrated. The residue was directly purified by high pressure preparative separation to obtain **compound 3** of Example 3.

MS (ESI) m/z 453.0 $(M+H)^+$
$^1H$ NMR (400 MHz, DMSO-$d_6$) $\delta$ 9.61 (d, $J$ = 8.4 Hz, 1H), 8.69 (s, 1H), 8.12 (d, $J$ = 5.1 Hz, 2H), 8.03 (d, $J$ = 3.0 Hz, 1H), 7.74 (dd, $J$ = 8.8, 3.0 Hz, 1H), 7.66 - 7.57 (m, 3H), 7.55 - 7.46 (m, 1H), 5.09 - 4.93 (m, 2H), 4.09 - 3.95 (m, 2H), 3.14 (m, 1H), 1.47 (d, $J$ = 6.1 Hz, 3H).

**Example 4:** Preparation of (S,$1^3$E,$1^4$E)-$1^6$-(2-chlorophenyl)-$4^5$-fluoro-6-methyl-5-oxa-2,8-diaza-1(5,3)-pyrazolo[1,5-a]pyrimidina-4(3,2)-pyridinacyclononaphan-9-one (compound 4):

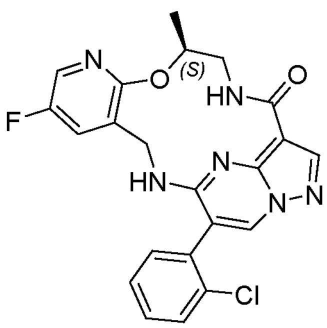

**4**

The preparation method was the same as that of Example 3, except that (2-chlorophenyl)boronic acid was used to replace (3-chlorophenyl)boronic acid. Compound 4 of Example 4 was obtained.

MS (ESI) m/z 453.0 $(M+H)^+$
$^1H$ NMR (400 MHz, DMSO-$d_6$) $\delta$ 9.72 - 9.63 (m, 1H), 8.72 (d, $J$ = 3.9 Hz, 1H), 8.13 (d, $J$ = 1.7 Hz, 1H), 8.07 - 7.93 (m, 2H), 7.77 - 7.47 (m, 5H), 5.12 - 4.89 (m, 2H), 4.07 - 3.95 (m, 2H), 3.14 (t, $J$ = 11.9 Hz, 1H), 1.47 (dd, $J$ = 6.1, 1.1 Hz, 3H).

**Example 5:** Preparation of (S,$1^3$E,$1^4$E)-$4^5$-fluoro-$1^6$-(2-methoxypyridin-4-yl)-6-methyl-5-oxa-2,8-diaza-1(5,3)-pyrazolo[1,5-a]pyrimidin-4(3, 2)-pyridinacyclononaphan-9-one (**compound 5**):

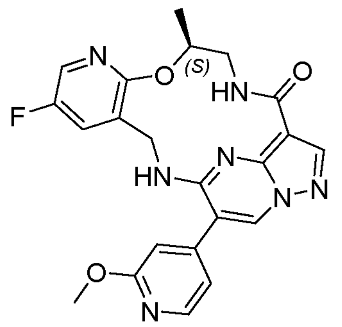

**5**

Using (2-methoxypyridin-4-yl)boronic acid to replace (3-chlorophenyl)boronic acid, **compound 5** of Example 5 was obtained by the preparation method same as that of Example 3.

MS (ESI) m/z 450.0 $(M+H)^+$
$^1$H NMR (400 MHz, DMSO-$d_6$) $\delta$ 9.59 (d, *J* = 8.8 Hz, 1H), 8.75 (s, 1H), 8.35 (dd, *J* = 5.3, 0.7 Hz, 1H), 8.20 (t, *J* = 6.0 Hz, 1H), 8.13 (s, 1H), 8.03 (d, *J* = 3.1 Hz, 1H), 7.75 (dd, *J* = 8.9, 3.1 Hz, 1H), 7.16 (dd, *J* = 5.2, 1.5 Hz, 1H), 7.02 (dd, *J* = 1.5, 0.7 Hz, 1H), 5.09 - 4.95 (m, 2H), 4.08 - 3.97 (m, 2H), 3.94 (s, 3H), 3.16 (d, *J* = 11.7 Hz, 1H), 1.47 (d, *J* = 6.1 Hz, 3H).

**Example 6:** Preparation of (S,$1^3$E,$1^4$E)-$1^6$-(2-fluorophenyl)-$4^5$-fluoro-6-methyl-5-oxa-2, 8-diaza-1(5,3)-pyrazolo[1,5-a]pyrimidina-4(3,2)-pyridinacyclononaphan-9-one (**compound 6**):

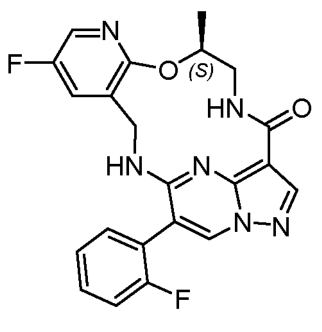

**6**

Using (2-fluorophenyl)boronic acid to replace (3-chlorophenyl)boronic acid, **compound 6** of Example 6 was obtained by the preparation method same as that of Example 3.

MS (ESI) m/z 437.1 $(M+H)^+$
$^1$H NMR (400 MHz, DMSO-$d_6$) $\delta$ 9.64 (d, *J* = 8.8 Hz, 1H), 8.74 (s, 1H), 8.13 (d, *J* = 5.8 Hz, 2H), 8.04 (d, *J* = 3.0 Hz, 1H), 7.72 - 7.38 (m, 5H), 5.10 - 4.95 (m, 2H), 4.03 (m, 2H), 3.14 (m, 1H), 1.47 (d, *J* = 6.1 Hz, 3H).

**Example 7:** Preparation of (S,$1^3$E,$1^4$E)-$1^6$-(3-fluorophenyl)-$4^5$-fluoro-6-methyl-5-oxa-2,8-diaza-1(5,3)-pyrazolo[1,5-a]pyrimidina-4(3,2)-pyridinacyclononaphan-9-one (compound 7):

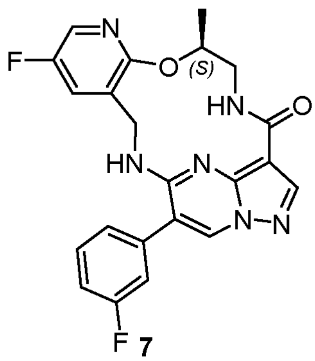

7

Using (3-fluorophenyl)boronic acid to replace (3-chlorophenyl)boronic acid, **compound 7** of Example 7 was obtained by the preparation method same as that of Example 3.

MS (ESI) m/z 437.1 $(M+H)^+$
$^1$H NMR (400 MHz, DMSO-$d_6$) δ 9.61 (dd, *J* = 9.0, 1.6 Hz, 1H), 8.67 (s, 1H), 8.12 (d, *J* = 6.8 Hz, 2H), 8.03 (d, *J* = 3.1 Hz, 1H), 7.75 (dd, *J* = 8.9, 3.1 Hz, 1H), 7.62 (m, 1H), 7.48 - 7.32 (m, 3H), 5.11 - 4.96 (m, 2H), 4.11 - 3.95 (m, 2H), 3.14 (m, 1H), 1.46 (d, *J* = 6.1 Hz, 3H).

**Example 8:** Preparation of (S,$1^3$E,$1^4$E)-$1^6$-(4-fluorophenyl)-$4^5$-fluoro-6-methyl-5-oxa-2,8-diaza-1(5,3)-pyrazolo[1,5-a]pyrimidina-4(3,2)-pyridinacyclononaphan-9-one (**compound 8**):

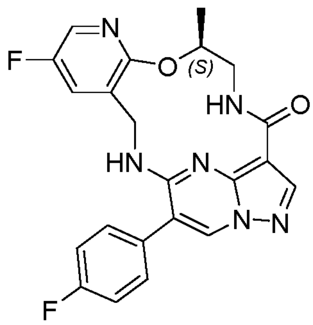

**8**

Using (4-fluorophenyl)boronic acid to replace (3-chlorophenyl)boronic acid, **compound 8** of Example 8 was obtained by the preparation method same as that of Example 3.

MS (ESI) m/z 437.1 $(M+H)^+$
$^1$H NMR (400 MHz, DMSO-$d_6$) δ 9.63 (d, *J* = 8.8 Hz, 1H), 8.63 (s, 1H), 8.10 (s, 1H), 8.09 - 7.98 (m, 2H), 7.75 (dd, *J* = 8.8, 3.1 Hz, 1H), 7.62 - 7.55 (m, 2H), 7.45 - 7.38 (m, 2H), 5.11 - 4.94 (m, 2H), 4.02 (m, 2H), 3.20 - 3.08 (m, 1H), 1.47 (d, *J* = 6.1 Hz, 3H).

**Example 9:** Preparation of (S,$1^3$E,$1^4$E)-$1^6$-(4-chlorophenyl)-$4^5$-fluoro-6-methyl-5-oxa-2,8-diaza-1(5,3)-pyrazolo[1,5-a]pyrimidina-4(3,2)-pyridinacyclononaphan-9-one (**compound 9**):

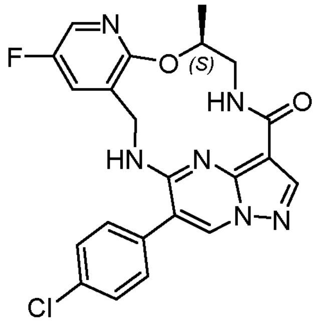

**9**

Using (4-chlorophenyl)boronic acid to replace (3-chlorophenyl)boronic acid, **compound 9** of Example 9 was obtained by the preparation method same as that of Example 3.

MS (ESI) m/z 453.1 $(M+H)^+$
$^1$H NMR (400 MHz, DMSO-$d_6$) δ 9.63 (m, 1H), 8.66 (s, 1H), 8.11 (s, 1H), 8.08 (m, 1H), 8.04 (m, 1H), 7.74 (m, 1H), 7.76 - 7.73 (m, 4H), 4.04-4.01 (m, 2H), 3.17-3.11 (m, 1H), 2.02-1.97 (m, 2H), 1.47-1.46 (m, 3H).

**Example 10:** Preparation of (S,$1^3$E,$1^4$E)-$1^6$-(4-phenoxyphenyl)-$4^5$-fluoro-6-methyl-5-oxa-2, 8-diaza-1(5,3)-pyrazolo[1,5-a]pyrimidina-4(3,2)-pyridinacyclononaphan-9-one (**compound 10**)

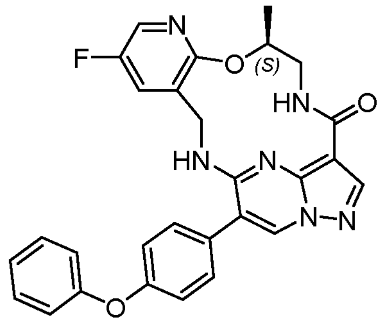

**10**

Using (4-phenoxyphenyl)boronic acid to replace (3-chlorophenyl)boronic acid, **compound 10** of Example 10 was obtained by the preparation method same as that of Example 3.

MS (ESI) m/z 511.2 $(M+H)^+$
$^1$H NMR (400 MHz, DMSO-$d_6$) δ 9.62 (d, *J* = 8.7 Hz, 1H), 8.61 (s, 1H), 8.13 - 7.98 (m, 3H), 7.78 (dd, *J* = 8.9, 3.1 Hz, 1H), 7.57 - 7.51 (m, 2H), 7.47 (dd, *J* = 8.5, 7.4 Hz, 2H), 7.27 - 7.19 (m, 1H), 7.19 - 7.06 (m, 4H), 5.12 - 4.92 (m, 2H), 4.12 - 3.88 (m, 2H), 3.19 - 3.05 (m, 1H), 1.47 (d, *J* = 6.1 Hz, 3H).

**Example 11:** Preparation of (S,$1^3$E,$1^4$E)-$1^6$-(4-methylphenyl)-$4^5$-fluoro-6-methyl-5-oxa-2,8-diaza-1(5,3)-pyrazolo[1,5-a]pyrimidina-4(3,2)-pyridinacyclononaphan-9-one (**compound 11**):

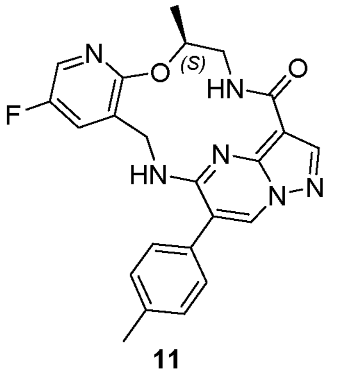

**11**

Using (4-methylphenyl)boronic acid to replace (3-chlorophenyl)boronic acid, **compound 11** of Example 11 was obtained by the preparation method same as that of Example 3.

MS (ESI) m/z 433.0 $(M+H)^+$
$^1$H NMR (400 MHz, DMSO-$d_6$) δ 9.63 (d, *J* = 8.8 Hz, 1H), 8.57 (s, 1H), 8.09 (s, 1H), 8.07 - 8.01 (m, 2H), 7.77 (dd, *J* = 8.9, 3.1 Hz, 1H), 7.45 - 7.33 (m, 4H), 5.02 (m, 2H), 4.08 - 3.96 (m, 2H), 3.19 - 3.09 (m, 1H), 2.41 (s, 3H), 1.47 (d, *J* = 6.1 Hz, 3H).

**Example 12:** Preparation of (S,1$^3$E,1$^4$E)-1$^6$-(3-methylphenyl)-4$^5$-fluoro-6-methyl-5-oxa-2,8-diaza-1(5,3)-pyrazolo[1,5-a]pyrimidina-4(3,2)-pyridinacyclononaphan-9-one (**compound 12**):

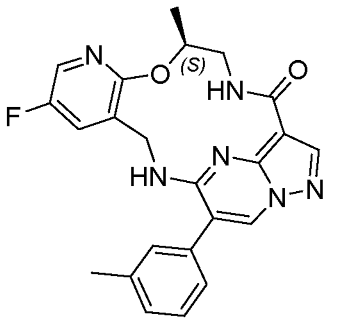

**12**

Using (3-methylphenyl)boronic acid to replace (3-chlorophenyl)boronic acid, **compound 12** of Example 12 was obtained by the preparation method same as that of Example 3.

MS (ESI) m/z 433.0 $(M+H)^+$
$^1$H NMR (400 MHz, DMSO-$d_6$) δ 9.62 (d, *J* = 8.8 Hz, 1H), 8.59 (s, 1H), 8.14 - 7.99 (m, 3H), 7.77 (dd, *J* = 9.0, 3.1 Hz, 1H), 7.53 - 7.43 (m, 1H), 7.39 - 7.30 (m, 3H), 5.10 - 4.95 (m, 2H), 4.11 - 3.96 (m, 2H), 3.14 (dd, *J* = 13.0, 9.8 Hz, 1H), 2.42 (s, 3H), 1.47 (d, *J* = 6.1 Hz, 3H)

**Example 13:** Preparation of (S,1$^3$E,1$^4$E)-1$^6$-(2-methylphenyl)-4$^5$-fluoro-6-methyl-5-oxa-2,8-diaza-1(5,3)-pyrazolo[1,5-a]pyrimidina-4(3,2)-pyridinacyclononaphan-9-one (**compound 13**):

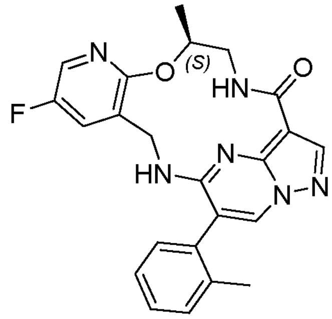

**13**

Using (2-methylphenyl)boronic acid to replace (3-chlorophenyl)boronic acid, **compound 13** of Example 13 was obtained by the preparation method same as that of Example 3.

MS (ESI) m/z 433.0 $(M+H)^+$
$^1H$ NMR (400 MHz, DMSO-$d_6$) δ 9.69 (t, *J* = 7.5 Hz, 1H), 8.56 (s, 1H), 8.10 (d, *J* = 2.1 Hz, 1H), 8.04 (dd, *J* = 10.5, 3.1 Hz, 1H), 7.83 - 7.60 (m, 2H), 7.50 - 7.23 (m, 4H), 5.09 - 4.91 (m, 2H), 4.02 (m, 2H), 3.19 - 3.08 (m, 1H), 2.21 (s, 1H), 2.06 (s, 2H), 1.47 (dd, *J* = 6.1, 1.3 Hz, 3H).

**Example 14:** Preparation of (S,$1^3$E,$1^4$E)-$1^6$-(4-trifluoromethylphenyl)-$4^5$-fluoro-6-methyl-5-oxa-2, 8-diaza-1(5,3)-pyrazolo[1,5-a]pyrimidina-4(3,2)-pyridinacyclononaphan-9-one **(compound 14)**:

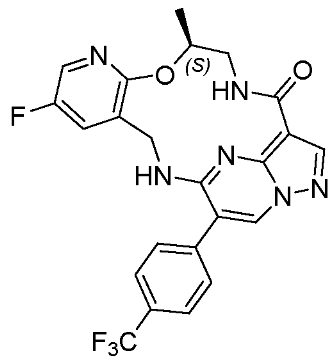

**14**

Using (4-trifluoromethylphenyl)boronic acid to replace (3-chlorophenyl)boronic acid, **compound 14** of Example 14 was obtained by the preparation method same as that of Example 3.

MS (ESI) m/z 487.1 $(M+H)^+$
$^1H$ NMR (400 MHz, DMSO-$d_6$) δ 9.61 (d, *J* = 8.8 Hz, 1H), 8.74 (s, 1H), 8.13 (d, *J* = 3.3 Hz, 2H), 8.04 (d, *J* = 3.0 Hz, 1H), 7.95 (d, *J* = 8.1 Hz, 2H), 7.80 - 7.72 (m, 3H), 5.19 - 4.92 (m, 2H), 4.21 - 3.92 (m, 2H), 3.15 (m, 1H), 1.47 (d, *J* = 6.1 Hz, 3H).

**Example 15:** Preparation of (S,$1^3$E,$1^4$E)-$1^6$-(3-trifluoromethylphenyl)-$4^5$-fluoro-6-methyl-5-oxa-2, 8-diaza-1(5,3)-pyrazolo[1,5-a]pyrimidina-4(3,2)-pyridinacyclononaphan-9-one (**compound 15**):

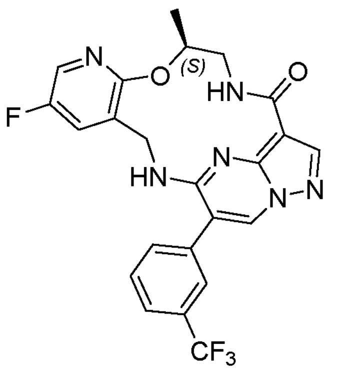

**15**

Using (3-trifluoromethylphenyl)boronic acid to replace (3-chlorophenyl)boronic acid, **compound 15** of Example 15 was obtained by the preparation method same as that of Example 3.

MS (ESI) m/z 487.1 $(M+H)^+$
$^1$H NMR (400 MHz, DMSO-$d_6$) δ 9.67 - 9.58 (m, 1H), 8.74 (s, 1H), 8.13 (d, *J* = 4.6 Hz, 2H), 8.04 (d, *J* = 3.1 Hz, 1H), 7.93 - 7.77 (m, 4H), 7.72 (dd, *J* = 8.8, 3.1 Hz, 1H), 5.19 - 4.90 (m, 2H), 4.03 (m, 2H), 3.20 - 3.07 (m, 1H), 1.47 (d, *J* = 6.1 Hz, 3H).

**Example 16:** Preparation of (S,1$^3$E,1$^4$E)-1$^6$-(2-trifluoromethylphenyl)-4$^5$-fluoro-6-methyl-5-oxa-2, 8-diaza-1(5,3)-pyrazolo[1,5-a]pyrimidina-4(3,2)-pyridinacyclononaphan-9-one (**compound 16**):

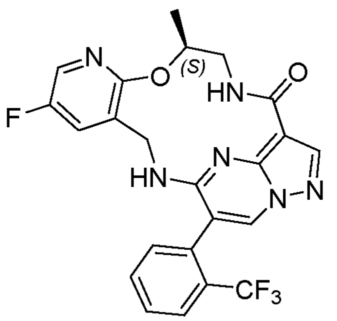

**16**

Using (2-trifluoromethylphenyl)boronic acid to replace (3-chlorophenyl)boronic acid, **compound 16** of Example 16 was obtained by the preparation method same as that of Example 3.

MS (ESI) m/z 487.1 $(M+H)^+$
$^1$H NMR (400 MHz, DMSO-$d_6$) δ 9.64 (t, *J* = 8.5 Hz, 1H), 8.70 (d, *J* = 9.9 Hz, 1H), 8.12 (s, 1H), 8.03 (dd, J = 8.9, 3.0 Hz, 1H), 7.97 (dd, *J* = 7.9, 1.4 Hz, 1H), 7.91 - 7.83 (m, 2H), 7.80 (t, *J* = 7.5 Hz, 1H), 7.63 (d, *J* = 7.5 Hz, 1H), 7.56 (m, 1H), 4.99 (m, 2H), 4.10 - 3.89 (m, 2H), 3.20 - 3.08 (m, 1H), 1.46 (dd, *J* = 6.1, 3.1 Hz, 3H).

**Example 17:** Preparation of (S,1$^3$E,1$^4$E)-4$^5$-fluoro-6-methyl-1$^6$-morpholino-5-oxa-2,8-diaza-1(5,3)-pyrazolo[1,5-a]pyrimidina-4(3,2)-pyridinacyclononaphan-9-one (**compound 17**):

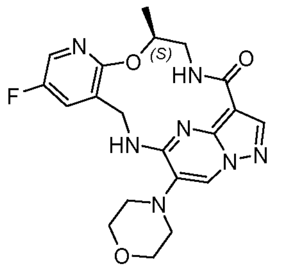

**17**

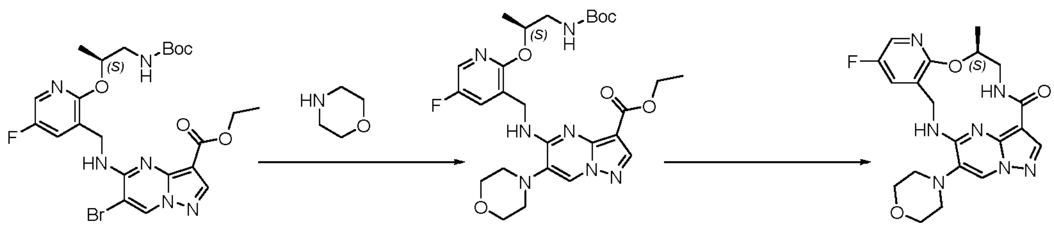

1H 17A 17

**Step 1:** The synthetic steps for the preparation of ethyl (S)-5-(((((2-((1-(((tert-butyloxycarbonyl)amino) propan-2-yl)oxy)-5-fluoropyridin-3-3-yl)methyl)amino)-6-morpholinopyrazolo[1,5-a]pyrimidin-3-carboxylat e (**compound 17A**) were shown as follows:

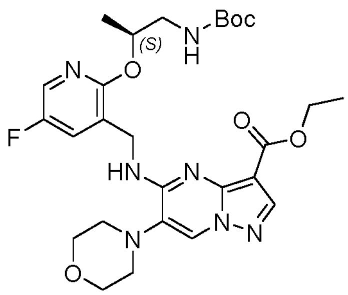

**17A**

Under the protection of argon, ethyl (S)-6-bromo-5-((((2-((1-(((tert-butyloxycarbonyl)amino) propan-2-yl)oxy)-5-fluoropyridin-3-3-yl)methyl)pyrazolo[1,5-a]pyrimidin-3-carboxylate (567.0 mg, 1.0 mmol) was dissolved in morpholine (5.0 mL), to which was added $Cs_2CO_3$ (1.0 mg, 3.0 mmol). Then, the reaction system was put into a microwave reactor and stirred at 130 °C for 2h. The reaction mixture was concentrated under reduced pressure. The residue was separated and purified by chromatographic column to obtain **compound 17A.**

MS (ESI) m/z 574.6 $(M+H)^+$

**Step 2:** Preparation of (S,$1^3$E,$1^4$E)-$4^5$-fluoro-6-methyl-$1^6$-morpholino-5-oxa-2,8-diaza-1(5,3)-pyrazolo[1,5-a]pyrimidina-4(3,2)-pyridinacyclononaphan-9-one (**compound 17**)

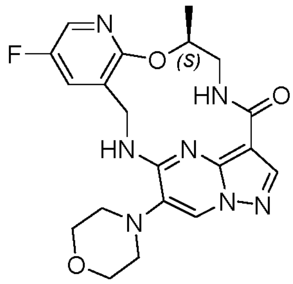

**17**

Ethyl ((S)-5-(((((2-((1-(((tert-butyloxycarbonyl)amino)propan-2-yl)oxy)-5-fluoropyridin-3-3-yl) methyl)amino)-6-morpholinopyrazolo[1,5-a]pyrimidin-3-carboxylate (344 mg, 0.60 mmol) was dissolved in a mixed solution of methanol and THF, to which was then added LiOH (288 mg, 12.09 mmol) aqueous solution. After addition, the reaction system was heated to 60 °C and stirred for 16 h. Then, the reaction system was cooled to 0 °C, and the pH was adjusted to 2-3 with 2N hydrochloric acid. The resultant mixture was extracted three times with DCM. The organic phases were combined, dried over anhydrous sodium sulfate, filtered, and concentrated to dryness. Then, the solution of HCl in 1,4-dioxane was added, and the resultant mixture was stirred at room temperature for 1 h, concentrated to dryness under reduced pressure, to which were then added DCM and DMF, followed by successive addition of DIPEA (15.78 g, 122.08 mmol) and FDPP (2.95 g, 7.69 mmol). After addition, the reaction system was stirred for 16 h at room temperature. The reaction system was concentrated to dryness under reduced pressure, and the residue was separated by high pressure preparative separation to obtain **compound 17** of Example 17.

MS (ESI) m/z 428.4 $(M+H)^+$
$^1$H NMR (400 MHz, DMSO-$d_6$) $\delta$ 9.71 - 9.58 (m, 1H), 8.53 (s, 1H), 8.28 (t, *J* = 6.1 Hz, 1H), 8.07 - 7.99 (m, 2H), 7.88 - 7.71 (m, 1H), 7.66 - 7.49 (m, 1H), 5.13 - 4.87 (m, 2H), 4.18 (dd, *J* = 14.5, 5.9 Hz, 1H), 3.99 (m, 1H), 3.91 - 3.72 (m, 4H), 3.52 (m, 1H), 3.11 (m, 1H), 2.89 (m, 2H), 1.45 (dd, *J* = 6.1, 4.1 Hz, 3H).

Example **18:** Preparation of N-(4-((S,1$^3$E,1$^4$E)-4$^5$-fluoro-6-methyl-9-oxo-5-oxa-2,8-diaza-1(5,3)-pyrazolo[1,5-a]pyrimidin-4(3,2)-pyridinacyclononaphan-1$^6$-yl)phenyl)acetamide (**compound 18**):

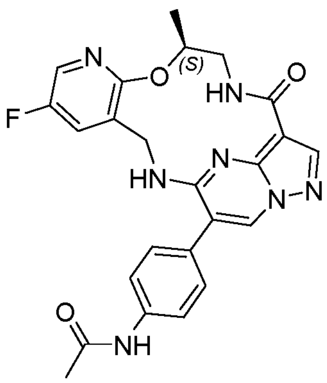

**18**

Using (4-acetylaminophenyl)boronic acid to replace (3-chlorophenyl)boronic acid, **compound 18** of Example 18 was obtained by the preparation method same as that of Example 3.

MS (ESI) m/z 476.2 $(M+H)^+$
$^1$H NMR (400 MHz, DMSO-$d_6$) $\delta$ 10.16 (s, 1H), 9.62 (d, *J* = 8.8 Hz, 1H), 8.58 (s, 1H), 8.16 - 8.00 (m, 3H), 7.85 - 7.72 (m, 3H), 7.46 (d, *J* = 8.5 Hz, 2H), 5.10 - 4.96 (m, 2H), 4.14 - 3.97 (m, 2H), 3.23 - 3.10 (m, 1H), 2.11 (s, 3H), 1.48 (d, *J* = 6.1 Hz, 3H).

**Example 19:** Preparation of 4-((S,$1^3$E,$1^4$E)-$4^5$-fluoro-6-methyl-9-oxo-5-oxa-2,8-diaza-1(5,3)-pyrazolo[1,5-a]pyrimidin-4(3,2)-pyridinacyclononaphan-$1^6$-yl)-N-methylbenzamide (**compound 19**):

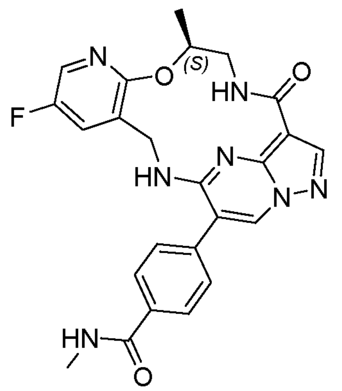

**19**

Using (4-(methylcarbamoyl)phenyl)boronic acid to replace (3-chlorophenyl)boronic acid, **compound 19** of Example 19 was obtained by the preparation method same as that of Example 3.

MS (ESI) m/z 476.2 $(M+H)^+$

$^1$H NMR (400 MHz, DMSO-$d_6$) δ 9.62 (d, *J* = 8.8 Hz, 1H), 8.69 (s, 1H), 8.58 (d, *J* = 4.7 Hz, 1H), 8.12 (d, *J* = 4.2 Hz, 2H), 8.08 - 7.99 (m, 3H), 7.76 (dd, *J* = 8.8, 3.1 Hz, 1H), 7.64 (d, *J* = 8.2 Hz, 2H), 5.03 (m, 2H), 4.04 (m, 2H), 3.16 (dd, *J* = 13.1, 10.1 Hz, 1H), 2.85 (d, *J* = 4.4 Hz, 3H), 1.48 (d, *J* = 6.1 Hz, 3H).

**Example 20:** Preparation of (S,$1^3$E,$1^4$E)-$4^5$-fluoro-6-methyl-$1^6$-(1-methyl-6-oxo-1,6-dihydropyridin-3-yl)-5-oxa-2,8-diaza-1 (5,3)-pyrazolo[1,5-a]pyrimidin-4(3,2)-pyridinacyclononaphan-9-on e (**compound 20**):

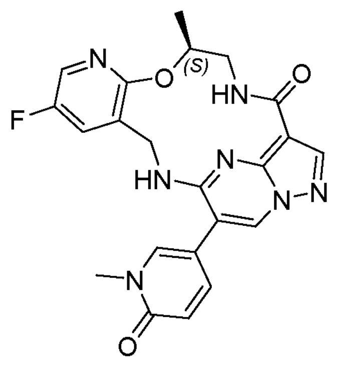

**20**

Using 1-methyl-5-(4,4,5,5-tetramethyl-1,3,2-dioxaborolan-2-yl)pyridin-2(1H)-one to replace (3-chlorophenyl)boronic acid, compound 20 of Example 20 was obtained by the preparation method same as that of Example 3.

MS (ESI) m/z 450.2 $(M+H)^+$

$^1$H NMR (400 MHz, DMSO-$d_6$) δ 9.62 (d, *J* = 8.5 Hz, 1H), 8.66 (s, 1H), 8.27 (t, *J* = 6.0 Hz, 1H), 8.04 (d, *J* = 3.1 Hz, 1H), 7.95 (d, *J* = 2.6 Hz, 1H), 7.76 (dd, *J* = 8.8, 3.1 Hz, 1H), 7.53 (dd, *J* = 9.3, 2.6 Hz, 1H), 6.56 (d, *J* = 9.3 Hz, 1H), 5.08 -4.95 (m, 2H), 4.09 - 3.98 (m, 2H), 3.50 (s, 3H), 3.19 - 3.12 (m, 1H), 1.46 (d, *J* = 6.1 Hz, 3H).

**Example 21:** Preparation of (S,$1^3$E,$1^4$E)-$1^6$-(2,4-dichlorophenyl)-$4^5$-fluoro-6-methyl-5-oxa-2,8-diaza-1(5,3)-pyrazolo[1,5-a]pyrimidin-4(3,2)-pyridinacyclononaphan-9-one (**compound 21**):

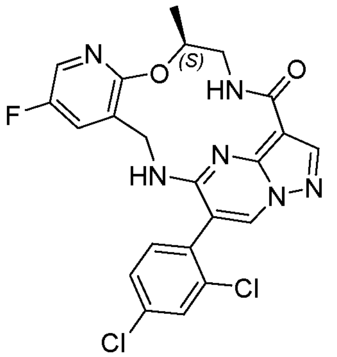

**21**

Using (2,4-dichlorophenyl)boronic acid to replace (3-chlorophenyl)boronic acid, **compound 21** of Example 21 was obtained by the preparation method same as that of Example 3.

MS (ESI) m/z 487.1 $(M+H)^+$

$^1$H NMR (400 MHz, DMSO-$d_6$) δ 9.66 (dd, *J* = 9.1, 4.0 Hz, 1H), 8.76 (d, *J*= 3.3 Hz, 1H), 8.14 (d, *J*= 1.5 Hz, 1H), 8.05 (m, 2H), 7.91 (dd, *J* = 5.6, 2.1 Hz, 1H), 7.73 - 7.59 (m, 2H), 7.59 - 7.51 (m, 1H), 5.10 - 4.90 (m, 2H), 4.09 - 3.92 (m, 2H), 3.15 (t, *J* = 12.0 Hz, 1H), 1.47 (d, *J* = 6.1 Hz, 3H).

**Example 22:** Preparation of (S,$1^3$*E*,$1^4$*E*)-$1^6$-(2,6-difluorophenyl)-$4^5$-fluoro-6-methyl-5-oxa-2,8-diaza-1(5,3)-pyrazolo[1,5-a]pyrimidin-4(3,2)-pyridinacyclononaphan-9-one (**compound 22**):

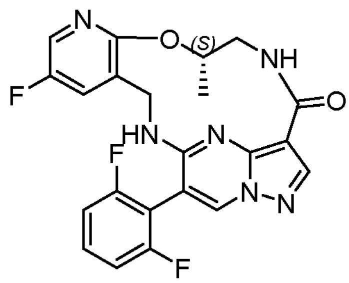

**22**

Using 2-(2,6-difluorophenyl)-4,4,5,5-tetramethyl-1,3,2-dioxaborolane to replace (3-chlorophenyl)boronic acid, **compound 22** of Example 22 was obtained by the preparation method same as that of Example 3.

MS (ESI) m/z 455.1 $(M+H)^+$

$^1$H NMR (400 MHz, DMSO-$d_6$) δ 9.60 (d, *J* = 8.9 Hz, 1H), 8.97 (s, 1H), 8.32 (t, *J* = 6.0 Hz, 1H), 8.16 (s, 1H), 8.05 (d, *J* = 3.1 Hz, 1H), 7.76 - 7.60 (m, 2H), 7.37 (m, 2H), 5.14 - 4.91 (m, 2H), 4.03 (m, 2H), 3.15 (m, 1H), 1.48 (d, *J* = 6.1 Hz, 3H).

**Example 23:** Preparation of (S,$1^3$E,$1^4$E)-$1^6$-cyclopropyl-$4^5$-fluoro-6-methyl-5-oxa-2,8-diaza-1(5,3)-pyrazolo[1,5-a]pyrimidin-4(3,2)-pyridinacyclononaphan-9-one (**compound 23**):

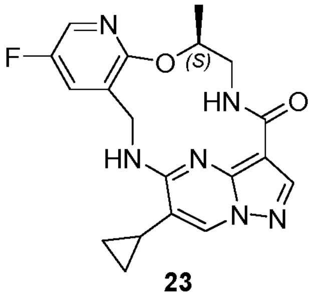

**23**

Using cyclopropylboronic acid to replace (3-chlorophenyl)boronic acid, **compound 23** of Example 23 was obtained by the preparation method same as that of Example 3.

MS (ESI) m/z 383.2 $(M+H)^+$

$^1$H NMR (400 MHz, DMSO-$d_6$) δ 9.68 (d, $J$ = 8.9 Hz, 1H), 8.64 (t, $J$ = 6.1 Hz, 1H), 8.44 (d, $J$ = 1.2 Hz, 1H), 8.04 (d, $J$ = 3.1 Hz, 1H), 8.02 (s, 1H), 7.83 (dd, $J$ = 8.7, 3.1 Hz, 1H), 5.12 - 4.97 (m, 2H), 4.16 (dd, $J$ = 14.5, 5.7 Hz, 1H), 4.00 (m, 1H), 3.17 - 3.07 (m, 1H), 1.47 (d, $J$ = 6.1 Hz, 3H), 0.98 (m, 2H), 0.89 - 0.82 (m, 1H), 0.77 (s, 1H), 0.67 (m, 1H).

**Example 24**: Preparation of ((S,1$^3$*E*,1$^4$*E*)-4$^5$-fluoro-6-methyl-1$^6$-(1-methyl-1H-pyrazol-4-yl)-5-oxa-2,8-diaza-1(5,3)-pyrazolo[1,5-a]pyrimidina-4(3,2)-pyridinacyclononaphan-9-one (**compound 24**):

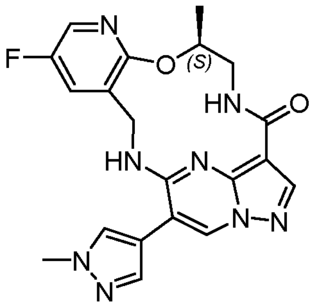

**24**

Using 1-methylpyrazol-4-boronic acid pinacol ester to replace (3-chlorophenyl)boronic acid, compound 24 of Example 24 was obtained by the preparation method same as that of Example 3.

MS (ESI) m/z 423.4 $(M+H)^+$

$^1$H NMR (400 MHz, DMSO-$d_6$) δ 9.59 (d, $J$ = 8.6 Hz, 1H), 8.65 (s, 1H), 8.23 - 8.10 (m, 2H), 8.08 (d, $J$ = 0.7 Hz, 1H), 8.03 (d, $J$ = 3.1 Hz, 1H), 7.92 - 7.84 (m, 1H), 7.78 (d, $J$ = 0.8 Hz, 1H), 5.19 - 4.89 (m, 2H), 4.10 (dd, $J$ = 14.5, 5.9 Hz, 1H), 4.01 (m, 1H), 3.96 (s, 3H), 3.15 (m, 1H), 1.47 (d, $J$ = 6.1 Hz, 3H).

Example 25: The synthetic steps for preparation of (S,1$^3$*E*,1$^4$*E*)-4$^5$-fluoro-6-methyl-1$^6$-phenylamino-5-oxa-2,8-diaza-1(5,3)-pyrazolo[1,5-a]pyrimidina-4(3,2)-pyridinacyclononaphan-9-one (**compound 25**) were shown as follows:

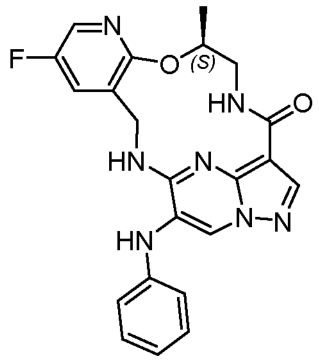

**25**

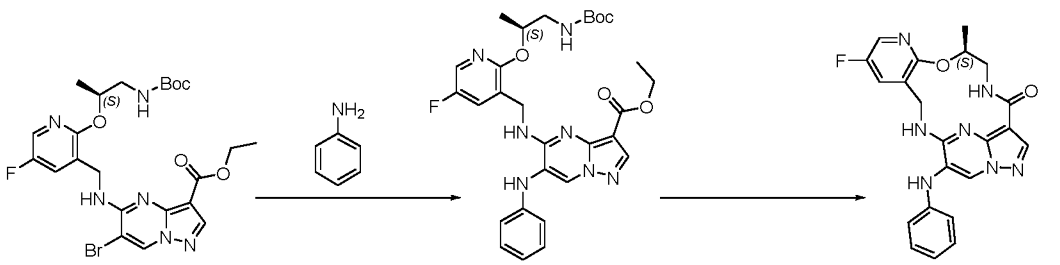

**1H** **25A** **25**

**Step 1:** Preparation of ethyl (S)-5-(((((2-((1-(((tert-butyloxycarbonyl)amino)propan-2-yl)oxy)-5-fluoropyridin-3-3-yl)methyl)amino)-6-(phenylaminopyrazolo[1,5-a]pyrimidin-3-carboxylate (**compound 25A):**

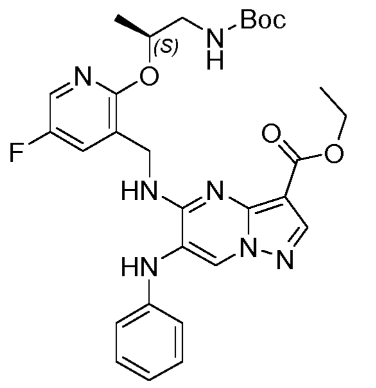

**25A**

Ethyl (S)-6-bromo-5-((((2-((1-(((tert-butyloxycarbonyl)amino)propan-2-yl)oxy)-5-fluoropyridin-3-3-yl)methyl)pyrazolo[1,5-a]pyrimidin-3-carboxylate (567.0 mg, 1.0 mmol), $Pd_2(dba)_3$ (91.5 mg, 0.1 mmol), t-BuXPhos (84.8 mg, 0.2 mmol), and $K_2CO_3$ (414.0 mg, 3.0 mmol) were dissolved in tert-butanol (5.0 mL), and then the reaction system was purged with argon. Finally, the reaction system was placed in a microwave reactor and stirred at 80 °C for 2 h. The reaction mixture was concentrated under reduced pressure. The residue was separated and purified by chromatographic column to obtain the intermediate **compound 25A.**
MS (ESI) m/z 580.2 $(M+H)^+$

**Step 2:** Preparation of (S,$1^3E$,$1^4E$)-$4^5$-fluoro-6-methyl-$1^6$-phenylamino-5-oxa-2,8-diaza-1(5,3)-pyrazolo[1,5-a]pyrimidina-4(3,2)-pyridinacyclononaphan-9-one (**compound 25**)

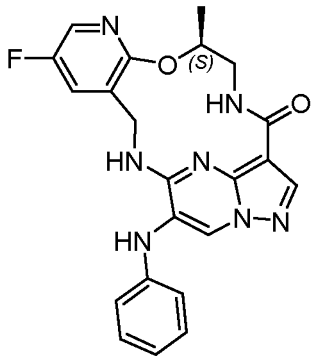

**25**

**Compound 25** of Example 25 was obtained by the same preparation method as that of Step 2 in Example 17.

MS (ESI) m/z 434.4 $(M+H)^+$

$^1$H NMR (400 MHz, DMSO-$d_6$) $\delta$ 9.72 (d, *J* = 9.0 Hz, 1H), 8.60 - 8.54 (m, 1H), 8.50 (t, *J* = 6.1 Hz, 1H), 8.05 (d, *J* = 5.2 Hz, 2H), 7.71 (dd, *J* = 8.8, 3.1 Hz, 1H), 7.60 (s, 1H), 7.26 - 7.07 (m, 2H), 6.87 - 6.77 (m, 1H), 6.77 - 6.67 (m, 2H), 5.17 - 4.83 (m, 2H), 4.27 - 3.84 (m, 2H), 3.14 (m, 1H), 1.47 (d, *J* = 6.1 Hz, 3H).

**Example 26:** The synthetic steps for preparation of ($1^3$E,$1^4$E,3R,6S)-$4^5$-fluoro-3,6-dimethyl-$1^6$-phenyl-5-oxa-2,8-diaza-1(5,3)-pyrazolo[1,5-a]pyrimidin-4(3,2)-pyridinacyclononaphan-9-one **(compound 26)** were shown as follows:

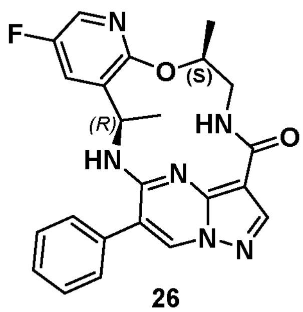

**26**

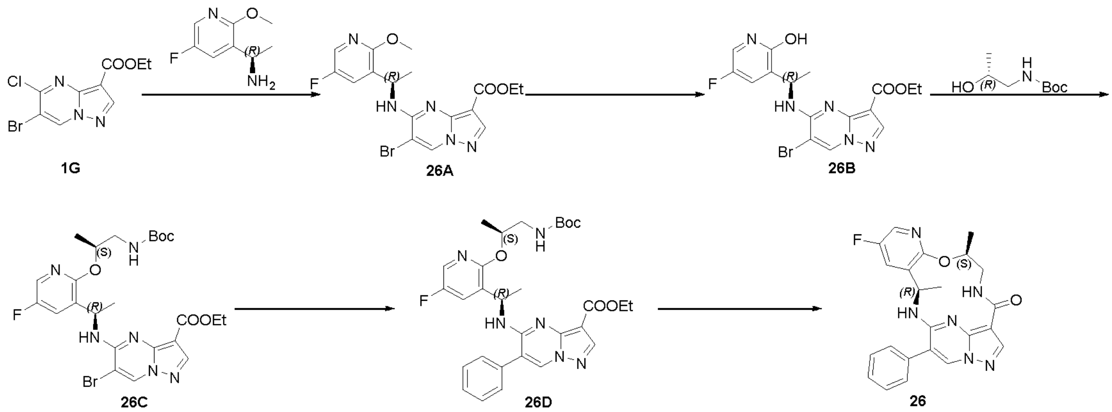

1G 26A 26B 26C 26D 26

**Step 1:** Preparation of ethyl (R)-6-bromo-5-((1-(5-fluoro-2-methoxypyridin-3-yl)ethyl)amino) pyrazolo[1,5-a]pyrimidin-3-carboxylate (**compound 26A**)

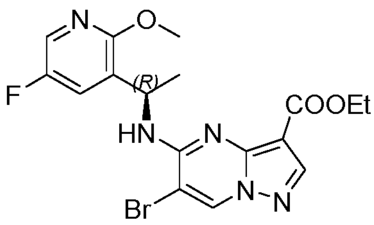

**26A**

Ethyl 6-bromo-5-chloropyrazolo[1,5-a]pyrimidin-3-carboxylate (500 mg, 1.64 mmol), (R)-1-(5-fluoro-2-methoxypyridin-3-yl)ethan-1-amine (335 mg, 1.97 mmol), TEA (497 mg, 4.92 mmol), and EtOH (10 mL) were placed in a reaction flask, and allowed to react for 5 h at 60 °C. After completion of the reaction, the residue was purified by column chromatography to obtain the **compound 26A**.
MS (ESI) m/z 438.0 $(M+H)^+$

**Step 2:** Preparation of ethyl (R)-6-bromo-5-((1-(5-fluoro-2-hydroxypyridin-3-yl)ethyl)amino) pyrazolo[1,5-a]pyrimidin-3-carboxylate **(compound 26B)**

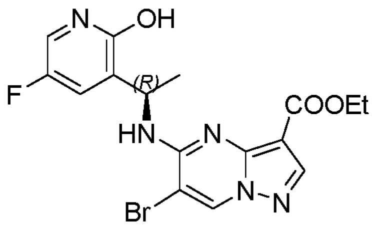

**26B**

Ethyl (R)-6-bromo-5-((1-(5-fluoro-2-methoxypyridin-3-yl)ethyl)amino)pyrazolo[1,5-a]pyrimidin-3-carboxylate (580 mg, 1.32 mmol) and the solution of HCl in 1,4-dioxane (4.0 M, 50 mL) were placed in a reaction flask, and allowed to react overnight at 80 °C. After completion of the reaction, the reaction system was naturally cooled to room temperature, and concentrated under reduced pressure, to which was then added NaHCOs solution (2.0 M, 20 mL). The resultant mixture was neutralized to pH 6-7 under stirring at room temperature, and then extracted three times with DCM. The organic phases were combined, dried over anhydrous sodium sulfate, filtered, and concentrated. The residue was purified by column chromatography to obtain **compound 26B**.
MS (ESI) m/z 424.0 $(M+H)^+$

**Step 3:** Preparation of ethyl 6-bromo-5-(((R)-1-(2-(((S)-1-((tert-butyloxycarbonyl)amino) propan-2-yl)oxy)-5-fluoropyridin-3-yl)ethyl)amino)pyrazolo[1,5-a]pyrimidin-3-carboxylate **(compound 26C)**

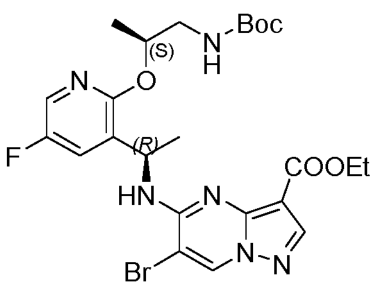

**26C**

Ethyl (R)-6-bromo-5-((1-(5-fluoro-2-hydroxypyridin-3-yl)ethyl)amino)pyrazolo[1,5-a]pyrimidin-3-carboxylate (300 mg, 0.71 mmol), tert-butyl (R)-(2-hydroxypropyl)carbamate (186 mg, 1.07 mmol), THF (5 mL) and $PPh_3$ (372 mg, 1.42 mmol) were successively added into a reaction flask, to which was added DIAD (247 mg, 1.42 mmol) dropwise

under argon atmosphere, and then the mixture was allowed to react overnight at room temperature. The reaction mixture was concentrated to dryness, and the residue was purified by column chromatography to obtain **compound 26C.**
MS (ESI) m/z 581.1 $(M+H)^+$

**Step 4**: Preparation of 5-(((R)-1-(2-(((S)-1-((tert-butyloxycarbonyl)amino)propan-2-yl)oxy)-5-fluoropyridin-3-yl)ethyl)aminoethyl)-6-phenylpyrazolo[1,5-a]pyrimidin-3-carboxylate (**compound 26D**)

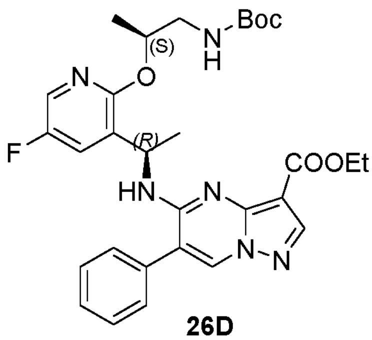

**26D**

Ethyl 6-bromo-5-(((R)-1-(2-(((S)-1-((tert-butyloxycarbonyl)amino)propan-2-yl)oxy)-5-fluoropyridin-3-yl)ethyl)amino)pyrazolo[1,5-a]pyrimidin-3-carboxylate (300 mg, 0.52 mmol), phenylboronic acid (82.5 mg, 0.68 mmol), $Pd(PPh_3)_4$ (60 mg, 0.052 mmol), $K_3PO_4$ (220 mg, 1.04 mmol), 1,4-dioxane (6 mL), and water (2 mL) were added into a reaction flask, and allowed to react for 3 h at 90 °C under argon atmosphere. After completion of the reaction, the reaction mixture was extracted three times with DCM. The organic phases were combined, dried over anhydrous sodium sulfate, filtered, and concentrated. The residue was purified by column chromatography to obtain **compound 26D.**
MS (ESI) m/z 579.3 $(M+H)^+$

**Step 5:** Preparation of ($1^3$E,$1^4$E,3R,6S)-$4^5$-fluoro-3,6-dimethyl-$1^6$-phenyl-5-oxa-2,8-diaza-1(5,3)-pyrazolo[1,5-a]pyrimidin-4(3,2)-pyridinacyclononaphan-9-one **(compound** 26)

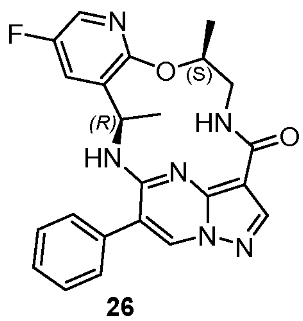

**26**

**compound 26** of Example 26 was obtained by the preparation method same as that of Step 2 in Example 17.

MS (ESI) m/z 433.2 $(M+H)^+$
$^1$H NMR (400 MHz, DMSO-$d_6$) δ 9.54 (d, *J* = 8.7 Hz, 1H), 8.61 (s, 1H), 8.11 (s, 1H), 8.02 (d, *J* = 8.2 Hz, 2H), 7.70 - 7.50 (m, 5H), 5.62 - 5.53 (m, 1H), 5.08 (m, 1H), 4.02 (m, 2H), 3.17 (d, *J* = 12.1 Hz, 1H), 1.49 (dd, *J* = 6.6, 4.2 Hz, 6H).

**Example 27:** The synthetic steps for preparation of methyl 3-chloro-4-((S,$1^3$*E*,$1^4$*E*)-$4^5$-fluoro-6-methyl-9-oxo-5-oxa-2,8-diaza-1(5,3)-pyrazolo[1,5-*a*]pyrimidin-4(3,2)-pyridinacyclononaphan-$1^6$-yl)phenyl)carbamate **(compound 27**) were shown as follows:

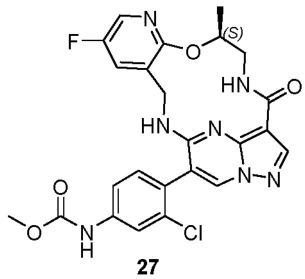

**27**

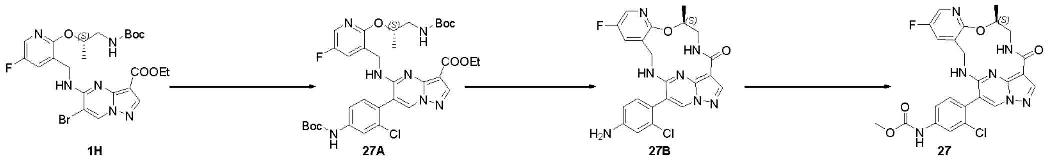

1H 27A 27B 27

**Step** 1: preparation of ethyl (S)-6-(4-((tert-butyloxycarbonyl)amino-2-chlorophenyl)-5-(((2-((1-(1-((tert-butyloxycarbonyl)amino)propan-2-yl)oxy)-5-fluoropyridin-3-3-yl)methyl)aminopyrazolo[1, 5-a]pyrimidin-3-carboxylate (**compound 27A**)

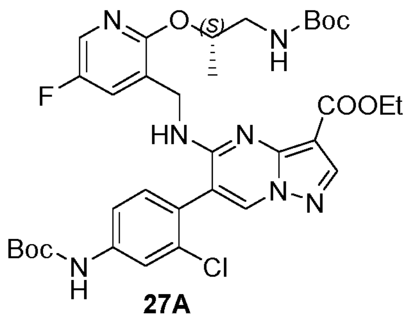

**27A**

Under argon atmosphere, ethyl (S)-6-bromo-5-((((2-((1-(((tert-butyloxycarbonyl)amino) propan-2-yl)oxy)-5-fluoropyridin-3-3-yl)methyl)pyrazolo[1,5-a]pyrimidin-3-carboxylate (BT113-114-016) (1.13 g, 2.0 mmol), 2-chloro-4-tert-butylamino-1-phenylboronic acid pinacol ester (848.0 mg, 1.2 mmol), Xphos Pd $G_3$ (180 mg, 0.1 mmol), Xphos (224 mg, 0.2 mmol), and $K_3PO_4$ (1.27 g, 3.0 mmol) were dissolved in 1,4-dioxane/water (3:1, 16.0 mL), and finally the reaction system was stirred for 3 h at 80°C. After completion of the reaction, the reaction mixture was poured into water, and the water phase was extracted three times with ethyl acetate. The organic phases were combined, dried over anhydrous sodium sulfate, filtered, and concentrated. The residue was separated and purified over column chromatography to obtain **compound 27A.**
MS (ESI) m/z 714.3 $(M+H)^+$

**Step 2:** Preparation of (S,$1^3E$,$1^4E$)-$1^6$-(4-amino-2-chlorophenyl)-$4^5$-fluoro-6-methyl-5-oxa-2,8-diaza-1(5,3)-pyrazolo[1,5-a]pyrimidina-4(3,2)-pyridinacyclononaphan-9-one (**compound 27B**)

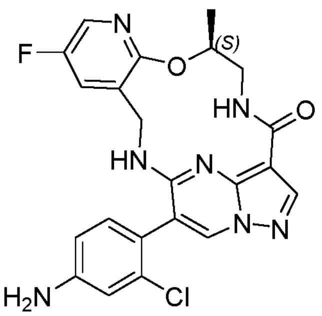

**27B**

Ethyl (S)-6-(4-((tert-butyloxycarbonyl)amino-2-chlorophenyl)-5-(((2-((1-(1-((tert-butyloxycarbonyl) amino)propan-2-yl)oxy)-5-fluoropyridin-3-3-yl)methyl)aminopyrazolo[1,5-a]pyrimidin-3-carboxylate (572.0 mg, 0.80 mmol) was dissolved in a mixed solution of methanol and THF, to which was then added LiOH (288 mg, 12.09 mmol) aqueous solution. After addition, the reaction system was heated to 60 °C and stirred for 16 h. After completion of the reaction, the reaction system was cooled to 0 °C, and the pH was adjusted to 2-3 with 2N hydrochloric acid. The resultant mixture was extracted three times with DCM. The organic phases were combined, dried over anhydrous sodium sulfate, and concentrated under reduced pressure. To the residue, was added the solution of HCl in 1,4-dioxane, and the resultant mixture was stirred at room temperature for 1 h. After completion of the reaction, the reaction mixture was concentrated under reduced pressure, and then to the residue were added DCM (120 mL) and DMF (60 mL), followed by successively adding DIPEA (15.78 g, 122.08 mmol) and FDPP (2.95 g, 7.69 mmol) to the above mixed solution. After addition, the reaction mixture was stirred for 16 h at room temperature. After completion of the reaction, the reaction mixture was concentrated under reduced pressure, and the residue was separated by high pressure preparative separation to obtain **compound 27B.**

MS (ESI) m/z 468.1 $(M+H)^+$

**Step 3:** methyl 3-chloro-4-((S,1$^3$*E*,1$^4$*E*)-4$^5$-fluoro-6-methyl-9-oxo-5-oxa-2,8-diaza-1(5,3)-pyrazolo[1,5-a]pyrimidin-4(3,2)-pyridinacyclononaphan-16-yl)phenyl)carbamate **(compound 27)**

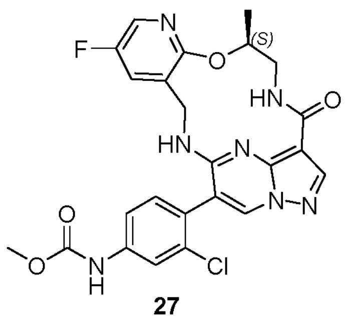

**27**

(S,1$^3$*E*,1$^4$*E*)-1$^6$-(4-amino-2-chlorophenyl)-4$^5$-fluoro-6-methyl-5-oxa-2,8-diaza-1(5,3)-pyrazolo[1,5-a] pyrimidina-4(3,2)-pyridinacyclononaphan-9-one (75.0 mg, 0.16 mmol) was dissolved in DCM (10.0 mL), to which was then added DIPEA (62.0 mg, 0.48 mmol), and finally methyl chlorocarboxylate (23.0 mg, 0.24 mmol) was added at 0 °C. Then, the reaction system was allowed to react at room temperature for 2 h. After completion of the reaction, the reaction mixture was concentrated under reduced pressure, and the residue was separated by high pressure preparative separation to obtain **compound 27** of Example 27.

MS (ESI) m/z 526.1 $(M+H)^+$

$^1$H NMR (400 MHz, DMSO-$d_6$) δ 10.12 (s, 1H), 9.74 - 9.61 (m, 1H), 8.67 (d, *J* = 2.1 Hz, 1H), 8.12 (s, 1H), 8.10 - 7.94 (m, 2H), 7.92 - 7.68 (m, 2H), 7.67 - 7.34 (m, 3H), 5.09 - 4.87 (m, 2H), 4.01 (m, 2H), 3.74 (s, 3H), 3.14 (t, *J* = 12.0 Hz, 1H), 1.47 (d, *J* = 6.1 Hz, 3H).

**Example 28:** The synthetic steps for preparation of (S,$1^3E$,$1^4E$)-$1^7$-amino-$4^5$-fluoro-6-methyl-$1^6$-phenyl-5-oxa-2,8-diaza-1(5,3)-pyrazolino[1,5-*a*]pyrimidin-4(3,2)-pyridinacyclononaphan-9-one **(compound 28)** were shown as follows:

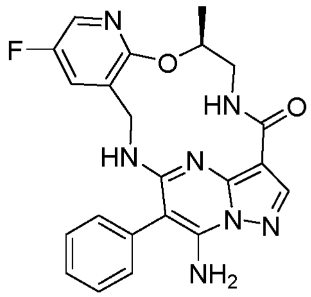

**28**

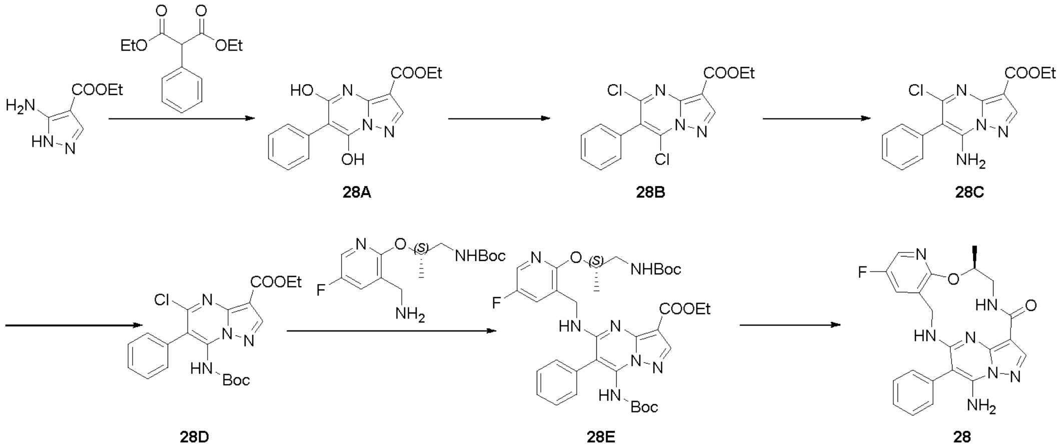

28A 28B 28C 28D 28E 28

**Step 1:** Preparation of ethyl 5,7-dihydroxyl-6-phenylpyrazolo[1,5-a]pyrimidin-3-carboxylate (**compound 28A)**

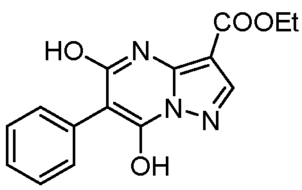

**28A**

ethyl 5-amino-1*H*-pyrazol-4-carboxylate (5 g, 32.23 mmol) was dissolved in ethanol, to which were successively added diethyl 2-phenylmalonate (15.2 g, 64.45 mmol) and sodium ethoxide (6.6 g, 96.68 mmol) in an ice bath. The reaction was heated to 90 °C and stirred for 16 h. After completion of the reaction, the reaction system was cooled to 0 °C, and filtered. The filter cake was rinsed with ethanol. The solid was dissolved in water, to which was added hydrochloric acid to adjust pH to 1. Lots of solid precipitated, which was collected by filtration. The filter cake was rinsed with water, and then dried to obtain **compound 28A.**
MS (ESI) m/z 300.1 $(M+H)^+$

**Step 2:** Preparation of ethyl 5,7-dichloro-6-phenylpyrazolo[1,5-a]pyrimidin-3-carboxylate **(compound 28B)**

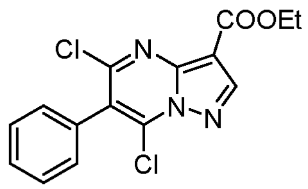

**28B**

At 0 °C, to a reaction flask containing ethyl 5,7-dihydroxyl-6-phenylpyrazolo[1,5-a]pyrimidin-3-carboxylate (4.6 g, 15.37 mmol), was added $POCl_3$ (100 mL), and then *N*,*N*-dimethylphenylamine (5.22 g, 43.04 mmol) was slowly added dropwise. After addition, the reaction was heated to 80°C, and then allowed to react at this temperature for 16 h. After completion of the reaction, the reaction mixture was slowly poured into the ice water for quenching the reaction, and then the resultant mixture was extracted twice with dichloromethane. The organic phases were combined, dried over anhydrous sodium sulfate, and concentrated under reduced pressure. The residue was separated and purified by column chromatography to provide **compound 28B.**
MS (ESI) m/z 336.0 $(M+H)^+$

**Step 3:** Preparation of ethyl 7-amino-5-chloro-6-phenylpyrazolo[1,5-a]pyrimidin-3-carboxylate(compound 28C)

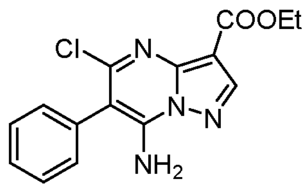

**28C**

Ethyl 5,7-dichloro-6-phenylpyrazolo[1,5-a]pyrimidin-3-carboxylate(2.5 g, 7.44 mmol) was dissolved in a solution of amine in ethanol (100 mL). After addition, the reaction system was stirred at room temperature for 3 h. After completion of the reaction, the reaction mixture was poured into water, and then a lot of yellow solid compound was precipitated. The filter cake was rinsed with water, and then dried to obtain **compound 28C.**
MS (ESI) m/z 317.1 $(M+H)^+$

Step 4: Preparation of ethyl 7-((tert-butyloxycarbonyl)amino)-5-chloro-6-phenylpyrazolo[1,5-a] pyrimidin-3-carboxylate(**compound 28D)**

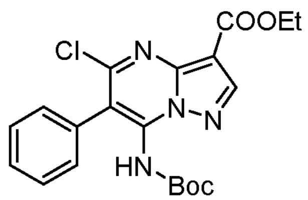

**28D**

Ethyl 7-amino-5-chloro-6-phenylpyrazolo[1,5-a]pyrimidin-3-carboxylate (2.0 g, 6.31 mmol) was dissolved in DCM, to which was then added DMAP (1.5 g, 12.63 mmol). The reaction system was cooled to 0°C and stirred for 0.5 h, and then $(Boc)_2O$ (2.8 g, 12.63 mmol) was slowly added dropwise. After addition, the reaction system was stirred at room temperature for 3 h. After completion of the reaction, the reaction mixture was concentrated under reduced pressure. The residue was separated and purified by column chromatography to provide **compound 28D.**

MS (ESI) m/z 417.1 $(M+H)^+$

**Step 5:** Preparation of ethyl (S)-7-((tert-butyloxycarbonyl)amino)-5-((2-((1-((tert-butyloxycarbonyl) amino)propan-2-yl)oxy)-5-fluoropyridin-3-yl)methyl)amino)-6-phenylpyrazolo[1,5-a]pyrimidin-3-carboxylate (**compound 28E**)

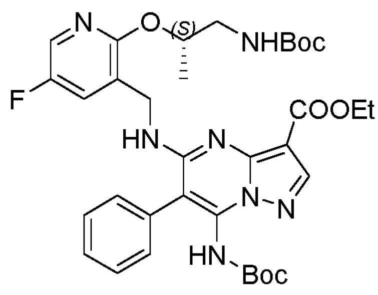

**28E**

Ethyl 7-((tert-butyloxycarbonyl)amino)-5-chloro-6-phenylpyrazolo[1,5-a]pyrimidin-3-carboxylate (600 mg, 1.44 mmol) was dissolved in ethanol, to which was then added tert-butyl (*S*)-(2-((3-(aminomethyl)-5-fluoropyridin-2-yloxy)propyl)carbamate (517 mg, 1.73 mmol) and DIPEA (372 mg, 2.88 mmol). The reaction system was heated to 80 °C and stirred for 16 h. After completion of the reaction, the reaction mixture was concentrated under reduced pressure. The residue was separated by column chromatography to provide **compound 28E.**
MS (ESI) m/z 680.3 $(M+H)^+$

**Step 6:** Preparation of ($S$,$1^3E$,$1^4E$)-$1^7$-amino-$4^5$-fluoro-6-methyl-$1^6$-phenyl-5-oxa-2,8-diaza-1(5,3)-pyrazolino[1,5-a]pyrimidin-4(3,2)-pyridinacyclononaphan-9-one (**compound 28**)

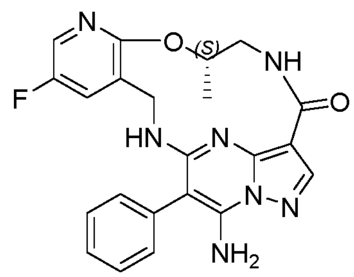

**28**

**Compound 28** of Example 28 was obtained by the preparation method same as that of Step 2 in Example 17.

MS (ESI) m/z 433.2 $(M+H)^+$
$^1$H NMR (400 MHz, DMSO-$d_6$) δ 9.87 (d, *J* = 8.8 Hz, 1H), 8.07 (s, 1H), 8.00 (d, *J* = 3.1 Hz, 1H), 7.70 (dd, *J* = 8.9, 3.1 Hz, 1H), 7.60 (t, *J* = 7.5 Hz, 2H), 7.51 (m, 1H), 7.35 (s, 2H), 6.98 (t, *J* = 6.2 Hz, 1H), 6.70 (s, 2H), 5.07 - 4.91 (m, 2H), 4.01 (m, 1H), 3.88 (dd, *J* = 14.6, 5.7 Hz, 1H), 3.12 (m, 1H), 1.46 (d, *J* = 6.1 Hz, 3H).

**Example 29:** The synthetic steps for preparation of (($3^1$s,$3^3$s,$6^3$E,$6^4$E)-$1^5$-fluoro-$6^6$-phenyl-2-oxa-4,7-diaza-6(3,5)-pyrazolo[1,5-a]pyrimidin-1(2,3)-pyridin-3(1,3)-cyclobutanacyclooctaphan-5-one (**compound 29**) were shown as follows:

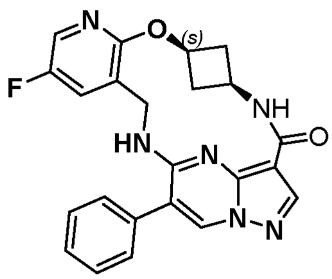

**29**

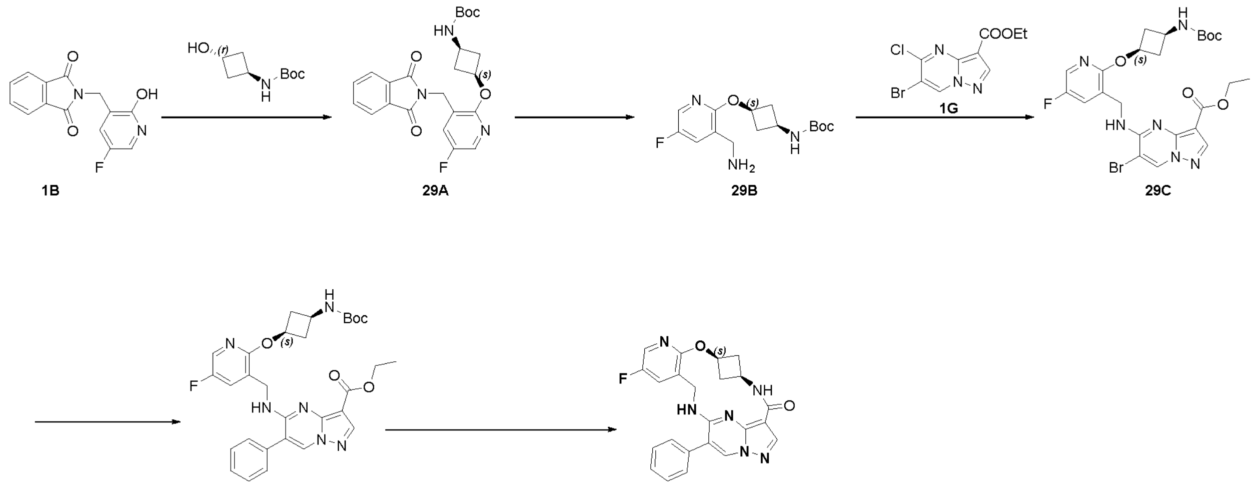


1B 29A 29B 29C 29D 29

**Step** 1: Preparation of tert-butyl ((1s,3s)-3-((3-((1,3-dioxoisoindol-2-yl)methyl)-5-fluoropyridin-2-yl) oxy)cyclobutanyl)carbamate (**compound 29A**)

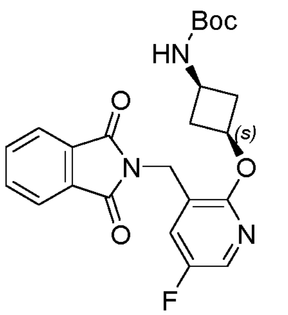

**29A**

Under the protection of argon, 2-((5-fluoro-2-hydroxypyridin-3-yl)methyl)isoindolin-1,3-dione (1 g, 3.68 mmol), tert-butyl ((1r,3r)-3-hydroxycyclobutanyl)carbamate(1.03 g, 5.51 mmol), $PPh_3$(1.44 g, 5.51 mmol), and DCM (40 mL) were added into a reaction flask, to which was then added DIAD (1.11 g, 5.51 mmol) dropwise at 0°C, and the mixture was allowed to react at room temperature for 2h. After completion of the reaction, the reaction mixture was concentrated to dryness under reduced pressure. The residue was purified by column chromatography to obtain **compound 29A.** MS (ESI) m/z 442.2 $(M+H)^+$.

**Step 2:** Preparation of tert-butyl ((1s,3s)-3-((3-(aminomethyl)-5-fluoropyridin-2-yl)ooy) cyclobutanyl)carbamate (**compound 29B**)

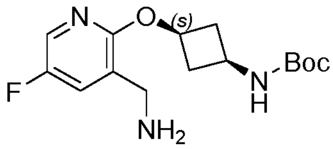

**29B**

Tert-Butyl ((1s,3s)-3-((3-((1,3-dioxoisoindol-2-yl)methyl)-5-fluoropyridin-2-yl)oxy) cyclobutanyl)carbamate (1 g, 2.27 mmol) was dissolved in ethanol (50 mL), to which was then added hydrazine hydrate (0.284 g, 4.54 mmol, 80%), and the reaction mixture was stirred overnight at 80 °C. After completion of the reaction, the reaction mixture was cooled to room temperature, and filtered. The filtrate was collected to provide **compound 29B.**
MS (ESI) m/z 312.2 $(M+H)^+$

**Step 3**: Preparation of ethyl 6-bromo-5-((((2-((1s,3s)-3-((tert-butyloxycarbonyl)amino)cyclobutoxy)-5-fluoropyridin-3-yl)methyl)pyrazolo[1,5-a]pyrimidin-3-carboxylate (**compound 29C**)

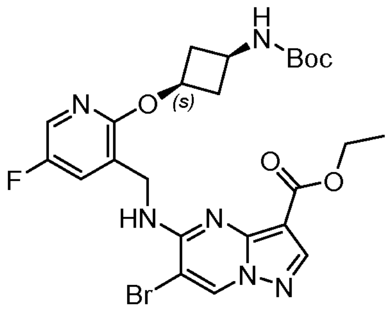

**29C**

Tert-butyl ((1s,3s)-3-((3-(aminomethyl)-5-fluoropyridin-2-yl)oxy)cyclobutanyl)carbamate (300 mg, 1 mmol), ethyl 6-bromo-5-chloropyrazolo[1 ,5-a]pyrimidin-3-carboxylate (308 mg, 1 mmol), DIPEA (226 mg, 2 mmol), and ethanol (30 mL) were added into a reaction flask, and allowed to react at 60°C for 3h. After completion of the reaction, the reaction mixture was concentrated to dryness under reduced pressure. The residue was purified by column chromatography to obtain **compound 29C.**
MS (ESI) m/z 579.1 $(M+H)^+$

**Step 4:** Preparation of ethyl 5-(((2-((1s,3s)-3-((tert-butyloxycarbonyl)amino)cyclobutoxy)-5-fluoropyridin-3--3-yl)methyl)aminoethyl-6-pyrazoloethyl[1,5-a]pyrimidin-3-carboxylate (**compound 29D**)

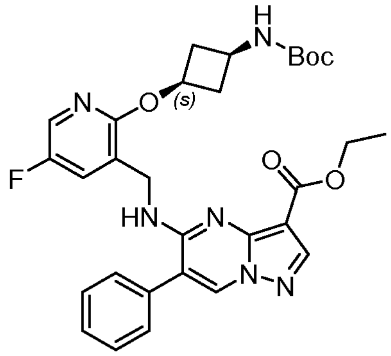

**29D**

Under the protection of argon, ethyl 6-bromo-5-((((2-((1s,3s)-3-((tert-butyloxycarbonyl)amino) cyclobutoxy)-5-fluoropyridin-3-yl)methyl)pyrazolo[1,5-a]pyrimidin-3-carboxylate (160 mg, 0.28 mmol), phenylboronic acid (41.2 mg, 0.34 mmol), Xphos (14.3 mg, 0.04 mmol), XphosPd G2 (25.4 mg, 0.03 mmol), $K_3PO_4$ (178 mg, 0.84 mmol), 1,4-dioxane (10 mL), and water (2.5 mL) were added into a reaction flask, and the mixture was allowed to react at 100 °C for 2 h. After completion of the reaction, the reaction mixture was diluted by adding water, and the resultant mixture was extracted three times with ethyl acetate. The organic phases were combined, dried over anhydrous sodium sulfate, filtered, and concentrated. The residue was purified by column chromatography to obtain **compound 29D.**
MS (ESI) m/z 577.2 $(M+H)^+$

**Step 5:** Preparation of (($3^1$s,$3^3$s,$6^3$E,$6^4$E)-$1^5$-fluoro-$6^6$-phenyl-2-oxa-4,7-diaza-6(3,5)-pyrazolo [1,5-a]pyrimidin-1(2,3 )-pyridin-3(1,3)-cyclobutanacyclooctaphan-5-one (**compound 29**)

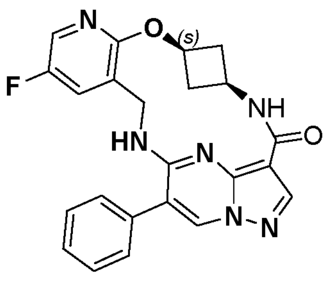

**29**

**Compound 29** of Example 29 was obtained by the preparation method same as that of Step 2 in Example 17.

MS (ESI) m/z 431.2 $(M+H)^+$
$^1$H NMR (400 MHz, DMSO-$d_6$) δ 9.20 (d, *J* = 10.8 Hz, 1H), 8.60 (s, 1H), 8.12 (s, 1H), 8.04 (d, *J* = 3.0 Hz, 1H), 7.96 (t, *J* = 6.4 Hz, 1H), 7.80 (dd, *J* = 8.8, 3.0 Hz, 1H), 7.66 - 7.53 (m, 4H), 5.26 (m, 1H), 5.14 (q, *J* = 4.5 Hz, 1H), 4.72 (m, 1H), 4.08 (dd, *J* = 14.8, 5.9 Hz, 1H), 3.07 (m, 1H), 2.97 - 2.83 (m, 1H), 2.14 (dd, *J* = 13.3, 7.5 Hz, 1H), 2.08 (s, 0H), 1.69 (dd, *J* = 13.7, 7.6 Hz, 1H)

**Example 30:** The synthetic steps for preparation of methyl (4-((S,$1^3$E,$1^4$E)-$4^5$-fluoro-6-methyl-9-oxo-5-oxa-2,8-diaza-1(5,3)-pyrazolo[1,5-a]pyrimidin-4(3,2)-pyridinacyclononaphan-$1^6$-yl)phenyl) carbamate (**compound 30**) were shown as follows:

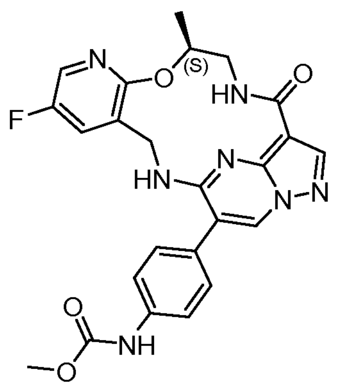

**30**

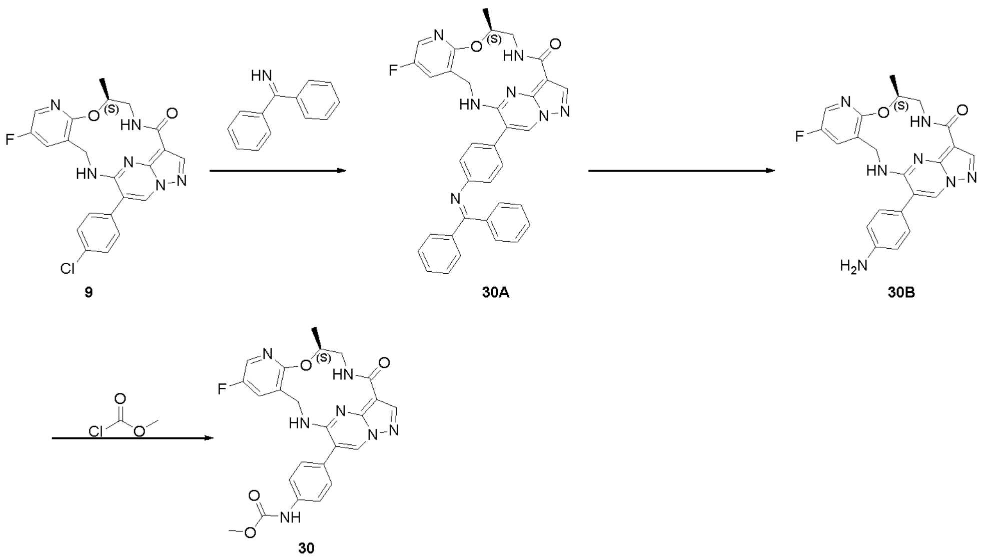

9 30A 30B 30

**Step** 1: Preparation of (S,$1^{3}$E,$1^{4}$E)-16-(4-(((diphenylmethylene)amino)phenyl)-$4^{5}$-fluoro-6-methyl-5-oxa-2,8-diaza-1(5,3)-pyrazolo[1,5-a]pyrimidin-4(3,2)-pyridinacyclononaphan-9-one **(compound 30A)**

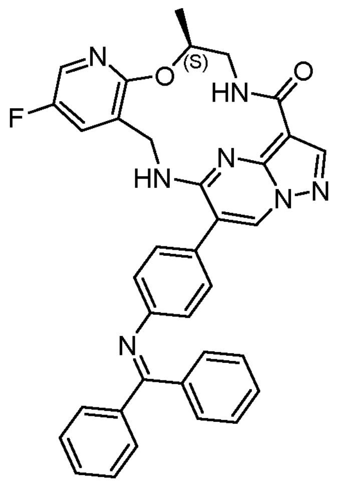

**30A**

(S,$1^{3}$E,$1^{4}$E)-$1^{6}$-(4-chlorophenyl)-$4^{5}$-fluoro-6-methyl-5-oxa-2,8-diaza-1(5,3)-pyrazolo[1,5-a]pyrimidin-4(3,2)-pyridinacyclononaphan-9-one (500 mg, 1.10 mmol), benzophenonimine (300 mg, 1.65 mmol), Xphos Pd G3 (87.5 mg, 0.11 mmol), $K_3PO_4$ (466 mg, 2.2 mmol), and 1,4-dioxane (10 mL) were added into a reaction flask, and the reaction system was purged with argon, then reacted overnight at 100 °C. After completion of the reaction, the reaction mixture was extracted three times with ethyl acetate. The organic phases were combined, dried over anhydrous sodium sulfate, filtered, and concentrated. The residue was purified by column chromatography to obtain **compound 30A.**
MS (ESI) m/z 598.2 $(M+H)^+$

**Step 2:** Preparation of (S,$1^3$E,$1^4$E)-$1^6$-(4-aminophenyl)-$4^5$-fluoro-6-methyl-5-oxa-2,8-diaza-1(5,3)-pyrazolo[1,5-a]pyrimidin-4(3,2)-pyridinacyclononaphan-9-one (**compound 30B**)

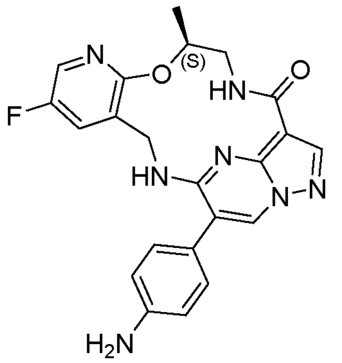

30B

(S,$1^3$E,$1^4$E)-16-(4-(((diphenylmethylene)amino)phenyl)-$4^5$-fluoro-6-methyl-5-oxa-2,8-diaza-1(5,3)-pyrazolo[1,5-a]pyrimidin-4(3,2)-pyridinacyclononaphan-9-one (250 mg, 0.42 mmol) was dissolved in THF (1 mL), to which was added concentrated hydrochloric acid (0.2 mL), and then the mixture was allowed to react at room temperature under stirring for 30 min. After completion of the reaction, the reaction mixture was concentrated under reduced pressure. The residue was directly used for the next reaction. MS (ESI) m/z 434.2 $(M+H)^+$

**Step 3:** Preparation of methyl (4-((S,$1^3$E,$1^4$E)-$4^5$-fluoro-6-methyl-9-oxo-5-oxa-2,8-diaza-1(5,3)-pyrazolo[1,5-a]pyrimidin-4(3,2)-pyridinacyclononaphan-$1^6$-yl)phenyl)carbamate (**compound 30**)

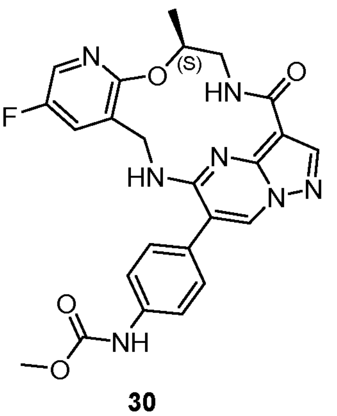

**30**

(S,$1^3$E,$1^4$E)-$1^6$-(4-aminophenyl)-$4^5$-fluoro-6-methyl-5-oxa-2,8-diaza-1(5,3)-pyrazolo[1,5-a]pyrimidin-4(3,2)-pyridinacyclononaphan-9-one (270 mg) was dissolved in THF (3 mL), to which was added DIPEA (402 mg, 3.12 mmol), and then under argon atmosphere, methyl chloroformate(88.4 mg, 0.94 mmol) was added dropwise at 0 °C. The mixture was slowly warmed to at room temperature and reacted under stirring for 5 h. After completion of the reaction, the reaction mixture was concentrated to dryness under reduced pressure. The residue was purified by high pressure preparative separation to provide **compound 30** of Example 30.

MS (ESI) m/z 492.2 $(M+H)^+$
$^1$H NMR (400 MHz, DMSO-$d_6$) δ 9.92 (s, 1H), 9.66 - 9.59 (m, 1H), 8.57 (s, 1H), 8.14 - 8.01 (m, 3H), 7.79 (dd, *J* = 8.8, 3.1 Hz, 1H), 7.66 (d, *J* = 8.5 Hz, 2H), 7.49 - 7.42 (m, 2H), 5.07-4.97 (m, 2H), 4.07-4.00 (m, 2H), 3.72 (s, 3H), 3.15 (dd, *J* = 12.9, 10.1 Hz, 1H), 1.48 (d, *J* = 6.1 Hz, 3H).

**Example 31:** The synthetic steps for preparation of iso-propyl (4-((S,1$^3$E,1$^4$E)-4$^5$-fluoro-6-methyl-9-oxo-5-oxa-2,8-diaza-1(5,3)-pyrazolo[1,5-a]pyrimidin-4(3,2)-pyridinacyclononaphan-1$^6$-yl)phenyl)carbamate (**compound 31**) were shown as follows:

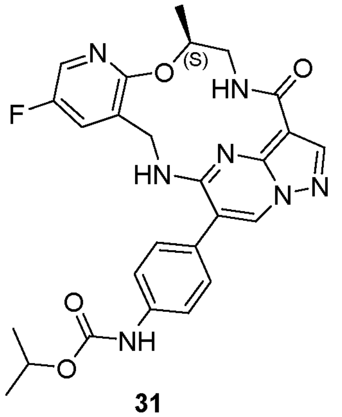

**31**

Using iso-propyl chloroformate to replace methyl chloroformate, compound 31 of Example 31 was obtained by the preparation method same as that of Step 3 in Example 30.

MS (ESI) m/z 520.2 $(M+H)^+$
$^1$H NMR (400 MHz, DMSO-$d_6$) δ 9.81 (s, 1H), 9.63 (d, *J* = 8.9 Hz, 1H), 8.57 (s, 1H), 8.12 - 8.01 (m, 3H), 7.79 (dd, *J* = 8.8, 3.1 Hz, 1H), 7.66 (d, *J* = 8.5 Hz, 2H), 7.48 - 7.40 (m, 2H), 5.11 - 4.87 (m, 3H), 4.11 - 3.96 (m, 2H), 3.15 (dd, *J* = 12.8, 10.2 Hz, 1H), 1.47 (d, *J* = 6.1 Hz, 3H), 1.29 (d, *J* = 6.3 Hz, 6H).

Example 32: The synthetic steps for preparation of 1-(4-((S,1$^3$E,1$^4$E)-4$^5$-fluoro-6-methyl-9-oxo-5-oxa-2,8-diaza-1(5,3)-pyrazolo[1,5-a]pyrimidin-4(3,2)-pyridinacyclononaphan-1$^6$-yl)phenyl)-3-methylurea (compound 32) were shown as follows:

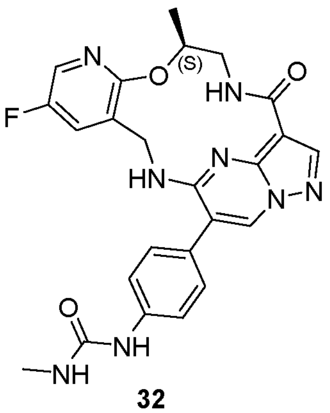

**32**

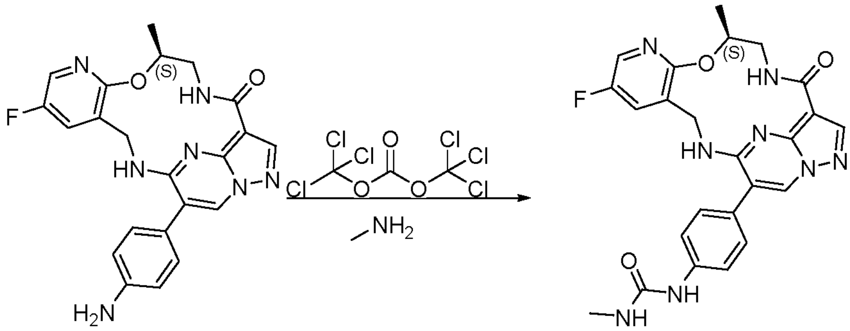

30B 32

(S,1$^3$E,1$^4$E)-1$^6$-(4-aminophenyl)-4$^5$-fluoro-6-methyl-5-oxa-2,8-diaza-1(5,3)-pyrazolo[1,5-a]pyrimidin-4(3,2)-pyridinacyclononaphan-9-one (270 mg) was dissolved in THF (3 mL), to which was added DIPEA (402 mg, 3.12 mmol), and then under argon atmosphere, the solution of triphosgene (62mg, 0.21 mmol) in THF was added dropwise

at 0 °C. The mixture was stirred at this temperature for 1h. Then, to the above reaction mixture, was added the solution of methanamine in THF (1 M, 0.5 mL) dropwise. After addition, the mixture was warmed to room temperature and reacted for 1h. After completion of the reaction, the reaction mixture was concentrated to dryness under reduced pressure. The residue was purified by high pressure preparative separation to provide **compound 32** of Example 32.

MS (ESI) m/z 491.2

$^1$H NMR (400 MHz, DMSO-$d_6$) δ 9.63 (d, *J* = 8.8 Hz, 1H), 8.77 (s, 1H), 8.54 (s, 1H), 8.08 (d, *J* = 6.4 Hz, 2H), 8.03 (d, *J* = 3.1 Hz, 1H), 7.80 (dd, *J* = 8.8, 3.1 Hz, 1H), 7.60 (d, *J* = 8.4 Hz, 2H), 7.38 (d, *J* = 8.5 Hz, 2H), 6.09 (q, *J* = 4.6 Hz, 1H), 5.11 - 4.94 (m, 2H), 4.12 - 3.96 (m, 2H), 3.20 - 3.09 (m, 1H), 2.68 (d, *J* = 4.5 Hz, 3H), 1.47 (d, *J* = 6.1 Hz, 3H).

Example 33: The synthetic steps for preparation of ((S,1$^3$E,1$^4$E)-4$^5$-fluoro-6-methyl-1$^6$-(pyridin-2-yl)-5-oxo-2,8-diaza-1(5,3)-pyrazolo[1,5-a]pyrimidin-4(3,2)-pyridinacyclononaphan-9-one (compound 33) were shown as follows:

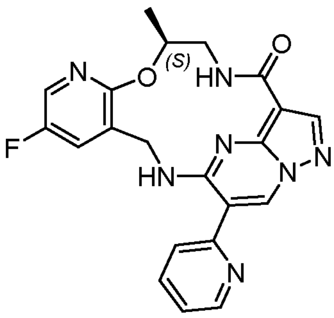

**33**

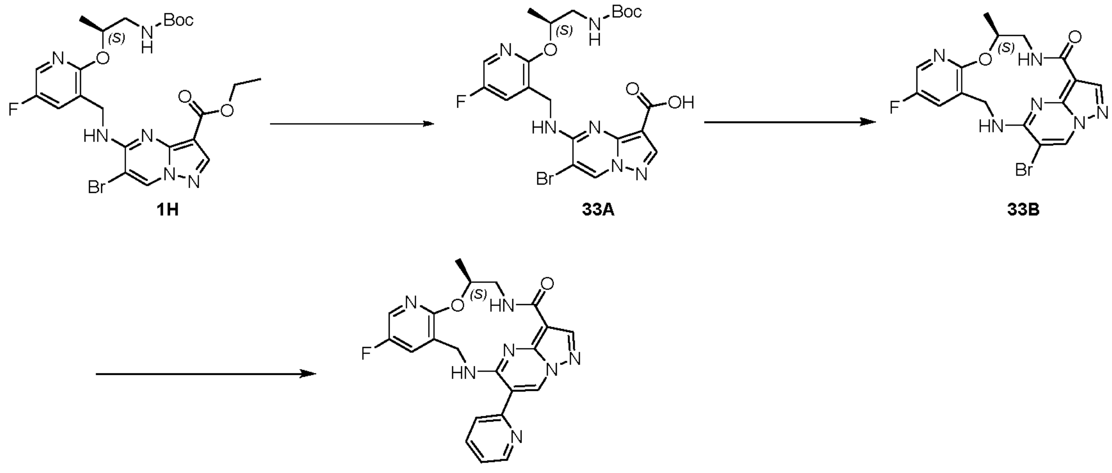

1H

33A

33B

33

**Step** 1: Preparation of (S)-6-bromo-5-((((2-((1-(((tert-butyloxycarbonyl)amino)propan-2-yl)oxy)-5-fluoropyridin-3-3-yl)methyl)pyrazolo[1,5-a]pyrimidin-3-carboxylic acid (**compound 33A**)

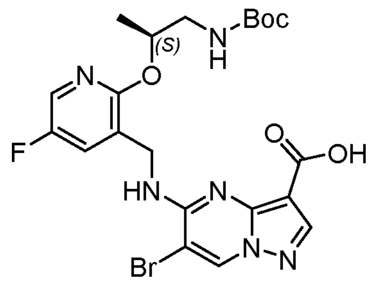

**33A**

Ethyl (S)-6-bromo-5-((((2-((1-(((tert-butyloxycarbonyl)amino)propan-2-yl)oxy)-5-fluoropyridin -3-3-yl)methyl)pyrazolo[1,5-a]pyrimidin-3-carboxylate (283.5 mg, 0.5mmol) was dissolved in anhydrous toluene (10.0 mL), and then under nitrogen protection, tributyltin-oxide (1.2 g, 2.0 mmol) was added. Finally, the reaction system was stirred at 130 °C for 5 d. After completion of the reaction, the reaction mixture was concentrated under reduced pressure. The residue was separated and purified by column chromatography to provide **compound 33A.**
MS (ESI) m/z 539.1 $(M+H)^+$

**Step 2:** Preparation of (S,1$^3$*E*,1$^4$*E*)-1$^6$-bromo-4$^5$-fluoro-6-methyl-5-oxa-2,8-diaza-1(5,3)-pyrazolo[1,5-a]pyrimidina-4(3,2)-pyridinacyclononaphan-9-one (**compound 33B**)

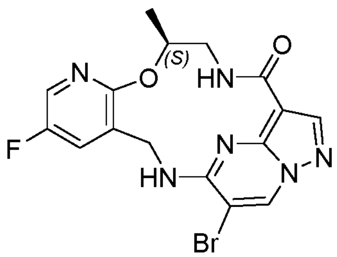

**33B**

(S)-6-bromo-5-((((2-((1-(((tert-butyloxycarbonyl)amino)propan-2-yl)oxy)-5-fluoropyridin-3-3-yl) methyl)pyrazolo[1,5-a]pyrimidin-3-carboxylic acid (108.0 mg, 0.2 mmol) was dissolved in DCM (5 mL), to which was then added the solution of HCl in 1,4-dioxane, and the mixture was stirred at room temperature for 1 h. After completion of the reaction, the above reaction mixture was concentrated under reduced pressure, and then the residue was dissolved in DCM (60 mL) and DMF (30 mL), followed by successive addition of DIPEA (516 mg, 4.0 mmol) and FDPP (768.0 mg, 2.0 mmol). After addition, the reaction system was stirred at room temperature for 16 h. After completion of the reaction, the reaction mixture was concentrated under reduced pressure. The residue was separated and purified by column chromatography to provide **compound 33B.**
MS (ESI) m/z 421.0 $(M+H)^+$

**Step 3:** Preparation of ((S,1$^3$E,1$^4$E)-4$^5$-fluoro-6-methyl-1$^6$-(pyridin-2-yl)-5-oxo-2,8-diaza-1(5,3)-pyrazolo[1,5-a]pyrimidin-4(3,2)-pyridinacyclononaphan-9-one (**compound 33**)

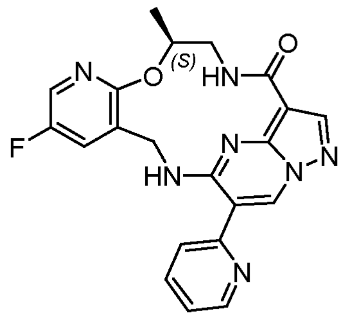

**33**

Under the protection of argon, (S,1$^3$*E*,1$^4$*E*)-1$^6$-bromo-4$^5$-fluoro-6-methyl-5-oxa-2,8-diaza-1(5,3)-pyrazolo[1,5-a]pyrimidina-4(3,2)-pyridinacyclononaphan-9-one (21.0 mg, 0.05 mmol) was dissolved in the mixed solvent of THF (5 mL) and DMSO (5 mL), to which were then added 2-(tributylstannyl)pyridine (73.6 mg, 0.2 mmol) and $Pd(PPh_3)_4$ (6.0 mg, 0.005 mmol). Finally, the reaction system was stirred at 90 °C for 5 h. After completion of the reaction, the reaction mixture was concentrated under reduced pressure. The residue was separated by high pressure preparative separation to provide **compound 33** of Example 33.

MS (ESI) m/z 420.2 $(M+H)^+$
$^1$H NMR (400 MHz, DMSO-$d_6$) δ 10.58 (s, 1H), 9.59 (d, *J* = 8.9 Hz, 1H), 9.40 (s, 1H), 8.79 (d, *J* = 4.9 Hz, 1H), 8.17 (d, *J* = 15.2 Hz, 2H), 8.01 (dd, *J* = 14.1, 5.5 Hz, 2H), 7.86 (dd, *J* = 8.7, 3.2 Hz, 1H), 7.52 (dd, *J* = 7.5, 4.9 Hz, 1H), 5.09 (m, 2H), 4.33 (dd, *J* = 14.4, 5.8 Hz, 1H), 4.03 (m, 1H), 3.16 (t, *J* = 11.7 Hz, 1H), 1.48 (d, *J* = 6.0 Hz, 3H).

**Example 34:** Preparation of (S,1$^3$E,1$^4$E)-1$^6$-(4-chloro-1H-indol-5-yl)-4$^5$-fluoro-6-methyl-5-oxo-2,8-diaza-1(5,3)-pyrazolo[1,5-a]pyrimidin-4(3,2)-pyridinacyclononaphan-9-one **(compound 34):**

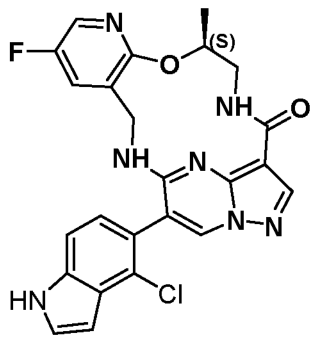

**34**

Using tert-butyl 4-chloro-5-(4,4,5,5-tetramethyl-1,3,2-dioxaborolan-2-yl)-1H-indol-1-carboxylate to replace (3-chlorophenyl)boronic acid, **compound 34** of Example 34 was obtained by the preparation method same as that of Example 3.

MS (ESI) m/z 492.1 $(M+H)^+$
$^1$H NMR (400 MHz, DMSO-$d_6$) δ 11.68 (s, 1H), 9.74 (t, *J* = 7.5 Hz, 1H), 8.65 (s, 1H), 8.12 (s, 1H), 8.04 (dd, *J* = 8.0, 3.1 Hz, 1H), 7.94 (m, 1H), 7.74 (dd, *J* = 8.9, 3.1 Hz, 1H), 7.62 - 7.55 (m, 2H), 7.16 (dd, *J* = 32.3, 8.2 Hz, 1H), 6.63 (m, 1H), 5.12 - 4.91 (m, 2H), 4.10 - 3.91 (m, 2H), 3.18 - 3.11 (m, 1H), 1.48 (d, *J* = 6.1 Hz, 3H).

**Example 35:** Preparation of 1-(3-chloro-4-((S,1$^3$E,1$^4$E)-4$^5$-fluoro-6-methyl-9-oxo-5-oxo-2,8-diaza-1(5,3)-pyrazolo[1,5-a]pyrimidin-4(3,2)-pyridinacyclononaphan-1$^6$-yl)phenyl)-3-methylurea **(compound 35):**

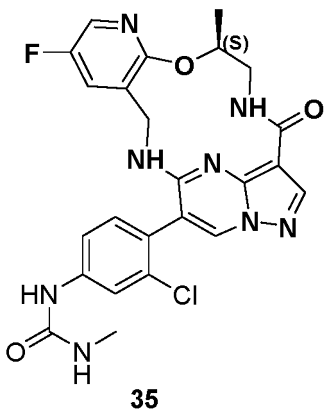

**35**

Using 1-(3-chloro-4-(4,4,5,5-tetramethyl-1 ,3,2-dioxaborolan-2-yl)phenyl)-3-methylurea to replace (3-chlorophenyl)boronic acid, **compound 35** of Example 35 was obtained by the preparation method same as that of Example 3.

MS (ESI) m/z 525.1 $(M+H)^+$
$^1$H NMR (400 MHz, DMSO-$d_6$) δ 9.69 (d, *J* = 9.0 Hz, 1H), 9.06 (s, 1H), 8.65 (d, *J* = 3.6 Hz, 1H), 8.12 (d, *J* = 1.6 Hz, 1H), 8.02 (m, 2H), 7.97 (d, *J* = 2.1 Hz, 1H), 7.76 (m, 1H), 7.65 - 7.53 (m, 1H), 7.46 - 7.28 (m, 2H), 5.09 - 4.90 (m, 2H), 4.08 - 3.95 (m, 2H), 3.14 (t, *J* = 12.1 Hz, 1H), 2.68 (d, *J* = 4.5 Hz, 3H), 1.47 (d, *J* = 6.0 Hz, 3H).

**Example 36:** Preparation of (S,$1^3$E,$1^4$E)-$1^6$-(4-aminophenyl)-$4^5$-fluoro-6-methyl-5-oxo-2,8-diaza-1(5,3)-pyrazolo[1,5-a]pyrimidin-4(3,2)-pyridinacyclononaphan-9-one **(compound 36):**

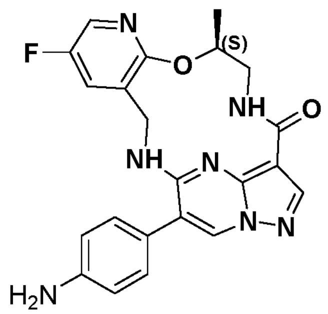

**36**

Using tert-butyl (4-(4,4,5,5-tetramethyl-1,3,2-dioxaborolan-2-yl)phenyl)carbamate to replace (3-chlorophenyl)boronic acid, **compound 36** of Example 36 was obtained by the preparation method same as that of Example 3.

MS (ESI) m/z 434.2 $(M+H)^+$
$^1$H NMR (400 MHz, DMSO-$d_6$) δ 9.65 (d, *J* = 8.6 Hz, 1H), 8.43 (s, 1H), 8.06 (s, 1H), 8.03 (t, *J* = 4.6 Hz, 2H), 7.80 (dd, *J* = 8.8, 3.1 Hz, 1H), 7.20 - 7.14 (m, 2H), 6.76 - 6.71 (m, 2H), 5.46 (s, 2H), 5.10 - 4.95 (m, 2H), 4.04 (m, 2H), 3.19 - 3.09 (m, 1H), 1.47 (d, *J* = 6.1 Hz, 3H).

**Example 37:** Preparation of (*S*,$1^3$*E*,$1^4$*E*)-$4^5$-fluoro-6-methyl-$1^6$-(1*H*-pyrazol-4-yl)-5-oxa-2,8-diaza-1(5,3)-pyrazolo[1,5-a]pyrimidin-4(3,2)-pyridinacyclononaphan-9-one **(compound 37):**

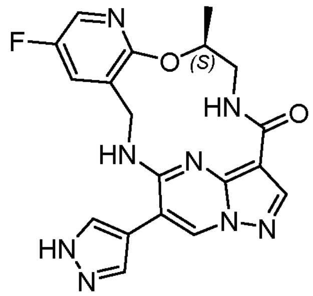

**37**

Using tert-butyl 4-(4,4,5,5-tetramethyl-1,3,2-dioxaborolan-2-yl)-1H-pyrazol-1-carboxylate to replace (3-chlorophenyl)boronic acid, **compound 37** of Example 37 was obtained by the preparation method same as that of Example 3.

MS (ESI) m/z 409.2 $(M+H)^+$

$^1$H NMR (400 MHz, DMSO-$d_6$) δ 13.30 (s, 1H), 9.60 (d, *J* = 8.8 Hz, 1H), 8.67 (s, 1H), 8.33 - 8.00 (m, 4H), 7.88 (dd, *J* = 8.8, 3.1 Hz, 2H), 5.14 - 4.92 (m, 2H), 4.20 - 3.94 (m, 2H), 3.14 (dd, *J* = 13.0, 10.2 Hz, 1H), 1.47 (d, *J* = 6.1 Hz, 3H).

**Example 38:** Preparation of (*S*,1$^3$*E*,1$^4$*E*)-4$^5$-fluoro-6-methyl-1$^6$-(2-oxo-2,3-dihydro-1*H*-benzo[*d*]imidazol-5-yl)-5-oxo-2,8-diaza-1(5,3)-pyrazolo[1,5-a]pyrimidin-4(3,2)-pyridinacyclononaphan -9-one **(compound 38)**:

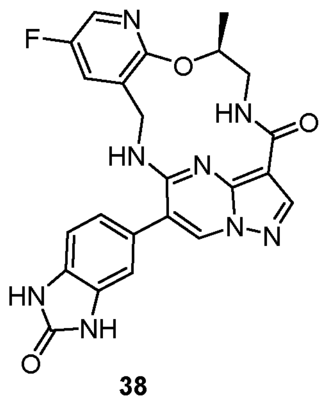

**38**

Using 5-(4,4,5,5-tetramethyl-1,3,2-dioxaborolan-2-yl)-1,3-dihydro-2H-benzo[d]imidazol-2-one to replace (3-chlorophenyl)boronic acid, **compound 38** of Example 38 was obtained by the preparation method same as that of Example 3.

MS (ESI) m/z 475.2 $(M+H)^+$

$^1$H NMR (400 MHz, DMSO-$d_6$) δ 10.85 (d, *J* = 9.7 Hz, 2H), 9.67 (d, *J* = 8.8 Hz, 1H), 8.56 (s, 1H), 8.11 - 8.02 (m, 2H), 7.75 (dd, *J* = 8.8, 3.1 Hz, 1H), 7.14 - 7.02 (m, 3H), 5.11 - 4.94 (m, 2H), 4.03 (m, 2H), 3.15 (dd, *J* = 13.0, 10.3 Hz, 1H), 1.47 (d, *J*= 6.1 Hz, 3H), 1.24 (s, 1H).

**Example 39:** Preparation of (S,1$^3$*E*,1$^4$*E*)-1$^6$-(4-methylamino-2-chlorophenyl)-4$^5$-fluoro-6-methyl-5-oxa-2,8-diaza-1(5,3)-pyrazolo[1,5-a]pyrimidina-4(3,2)-pyridinacyclononaphan-9-one (**compound 39**):

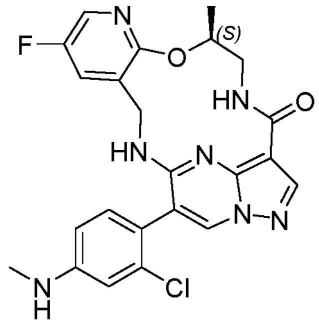

39

Using 2-chloro-4-tert-butylmethylamino-1-phenylboronic acid pinacol ester to replace (3-chlorophenyl)boronic acid, **compound 39** of Example 39 was obtained by the preparation method same as that of Example 3.

MS (ESI) m/z 482.1 $(M+H)^+$

$^1H$ NMR (400 MHz, DMSO-$d_6$) δ 9.71 (d, *J*= 8.9 Hz, 1H), 8.54 (s, 1H), 8.17 - 7.90 (m, 3H), 7.83 - 7.46 (m, 2H), 7.16 (dd, *J* = 28.7, 8.5 Hz, 1H), 6.70 (dd, *J* = 43.0, 6.9 Hz, 1H), 6.39 - 6.24 (m, 1H), 5.16 - 4.85 (m, 2H), 4.00 (m, 2H), 3.13 (t, *J* = 11.9 Hz, 1H), 2.75 (d, *J* = 4.9 Hz, 3H), 1.46 (d, *J* = 6.0 Hz, 3H).

**Example 40:** The synthetic steps for preparation of (S,$1^3$E,$1^4$E)-$1^6$-(2-chloro-4-(2-oxaoxazolidin-3-yl) phenyl)-$4^5$-fluoro-6-methyl-5-oxa-2,8-diaza-1(5,3)-pyrazolo[1,5-a]pyrimidin-4(3,2)-pyridinacyclononapha n-9-one (compound **40**) were shown as follows:

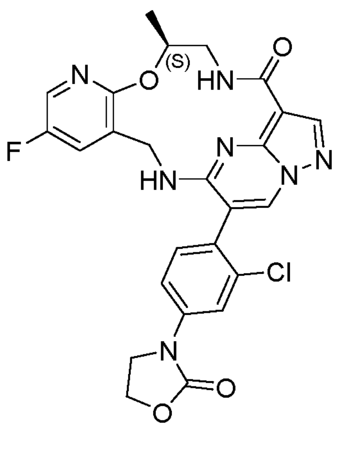

40

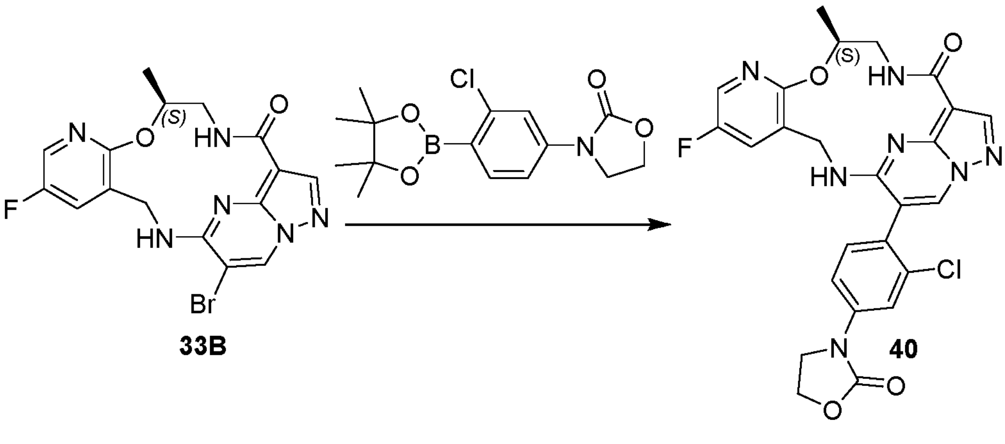

33B

40

**Step 1:** Preparation of S,$1^3$E,$1^4$E)-$1^6$-(2-chloro-4-(2-oxaoxazolidin-3-yl)phenyl)-$4^5$-fluoro-6-methyl-5-oxa-2,8-diaza-1(5,3)-pyrazolo[1,5-a]pyrimidin-4(3,2)-pyridinacyclononaphan-9-one (compound **40**)

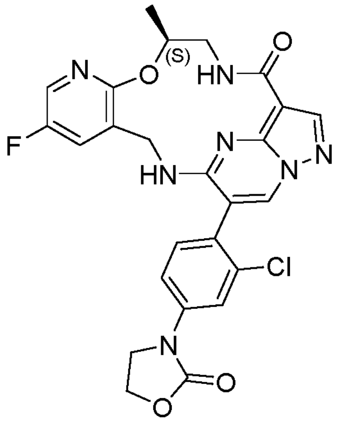

**40**

Under the protection of argon, (S,$1^3$E,$1^4$E)-$1^6$-bromo-$4^5$-fluoro-6-methyl-5-oxa-2,8-diaza-1 (5,3)-pyrazolo[1 ,5-a]pyrimidin-4(3,2)-pyridinacyclononaphan-9-one (50 mg, 0.12 mmol), 3-(3-chloro-4-(4,4,5,5-tetramethyl-1 ,3,2-dioxaborolan-2-yl)phenyl)oxazolidin-2-one (77.5 mg, 0.24 mmol), Pd(dppf)$_2$Cl$_2$ (8.8 mg, 0.012 mmol), $K_3PO_4$ (51 mg, 0.24 mmol), dioxane (3 mL), and water (1 mL) were added into a reaction flask, and the mixture was allowed to react at 80 °C for 16 h. After completion of the reaction, the reaction was quenched by adding water. The resultant mixture was extracted three times with ethyl acetate. The extracted solution was combined, dried over $Na_2SO_4$, filtered, and concentrated. The residue was separated by high pressure preparative separation to provide **compound 40.**

MS (ESI) m/z 538.1 $(M+H)^+$

$^1$H NMR (400 MHz, DMSO-$d_6$) $\delta$ 9.68 (d, *J*= 9.0 Hz, 1H), 8.70 (d, *J*= 3.5 Hz, 1H), 8.13 (d, *J*= 1.5 Hz, 1H), 8.10 - 8.00 (m, 2H), 7.97 (d, *J* = 2.2 Hz, 1H), 7.72 (m, 1H), 7.67 - 7.50 (m, 2H), 5.10 - 4.93 (m, 2H), 4.53 (t, *J* = 8.0 Hz, 2H), 4.17 (t, *J*= 8.2 Hz, 2H), 4.01 (dd, *J* = 14.4, 9.8 Hz, 2H), 3.15 (t, *J*= 12.0 Hz, 1H), 1.48 (d, *J* = 6.0 Hz, 3H).

**Example 41:** The synthetic steps for preparation of (S,$1^3$E,$1^4$E)-$1^6$-(cyclopent-1-en-1-yl)-$4^5$-fluoro-6-methyl-5-oxa-2,8-diaza-1(5,3)-pyrazolo[1,5-a]pyrimidin-4(3,2)-pyridinacyclononaphan-9-one (compound **41**) were shown as follows:

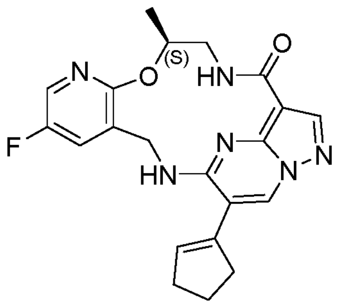

**41**

Using 2-(cyclopent-1-en-1-yl)-4,4,5,5-tetramethyl-1,3,2-dioxaborolane to replace 3-(3-chloro-4-(4,4,5,5-tetramethyl-1,3,2-dioxaborolan-2-yl)phenyl)oxazolidin-2-one, **compound 41** of Example 41 was obtained by the preparation method same as that of Example 40.

MS (ESI) m/z 409.2 $(M+H)^+$

$^1$H NMR (400 MHz, DMSO-$d_6$) δ 9.53 (d, $J$ = 8.7 Hz, 1H), 8.58 (s, 1H), 8.17 (t, $J$ = 6.0 Hz, 1H), 8.11 - 8.00 (m, 2H), 7.85 (dd, $J$ = 8.8, 3.1 Hz, 1H), 6.29 (t, $J$ = 2.2 Hz, 1H), 5.03 (m, 2H), 4.16 (dd, $J$ = 14.5, 5.8 Hz, 1H), 4.01 (m, 1H), 3.14 (m, 1H), 2.72 (m, 2H), 2.61 (m, 2H), 2.00 (m, 2H), 1.47 (d, $J$ = 6.1 Hz, 3H).

**Example 42:** The synthetic steps for preparation of ($S$,1$^3E$,1$^4E$)-4$^5$-fluoro-6-methyl-1$^6$-(4-(oxazol-2-amino)phenyl)-5-oxa-2,8-diaza-1(5,3)-pyrazolo[1,5-a] pyrimidin-4(3,2)-pyridinacyclononaphan-9-one (**compound 42**) were shown as follows:

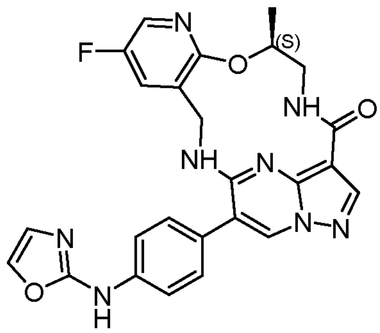

**42**

Using 4-(oxazol-2-amino)phenylboronic acid to replace 3-(3-chloro-4-(4,4,5,5-tetramethyl-1,3,2-dioxaborolan-2-yl)phenyl)oxazolidin-2-one, compound **42** of Example 42 was obtained by the preparation method same as that of Example 40.

MS (ESI) m/z 501.2 $(M+H)^+$

$^1$H NMR (400 MHz, DMSO-$d_6$) δ 9.63 (d, $J$ = 8.8 Hz, 1H), 8.66 (s, 1H), 8.15 (t, $J$ = 6.1 Hz, 1H), 8.11 (s, 1H), 8.04 (d, $J$ = 3.1 Hz, 1H), 7.77 (dd, $J$ = 8.8, 3.1 Hz, 1H), 7.69 (d, $J$ = 8.2 Hz, 2H), 7.58 (t, $J$ = 5.8 Hz, 2H), 5.02 (m, 2H), 4.11 - 3.96 (m, 2H), 3.18 - 3.11 (m, 1H), 1.47 (d, $J$ = 6.1 Hz, 3H).

**Example 43:** The synthetic steps for preparation of methyl (4-((S,1$^3$E,1$^4$E)-4$^5$-fluoro-6-methyl-9-oxo-5-oxa-2,8-diaza-1(5,3)-pyrazolo[1,5-a]pyrimidin-4(3,2)-pyridinacyclononaphan-1$^6$-yl)cyclohex-3-en-1-yl)carbamate (**compound 43**) were shown as follows:

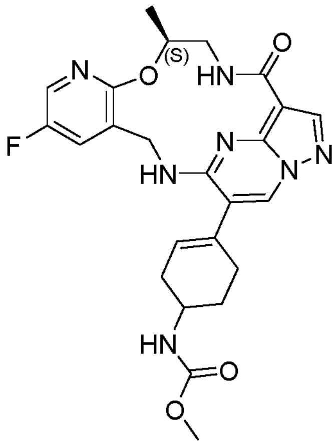

**43**

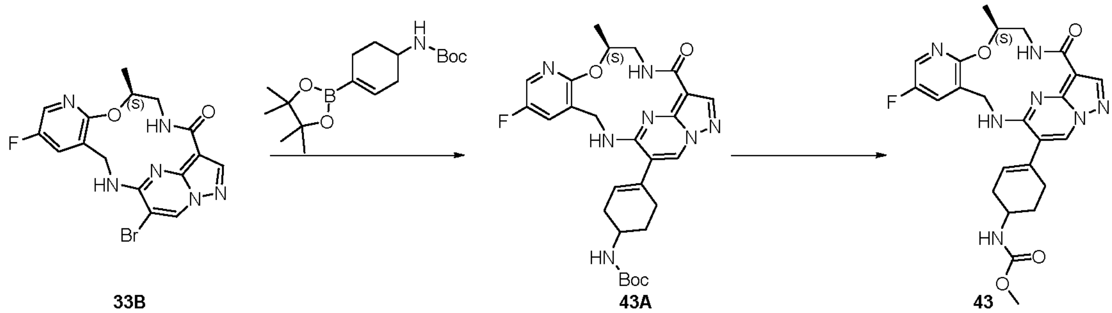

33B 43A 43

**Step 1:** Preparation of tert-butyl (4-(((S,1$^3$E,1$^4$E)-4$^5$-fluoro-6-methyl-9-oxo-5-oxa-2,8-diaza-1(5,3)-pyrazolo[1,5-a]pyrimidin-4(3,2)-pyridinacyclononaphan-16-yl)cyclohex-3-en-1-yl)carbamate **(compound 43A)**

(S,1$^3$E,1$^4$E)-1$^6$-bromo-4$^5$-fluoro-6-methyl-5-oxa-2,8-diaza-1(5,3)-pyrazolo[1,5-a]pyrimidin-4(3,2)-pyridinacyclononaphan-9-one (100 mg, 0.24 mmol), 2-(cyclopent-1-en-1-yl)-4,4,5,5-tetramethyl-1,3,2-dioxaborolane (155 mg, 0.48 mmol), $Pd(dppf)_2Cl_2$ (17.6 mg, 0.024 mmol), $K_3PO_4$ (102 mg, 0.48 mmol), dioxane (6 mL), and water (2 mL) were added into a reaction flask. The mixture was allowed to react at 80 °C for 16 h. After completion of the reaction, the reaction was quenched by adding water. The resultant mixture was extracted three times with ethyl acetate. The extracted solution was combined, dried over $Na_2SO_4$, filtered, and concentrated. The residue was separated and purified by column chromatography to provide the intermediate compound **43A**.
MS (ESI) m/z 538.2 $(M+H)^+$.

**Step 2:** Preparation of methyl (4-((S,1$^3$E,1$^4$E)-4$^5$-fluoro-6-methyl-9-oxo-5-oxa-2,8-diaza-1(5,3)-pyrazolo[1,5-a]pyrimidin-4(3,2)-pyridinacyclononaphan-1$^6$-yl)cyclohex-3-en-1-yl)carbamate (**compound** 43)

Tert-butyl (4-(((S,1$^3$E,1$^4$E)-4$^5$-fluoro-6-methyl-9-oxo-5-oxa-2,8-diaza-1(5,3)-pyrazolo[1,5-a] pyrimidin-4(3,2)-pyridinacyclononaphan-1$^6$-yl)cyclohex-3-en-1-yl)carbamate (80 mg, 0.15 mmol) was dissolved in DCM (2 mL), to which was added 4 M solution of HCl in dioxane (10 mL), and the mixture was stirred at room temperature for 2 h. After completion of the reaction, the reaction mixture was concentrated to remove the solvent and obtain the crude product, which was dissolved in DCM (5 mL), followed by addition of TEA (45 mg, 0.45 mmol). The mixture was cooled to 0 °C, to which was added methyl chloroformate (21.3 mg, 0.225 mmol) dropwise. After addition, the ice bath was removed, and the mixture was allowed to react at room temperature for 6 h. After completion of the reaction, the solvent was removed by concentration. The residue was separated by high pressure preparative separation to provide **compound 43.**

MS (ESI) m/z 496.2 $(M+H)^+$
$^1$H NMR (400 MHz, OMSO-$d_6$) $\delta$ 9.60 (d, $J$ = 8.6 Hz, 1H), 8.51 (d, $J$= 1.5 Hz, 1H), 8.18 (d, $J$ = 2.3 Hz, 1H), 8.09 - 7.99 (m, 2H), 7.75 (m, 1H), 7.29 (t, $J$ = 6.7 Hz, 1H), 5.82 (s, 1H), 5.13 - 4.93 (m, 2H), 4.15 - 3.96 (m, 2H), 3.80 (s, 1H), 3.57 (s, 3H), 3.13 (dd, $J$ = 12.9, 10.1 Hz, 1H), 2.44-2.51 (m, 2H), 2.12 (m, 2H), 1.89(s, 1H)1.69 (d, $J$ = 39.3 Hz, 1H), 1.47 (d, $J$ = 6.1 Hz, 3H).

**Example 44:** Preparation of N-(3-chloro-4-((S,1$^3$E,1$^4$E)-4$^5$-fluoro-6-methyl-9-oxo-5-oxa-2,8-diaza-1(5,3)-pyrazolo[1,5-a]pyrimidinyl-4(3,2)-pyridinacyclononaphan-1$^6$-yl)phenyl)methanesulfonamide **(compound 44):**

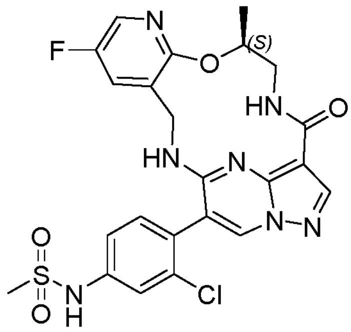

**44**

Using methanesulfonyl chloride to replace methyl chloroformate, **compound 44** of Example 44 was obtained by the preparation method same as that of Step 3 in Example 27.

MS (ESI) m/z 546.2 $(M+H)^+$

$^1H$ NMR (400 MHz, OMSO-$d_6$) δ 10.34 (s, 1H), 9.67 (dd, *J* = 9.0, 3.6 Hz, 1H), 8.68 (d, *J* = 2.2 Hz, 1H), 8.13 (s, 1H), 8.03 (m, 2H), 7.82 - 7.70 (m, 1H), 7.66 - 7.57 (m, 1H), 7.57 - 7.50 (m, 1H), 7.49 - 7.43 (m, 1H), 7.33 (m, 1H), 5.11 -4.91 (m, 2H), 4.10 - 3.94 (m, 2H), 3.19 (s, 4H), 1.47 (d, *J* = 6.1 Hz, 3H).

**Example 45:** Preparation of iso-propyl 3-chloro-4-((S,$1^3$*E*,$1^4$*E*)-$4^5$-fluoro-6-methyl-9-oxo-5-oxa-2,8-diaza-1(5,3)-pyrazolo[1,5-*a*]pyrimidin-4(3,2)-pyridinacyclononaphan-$1^6$-yl)phenyl)carbamate (**compound 45**):

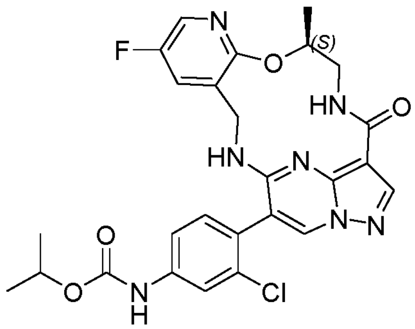

**45**

Using iso-propyl chloroformate to replace methyl chloroformate, **compound 45** of Example 45 was obtained by the preparation method same as that of Step 3 in Example **27.**

MS (ESI) m/z 554.2 $(M+H)^+$

$^1H$ NMR (400 MHz, OMSO-$d_6$) δ 10.04 (d, *J* = 1.7 Hz, 1H), 9.68 (dd, *J* = 9.1, 3.1 Hz, 1H), 8.67 (d, *J* = 2.2 Hz, 1H), 8.12 (s, 1H), 8.07 - 7.95 (m, 2H), 7.86 (dd, *J* = 10.4, 2.1 Hz, 1H), 7.67 (m, 1H), 7.55 (m, 1H), 7.42 (dd, *J* = 26.8, 8.4 Hz, 1H), 5.04 (m, 1H), 4.95 (m, 2H), 4.10 - 3.93 (m, 2H), 3.21 - 3.09 (m, 1H), 1.47 (d, *J* = 6.1 Hz, 3H), 1.30 (d, *J* = 6.2 Hz, 6H).

**Example 46:** Preparation of cyclopropylmethyl (3-chloro-4-((S,$1^3$E,$1^4$E)-$4^5$-fluoro-6-methyl-9-oxo-5-oxadiaza-2,8-1(5,3)-pyrazolo[1,5-a]pyrimidin-4(3,2)-pyridinacyclononaphan-$1^6$-yl)phenyl)carbamate (**compound 46**):

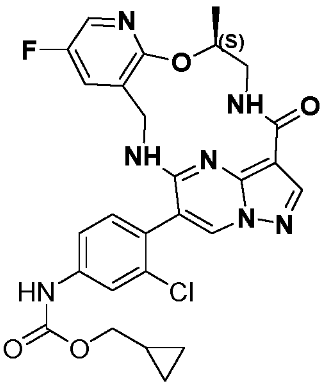

46

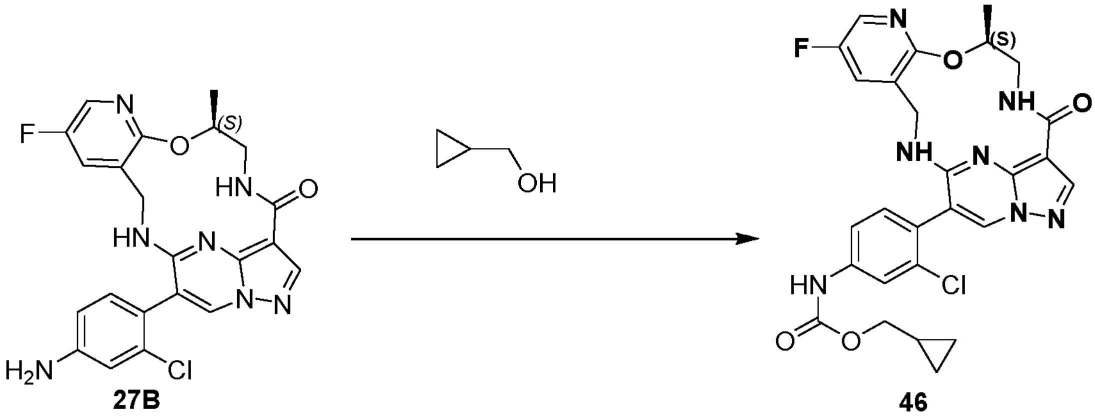

27B

46

**Step 1:** Preparation of cyclopropylmethyl (3-chloro-4-((S,$1^3$E,$1^4$E)-$4^5$-fluoro-6-methyl-9-oxo-5-oxadiaza-2,8-1(5,3)-pyrazolo[1,5-a]pyrimidin-4(3,2)-pyridinacyclononaphan-$1^6$-yl)phenyl)carbamate **(compound 46)**

(S,$1^3$E,$1^4$E)-$1^6$-(4-amino-2-chlorophenyl)-$4^5$-fluoro-6-methyl-5-oxo-2,8-diaza-1(5,3)-pyrazolo[1,5-a] pyrimidin-4(3,2)-pyridinacyclononaphan-9-one (70 mg, 0.15 mmol), DIPEA (58 mg, 0.45 mmol) and CDI (97.2 mg, 0.6 mmol) were dissolved in DCM (10 mL), and the mixture was allowed to react overnight at 40 °C. To above reaction system, was added cyclopropylmethanol (21.6 mg, 0.3 mmol) dropwise. After addition, the mixture was further allowed to react overnight at 40 °C. After completion of the reaction, the mixture was concentrated under reduced pressure, and the residue was separated by high pressure preparative separation to provide **compound 46.**

MS (ESI) m/z 566.2 $(M+H)^+$

$^1$H NMR (400 MHz, DMSO-$d_6$) δ 10.18 (s, 1H), 9.68 (dd, *J* = 9.0, 3.7 Hz, 1H), 8.68 (d, *J* = 2.7 Hz, 1H), 8.12 (d, *J*= 1.2 Hz, 1H), 8.06 - 7.98 (m, 2H), 7.85 (dd, *J* = 12.9, 2.1 Hz, 1H), 7.57 (m, 2H), 7.43 (dd, *J* = 26.3, 8.4 Hz, 1H), 5.09 -4.92 (m, 2H), 4.07 - 3.97 (m, 4H), 3.14 (t, *J* = 11.8 Hz, 1H), 1.47 (d, *J* = 6.0 Hz, 3H), 1.18 (m, 1H), 0.61 - 0.51 (m, 2H), 0.38 - 0.31 (m, 2H).

**Example 47:** The synthetic steps for preparation of (S,$1^3$*E*,$1^4$*E*)-$4^5$-chloro-6-methyl-$1^6$-phenyl-5-oxa-2,8-diaza-1(5,3)-pyrazolo[1,5-a]pyrimidina-4(3,2)-pyridinacyclononaphan-9-one **(compound 47**) were shown as follows:

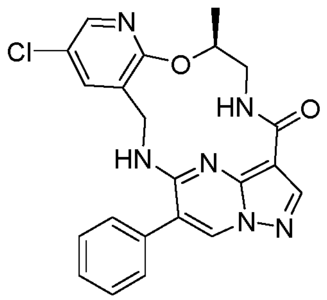

**47**

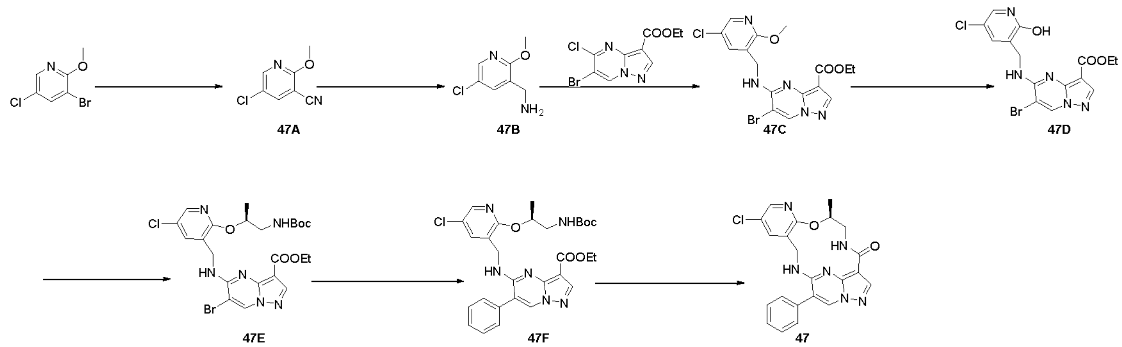

47A 47B 47C 47D 47E 47F 47

**Step 1:** Preparation of 5-chloro-2-methoxynicotinonitrile (**compound 47A**)

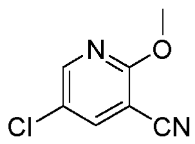

**47A**

3-Bromo-5-chloro-2-methoxypyridine (22.0 g, 100.0 mmol) was dissolved in DMF (200.0 mL), to which were added zinc cyanide (17.5 g, 150.0 mmol) and $Pd(PPh_3)_4$ (23.0 g, 20.0 mmol) at room temperature, and then the reaction system was purged with nitrogen. Finally, under nitrogen protection, the mixture was allowed to react at 120 °C for 6 h. After completion of the reaction, the reaction mixture was poured into ethyl acetate, and filtered with diatomite. The filtrate was poured into water, and the water phase was extracted with ethyl acetate. The organic phases were combined, dried over $Na_2SO_4$, filtered, and concentrated under reduced pressure. The residue was separated and purified over column chromatography to provide **compound 47A.**

MS (ESI) m/z 169.6 $(M+H)^+$

**Step 2:** Preparation of (5-chloro-2-methoxypyridin-3-yl)methanamine (**compound 47B**)

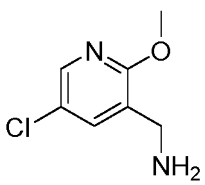

**47B**

5-Chloro-2-methoxynicotinonitrile (8.4 g, 50.0 mmol) was dissolved in THF (80.0 mL), to which was added the solution of borane in THF (150.0 mL, 1 M in THF, 150.0 mmol) in an ice bath, and after addition, the reaction mixture was stirred and reacted at 60 °C for 8 h. After completion of the reaction, methanol was added into the reaction mixture to quench the excess borane. Then, after filtration, the filtrate was evaporated to dryness, and the residue was subjected to column chromatography to obtain **compound 47B.**
MS (ESI) m/z 173.6 $(M+H)^+$.

**Step 3:** Preparation of ethyl 6-bromo-5-((((5-chloro-2-methoxypyridin-3-yl)methyl)amino)amino) pyrazolo[1,5-a]pyrimidin-3-carboxylate (**compound 47C**)

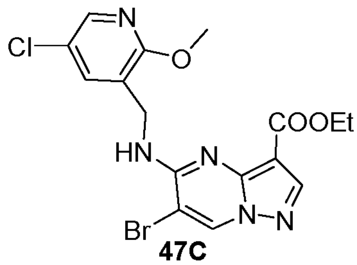

**47C**

Ethyl 5-chloro-6-bromopyrazolo[1,5-a]pyrimidin-3-carboxylate (6.1 g, 20.0 mmol) was dissolved in ethanol, to which were then added (5-chloro-2-methoxypyridin-3-yl)methanamine (3.5 g, 20.0 mmol) and DIPEA (5.1 g, 40.0 mmol), and the reaction system was heated to 80 °C and stirred for 3 h. After completion of the reaction, ethanol was removed by concentration under reduced pressure, and then water was added. The aqueous phase was extracted three times with DCM. The organic phases were combined, dried over $Na_2SO_4$, filtered, and concentrated. The residue was purified by column chromatography to obtain **compound 47C.**
MS (ESI) m/z 441.7 $(M+H)^+$.

**Step 4:** Preparation of ethyl 6-bromo-5-((((5-chloro-2-hydroxypyridin-3-yl)methyl)amino)amino) pyrazolo[1,5-a]pyrimidin-3-carboxylate(**compound 47D**)

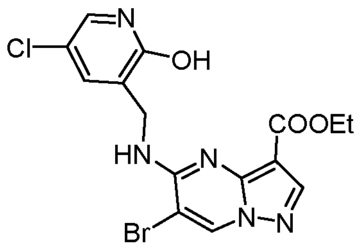

**47D**

Ethyl 6-bromo-5-((((5-chloro-2-methoxypyridin-3-yl)methyl)amino)amino)pyrazolo[1,5-a] pyrimidin-3-carboxylate (4.4 g, 10.0 mmol) was dissolved in the solution of HCl in 1,4-dioxane (5.0 mL, 20.0 mmol), and then the system was stirred and reacted overnight at 70 °C. After completion of the reaction, the reaction mixture was concentrated under reduced pressure. The residue was purified by column chromatography to obtain **compound 47D**.
MS (ESI) m/z 427.7 $(M+H)^+$.

**Step 5:** Preparation of ethyl (S)-6-bromo-5-((((2-((1-(((tert-butyloxycarbonyl)amino)propan-2-yl) oxy)-5-chloropyridin-3-3-yl)methyl)pyrazolo[1,5-a]pyrimidin-3-carboxylate **(compound 47E**)

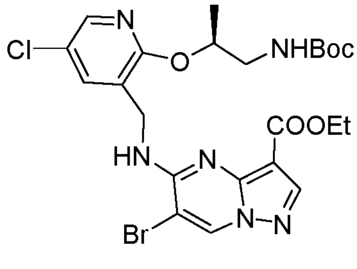

**47E**

Ethyl 6-bromo-5-((((5-chloro-2-hydroxypyridin-3-yl)methyl)amino)amino)pyrazolo[1,5-a] pyrimidin-3-carboxylate (3.4 g, 8.0 mmol) was dissolved in THF (15.0 mL), to which were then successively added tert-butyl (R)-(2-hydroxypropyl)carbamate (1.68 g, 9.6 mmol) and $PPh_3$ (3.15 g, 12.0 mmol). Finally, under nitrogen protection, diisopropyl azodicarboxylate (2.4 g, 12.0 mmol) was slowly added. After addition, the reaction mixture was allowed to react at room temperature for 3h. After completion of the reaction, the solution was concentrated under reduced pressure. The residue was purified by column chromatography to obtain **compound 47E.**
MS (ESI) m/z 584.1 $(M+H)^+$

**Step 6:** Preparation of ethyl (S)-6-phenyl-5-((((2-((1-(((tert-butyloxycarbonyl)amino)propan-2-yl) oxy)-5-chloropyridin-3-3-yl)methyl)pyrazolo[1,5-a]pyrimidin-3-carboxylate (**compound 47F**)

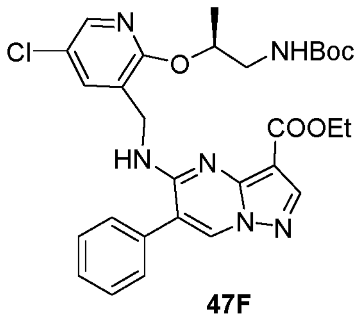

**47F**

Under nitrogen protection, ethyl (S)-6-bromo-5-((((2-((1-(((tert-butyloxycarbonyl)amino)propan-2-yl)oxy)-5-chloropyridin-3-3-yl)methyl)pyrazolo[1,5-a]pyrimidin-3-carboxylate (582.0 mg, 1.0 mmol), phenylboronic acid (147 mg, 1.2 mmol), $Pd(dppf)Cl_2$ (73.2 mg, 0.1 mmol), and $K_2CO_3$ (414.0 mg, 3.0 mmol) were dissolved in 1,4-dioxane/water (3:1, 16.0 mL), and finally, the reaction system was stirred at 90 °C for 3 h. After completion of the reaction, the reaction mixture was poured into water, and the aqueous phase was extracted twice with ethyl acetate. The organic phases were combined, dried over $Na_2SO_4$, filtered, and concentrated. The residue was separated and purified by column chromatography to obtain **compound 47F.**
MS (ESI) m/z 582.1 $(M+H)^+$

**Step 7:** Preparation of (S,$1^3$E,$1^4$E)-$4^5$-chloro-6-methyl-$1^6$-phenyl-5-oxa-2,8-diaza-1(5,3)-pyrazolo[1,5-a]pyrimidina-4(3,2)-pyridinacyclononaphan-9-one (**compound 47**)

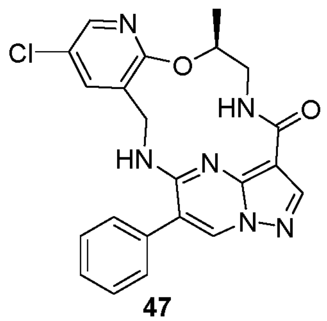

**47**

Ethyl (S)-6-phenyl-5-((((2-((1-(((tert-butyloxycarbonyl)amino)propan-2-yl)oxy)-5-chloropyridin-3-3-yl) methyl)pyrazolo[1,5-a]pyrimidin-3-carboxylate(348.0 mg, 0.60 mmol) was dissolved in a mixed solution of methanol and THF, to which was then added LiOH (288 mg, 12.09 mmol) aqueous solution. After addition, the reaction system was heated to 60 °C and stirred for 16 h. Then, the reaction system was cooled to 0 °C, and the pH was adjusted to 2-3 with 2N hydrochloric acid. The resultant mixture was extracted three times with DCM. The organic phases were combined, dried over anhydrous sodium sulfate, filtered, and concentrated to dryness. Then, the solution of HCl in 1,4-dioxane was added, and the resultant mixture was stirred at room temperature for 1 h. The reaction system was concentrated to dryness, to which were then added DCM and DMF, followed by successive addition of DIPEA (15.78 g, 122.08 mmol) and FDPP (2.95 g, 7.69 mmol). After addition, the reaction system was stirred for 16 h at room temperature. The reaction system was concentrated to dryness under reduced pressure, and the residue was separated by high pressure preparative separation to obtain **compound 47** of Example 50.

MS (ESI) m/z 435.1 $(M+H)^+$
$^1H$ NMR (400 MHz, DMSO-$d_6$) δ 9.59 (d, *J* = 8.8 Hz, 1H), 8.61 (s, 1H), 8.13 - 8.02 (m, 3H), 7.94 (d, *J* = 2.7 Hz, 1H), 7.63 - 7.49 (m, 5H), 5.14 - 5.04 (m, 1H), 4.99 (dd, *J* = 14.4, 6.3 Hz, 1H), 4.04 (m, 2H), 3.15 (dd, *J* = 13.2, 10.2 Hz, 1H), 1.47 (d, *J*= 6.1 Hz, 3H).

**Example 48:** The synthetic steps for preparation of (S,$1^3$E,$1^4$E)-6-methyl-$1^6$-phenyl-$4^5$-(pyridin-2-ylamino)-5-oxa-2,8-diaza-1 (5,3)-pyrazolo[1,5-a]pyrimidina-4(3,2)-pyridinacyclononaphan-9-one **(compound 48)** were shown as follows:

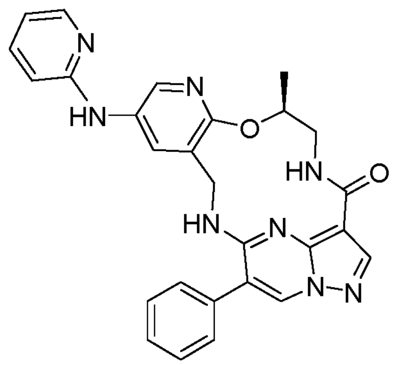

**48**

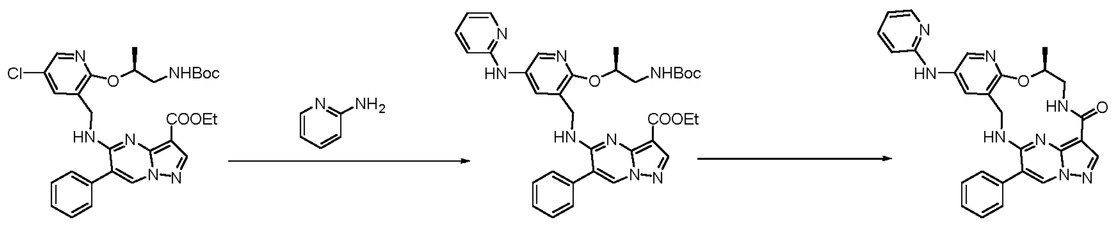

47F 48A 48

**Step 1:** Preparation of ethyl (S)-5-(((((2-((1-(((tert-butyloxycarbonyl)amino)propan-2-yl)oxy)-5-(pyridin-2-ylamino)pyridin-3-yl)methyl)amino)-6-phenylpyrazolo[1,5-a]pyrimidin-3-carboxylate **(compound 48A)**

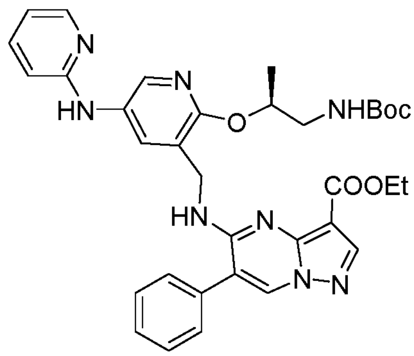

**48A**

Under nitrogen protection, ethyl (S)-6-phenyl-5-((((2-((1-(((tert-butyloxycarbonyl)amino)propan-2-yl)oxy)-5-chloropyridin-3-3-yl)methyl)pyrazolo[1,5-a]pyrimidin-3-carboxylate (580.0 mg, 1.0 mmol) was dissolved in tert-butanol (30.0 mL), to which were successively added 2-aminopyridine (122.0 mg, 1.3 mmol), $Pd_2(dba)_3$ (91.5 mg, 0.1 mmol), BrettPhos (107.2 mg, 0.2 mmol) and $Cs_2CO_3$ (984.0 mg, 3.0 mmol) at room temperature, and the above system was allowed to react at 110 °C for 3 h. After completion of the reaction, the reaction mixture was concentrated under reduced pressure. The residue was separated and purified over column chromatography to provide **compound 48A.**
MS (ESI) m/z 639.3 $(M+H)^+$

**Step 2:** Preparation of (S,$1^3$*E*,$1^4$*E*)-6-methyl-$1^6$-phenyl-$4^5$-(pyridin-2-ylamino)-5-oxa-2,8-diaza-1(5,3)-pyrazolo[1,5-a]pyrimidina-4(3,2)-pyridinacyclononaphan-9-one (**compound 48**)

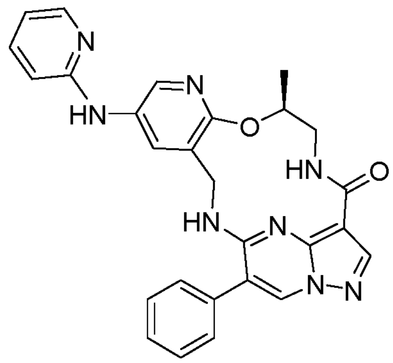

**48**

Ethyl (S)-5-(((((2-((1-(((tert-butyloxycarbonyl)amino)propan-2-yl)oxy)-5-(pyridin-2-ylamino) pyridin-3-yl)methyl)amino)-6-phenylpyrazolo[1,5-*a*]pyrimidin-3-carboxylate (382.0 mg, 0.60 mmol) was dissolved in a mixed solution of methanol and THF, to which was then added LiOH (288 mg, 12.09 mmol) aqueous solution. After addition, the reaction system was heated to 60 °C and stirred for 16 h. Then, the reaction system was cooled to 0 °C, and the pH was adjusted to 2-3 with 2N hydrochloric acid. The resultant mixture was extracted three times with DCM. The organic phases were combined, dried over anhydrous sodium sulfate, filtered, and concentrated to dryness. Then, the solution of HCl in 1,4-dioxane was added, and the resultant mixture was stirred at room temperature for 1 h. The reaction system was concentrated to dryness, to which were then added DCM and DMF, followed by successive addition of DIPEA (15.78 g, 122.08 mmol) and FDPP (2.95 g, 7.69 mmol). After addition, the reaction system was stirred for 16 h at room temperature. The reaction system was concentrated to dryness under reduced pressure, and the residue was separated by high pressure preparative separation to obtain compound **48** of Example 51.

MS (ESI) m/z 493.5 $(M+H)^+$

$^1$H NMR (400 MHz, DMSO-$d_6$) $\delta$ 9.67 (dd, $J$ = 8.9, 1.7 Hz, 1H), 8.89 (s, 1H), 8.59 (s, 1H), 8.23 - 8.17 (m, 2H), 8.13 (d, $J$ = 2.7 Hz, 1H), 8.09 (s, 1H), 8.07 (dd, $J$ = 5.2, 1.8 Hz, 1H), 7.61 - 7.49 (m, 6H), 6.76 -6.67 (m, 2H), 5.08 (m, 1H), 4.99 (dd, $J$ = 14.2, 5.7 Hz, 1H), 4.10 - 3.95 (m, 2H), 3.13 (m, 1H), 1.47 (d, $J$ = 6.1 Hz, 3H).

**Example 49:** The synthetic steps for preparation of methyl (4-((*S*,1$^3$*E*,1$^4$*E*)-1$^7$-amino-4$^5$-fluoro-6-methyl-9-oxo-5-oxa-2,8-diaza-1(5,3)-pyrazolino[1,5-a]pyrimidin-4(3,2)-pyridinacyclononaphan-1$^6$-yl)-phenyl)carbamate (**compound 49**) were shown as follows:

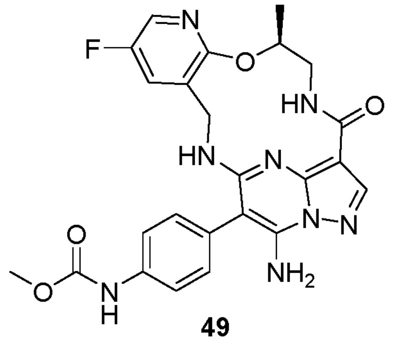

**49**

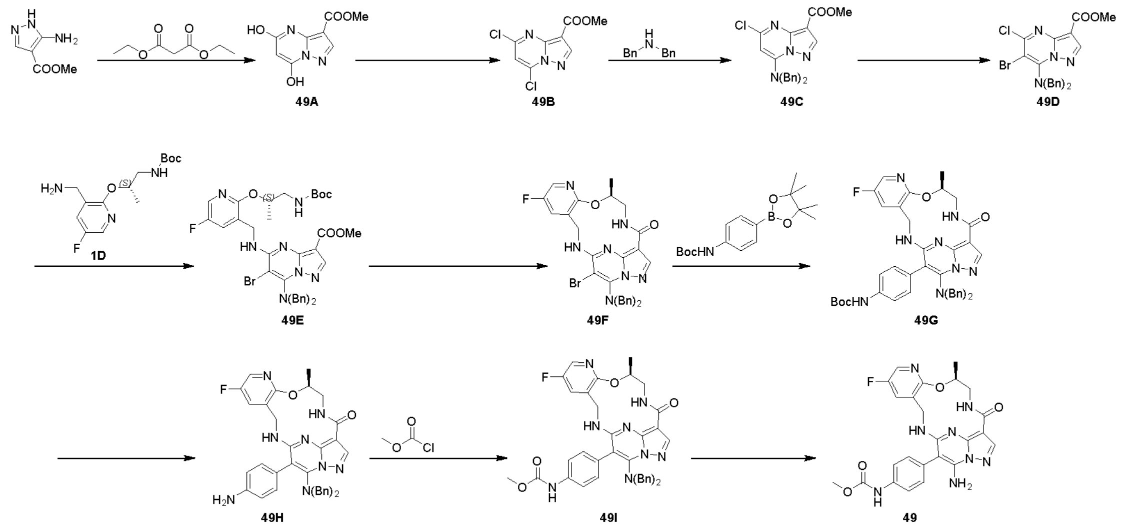


**Step 1:** Preparation of methyl 5,7-dihydroxylpyrazolo[1,5-*a*]pyrimidin-3-carboxylate (**compound 49A**)

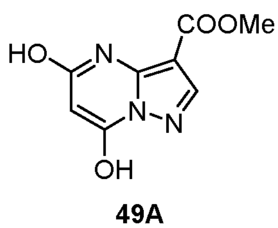

**49A**

Methyl 5-amino-1*H*-pyrazol-4-carboxylate (28.2 g, 200.0 mmol) was dissolved in methanol, to which were successively added diethyl malonate (64.0 g, 400.0 mmol) and sodium methoxide (32.4 g, 600.0 mmol) in an ice-water bath. The reaction was heated to 90 °C and stirred for 16 h. After completion of the reaction, the reaction system was cooled to 0 °C, and filtered. The filter cake was rinsed with ethanol. The solid was dissolved in water, and then hydrochloric

acid was added to adjust pH 1. A lot of soild was precipitated and filtered. The filter cake was rinsed with water, and then dried to provide **compound 49A.**
MS (ESI) m/z 210.1 $(M+H)^+$

**Step 2:** Preparation of methyl 5,7-dichloropyrazolo[1,5-*a*]pyrimidin-3-carboxylate (**compound 49B**)

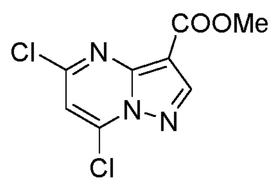

**49B**

In an ice bath, to a reaction flask containing methyl 5,7-dihydroxylpyrazolo[1,5-*a*]pyrimidin-3-carboxylate (10.45 g, 50.0 mmol), was added $POCl_3$ (100 mL), and then *N,N*-dimethylphenylamine (15.1g, 125.0 mmol) was slowly added dropwise. After addition, the reaction was heated to 80 °C, and then allowed to react at this temperature for 16 h. After completion of the reaction, the reaction mixture was slowly poured into ice water for quenching the reaction. The resultant mixture was extracted twice with DCM. The organic phases were combined, dried over anhydrous sodium sulfate, filtered, and concentrated. The residue was separated and purified over column chromatography to provide **compound 49B.**
MS (ESI) m/z 246.0 $(M+H)^+$.

**Step 3:** Preparation of methyl 5-chloro-7-(dibenzylamino)pyrazolo[1,5-a]pyrimidin-3-carboxylate (**compound 49C**)

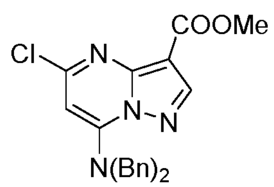

**49C**

Methyl 5,7-dichloropyrazolo[1,5-*a*]pyrimidin-3-carboxylate (7.35 g, 30.0 mmol) was dissolved in a mixed solution of 1,4-dioxane and DCM (dioxane/DCM=15.0mL /30.0 mL), to which were successively added DIPEA (7.7 g, 60.0 mmol) and dibenzylamine (7.1 g, 36.0 mmol). After addition, the reaction system was stirred at room temperature for 3h. After completion of the reaction, the reaction mixture was concentrated to remove excess $POCl_3$. The residue was purified by column chromatography to obtain **compound 49C.**
MS (ESI) m/z 407.1 $(M+H)^+$.

**Step 4:** Preparation of methyl 6-bromo-5-chloro-7-(dibenzylamino)pyrazolo[1,5-a]pyrimidin-3-carboxylate (**compound 49D**)

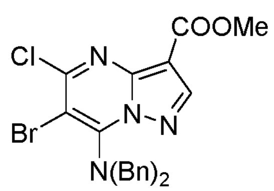

**49D**

Methyl 5-chloro-7-(dibenzylamino)pyrazolo[1,5-*a*]pyrimidin-3-carboxylate (8.2 g, 20.0 mmol) was dissolved in

DCM, to which was added NBS (4.3 g, 24.0 mmol). After addition, the reaction system was stirred at room temperature for 16 h. After completion of the reaction, the reaction mixture was concentrated under reduced pressure, and the residue was purified by column chromatography to obtain **compound 49D**.
MS (ESI) m/z 485.0 $(M+H)^+$.

**Step 5:** Preparation of methyl (*S*)-6-bromo-5-((((2-((1-(((tert-butyloxycarbonyl)amino)propan-2-yl) oxy)-5-fluoropyridin-3-yl)methyl)amino)-7-(dibenzylamino)pyrazolo[1,5-*a*]pyrimidin-3-carboxylate (**compound 49E**)

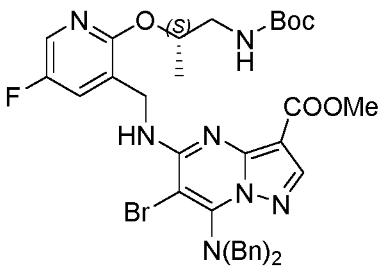

**49E**

Methyl 6-bromo-5-chloro-7-(dibenzylamino)pyrazolo[1,5-a]pyrimidin-3-carboxylate (7.26 g, 15.0 mmol) was dissolved in ethanol, to which were then added tert-butyl (*S*)-(2-((3-(aminomethyl)-5-fluoropyridin-2-yloxy)propyl)carbamate (6.78 g, 22.5 mmol) and DIPEA (5.8 g, 45.0 mmol), and the reaction system was heated to 80 °C and stirred for 16 h. After completion of the reaction, the reaction mixture was concentrated under reduced pressure. The residue was purified by column chromatography to obtain **compound 49E.**
MS (ESI) m/z 748.7 $(M+H)^+$

**Step 6:** Preparation of (*S*,$1^3$*E*,$1^4$*E*)-$1^6$-bromo-$1^7$-(dibenzylamino)-$4^5$-fluoro-$1^{12}$,6-dimethyl-5-oxa-2,8-diaza-1(5,3)-pyrazolino[1,5-a]pyrimidin-4(3,2)-pyridinacyclononaphan-9-one **(compound 49F**)

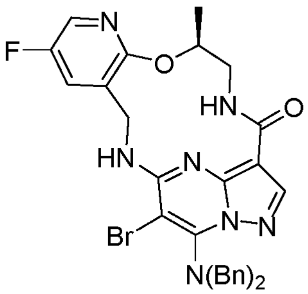

**49F**

Methyl (*S*)-6-bromo-5-((((2-((1-(((tert-butyloxycarbonyl)amino)propan-2-yl)oxy)-5-fluoropyridin-3-yl)methyl)amino)-7-(dibenzylamino)pyrazolo[1,5-*a*]pyrimidin-3-carboxylate (7.47 g, 10.0 mol) was dissolved in a mixed solution of methanol and THF, to which was then added LiOH (4.8 g, 200.0 mmol) aqueous solution. After addition, the reaction system was heated to 60 °C and stirred for 16 h. Then, the reaction system was cooled to 0 °C, and the pH was adjusted to 2-3 with 2N hydrochloric acid. The resultant mixture was extracted three times with DCM. The organic phases were combined, dried over anhydrous sodium sulfate, filtered, and concentrated to dryness. Then, the solution of HCl in 1,4-dioxane was added, and the resultant mixture was stirred at room temperature for 1 h. The reaction system was concentrated to dryness, to which were then added DCM and DMF, followed by successive addition of DIPEA (19.32 g, 150.0 mmol) and FDPP (19.2 g, 50.0 mmol). After addition, the reaction system was stirred for 16 h at room temperature. The reaction system was concentrated to dryness under reduced pressure, and the residue was separated by C-18 reversed-phase preparative separation to obtain **compound 49F.**
MS (ESI) m/z 630.2 $(M+H)^+$

**Step 7:** Preparation of ($S$,1$^3E$,1$^4E$)-1$^6$-((4-tert-butyloxycarbonylamino)phenyl)-1$^7$-(dibenzylamino)-4$^5$-fluoro-1$^{12}$,6-dimethyl-5-oxa-2,8-diaza-1(5,3)-pyrazolino[1,5-a]pyrimidin-4(3,2)-pyridinacyclononaphan-9-one (**compound 49G**)

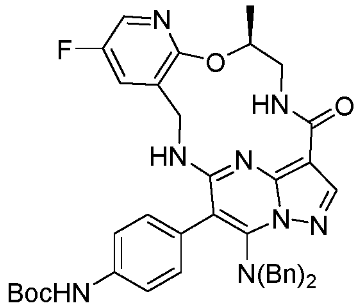

**49G**

Under nitrogen protection, ($S$,1$^3E$,-1$^4E$)-1$^6$-bromo-1$^7$-(dibenzylamino)-4$^5$-fluoro-1$^{12}$,6-dimethyl-5-oxa-2,8-diaza-1(5,3)-pyrazolino[1,5-a]pyrimidin-4(3,2)-pyridinacyclononaphan-9-one (6.29 g, 10.0 mmol) and 4-tert-butyloxycarbonylaminophenylboronic acid pinacol ester (3.83g, 12.0 mmol) were added into a reaction flask containing 1,4-dioxane/water (60.0 mL), to which were then added $Pd(PPh_3)_4$ (1.2 g, 1.0 mmol) and $Cs_2CO_3$ (9.84 g, 30.0 mmol), and finally, the reaction mixture was stirred at 80 °C for 3h. After completion of the reaction, the reaction system was concentrated under reduced pressure. The residue was separated and purified by column chromatography to provide **compound 49G.**
MS (ESI) m/z 729.8 $(M+H)^+$

**Step 8:** Preparation of ($S$,1$^3$E,1$^4$E)-1$^6$-(4-amino)phenyl)-1$^7$-(dibenzylamino)-4$^5$-fluoro-1$^{12}$,6-dimethyl-5-oxa-2,8-diaza-1(5,3)-pyrazolino[1,5-a]pyrimidin-4(3,2)-pyridinacyclononaphan-9-one **(compound 49H)**

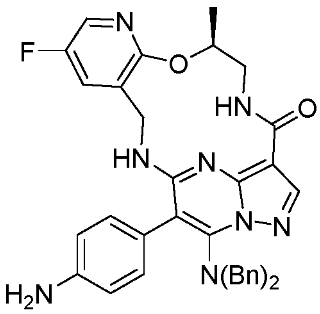

**49H**

($S$,1$^3E$,1$^4E$)-1$^6$-((4-tert-butyloxycarbonylamino)phenyl)-1$^7$-(dibenzylamino)-4$^5$-fluoro-1$^{12}$,6-dimethyl-5 -oxa-2,8-diaza-1(5,3)-pyrazolino[1,5-*a*]pyrimidin-4(3,2)-pyridinacyclononaphan-9-one (3.28 g, 4.5 mmol) was dissolved in DCM (60.0mL), to which was then added the solution of HCl in 1,4-dioxane, and the mixture was stirred at room temperature for 3h. After completion of the reaction, the reaction mixture was concentrated under reduced pressure to provide **compound 49H.**
MS (ESI) m/z 629.8 $(M+H)^+$

**Step 9:** Preparation of methyl (4-(($S$,1$^3E$,1$^4E$)-1$^7$-(dibenzylamino)-4$^5$-fluoro-6-methyl-9-oxo-5-oxa-2,8-diaza-1(5,3)-pyrazolino[1,5-*a*]pyrimidin-4(3,2)-pyridinacyclononaphan-1$^6$-yl)-phenyl)carbamate **(compound 49I)**

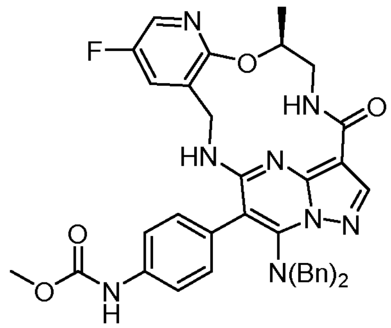

**49l**

($S$,1$^3$$E$,1$^4$$E$)-1$^6$-(4-amino)phenyl)-1$^7$-(dibenzylamino)-4$^5$-fluoro-1$^{12}$,6-dimethyl-5-oxa-2,8-diaza-1(5,3)-pyrazolino[1,5-*a*]pyrimidin-4(3,2)-pyridinacyclononaphan-9-one (628 mg, 1.0 mmol) was dissolved in DCM (10.0mL), to which was then added DIPEA (387mg, 3.0 mmol) in an ice bath, and finally methyl chloroformate (141 mg, 1.5 mmol) was slowly added dropwise. After addition, the reaction system was stirred and reacted at room temperature for 3 h. After completion of the reaction, the reaction system was concentrated under reduced pressure. The residue was separated and purified by column chromatography to provide **compound 49l.**
MS (ESI) m/z 687.3 $(M+H)^+$

**Step 10:** Preparation of methyl (4-(($S$,1$^3$$E$,1$^4$$E$)-1$^7$-amino-4$^5$-fluoro-6-methyl-9-oxo-5-oxa-2,8-diaza-1(5,3)-pyrazolino[1,5-*a*]pyrimidin-4(3,2)-pyridinacyclononaphan-1$^6$-yl)-phenyl)carbamate (**compound 49**)

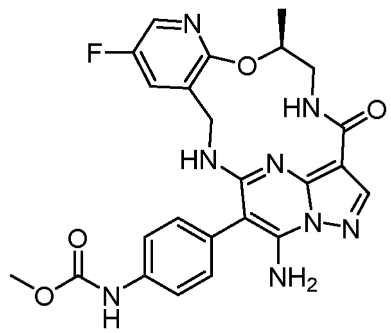

**49**

Methyl (4-(($S$,1$^3$E,1$^4$$E$)-1$^7$-(dibenzylamino)-4$^5$-fluoro-6-methyl-9-oxo-5-oxa-2,8-diaza-1(5,3)-pyrazolino[1,5-*a*]pyrimidin-4(3,2)-pyridinacyclononaphan-1$^6$-yl)-phenyl)carbamate (68.6 mg, 0.1 mmol) was dissolved in DCM (10.0 mL), to which was added trifluoromethanesulfonic acid (150.0 mg, 1.0 mmol) in an ice bath, and then the mixture was stirred and reacted in the ice bath for 2 h. After completion of the reaction, the reaction mixture was concentrated under reduced pressure, and the residue was separated by high pressure preparative separation, to obtain **compound 49** of Example 52.

MS (ESI) m/z 507.2 $(M+H)^+$
$^1$H NMR (400 MHz, DMSO-$d_6$) δ 9.89 (d, $J$ = 6.9 Hz, 2H), 8.05 (s, 1H), 7.99 (d, $J$ = 3.1 Hz, 1H), 7.72 - 7.64 (m, 3H), 7.25 (d, $J$ = 18.1 Hz, 2H), 6.99 (t, $J$ = 6.2 Hz, 1H), 6.69 (s, 2H), 5.01 (m, 1H), 4.93 (m, 1H), 4.01 (m, 1H), 3.87 (dd, $J$ = 14.6, 5.7 Hz, 1H), 3.72 (s, 3H), 3.11 (m, 1H), 1.46 (d, $J$ = 6.1 Hz, 3H).

**Example 50:** The synthetic steps for preparation of ($S$,1$^3$$E$,1$^4$$E$)-1$^7$-amino-1$^6$-(4-chlorophenyl)-4$^5$-fluoro-6-methyl-5-oxa-2,8-diaza-1(5,3)-pyrazolino[1,5-*a*]pyrimidin-4(3,2)-pyridinacyclononaphan-9-one **(compound 50)** were shown as follows:

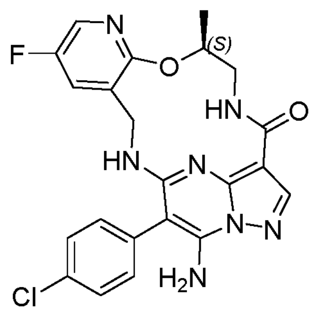

**50**

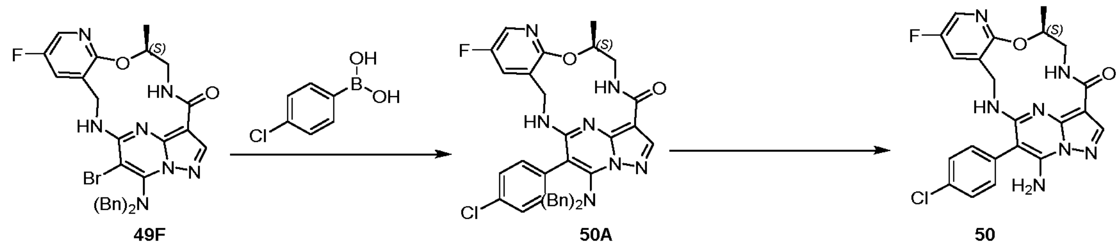

**Step 1:** Preparation of ($S$,1$^3E$,1$^4E$)-1$^6$-((4-chlorophenyl)-1$^7$-(dibenzylamino)-4$^5$-fluoro-1,6-dimethyl-5-oxa-2,8-diaza-1(5,3)-pyrazolino[1,5-a]pyrimidin-4(3,2)-pyridinacyclononaphan-9-one **(compound 50A**)

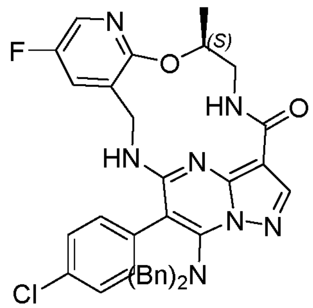

**50A**

Under nitrogen protection, ($S$,1$^3E$,1$^4E$)-1$^6$-bromo-1$^7$-(dibenzylamino)-4$^5$-fluoro-1,6-dimethyl-5-oxa-2,8-diaza-1(5,3)-pyrazolino[1,5-a]pyrimidin-4(3,2)-pyridinacyclononaphan-9-one (6.29 g, 10.0 mmol) and 4-chlorophenylboronic acid (1.87 g, 12.0 mmol) were added into a reaction flask containing 1,4-dioxane/water (60.0 mL), to which were then added $Pd(PPh_3)_4$ (1.2 g, 1.0 mmol) and $Cs_2CO_3$ (9.84 g, 30.0 mmol). Then, the system was purged twice with nitrogen, and finally, the reaction mixture was stirred at 80 °C for 3 h. After completion of the reaction, the reaction mixture was concentrated under reduced pressure. The residue was separated and purified by column chromatography to provide **compound 50A.**

MS (ESI) m/z 648.2 $(M+H)^+$

**Step 2:** Preparation of ($S$,1$^3E$,1$^4E$)-1$^7$-amino-1$^6$-(4-chlorophenyl)-4$^5$-fluoro-6-methyl-5-oxa-2,8-diaza-1(5,3)-pyrazolino[1,5-a]pyrimidin-4(3,2)-pyridinacyclononaphan-9-one (**compound 50**)

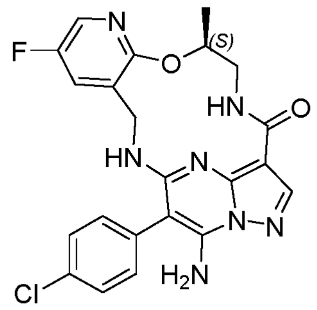

**50**

Methyl (4-(($S$,1$^3E$,1$^4E$)-1$^7$-(dibenzylamino)-4$^5$-fluoro-6-methyl-9-oxo-5-oxa-2,8-diaza-1(5,3)-pyrazolino[1,5-*a*]pyrimidin-4(3,2)-pyridinacyclononaphan-1$^6$-yl)-phenyl)carbamate (64.7 mg, 0.1 mmol) was dissolved in DCM (10.0 mL), to which was then added trifluoromethanesulfonic acid (150 mg, 1.0 mmol) in an ice bath, and the mixture was allowed to react for 2 h in the ice bath. After completion of the reaction, the reaction mixture was concentrated under reduced pressure. The residue was separated and purified over column chromatography to provide **compound 50.**

MS (ESI) m/z 468.1 $(M+H)^+$.
$^1$H NMR (400 MHz, DMSO-$d_6$) δ 9.87 (d, $J$ = 8.9 Hz, 1H), 8.06 (s, 1H), 7.99 (d, $J$ = 3.1 Hz, 1H), 7.70 - 7.58 (m, 3H), 7.35 (d, $J$ = 18.6 Hz, 2H), 7.03 (t, $J$ = 6.2 Hz, 1H), 6.88 (s, 2H), 5.08 - 4.88 (m, 2H), 4.00 (m, $J$ = 13.2, 9.1, 4.0 Hz, 1H), 3.84 (dd, $J$ = 14.6, 5.7 Hz, 1H), 3.10 (m, $J$= 13.5, 10.1, 1.4 Hz, 1H), 1.45 (d, $J$ = 6.1 Hz, 3H).

**Example 51:** The synthetic steps for preparation of ($S$,1$^3E$,1$^4E$)-1$^7$-amino-1$^6$-(4-fluorophenyl)-4$^5$-fluoro-6-methyl-5-oxa-2,8-diaza-1(5,3)-pyrazolino[1,5-*a*]pyrimidin-4(3,2)-pyridinacyclononaphan-9-one **(compound 51)** were shown as follows:

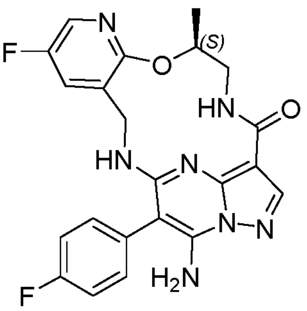

**51**

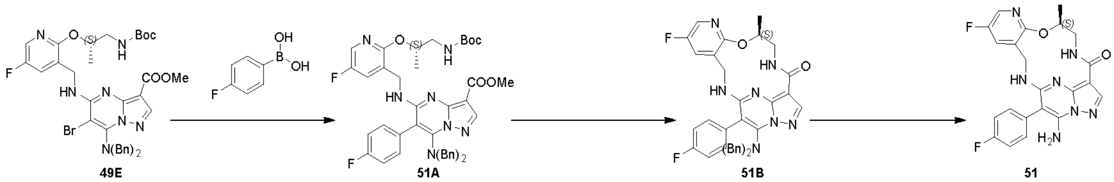

49E 51A 51B 51

**Step 1:** Preparation of methyl (S)-5-(((2-((1-(((tert-butyloxycarbonyl)amino)propan-2-yl)oxy)-5-fluoropyridin-3-3-yl)methyl)amino)-7-(dibenzylamino)-6-(4-fluorophenyl)pyrazolo[1,5-a]pyrimidin-3-carboxylate (**compound 51A**)

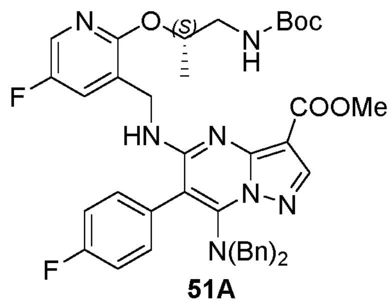

**51A**

Under nitrogen protection, methyl (*S*)-6-bromo-5-((((2-((1-(((tert-butyloxycarbonyl)amino) propan-2-yl)oxy)-5-fluoropyridin-3-yl)methyl)amino)-7-(dibenzylamino)pyrazolo[1,5-a]pyrimidin-3-carboxylate (1.5 g, 2.0 mmol), p-fluorophenylboronic acid (848.0 mg, 1.2 mmol), Xphos Pd $G_3$ (180 mg, 0.1 mmol), Xphos (224 mg, 0.2 mmol), and $K_3PO_4$ (1.27g, 3.0 mmol) were dissolved in 1,4-dioxane/water (3:1, 16.0 mL), and finally, the system was stirred at 80 °C for 3 h. After completion of the reaction, the reaction mixture was poured into water, and the aqueous phase was extracted twice with ethyl acetate. The organic phases were combined, dried over anhydrous sodium sulfate, and concentrated under reduced pressure. The residue was separated and purified by column chromatography to obtain **compound 51A.**
MS (ESI) m/z 764.3 $(M+H)^+$

**Step 2:** Preparation of (*S*,$1^3$*E*,$1^4$*E*)-$1^7$-(dibenzylamino)-$4^5$-fluoro-$1^6$-(4-fluorophenyl)-6-methyl-5-oxa-2,8-diaza-1(5,3)-pyrazolino[1 ,5-a]pyrimidin4(3,2)-pyridinacyclononaphan-9-one (**compound 51B**)

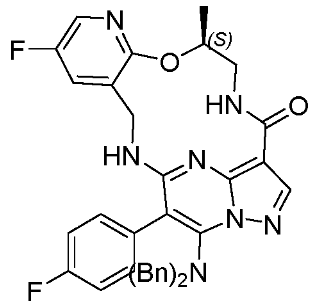

**51B**

Methyl (S)-5-(((2-((1-(((tert-butyloxycarbonyl)amino)propan-2-yl)oxy)-5-fluoropyridin-3-3-yl) methyl)amino)-7-(dibenzylamino)-6-(4-fluorophenyl)pyrazolo[1,5-a]pyrimidin-3-carboxylate (610.0 mg, 0.80 mmol) was dissolved in a mixed solution of methanol and THF, to which was then added LiOH (288 mg, 12.09 mmol) aqueous solution. After addition, the reaction system was heated to 60 °C and stirred for 16 h. Then, the reaction system was cooled to 0 °C, and the pH was adjusted to 2-3 with 2N hydrochloric acid. The resultant mixture was extracted three times with DCM. The organic phases were combined, dried over anhydrous sodium sulfate, filtered, and concentrated to dryness. Then, the solution of HCl in 1,4-dioxane was added, and the resultant mixture was stirred at room temperature for 1 h. The reaction system was concentrated to dryness, to which were then added DCM and DMF, followed by successive addition of DIPEA (15.78 g, 122.08 mmol) and FDPP (2.95 g, 7.69 mmol). After addition, the reaction system was stirred for 16 h at room temperature. The reaction system was concentrated to dryness under reduced pressure, and the residue was separated by C-18 reversed-phase preparative separation to obtain **compound 51B.**
MS (ESI) m/z 632.3 $(M+H)^+$.

**Step 3:** Preparation of (*S*,$1^3$*E*,$1^4$*E*)-$1^7$-amino-$1^6$-(4-fluorophenyl)-$4^5$-fluoro-6-methyl-5-oxa-2,8-diaza-1(5,3)-pyrazolino[1,5-a]pyrimidin4(3,2)-pyridinacyclononaphan-9-one (**compound 51**)

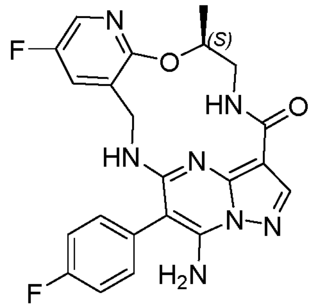

**51**

(*S*,1$^3$*E*,1$^4$*E*)-1$^7$-(dibenzylamino)-4$^5$-fluoro-1$^6$-(4-fluorophenyl)-6-methyl-5-oxa-2,8-diaza-1(5,3)-pyrazolino[1,5-*a*]pyrimidin4(3,2)-pyridinacyclononaphan-9-one (63.1 mg, 0.1 mmol) was dissolved in DCM (10.0 mL), to which was added trifluoromethanesulfonic acid (150 mg, 1.0 mmol) in an ice bath, and then the mixture was allowed to react in the ice bath for 2 h. After completion of the reaction, the reaction mixture was concentrated under reduced pressure. The residue was separated and purified by high pressure preparative separation to provide **compound 51.**

MS (ESI) m/z 452.2 $(M+H)^+$.
$^1$H NMR (400 MHz, DMSO-$d_6$) δ 9.90 (d, *J*= 8.8 Hz, 1H), 8.07 (s, 1H), 7.99 (d, *J*= 3.0 Hz, 1H), 7.68 (dd, *J* = 8.9, 3.1 Hz, 1H), 7.38 (dd, *J* = 20.5, 7.4 Hz, 4H), 7.00 (t, *J* = 6.2 Hz, 1H), 6.83 (s, 2H), 5.06 - 4.89 (m, 2H), 4.01 (m, 1H), 3.86 (dd, *J* = 14.7, 5.7 Hz, 1H), 3.18 - 3.06 (m, 1H), 1.46 (d, *J* = 6.1 Hz, 3H).

**Example 52:** Preparation of (*S*,1$^3$*E*,1$^4$*E*)-1$^7$-amino-1$^6$-(1H-benzo[*d*]imidazol-6-yl)-4$^5$-fluoro-6-methyl-5-oxo-2,8-diaza-1(5,3)-pyrazolo[1,5-*a*]pyrimidin-4(3,2)-pyridinacyclononaphan-9-one **(compound 52):**

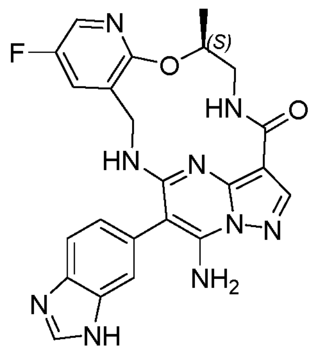

**52**

Using tert-butyl 5-(4,4,5,5-tetramethyl-1,3,2-dioxaborolan-2-yl)-1*H*-benzo[*d*]imidazol-1-carboxylate to replace p-fluorophenylboronic acid, **compound 52** of Example 52 was obtained by the preparation method same as that of Example **51.**

MS (ESI) m/z 474.2 $(M+H)^+$
$^1$H NMR (400 MHz, DMSO-$d_6$) δ 12.63 (s, 1H), 9.94 (d, *J*= 9.0 Hz, 1H), 8.33 (s, 1H), 8.07 (s, 1H), 8.00 (d, *J* = 3.0 Hz, 1H), 7.80 (t, *J* = 7.1 Hz, 1H), 7.74 - 7.61 (m, 1H), 7.61 - 7.48 (m, 1H), 7.19 - 7.04 (m, 1H), 6.99 (q, *J*= 5.6 Hz, 1H), 6.69 (s, 2H), 5.09 - 4.86 (m, 2H), 4.02 (m, 1H), 3.91 - 3.77 (m, 1H), 3.12 (dd, *J* = 13.1, 10.3 Hz, 1H), 1.46 (d, *J* = 6.1 Hz, 3H).

**Example 53:** Preparation of (S,1$^3$E,1$^4$E)-1$^7$-amino-4$^5$-fluoro-1$^6$-(1H-indol-5-yl)-6-methyl-5-oxo-2,8-diaza-1(5,3)-pyrazolo[1,5-a]pyrimidin-4(3,2)-pyridinacyclononaphan-9-one (**compound 53**):

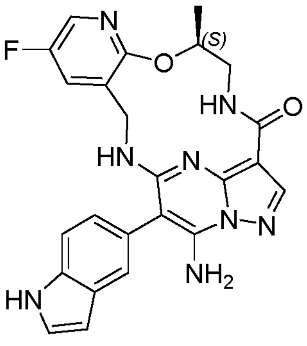

**53**

Using tert-butyl 5-(4,4,5,5-tetramethyl-1,3,2-dioxaborolan-2-yl)-1H-indol-1-carboxylate to replace p-fluorophenylboronic acid, **compound 53** of Example 53 was obtained by the preparation method same as that of Example **51.**

MS (ESI) m/z 473.2 $(M+H)^+$

$^1$H NMR (400 MHz, DMSO-$d_6$) δ 11.15 (s, 1H), 9.93 (dd, *J* = 17.5, 9.0 Hz, 1H), 8.10 - 7.97 (m, 2H), 7.81 - 7.56 (m, 2H), 7.15 - 6.90 (m, 2H), 6.79 - 6.41 (m, 2H), 6.65(m, 1H), 4.98 (d, *J* = 27.9 Hz, 2H), 3.90 (s, 2H), 3.46 (dd, *J* = 15.8, 8.2 Hz, 2H), 3.12 (t, *J*= 11.8 Hz, 1H), 1.46 (d, *J* = 6.1 Hz, 3H).

**Example 54:** Preparation of (*S*,1$^3$*E*,1$^4$*E*)-1$^7$-amino-4$^5$-fluoro-6-methyl-1$^6$-(4-(methylamino)phenyl)-5-oxo-2,8-diaza-1(5,3)-pyrazolo[1,5-a]pyrimidin-4(3,2)-pyridinacyclononaphan-9-one (**compound 54**):

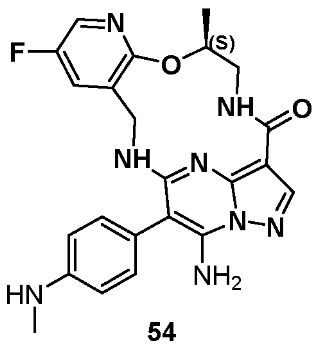

**54**

Using tert-butylmethyl (4-(4,4,5,5-tetramethyl-1,3,2-dioxaborolan-2-yl)phenyl)carbamate to replace p-fluorophenylboronic acid, **compound 54** of Example 54 was obtained by the preparation method same as that of Example **51.**

MS (ESI) m/z 463.3$(M+H)^+$

$^1$H NMR (400 MHz, DMSO-$d_6$) δ 9.91 (d, *J*= 8.8 Hz, 1H), 8.04 (s, 1H), 7.99 (d, *J*= 3.1 Hz, 1H), 7.71 (dd, *J* = 8.9, 3.1 Hz, 1H), 7.11 - 6.94 (m, 3H), 6.75 (d, *J* = 7.9 Hz, 2H), 6.49 (s, 2H), 5.94 (q, *J* = 5.0 Hz, 1H), 5.01 (m, 1H), 4.93 (m, 1H), 4.00 (m, 1H), 3.89 (dd, *J* = 14.6, 5.7 Hz, 1H), 3.15 - 3.07 (m, 1H), 2.77 (d, *J* = 4.8 Hz, 3H), 1.46 (d, *J* = 6.1 Hz, 3H).

**Example 55:** Preparation of 1-(4-((S,1$^3$E,1$^4$E)-1$^7$-amino-4$^5$-fluoro-6-methyl-9-oxo-5-oxo-2,8-diaza-1(5,3)-pyrazolo[1,5-a]pyrimidin-4(3,2)-pyridinacyclononaphan-1$^6$-yl)phenyl)-3-methylurea (**compound 55**):

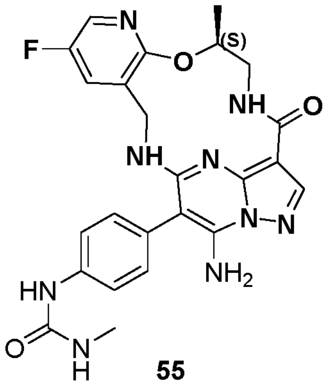

55

Using 1-methyl-3-(4-(4,4,5,5-tetramethyl-1,3,2-dioxaborolan-2-yl)phenyl)urea to replace p-fluorophenylboronic acid, **compound 55** of Example 55 was obtained by the preparation method same as that of Example **51.**

MS (ESI) m/z 506.2 $(M+H)^+$

$^1$H NMR (400 MHz, DMSO-$d_6$) δ 9.89 (d, *J* = 9.0 Hz, 1H), 8.72 (s, 1H), 8.05 (s, 1H), 8.00 (d, *J* = 3.1 Hz, 1H), 7.70 (dd, *J* = 8.9, 3.1 Hz, 1H), 7.61 (d, *J*= 14.1 Hz, 2H), 7.17 (d, *J*= 18.4 Hz, 2H), 7.00 (t, *J* = 6.2 Hz, 1H), 6.65 (s, 2H), 6.06 (q, *J* = 4.6 Hz, 1H), 5.01 (m, 1H), 4.93 (m, 1H), 4.00 (m, 1H), 3.88 (dd, *J* = 14.6, 5.8 Hz, 1H), 3.11 (dd, *J* = 12.9, 10.3 Hz, 1H), 2.68 (d, *J* = 4.5 Hz, 3H), 1.46 (d, *J* = 6.1 Hz, 3H).

**Example 56:** Preparation of S,1$^3$E,1$^4$E)-1$^7$-amino-4$^5$-fluoro-6-methyl-1$^6$-(*p*-methylphenyl)-5-oxo-2,8-diaza-1(5,3)-pyrazolo[1,5-a]pyrimidin-4(3,2)-pyridinacyclononaphan-9-one (**compound 56**):

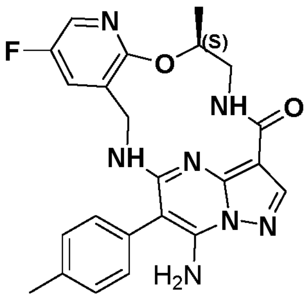

**56**

Using 4-(4,4,5,5-tetramethyl1,3,2-dioxaborolan-diyl)toluene to replace p-fluorophenylboronic acid, **compound 56** of Example 56 was obtained by the preparation method same as that of Example **51.**

MS (ESI) m/z 448.2 $(M+H)^+$

$^1$H NMR (400 MHz, DMSO-$d_6$) δ 9.89 (d, *J* = 8.9 Hz, 1H), 8.06 (s, 1H), 8.00 (d, *J* = 3.1 Hz, 1H), 7.69 (dd, *J* = 8.9, 3.1 Hz, 1H), 7.41 (d, *J*= 7.6 Hz, 2H), 7.29 - 7.16 (m, 2H), 6.97 (t, *J* = 6.2 Hz, 1H), 6.67 (s, 2H), 5.01 (m, 1H), 4.93 (m, 1H), 4.01 (m, 1H), 3.87 (dd, *J* = 14.6, 5.7 Hz, 1H), 3.11 (dd, *J* = 13.0, 10.3 Hz, 1H), 2.42 (s, 3H), 1.46 (d, *J* = 6.1 Hz, 3H).

**Example 57:** Preparation of (S,1$^3$E,1$^4$E)-1$^7$-amino-4$^5$-fluoro-1$^6$-(4-(isopropylamino)phenyl)-6-methyl-5-oxo-2,8-diaza-1(5,3)-pyrazolo[1,5-a]pyrimidin-4(3,2)-pyridinacyclononaphan-9-one (**compound 57**):

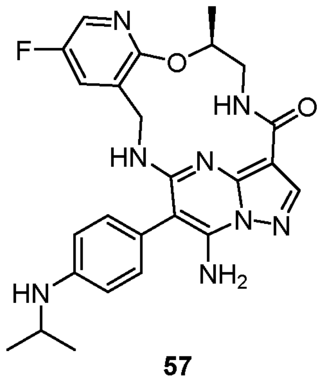

**57**

Using 4-isopropylaminophenylboronic acid pinacol ester to replace p-fluorophenylboronic acid, **compound 57** of Example 57 was obtained by the preparation method same as that of Example **51.**

MS (ESI) m/z 491.2 $(M+H)^+$
$^1H$ NMR (400 MHz, DMSO-$d_6$) δ 9.91 (d, *J*= 8.9 Hz, 1H), 8.04 (s, 1H), 7.99 (d, *J*= 3.1 Hz, 1H), 7.72 (dd, *J* = 9.0, 3.1 Hz, 1H), 7.07 - 6.95 (m, 3H), 6.80 - 6.69 (m, 2H), 6.50 (s, 2H), 5.76 (d, *J* = 7.6 Hz, 1H), 4.97 (m, 2H), 4.00 (m, 1H), 3.90 (dd, *J* = 14.6, 5.7 Hz, 1H), 3.59 (m, 1H), 3.16 - 3.05 (m, 1H), 1.45 (d, *J* = 6.1 Hz, 3H), 1.20 (dd, *J* = 6.3, 3.0 Hz, 6H).

**Example 58:** Preparation of (S,$1^3$E,$1^4$E)-$1^7$-amino-$1^6$-(4-(dimethylamino)phenyl)-$4^5$-fluoro-6-methyl-5-oxo-2,8-diaza-1(5,3)-pyrazolo[1,5-a]pyrimidin-4(3,2)-pyridinacyclononaphan-9-one (**compound 58**):

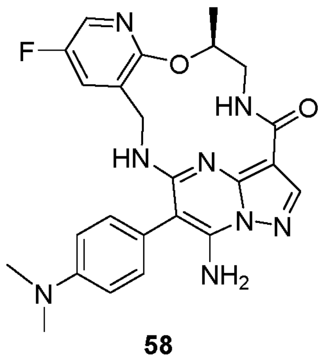

**58**

Using 4-(N,N-dimethylamino)phenylboronic acid pinacol ester to replace p-fluorophenylboronic acid, **compound 58** of Example 58 was obtained by the preparation method same as that of Example **51.**

MS (ESI) m/z 477.2$(M+H)^+$
$^1H$ NMR (400 MHz, DMSO-$d_6$) δ 9.91 (d, *J*= 9.0 Hz, 1H), 8.04 (s, 1H), 8.00 (d, *J*= 3.0 Hz, 1H), 7.71 (dd, *J* = 8.9, 3.1 Hz, 1H), 7.19 - 7.07 (m, 2H), 7.02 - 6.90 (m, 3H), 6.54 (s, 2H), 5.07 - 4.89 (m, 2H), 4.01 (m, 1H), 3.89 (dd, *J* = 14.6, 5.7 Hz, 1H), 3.11 (dd, *J* = 13.0, 10.3 Hz, 1H), 2.53 (s, 6 H), 1.46 (d, *J* = 6.1 Hz, 3H).

**Example 59:** Preparation of (S,$1^3$*E*,$1^4$*E*)-$1^7$-amino-$4^5$-fluoro-$1^6$-(4-methoxyphenyl)-6-methyl-5-oxo-2,8-diaza-1(5,3)-pyrazolo[1,5-a]pyrimidin-4(3,2)-pyridinacyclononaphan-9-one (**compound 59**):

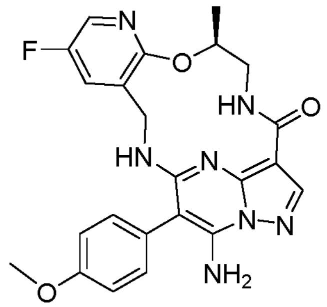

**59**

Using 4-(N,N-dimethylamino)phenylboronic acid pinacol ester to replace p-fluorophenylboronic acid, **compound 59** of Example 59 was obtained by the preparation method same as that of Example **51.**

MS (ESI) m/z 464.2 $(M+H)^+$

$^1$H NMR (400 MHz, DMSO-$d_6$) δ 9.90 (d, *J*= 8.9 Hz, 1H), 8.05 (s, 1H), 8.00 (d, *J*= 3.1 Hz, 1H), 7.69 (dd, *J* = 8.9, 3.1 Hz, 1H), 7.33 - 7.12 (m, 4H), 6.97 (t, *J* = 6.2 Hz, 1H), 6.67 (s, 2H), 5.07 - 4.89 (m, 2H), 4.01 (m, 1H), 3.85 (s, 4H), 3.16 - 3.07 (m, 1H), 1.46 (d, *J* = 6.1 Hz, 3H).

**Example 60:** The synthetic steps for preparation of (*S*,$1^3$*E*,$1^4$*E*)-$1^7$-amino-$1^6$-(4-aminophenyl)-$4^5$-fluoro-6-methyl-5-oxa-2,8-diaza-1(5,3)-pyrazolino[1,5-*a*] pyrimidin-4(3,2)-pyridinacyclononaphan-9-one (**compound 60**) were shown as follows:

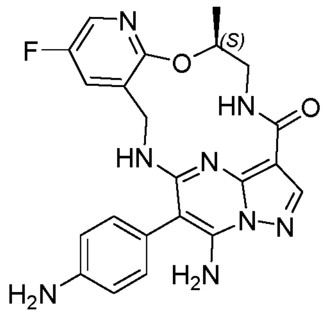

**60**

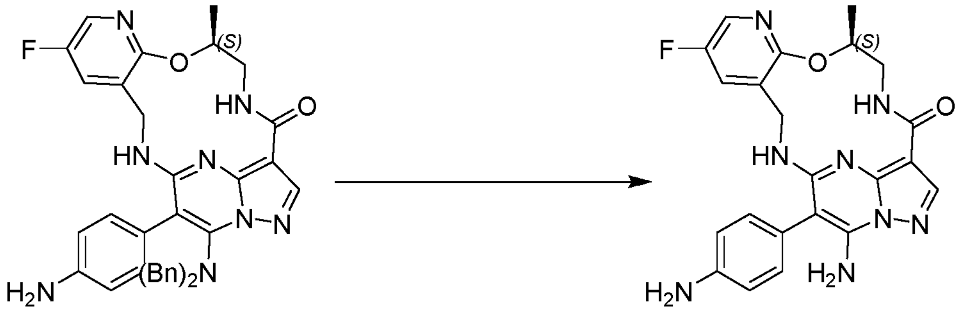

**49H** **60**

**Step 1:** Preparation of (*S*,$1^3$*E*,$1^4$*E*)-$1^7$-amino-$1^6$-(4-aminophenyl)-$4^5$-fluoro-6-methyl-5-oxa-2,8-diaza-1(5,3)-pyrazolino[1,5-*a*]pyrimidin-4(3,2)-pyridinacyclononaphan-9-one (**compound 60**)

(*S*,$1^3$*E*,$1^4$*E*)-$1^6$-(4-amino)phenyl)-$1^7$-(dibenzylamino)-$4^5$-fluoro-$1^{12}$,6-dimethyl-5-oxa-2,8-diaza-1(5,3)-pyrazolino[1,5-*a*]pyrimidin-4(3,2)-pyridinacyclononaphan-9-one (62.8mg, 0.1 mmol) was dissolved in DCM (10.0 mL), to which was added trifluoromethanesulfonic acid (150 mg, 1.0 mmol) in an ice bath, and then the mixture was allowed to react in the ice bath for 2h. After completion of the reaction, the reaction mixture was concentrated under reduced

pressure. The residue was separated and purified over column chromatography to provide **compound 60.**

MS (ESI) m/z 449.2 $(M+H)^+$.

$^1H$ NMR (400 MHz, DMSO-$d_6$) $\delta$ 9.91 (d, *J*= 8.9 Hz, 1H), 8.04 (s, 1H), 7.99 (d, *J*= 3.1 Hz, 1H), 7.71 (dd, *J* = 8.9, 3.1 Hz, 1H), 6.96 (m, 3H), 6.76 (d, *J* = 8.0 Hz, 2H), 6.50 (s, 2H), 5.35 (s, 2H), 5.01 (m, 1H), 4.92 (m, 1H), 4.00 (m, 1H), 3.89 (dd, *J* = 14.6, 5.7 Hz, 1H), 3.11 (dd, *J* = 13.1, 10.3 Hz, 1H), 1.45 (d, *J* = 6.1 Hz, 3H).

**Example 61:** The synthetic steps for preparation iso-propyl of 4-((*S*,$1^3$*E*,$1^4$*E*)-$1^7$-amino-$4^5$-fluoro-6-methyl-9-oxo-5-oxa-2,8-diaza-1(5,3)-pyrazolino[1,5-a]pyrimidin-4(3, 2)-pyridinacyclononaphan-$1^6$-yl)-phenyl)carbamate (**compound 61**) were shown as follows:

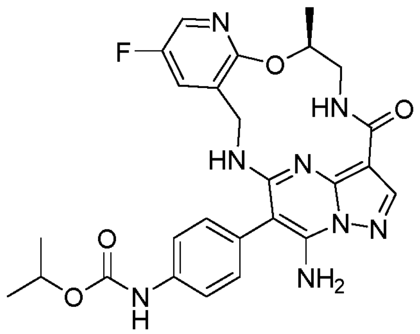

**61**

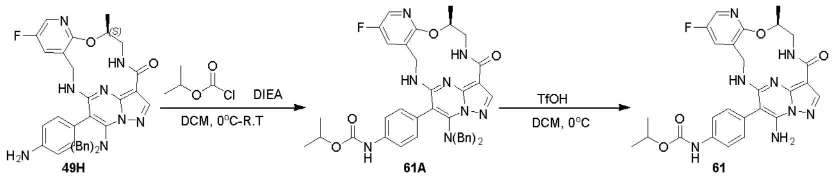


49H

61A

61

**Step 1:** Preparation of iso-propyl (4-((*S*,$1^3$*E*,$1^4$*E*)-$1^7$-(dibenzylamino)-$4^5$-fluoro-6-methyl-9-oxo-5-oxa-2,8-diaza-1(5,3)-pyrazolino[1,5-a]pyrimidin-4(3,2)-pyridinacyclononaphan-$1^6$-yl)-phenyl)carbamate **(compound 61A)**

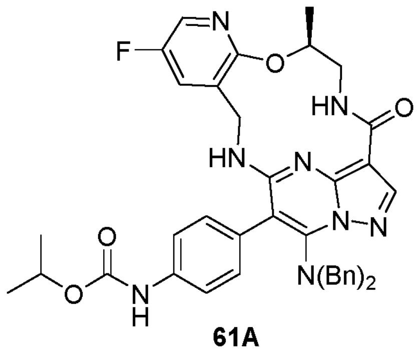

**61A**

(*S*,$1^3$E,$1^4$E)-$1^6$-(4-amino)phenyl)-$1^7$-(dibenzylamino)-$4^5$-fluoro-$1^{12}$,6-dimethyl-5-oxa-2,8-diaza-1(5,3)-pyrazolino[1,5-a]pyrimidin-4(3,2)-pyridinacyclononaphan-9-one (628 mg, 1.0 mmol) was dissolved in DCM (10.0mL), to which was then added DIPEA (387 mg, 3.0 mmol) in an ice bath, and finally, iso-propyl chloroformate (183.0 mg, 1.5 mmol) was slowly added dropwise in the ice bath. After addition, the reaction system was warmed to room temperature and allowed to react under stirring for 3 h. After completion of the reaction, the reaction mixture was concentrated under reduced pressure, and the residue was separated and purified over column chromatography to provide **compound 61A.** MS (ESI) m/z 715.3 $(M+H)^+$.

**Step 2:** Preparation of iso-propyl (4-((*S*,$1^3$*E*,$1^4$*E*)-$1^7$-amino-$4^5$-fluoro-6-methyl-9-oxo-5-oxa-2,8-diaza-1(5,3)-pyrazolino[1,5-*a*]pyrimidin-4(3,2)-pyridinacyclononaphan-$1^6$-yl)-phenyl)carbamate **(compound 61)**

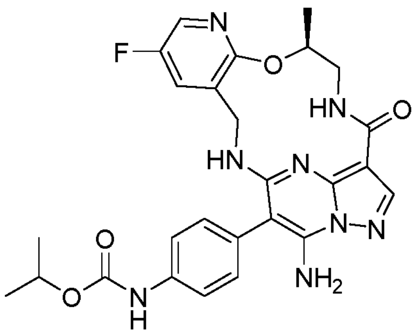

**61**

Iso-propyl (4-((*S*,$1^3$*E*,$1^4$*E*)-$1^7$-(dibenzylamino)-$4^5$-fluoro-6-methyl-9-oxo-5-oxa-2,8-diaza-1(5,3)-pyrazolino[1,5-*a*]pyrimidin-4(3,2)-pyridinacyclononaphan-$1^6$-yl)-phenyl)carbamate (68.6 mg, 0.1 mmol) was dissolved in DCM (10.0 mL), to which was added trifluoromethanesulfonic acid (150mg, 1.0 mmol) in an ice bath, and then the mixture was allowed to react in the ice bath for 2 h. After completion of the reaction, the reaction mixture was concentrated under reduced pressure, and the residue was separated and purified by high pressure preparative separation to provide **compound 61.**

MS (ESI) m/z 535.2 $(M+H)^+$.

$^1$H NMR (400 MHz, DMSO-$d_6$) δ 9.89 (d, *J* = 9.0 Hz, 1H), 9.79 (s, 1H), 8.05 (s, 1H), 8.00 (d, *J* = 3.1 Hz, 1H), 7.69 (dd, *J* = 8.6, 3.9 Hz, 3H), 7.32 - 7.14 (m, 2H), 6.98 (t, *J* = 6.2 Hz, 1H), 6.70 (s, 2H), 5.06 - 4.97 (m, 1H), 4.97 - 4.88 (m, 2H), 4.00 (m, 1H), 3.87 (dd, *J* = 14.6, 5.7 Hz, 1H), 3.11 (dd, *J* = 13.1, 10.3 Hz, 1H), 1.46 (d, *J* = 6.1 Hz, 3H), 1.29 (d, *J* = 6.2 Hz, 6H).

**Example 62:** Preparation of ethyl (4-((*S*,$1^3$*E*,$1^4$*E*)-$1^7$-amino-$4^5$-fluoro-6-methyl-9-oxo-5-oxa-2,8-diaza-1(5,3)-pyrazolino[1,5-*a*]pyrimidin-4(3,2)-pyridinacyclononaphan-$1^6$-yl)-phenyl)carbamate **(compound 62):**

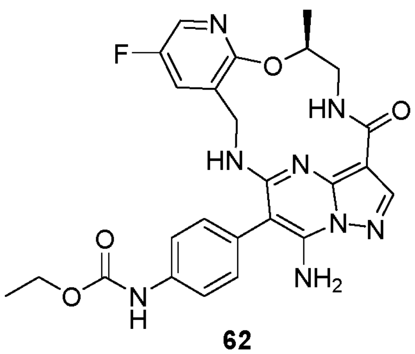

**62**

Using ethyl chloroformate to replace iso-propyl chloroformate, **compound 62** of Example 62 was obtained by the preparation method same as that of Example **61.**

MS (ESI) m/z 521.2 $(M+H)^+$

$^1$H NMR (400 MHz, DMSO-$d_6$) δ 9.94 - 9.84 (m, 2H), 8.05 (s, 1H), 8.00 (d, *J* = 3.1 Hz, 1H), 7.69 (m, 3H), 7.24 (d, *J* = 19.7 Hz, 2H), 6.99 (t, *J* = 6.2 Hz, 1H), 6.70 (s, 2H), 5.01 (m, 1H), 4.93 (m, 1H), 4.18 (q, *J* = 7.1 Hz, 2H), 4.00 (m, 1H), 3.87 (dd, *J* = 14.6, 5.7 Hz, 1H), 3.11 (dd, *J* = 13.1, 10.2 Hz, 1H), 1.46 (d, *J* = 6.1 Hz, 3H), 1.28 (t, *J* = 7.1 Hz, 3H).

**Example 63:** The synthetic steps for preparation of cyclopropylmethyl (4-((S,1$^3$E,1$^4$E)-1$^7$-amino-4$^5$-fluoro-6-methyl-9-oxo-5-oxo-2,8-diaza-1(5,3)-pyrazolo[1,5-a]pyrimidin-4(3,2)-pyridinacyclononaphan-1$^6$-yl)phenyl)carbamate (**compound 63**) were shown as follows:

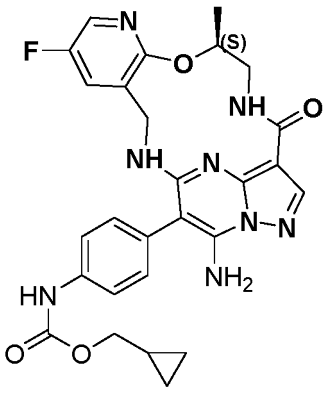

**63**

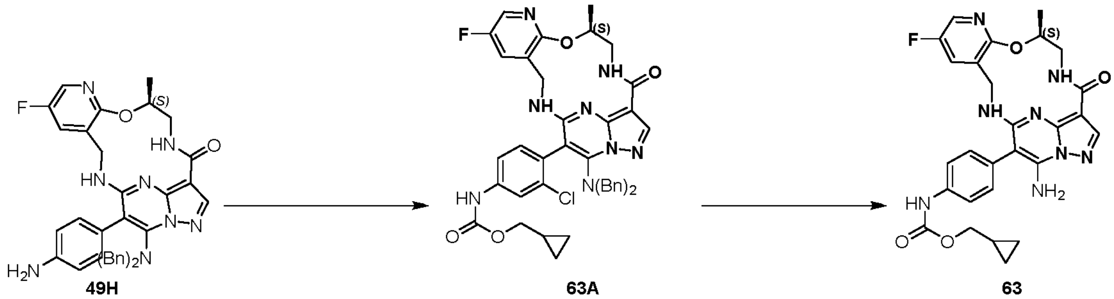

49H 63A 63

**Step 1:** Preparation of cyclopropylmethyl (3-chloro-4-((S,1$^3$E,1$^4$E)-1$^7$-(dibenzylamino)-4$^5$-fluoro-6-methyl-9-oxo-5-oxo-2,8-diaza-1(5,3)-pyrazolo[1,5-a]pyrimidin-4(3,2)-pyridinacyclononaphan-1$^6$-yl)phenyl)carbamate (**compound 63A**)

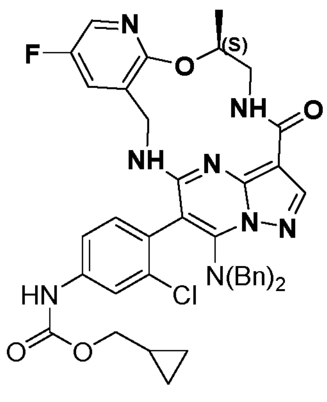

**63A**

(S,1$^3$E,1$^4$E)-1$^6$-(4-aminophenyl)-1$^7$-(dibenzylamino)-4$^5$-fluoro-6-methyl-5-oxo-2,8-diaza-1(5,3)-pyrazolo[1,5-a]pyrimidin-4(3,2)-pyridinacyclononaphan-9-one (160 mg, 0.25 mmol) and DIPEA (97 mg, 0.75 mmol) were dissolved in DCM (10 mL), and then CDI (203 mg, 1.25 mmol) was added into the reaction flask. After the mixture was reacted overnight at 40 °C, cyclopropylmethanol (36 mg, 0.5 mmol) was added dropwise. After addition, the mixture was allowed to react overnight at 40 °C. After completion of the reaction, the reaction mixture was concentrated under reduced pressure, and the residue was separated and purified over column chromatography to provide **compound 63A**.

MS (ESI) m/z 761.3 (M+H)+.

**Step 2**: Preparation of cyclopropylmethyl (4-((S,$1^3$E,$1^4$E)-$1^7$-(dibenzylamino)-$4^5$-fluoro-6-methyl-9-oxo-5-oxo-2,8-diaza-1(5,3)-pyrazolo[1,5-a]pyrimidin-4(3,2)-pyridinacyclononaphan-$1^6$-yl)phenyl) carbamate (**compound 63**)

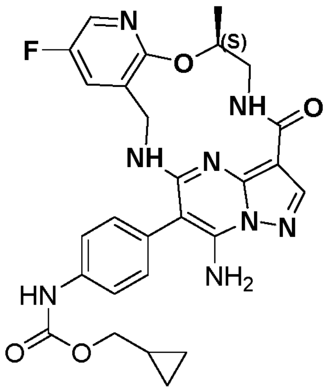

**63**

Cyclopropylmethyl (3-chloro-4-((S,$1^3$E,$1^4$E)-$1^7$-(dibenzylamino)-$4^5$-fluoro-6-methyl-9-oxo-5-oxo-2,8-diaza-1(5,3)-pyrazolo[1,5-a]pyrimidin-4(3,2)-pyridinacyclononaphan-$1^6$-yl)phenyl)carbam ate (160 mg, 0.2 mmol) and dichloromethane (10 mL) were added into a reaction flask, to which were added palladium on carbon (100 mg) and palladium hydroxide (100 mg). Hydrogen was introduced to replace air, and then the mixture was allowed to react overnight at room temperature. After completion of the reaction, the reaction mixture was filtered and concentrated. The residue was separated and purified by high pressure preparative separation to provide **compound 63.**

MS (ESI) m/z 547.2 (M+H)+.
$^1$H NMR (400 MHz, DMSO-$d_6$) δ 9.91 - 9.83 (m, 2H), 8.04 (s, 1H), 7.99 (d, *J* = 3.0 Hz, 1H), 7.71 - 7.63 (m, 3H), 7.23 (m, 2H), 6.96 (t, *J* = 6.2 Hz, 1H), 6.67 (s, 2H), 5.05 - 4.88 (m, 3H), 3.99 (m, 1H), 3.86 (dd, *J* = 14.6, 5.7 Hz, 1H), 3.10 (dd, *J* = 12.9, 10.2 Hz, 1H), 2.39 - 2.28 (m, 3H), 2.14 - 2.01 (m, 2H), 1.78 (q, *J* = 10.4 Hz, 1H), 1.63 (m, 1H), 1.45 (d, *J* = 6.1 Hz, 2H).

**Example 64:** The synthetic steps for preparation of (*S*,$1^3$*E*,$1^4$*E*)-$4^5$-fluoro-6-methyl-$1^7$-(methylamino)-$1^6$-phenyl-5-oxa-2,8-diaza-1(5,3)-pyrazolopyrimidin-4(3,2)-pyridinacyclononaphan-9-one **(compound 64)** were shown as follows:

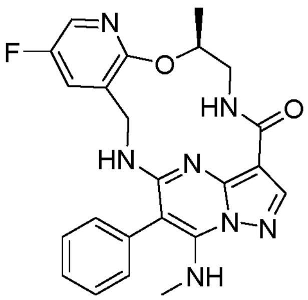

**64**

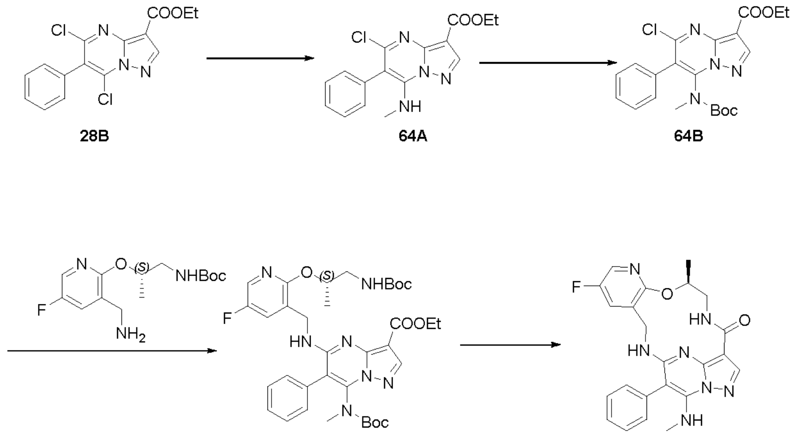

28B 64A 64B

64C 64

**Step 1:** Preparation of ethyl 5-chloro-7-(methylamino)-6-phenylpyrazolino[1,5-a]pyrimidin-3-carboxylate **(compound 64A)**

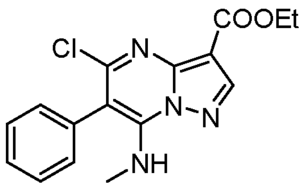

**64A**

Ethyl 5,7-dichloro-6-phenylpyrazolo[1,5-*a*]pyrimidin-3-carboxylate (1.5 g, 4.46 mmol) was dissolved in a mixed solution of methanamine in THF and ethanol (100 mL), to which was then added $K_2CO_3$ (617 mg, 4.46 mmol). The above reaction system was stirred at room temperature for 3 h. After completion of the reaction, the reaction mixture was poured into water, and then a lot of yellow solid compound was precipitated and filtered. The filter cake was rinsed with water and then dried to obtain **compound 64A.** MS (ESI) m/z 331.1 $(M+H)^+$.

**Step 2:** Preparation of ethyl 7-((tert-butyloxycarbonyl)(methyl)amino)-5-chloro-6-phenylpyrazolo [1,5-a]pyrimidin-3-carboxylate (**compound 64B**)

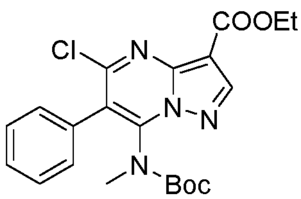

**64B**

Ethyl 5-chloro-7-(methylamino)-6-phenylpyrazolino[1,5-a]pyrimidin-3-carboxylate (1.26 g, 3.81 mmol) was dissolved in DCM, to which was then added DMAP (47 mg, 0.38 mmol). The reaction system was cooled to 0 °C, and stirred for 0.5 h. Then, $(Boc)_2O$ (1.66 g, 7.62 mmol) was slowly added dropwise. After addition, the reaction system was stirred at room temperature for 3 h, and then concentrated under reduced pressure. The residue was separated and purified over column chromatography to provide **compound 64B**.
MS (ESI) m/z 431.1 $(M+H)^+$.

**Step 3:** Preparation of ethyl (S)-7-(((tert-butyloxycarbonyl)(methyl)amino)-5-((((2-((1-(((*tert*-butyloxycarbonyl)amino)propan-2-yl)oxy]-5-fluoropyridin)ethyl-3-yl)methyl)amino)-6-phenyl pyrazolo[1,5-a]pyrimidin-3-carboxylate (**compound 64C**)

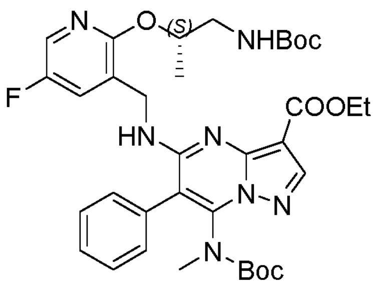

**64C**

Ethyl 7-((tert-butyloxycarbonyl)(methyl)amino)-5-chloro-6-phenylpyrazolo[1,5-*a*]pyrimidin-3-carboxylate (1.44 g, 3.34 mmol) was dissolved in ethanol, to which were then added tert-butyl (*S*)-(2-((3-(aminomethyl)-5-fluoropyridin-2-yloxy)propyl)carbamate (1.5 g, 5.01 mmol) and DIPEA (1.30 g, 10.03 mmol). The reaction system was heated to 80 °C and stirred for 16 h. After completion of the reaction, the reaction system was concentrated under reduced pressure, and the residue was separated and purified over column chromatography to provide **compound 64C.**
MS (ESI) m/z 694.3$(M+H)^+$.

**Step 4:** Preparation of (*S*,$1^3$*E*,$1^4$*E*)-$4^5$-fluoro-6-methyl-$1^7$-(methylamino)-$1^6$-phenyl-5-oxa-2,8-diaza-1(5,3)-pyrazolopyrimidin-4(3,2)-pyridinacyclononaphan-9-one (**compound 64**)

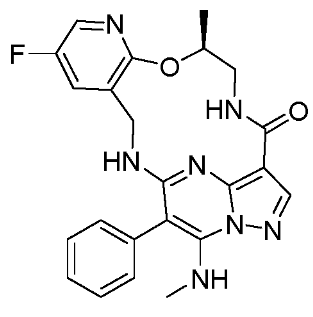

**64**

Ethyl (S)-7-(((tert-butyloxycarbonyl)(methyl)amino)-5-((((2-((1-(((tert-butyloxycarbonyl)amino) propan-2-yl)oxy]-5-fluoropyridin)ethyl-3-yl)methyl)amino)-6-phenylpyrazolo[1,5-*a*]pyrimidin-3-carboxylate (684 mg, 0.99 mmol) was dissolved in a mixed solution of methanol and THF, to which was then added LiOH (472 mg, 19.72 mmol) aqueous solution. After addition, the reaction system was heated to 60 °C and stirred for 16 h. Then, the reaction system was cooled to 0 °C, and the pH was adjusted to 2-3 with 2N hydrochloric acid. The resultant mixture was extracted three times with DCM. The organic phases were combined, dried over anhydrous sodium sulfate, filtered, and concentrated to dryness. Then, the solution of HCl in 1,4-dioxane was added, and the resultant mixture was stirred at room temperature for 1 h. The reaction system was concentrated to dryness, to which were then added DCM and DMF, followed by successive addition of DIPEA (17.2 g, 133.10 mmol) and FDPP (3.41 g, 8.87 mmol). After addition, the reaction system was stirred for 16 h at room temperature, and then concentrated to dryness under reduced pressure. The residue was separated by high pressure preparative separation to obtain **compound 64.**

MS (ESI) m/z 448.2 $(M+H)^+$.
$^1$H NMR (400 MHz, DMSO-$d_6$) $\delta$ 9.86 (d, *J*= 8.9 Hz, 1H), 8.06 (s, 1H), 7.99 (d, *J*= 3.1 Hz, 1H), 7.66 (dd, *J* = 8.9, 3.1 Hz, 1H), 7.54 (m, 3H), 7.48 - 7.36 (m, 2H), 7.27 (q, *J* = 5.2 Hz, 1H), 6.79 (t, *J* = 6.1 Hz, 1H), 5.02 (m, 1H), 4.92 (m, 1H), 4.01 (m, 1H), 3.88 (dd, *J* = 14.6, 5.7 Hz, 1H), 3.11 (m, 1H), 2.26 (d, *J*= 5.2 Hz, 3H), 1.46 (d, *J* = 6.1 Hz, 3H).

**Example 65:** The synthetic steps for preparation of ($1^3E$,$1^4E$)-$1^7$-amino-$4^5$-fluoro-$1^6$-phenyl-5-oxa-2,8-diaza-1(5,3)-pyrazolino[1,5-*a*]pyrimidin-4(3,2)-pyridinacyclononaphan-9-one (**compound 65**) were shown as follows:

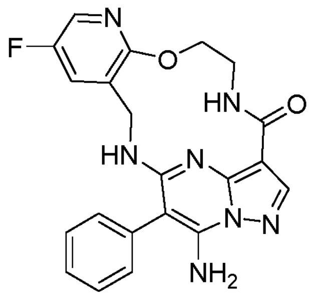

**65**

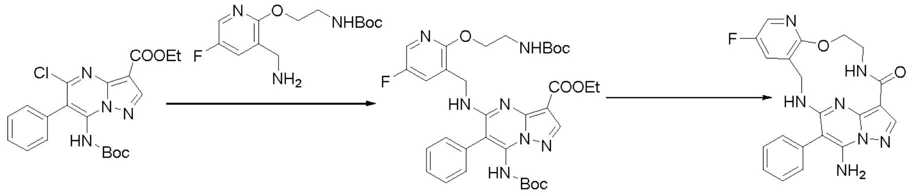

28D 65A 65

**Step 1:** Preparation of ethyl 7-((tert-butyloxycarbonyl)amino)-5-((((2-(2-(((tert-butyloxycarbonyl)amino) ethoxy)-5-fluoropyridin-3-yl)methyl)amino)-6-phenylpyrazolo[1,5-a]pyrimidin-3-carboxylate **(compound 65A)**

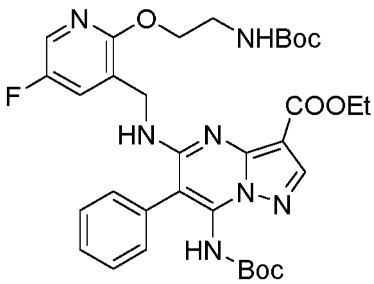

**65A**

Ethyl 7-((tert-butyloxycarbonyl)amino)-5-chloro-6-phenylpyrazolo[1,5-a]pyrimidin-3-carboxylate (500 mg, 1.20 mmol) was dissolved in ethanol, to which were then added tert-butyl (2-((3-(aminomethyl)-5-fluoropyridin-2-yl)oxy)ethyl)carbamate (411 mg, 1.44 mmol) and DIPEA (465 mg, 3.60 mmol). The reaction system was heated to 80 °C and stirred for 16 h. After completion of the reaction, the reaction mixture was concentrated under reduced pressure. The residue was separated by column chromatography to provide **compound 65A**.
MS (ESI) m/z 666.3 $(M+H)^+$

**Step 2:** Preparation of ($1^3E$,$1^4E$)-$1^7$-amino-$4^5$-fluoro-$1^6$-phenyl-5-oxa-2,8-diaza-1(5,3)-pyrazolino[1,5-*a*]pyrimidin-4(3,2)-pyridinacyclononaphan-9-one (**compound 65**)

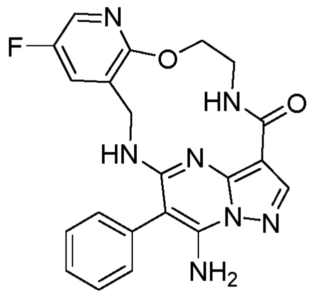

**65**

**Compound 65** of Example **65** was obtained by the preparation method same as that of step 2 in Example 17.

MS (ESI) m/z 420.2 $(M+H)^+$

$^1H$ NMR (400 MHz, DMSO-$d_6$) $\delta$ 9.73 (dd, *J* = 7.8, 2.4 Hz, 1H), 8.08 (s, 1H), 8.02 (d, *J* = 3.1 Hz, 1H), 7.72 (dd, *J* = 8.8, 3.0 Hz, 1H), 7.60 (t, *J* = 7.5 Hz, 2H), 7.55 - 7.48 (m, 1H), 7.36 (d, *J* = 17.9 Hz, 2H), 6.93 (t, *J* = 6.1 Hz, 1H), 6.70 (s, 2H), 4.99 (m, 1H), 4.59 (m, 1H), 4.36 (m, 1H), 3.91 (m, 2H), 3.51 - 3.38 (m, 1H).

**Example 66:** The synthetic steps for preparation of ((S,1$^3$E,1$^4$E)-1$^7$-amino-4$^5$-fluoro-1$^6$-(2-fluorophenyl)-6-methyl-5-oxo-2,8-diaza-1 (5,3)-pyrazolo[1,5-a]pyrimidin-4(3,2)-pyridinacyclononaphan -9-one (**compound 66**) were shown as follows:

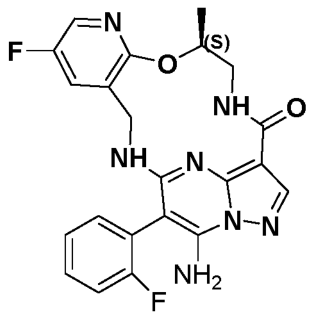

**66**

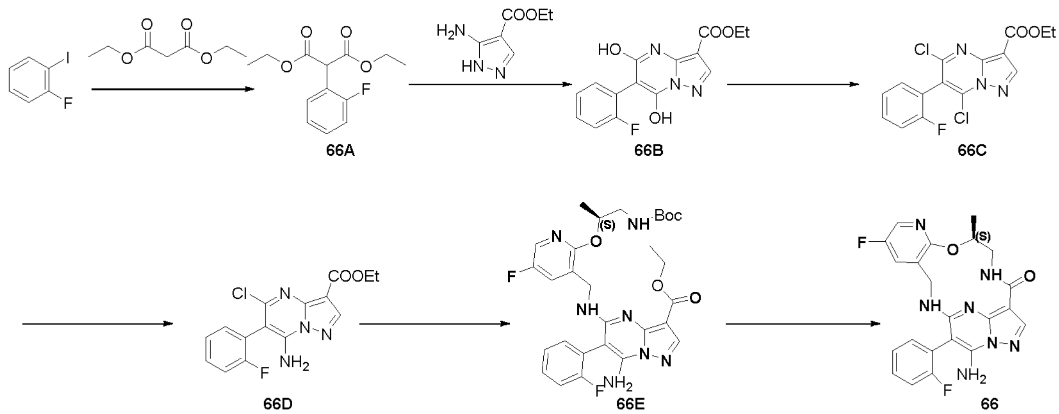

66A

66B

66C

66D

66E

66

**Step 1:** Preparation of diethyl 2-(2-fluorophenyl)malonate (**compound 66A**)

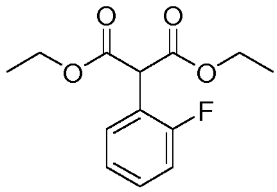

**66A**

Under nitrogen protection, 2-fluoroiodobenzene (21 g, 94.6 mmol), THF (600 mL), diethyl malonate (30.3 g, 189.2 mmol), CuI (0.9 g, 4.73 mmol), 2-pyridinecarboxylic acid (2.32 g, 18.9 mmol) and $Cs_2CO_3$ (46.15 g, 142 mmol) were successively added into a reaction flask, and the mixture was allowed to react overnight at 70 °C. After completion of the reaction, the reaction system was cooled to room temperature, filtered, and concentrated. The residue was purified by column chromatography to obtain **compound 66A.**
MS (ESI) m/z 255.1 $(M+H)^+$.

**Step 2:** Preparation of ethyl 6-(2-fluorophenyl)-5,7-dihydroxylpyrazolo[1 ,5-a]pyrimidin-3-carboxylate (**compound 66B**)

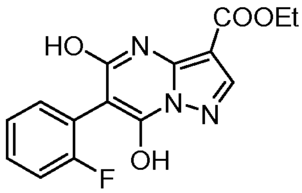

**66B**

Diethyl 2-(2-fluorophenyl)malonate (22 g, 86.6 mmol), ethyl 5-amino-1H-pyrazol-4-carboxylate (10.3 g, 66.6 mmol), and tri-n-butylamine (13.6 g, 73.3 mmol) were allowed to react for 8 h at 180 °C. After completion of the reaction, the crude product was directly used in the next reaction.
MS (ESI) m/z 318.1$(M+H)^+$.

**Step 3:** Preparation of ethyl 5,7-dichloro-6-(2-fluorophenyl)pyrazolo[1,5-a]pyrimidin-3-carboxylate (**compound 66C**)

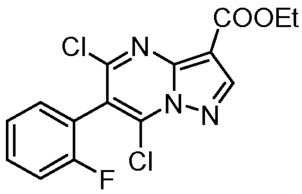

**66C**

Ethyl 6-(2-fluorophenyl)-5,7-dihydroxylpyrazolo[1 ,5-a]pyrimidin-3-carboxylate (20 g, 63.1 mmol), $PCl_5$ (19.68 g, 94.65 mmol), and $POCl_3$ (150 mL) were added into a reaction flask, and then the reaction system was allowed to react overnight at 100 °C. After completion of the reaction, the reaction mixture was concentrated under reduced pressure, and poured into ice water. The aqueous phase was extracted with ethyl acetate. The organic phase was dried over anhydrous sodium sulfate, filtered, and concentrated. The residue was separated and purified over column chromatography to provide **compound 66C**.
MS (ESI) m/z 354.0 $(M+H)^+$.

**Step 4:** Preparation of ethyl 7-amino-5-chloro-6-(2-fluorophenyl)pyrazolo[1,5-a]pyrimidin-3-carboxylate **(compound 66D)**

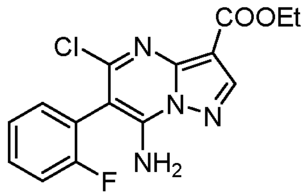

**66D**

Ethyl 5,7-dichloro-6-(2-fluorophenyl)pyrazolo[1,5-a]pyrimidin-3-carboxylate (5 g, 14.2 mmol), DCM (20 mL), and the solution of ammonia in methanol (20 mL) were added into a reaction flask, and then the mixture was allowed to react overnight at room temperature. After completion of the reaction, the reaction mixture was diluted by adding water, filtered, rinsed with ethyl acetate to obtain **compound 66D.** MS (ESI) m/z 335.1 $(M+H)^+$.

**Step 5:** Preparation of ethyl (S)-7-amino-5-((((2-((1-(((tert-butyloxycarbonyl)amino)propan-2-yl)oxy)-5-fluoropyridin-3-yl)methyl)amino)-6-(2-fluorophenyl)pyrazolo[1,5-a]pyrimidin-3-carboxylate (**compound 66E**)

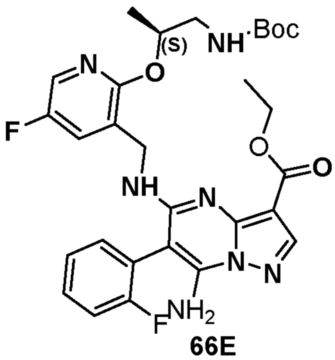

**66E**

Ethyl 7-amino-5-chloro-6-(2-fluorophenyl)pyrazolo[1,5-a]pyrimidin-3-carboxylate (500 mg, 1.5 mmol), tert-butyl (S)-(2-((3-(aminomethyl)-5-fluoropyridin-2-yl)oxy)propyl)carbamate (900 mg, 3 mmol), DIPEA (580 mg, 4.5 mmol), and n-butanol (10 mL) were added into a 30 mL sealed tube. The mixture was allowed to react overnight at 120 °C under stirring, followed by stirring for 2 d. After completion of the reaction, the reaction mixture was diluted by adding water, and then extracted with ethyl acetate. The organic phase was successively washed with water and saturated brine, dried over anhydrous sodium sulfate, and concentrated under reduced pressure. The residue was separated by column chromatography to provide **compound 66E.**
MS (ESI) m/z 598.3 $(M+H)^+$

**Step 6:** Preparation of ((S,$1^3$E,$1^4$E)-$1^7$-amino-$4^5$-fluoro-$1^6$-(2-fluorophenyl)-6-methyl-5-oxo-2,8-diaza-1(5,3)-pyrazolo[1,5-a]pyrimidin-4(3,2)-pyridinacyclononaphan-9-one (**compound 66**)

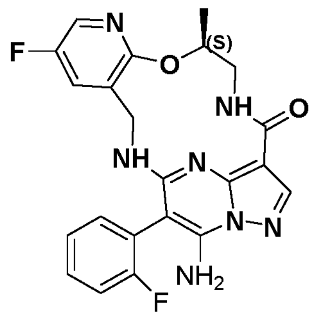

**66**

**Compound 66** of Example **66** was obtained by the preparation method same as that of step 2 in Example 17.

MS (ESI) m/z 452.2 $(M+H)^+$

$^1H$ NMR (400 MHz, DMSO-$d_6$) δ 9.87 (d, *J* = 9.0 Hz, 1H), 8.08 (s, 1H), 8.01 (t, *J* = 2.7 Hz, 1H), 7.67 (m, 1H), 7.58 (m, 1H), 7.47 - 7.33 (m, 3H), 7.12 (m, 1H), 7.00 (d, *J* = 3.6 Hz, 2H), 5.07 - 4.90 (m, 2H), 4.02 (m, 1H), 3.86 (m, 1H), 3.12 (m, 1H), 1.46 (d, *J* = 6.0 Hz, 3H).

**Example 67:** Preparation of propyl (4-((*S*,$1^3$*E*,$1^4$*E*)-$1^7$-amino-$4^5$-fluoro-6-methyl-9-oxo-5-oxa-2,8-diaza-1(5,3)-pyrazolino[1,5-*a*]pyrimidin-4(3, 2)-pyridinacyclononaphan-$1^6$-yl)-phenyl)carbamate (**compound 67**):

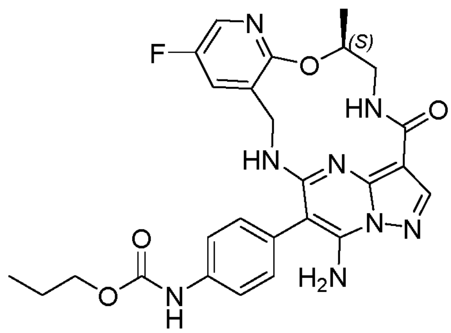

**67**

Using n-propyl chloroformate to replace methyl chloroformate, **compound 67** of Example 67 was obtained by the preparation method same as that of Example **61.**

MS (ESI) m/z 535.2$(M+H)^+$

$^1H$ NMR (400 MHz, DMSO-$d_6$) δ 9.99 - 9.76 (m, 2H), 8.05 (s, 1H), 8.00 (d, *J* = 3.1 Hz, 1H), 7.69 (dd, *J* = 8.7, 3.6 Hz, 3H), 7.32 - 7.14 (m, 2H), 6.99 (t, *J* = 6.2 Hz, 1H), 6.70 (s, 2H), 5.01 (m, 1H), 4.93 (m, 1H), 4.09 (t, *J* = 6.7 Hz, 2H), 4.01 (m, 2H), 3.87 (dd, *J* = 14.6, 5.7 Hz, 1H), 3.11 (m, 1H), 1.68 (m, 2H), 1.46 (d, *J* = 6.1 Hz, 3H), 0.96 (t, *J* = 7.4 Hz, 3H).

**Example 68:** Preparation of trifluoroethyl (4-((*S*,$1^3$*E*,$1^4$*E*)-$1^7$-amino-$4^5$-fluoro-6-methyl-9-oxo-5-oxa-2,8-diaza-1(5,3)-pyrazolino[1,5-*a*]pyrimidin-4(3,2)-pyridinacyclononaphan-$1^6$-yl)-phenyl)carbamat e (**compound 68**):

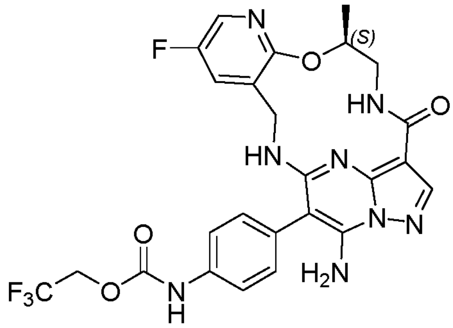

**68**

Using trifluoroethanol to replace cyclopropylmethanol, **compound 68** of Example 68 was obtained by the preparation method same as that of Example **63.**

MS (ESI) m/z 575.2 $(M+H)^+$

$^1$H NMR (400 MHz, DMSO-$d_6$) δ 10.36 (s, 1H), 9.88 (d, *J* = 8.9 Hz, 1H), 8.06 (s, 1H), 8.00 (d, *J*= 3.0 Hz, 1H), 7.77 - 7.63 (m, 3H), 7.28 (d, *J* = 12.2 Hz, 2H), 6.99 (t, *J* = 6.2 Hz, 1H), 6.72 (s, 2H), 5.02 - 4.99 (m, 1H), 4.98 - 4.92 (m, 1H), 4.87 (q, *J* = 9.2 Hz, 2H), 4.04 - 3.97 (m, 1H), 3.87 (dd, *J* = 14.7, 5.7 Hz, 1H), 3.11 (dd, *J* = 13.1, 10.3 Hz, 1H), 1.46 (d, *J* = 6.1 Hz, 3H).

**Example 69:** The synthetic steps for preparation of ethyl (4-((*S*,$1^3$*E*,$1^4$*E*)-$1^7$-amino-$4^5$-fluoro-6-methyl-9-oxo-5-oxa-2,8-diaza-1(5,3)-pyrazolo[1,5-a]pyrimidin-4(3,2)-pyridinacyclononaphan-$1^6$-yl)-2-fluorophenyl)carbamate (**compound 69**) were shown as follows:

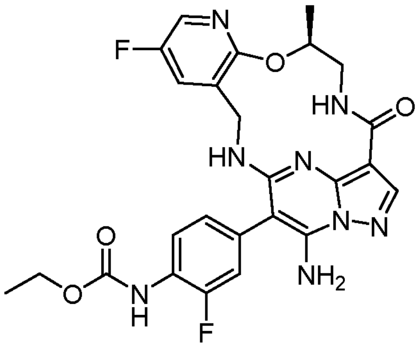

**69**

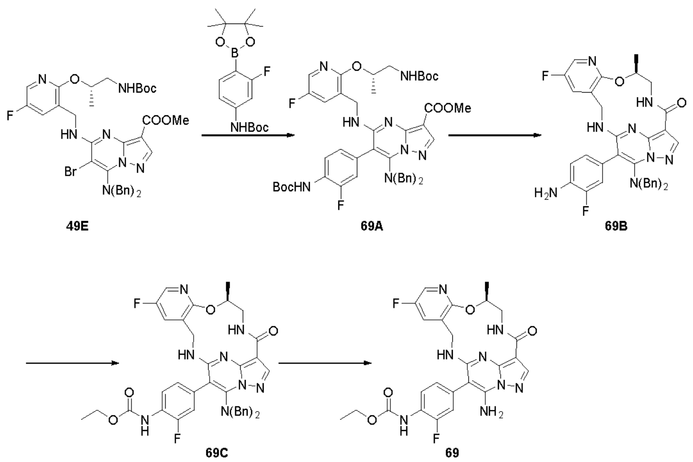

49E 69A 69B

69C 69

**Step 1:** Preparation of methyl (*S*)-6-(4-((tert-butyloxycarbonyl)amino)-3-fluorophenyl)-5-(((2-((1-(1-((tert-butyloxycarbonyl)amino)propan-2-yl]oxy)-5-fluoropyridin-3-yl)methyl)amino)-7-(dibenzylamino) pyrazolo[1,5-*a*]pyrimidin-3-carboxylate (**compound 69A**)

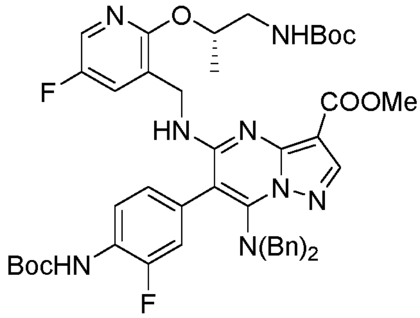

**69A**

Methyl (*S*)-6-bromo-5-(((2-((1-((tert-butyloxycarbonyl)amino)propan-2-yl)oxy)-5-fluoropyridin-3-yl) methyl)amino)-7-(dibenzylamino)pyrazolo[1,5-a]pyrimidin-3-carboxylate (3.0 g, 4.01 mmol) was dissolved in a mixed solvent of 1,4-dioxane and water, to which were added tert-butyl (2-fluoro-4-(4,4,5,5-tetramethyl-1,3,2-dioxaborolan-2-yl)phenyl)carbamate (2.43 g, 7.21 mmol), $Pd_2(dba)_3$ (339 mg, 0.40 mmol), Xphos (191 mg, 0.40 mmol), and sodium phosphate (1.27 g, 12.02 mmol). After addition, the system was heated to 80 °C and stirred for 4 h. After completion of the reaction, the reaction mixture was filtered through diatomite, and then concentrated under reduced pressure. The residue was separated and purified over column chromatography to provide **compound 69A.**
MS (ESI) m/z 879.4 $(M+H)^+$.

**Step 2:** Preparation of (*S*,1$^3$*E*,1$^4$*E*)-1$^6$-(4-amino-3-fluorophenyl)-1$^7$-(dibenzylamino)-4$^5$-fluoro-6-methyl-5-oxo-2,8-diaza-1(5,3)-pyrazolo[1,5-*a*]pyrimidin-4(3,2)-pyridinacyclononaphan-9-one **(compound 69B)**

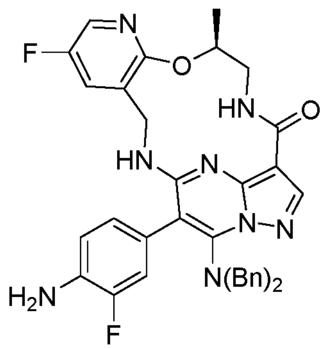

**69B**

Methyl (*S*)-6-(4-((tert-butyloxycarbonyl)amino)-3-fluorophenyl)-5-(((2-((1-(1- ((tert-butyloxycarbonyl) amino)propan-2-yl]oxy)-5-fluoropyridin-3-yl)methyl)amino)-7-(dibenzylamino)pyrazolo[1,5-*a*]pyrimidin-3-carboxylate (2.68 g, 3.05 mmol) was dissolved in a mixed solution of methanol and THF, to which was then added LiOH (1.46 g, 60.98 mmol) aqueous solution. After addition, the reaction system was heated to 60 °C and stirred for 16 h. Then, the reaction system was cooled to 0 °C, and the pH was adjusted to 2-3 with 2N hydrochloric acid. The resultant mixture was extracted three times with DCM. The organic phases were combined, dried over anhydrous sodium sulfate, filtered, and concentrated to dryness. Then, the solution of HCl in 1,4-dioxane was added, and the resultant mixture was stirred at room temperature for 1 h. The reaction system was concentrated to dryness, to which were then added DCM and DMF, followed by successive addition of DIPEA (53.20 g, 411.61 mmol) and FDPP (9.96 g, 25.92 mmol). After addition, the reaction system was stirred for 16 h at room temperature. The reaction system was concentrated to dryness under reduced pressure, and the residue was separated by C-18 reversed-phase preparative separation to obtain **compound**

**69B**.
MS (ESI) m/z 647.3 $(M+H)^+$

**Step 3**: Preparation of ethyl (4-((*S*,1$^3$*E*,1$^4$*E*)-1$^7$-(dibenzylamino)-4$^5$-fluoro-6-methyl-9-oxo-5-oxa-2,8-diaza-1(5,3)-pyrazolo[1,5-*a*]pyrimidin-4(3,2)-pyridinacyclononaphan-1$^6$-yl)-2-fluorophenyl)carbamate (**compound 69C**)

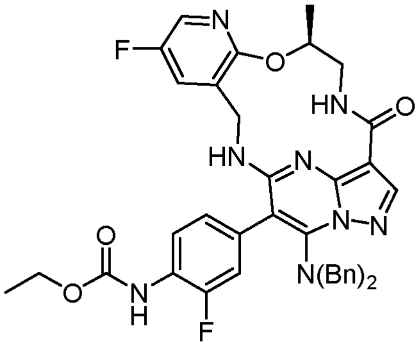

**69C**

(*S*,1$^3$*E*,1$^4$*E*)-1$^6$-(4-amino-3-fluorophenyl)-1$^7$-(dibenzylamino)-4$^5$-fluoro-6-methyl-5-oxo-2,8-diaza-1(5, 3)-pyrazolo[1,5-*a*]pyrimidin-4(3,2)-pyridinacyclononaphan-9-one (1.80 g, 2.78 mmol) was dissolved in THF (20 mL), and then the reaction system was cooled to 0 °C, to which was added N,N-diisopropylethylamine (1.08 g, 8.35 mmol). Then, ethyl chloroformate (604 mg, 5.57 mmol) was slowly added dropwise. After addition, the reaction system was naturally warmed to room temperature and stirred for 16 h. After completion of the reaction, the reaction was quenched by adding water, and the resultant mixture was extracted with ethyl acetate. The organic phases were combined, dried over anhydrous sodium sulfate, filtered, and concentrated. The residue was separated and purified over column chromatography to provide **compound 69C.**
MS (ESI) m/z 719.3 $(M+H)^+$

**Step 4:** Preparation of ethyl (4-((*S*,*1*$^3$*E*,*1*$^4$*E*)-1$^7$-amino-4$^5$-fluoro-6-methyl-9-oxo-5-oxa-2,8-diaza-1(5,3)-pyrazolo[1,5-*a*]pyrimidin-4(3,2)-pyridinacyclononaphan-1$^6$-yl)-2-fluorophenyl)carbamate **(compound 69)**

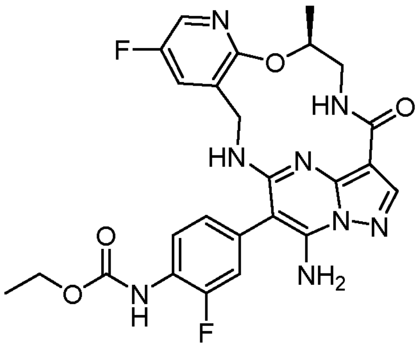

**69**

Ethyl (4-((*S*,1$^3$E,1$^4$*E*)-1$^7$-(dibenzylamino)-4$^5$-fluoro-6-methyl-9-oxo-5-oxa-2,8-diaza-1(5,3)-pyrazolo[1,5-*a*]pyrimidin-4(3,2)-pyridinacyclononaphan-1$^6$-yl)-2-fluorophenyl)carbamate (980 mg, 1.36 mmol) was dissolved in DCM (10 mL), and the reaction system was cooled to 0 °C. Then, trifluoromethanesulfonic acid (204 mg, 1.36 mmol) was added dropwise. After addition, the reaction system was stirred at 0 °C for 10 min. After completion of the reaction, the pH value was adjusted to be 9 by slowly adding triethylamine in an ice bath, and then the crude product was separated and purified by high pressure preparative separation to provide **compound 69.**

MS (ESI) m/z 539.2 $(M+H)^+$.

$^1$H NMR (400 MHz, DMSO-$d_6$) δ 9.87 (d, *J* = 9.0 Hz, 1H), 9.55 (s, 1H), 8.06 (s, 1H), 8.00 (d, *J* = 3.1 Hz, 1H), 7.92 (t, *J*= 8.4 Hz, 1H), 7.69 (s, 1H), 7.25 - 7.04 (m, 3H), 6.88 (s, 2H), 5.09 - 4.88 (m, 2H), 4.19 (q, *J* = 7.1 Hz, 2H), 4.00 (m, 1H), 3.87 (m, 2H), 3.11 (m, 1H), 1.46 (d, *J* = 6.1 Hz, 3H), 1.29 (t, *J* = 7.1 Hz, 3H).

**Example 70:** Preparation of (*S*,1$^3$*E*,1$^4$*E*)-1$^7$-amino-4$^5$-fluoro-6-methyl-1$^6$-(6$^{2,6}$-difluoro-6$^1$-(ethoxycarbonyl)amino-4-yl)phenyl-5-oxa-2,8-diaza-1(5,3)-pyrazolo[1,5-*a*]pyrimidinyl-4(3,2)-pyridinacyclononaphan-9-one (**compound 70**):

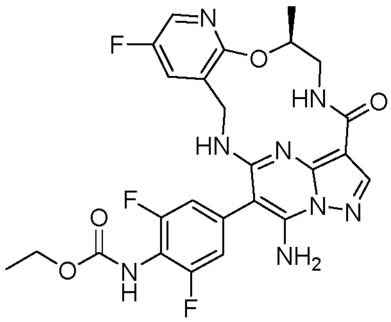

**70**

Using 2,6-difluoro-4-pinacolboronate phenylamine bis(tert-butyloxy)carbonyl ester to replace tert-butyl (2-fluoro-4-(4,4,5,5-tetramethyl-1,3,2-dioxaborolan-2-yl)phenyl)carbamate, **compound 70** of Example 70 was obtained by the preparation method same as that of Example **69.**

MS (ESI) m/z 557.2 $(M+H)^+$

$^1$H NMR (400 MHz, DMSO-$d_6$) δ 9.84 (d, *J* = 8.8 Hz, 1H), 9.38 (s, 1H), 8.07 (s, 1H), 8.00 (d, *J* = 3.1 Hz, 1H), 7.70 (dd, *J* = 8.8, 3.1 Hz, 1H), 7.21 (t, *J* = 6.1 Hz, 1H), 7.17 - 7.04 (m, 3H), 5.02 (m, 1H), 4.94 (dd, *J* = 14.3, 6.5 Hz, 1H), 4.16 (q, *J*= 7.1 Hz, 2H), 4.05 - 3.95 (m, 2H), 3.88 (dd, *J* = 14.7, 5.7 Hz, 1H), 3.13 (q, *J* = 12.0, 10.3 Hz, 1H), 1.46 (d, *J* = 6.1 Hz, 3H), 1.27 (t, *J*= 7.1 Hz, 3H).

**Example 71:** The synthetic steps for preparation of ethyl (4-((S,1$^3$E,1$^4$E)-1$^7$-amino-4$^5$-fluoro-6-methyl-9-oxo-5-oxo-2,8-diaza-1(5,3)-pyrazolo[1,5-a]pyrimidin-4(3,2)-pyridinacyclononaphan-1$^6$-yl)-3-fluorophenyl)carbamate (**compound 71**) were shown as follows:

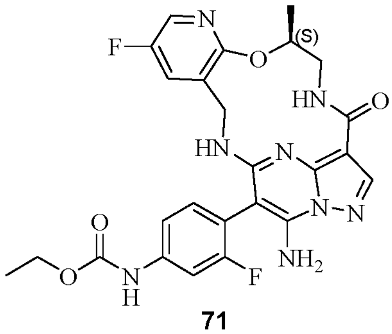

**71**

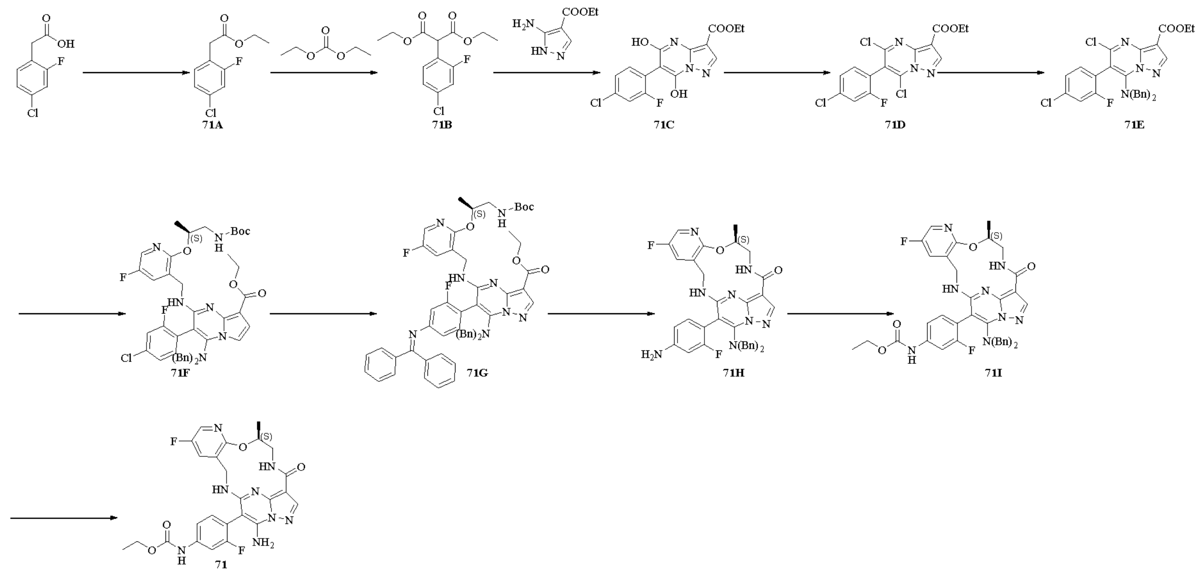


**Step 1:** Preparation of ethyl 2-(4-chloro-2-fluorophenyl)acetate (**compound 71A**)

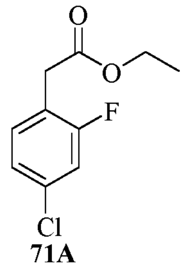

**71A**

2-(4-Chloro-2-fluorophenyl)acetic acid (25.00 g, 0.13 mol) was dissolved in EtOH (200 mL), to which was added concentrated sulfuric acid (4 mL) dropwise. After addition, the reaction system was allowed to react overnight at 80 °C. After completion of the reaction, the system was cooled to room temperature, and then concentrated under reduced pressure, to which was further added saturated sodium carbonate solution for dilution. The resultant mixture was extracted with ethyl acetate, dried over sodium sulfate, filtered, and concentrated to obtain **compound 71A.**
MS (ESI) m/z 217.0 $(M+H)^+$.

**Step 2:** Preparation of diethyl 2-(4-chloro-2-fluorophenyl)malonate (**compound 71B**)

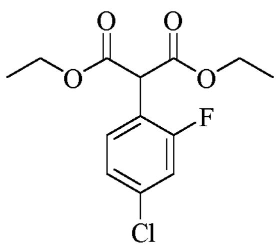

**71B**

Diethyl carbonate (30.70 g, 0.26 mol) was dissolved in THF (400 mL), to which was added sodium hydride (13.20 g, 0.33 mol) in batches at 0 °C, and after stirred for 10 min, ethyl 2-(4-chloro-2-fluorophenyl)acetate was added to the above system. Then, the system was allowed to react overnight at 60 °C. After completion of the reaction, the

reaction system was cooled to room temperature, and the reaction mixture was poured into ammonium chloride aqueous solution, then The resultant mixture was extracted with diethyl ether. The organic phase was dried over anhydrous sodium sulfate, filtered, and concentrated. The residue was separated and purified over column chromatography, to obtain **compound 71B.**
MS (ESI) m/z 289.1 $(M+H)^+$.

**Step 3:** Preparation of ethyl 6-(4-chloro-2-fluorophenyl)-5,7-dihydroxylpyrazolo[1 ,5-a]pyrimidin-3-carboxylate (**compound 71C**)

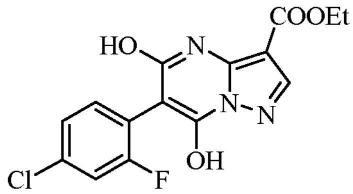

**71C**

Diethyl 2-(4-chloro-2-fluorophenyl)malonate (34.00 g, 0.12 mol), ethyl 5-amino-1H-pyrazol-4-carboxylate (15.50 g, 0.10 mol), and tri-n-butylamine (20.30 g, 0.11 mol) were added into a reaction flask, and then the above system was allowed to react at 180 °C for 10 h. After completion of the reaction, the system was cooled, and directly used in the next step.
MS (ESI) m/z 352.0 $(M+H)^+$.

**Step 4:** Preparation of ethyl 5,7-dichloro-6-(4-chloro-2-fluorophenyl)pyrazolo[1,5-a]pyrimidin-3-carboxylate (**compound 71D**)

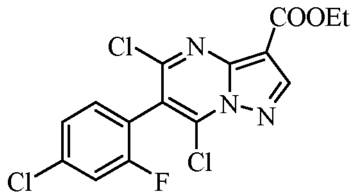

**71D**

Ethyl 6-(4-chloro-2-fluorophenyl)-5,7-dihydroxylpyrazolo[1,5-a]pyrimidin-3-carboxylate (36 g, 0.10 mol), $PCl_5$ (31.20 g, 0.15 mol), and $POCl_3$ (200 mL) were added into a reaction flask, and then the above system was allowed to react overnight at 100 °C. After completion of the reaction, the system was cooled, and concentrated under reduced pressure. The residue was poured into ice water for quenching, and then the solution was extracted twice with ethyl acetate. The organic phase was dried over sodium sulfate, filtered, and concentrated. The residue was purified by column chromatography to obtain **compound 71D.**
MS (ESI) *m/z* 388.0 $(M+H)^+$.

**Step 5:** Preparation of ethyl 5-chloro-6-(4-chloro-2-fluorophenyl)-7-(dibenzylamino) pyrazolo[1,5-a]pyrimidin-3-carboxylate (**compound 71E**)

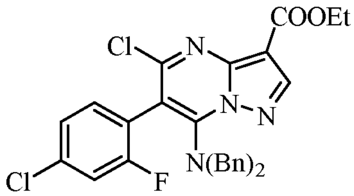

**71E**

Ethyl 5,7-dichloro-6-(4-chloro-2-fluorophenyl)pyrazolo[1,5-a]pyrimidin-3-carboxylate (6.00 g, 15.50 mmol) was dissolved in DCM (80 mL), to which were then added DIPEA (6.00 g, 46.50 mmol) and dibenzylamine (6.14 g, 31.00 mmol). After addition, the mixture was allowed to react at room temperature overnight. After completion of the reaction, the reaction mixture was concentrated under reduced pressure, and the residue was separated by column chromatography to provide **compound 71E.**
MS (ESI) m/z 549.1 $(M+H)^+$

**Step 6:** Preparation of ethyl (S)-2-(((2-((1-((tert-butyloxycarbonyl)amino)propan-2-yl)oxy)-5-fluoropyridin-3-yl)methyl)amino)-3-(4-chloro-2-fluorophenyl)-4-(dibenzylamino)pyrrolo[1,2-a]pyrimidin-8-carboxylate (compound **71F**)

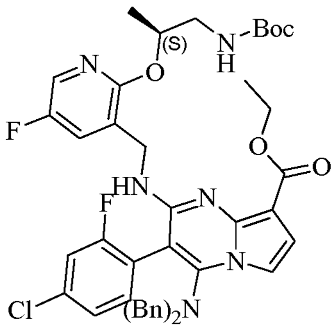

**71F**

Ethyl 5-chloro-6-(4-chloro-2-fluorophenyl)-7-(dibenzylamino)pyrazolo[1,5-a]pyrimidin-3-carboxylate (4.5 g, 8.2 mmol), tert-butyl (S)-(2-((3-(aminomethyl)-5-fluoropyridin-2-yl)oxy)propyl)carbamate (2.95 g, 9.84 mmol), ethanol (100 mL), and DIPEA (3.2 g, 24.60 mmol) were added into a reaction flask, and then the system was allowed to react at 80 °C for 24 h. After completion of the reaction, the system was cooled to room temperature, and concentrated under reduced pressure. The residue was purified by column chromatography to provide **compound 71F.**
MS (ESI) m/z 812.1 $(M+H)^+$

**Step 7:** Preparation of ethyl (S)-5-(((2-((1-((tert-butyloxycarbonyl)amino)propan-2-yl)oxy)-5-fluoropyridin-3-yl)methyl)amino)-7-(dibenzylamino)-6-(4-((diphenylmethylene)amino)-2-fluorophenyl) pyrazolo[1,5-a]pyrimidin-3-carboxylate (**compound 71G**)

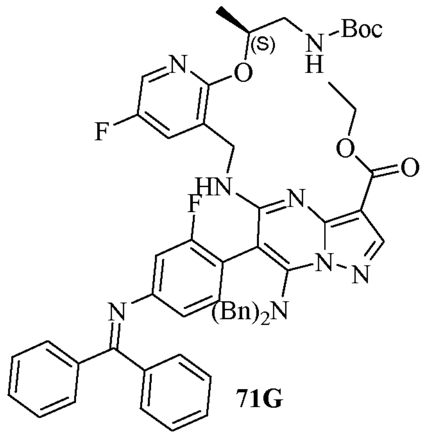

71G

Under nitrogen protection, ethyl (S)-2-(((2-((1-((tert-butyloxycarbonyl)amino)propan-2-yl) oxy)-5-fluoropyridin-3-yl)methyl)amino)-3-(4-chloro-2-fluorophenyl)-4-(dibenzylamino)pyrrolo[1,2-a] pyrimidin-8-carboxylate (1.70 g, 2.10 mmol), benzophenonimine (765 mg, 4.20 mmol), Xphos PdG3 (178 mg, 0.21 mmol), $K_3PO_4$ (1.34 g, 6.30 mmol), and 1,4-dioxane(50 mL) were added into a reaction flask, and then the above system was allowed to react overnight at 100 °C. After completion of the reaction, the reaction system was concentrated under reduced pressure. The residue was purified by column chromatography to obtain **compound 71G.**
MS (ESI) m/z 957.4 $(M+H)^+$

**Step 8:** Preparation of (S,$1^3$E,$1^4$E)-$1^6$-(4-amino-2-fluorophenyl)-$1^7$-(dibenzylamino)-$4^5$-fluoro-6-methyl-5-oxo-2,8-diaza-1(5,3)-pyrazolo[1,5-a]pyrimidin-4(3,2)-pyridinacyclononaphan-9-one **(compound 71H)**

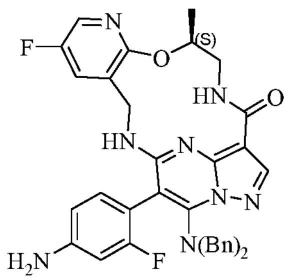

71H

Ethyl (S)-5-(((2-((1-((tert-butyloxycarbonyl)amino)propan-2-yl)oxy)-5-fluoropyridin-3-yl) methyl)amino)-7-(dibenzylamino)-6-(4-((diphenylmethylene)amino)-2-fluorophenyl)pyrazolo[1,5-a] pyrimidin-3-carboxylate (1.00 g, 1.05 mmol) was dissolved in a mixed solution of methanol (30 mL), THF (10 mL), and water (10 mL), to which was then added LiOH (504 mg, 21.00 mmol). After addition, the reaction system was heated to 60 °C and stirred for 16 h. Then, the reaction system was cooled to 0 °C, and the pH was adjusted to 2-3 with 2N hydrochloric acid. The resultant mixture was extracted with DCM. The organic phases were combined, dried over anhydrous sodium sulfate, and concentrated under reduced pressure. Then, the residue was dissolved in dichloromethane (10 mL), to which was added the solution of HCl in 1 ,4-dioxane (8 mL), and the resultant system was allowed to react at room temperature for 2 h. After completion of the reaction, the reaction mixture was concentrated under reduced pressure, to which were successively added DMF (170 mL), DCM (340 mL), DIPEA (5.00 g, 39.00 mmol), and FDPP (1.50 g, 3.90 mmol). After addition, the reaction system was stirred for 1 h at room temperature. After completion of the reaction, saturated $Na_2CO_3$ solution was added to quench the reaction, and then the resultant mixture was extracted twice with DCM:MeOH=(10:1). The organic phases were combined, dried over anhydrous sodium sulfate, filtered, and concentrated. The residue was purified by column chromatography, to obtain **compound 71H.**
MS (ESI) m/z 647.3 $(M+H)^+$

**Step 9:** Preparation of ethyl (4-((S,$1^3$E,$1^4$E)-$1^7$-(dibenzylamino)-$4^5$-fluoro-6-methyl-9-oxo-5-oxo-2,8-diaza-1(5,3)-pyrazolo[1,5-a] pyrimidin-4(3,2)-pyridinacyclononaphan-$1^6$-yl)-3-fluorophenyl)carbamate (**compound 71l**)

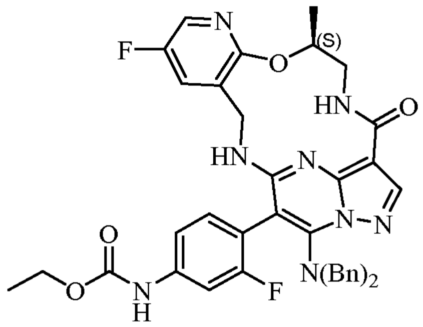

**71I**

(S,$1^3$E,$1^4$E)-$1^6$-(4-amino-2-fluorophenyl)-$1^7$-(dibenzylamino)-$4^5$-fluoro-6-methyl-5-oxo-2,8-diaza-1(5, 3)-pyrazolo[1,5-a]pyrimidin-4(3,2)-pyridinacyclononaphan-9-one (500 mg, 0.47 mmol), DIPEA (300 mg, 2.31 mmol), and DCM (20 mL) were added into a reaction flask, to which was added ethyl chloroformate (125 mg, 1.16 mmol) dropwise at 0 °C. After addition, the reaction system was allowed to react at room temperature overnight. After completion of the reaction, the reaction mixture was concentrated under reduced pressure, and the residue was purified by column chromatography to obtain **compound 71l.**
MS (ESI) m/z 719.3 $(M+H)^+$

**Step 10:** Preparation of ethyl (4-((S,$1^3$E,$1^4$E)-$1^7$-amino-$4^5$-fluoro-6-methyl-9-oxo-5-oxo-2,8-diaza-1(5,3)-pyrazolo[1,5-a]pyrimidin-4(3,2)-pyridinacyclononaphan-$1^6$-yl)-3-fluorophenyl)carbamate **(compound 71)**

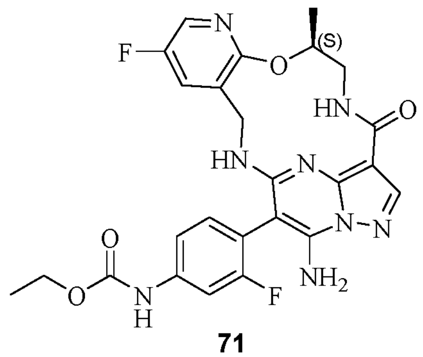

**71**

Ethyl (4-((S,$1^3$E,$1^4$E)-$1^7$-(dibenzylamino)-$4^5$-fluoro-6-methyl-9-oxo-5-oxo-2,8-diaza-1(5,3)-pyrazolo[1,5-a]pyrimidin-4(3,2)-pyridinacyclononaphan-$1^6$-yl)-3-fluorophenyl)carbamate (500 mg, 0.70 mmol) was dissolved in DCM (10 mL), to which was added trifluoromethanesulfonic acid (2 mL) dropwise at 0 °C. After addition, the reaction system was allowed to react at 0 °C for 1h. After completion of the reaction, triethylamine was added in an ice bath, and then the solution was concentrated under reduced pressure. The residue was purified by high pressure preparative separation to provide **compound 71.**

MS (ESI) m/z 539.2 $(M+H)^+$
$^1$H NMR (400 MHz, DMSO-$d_6$) $\delta$ 10.06 (m, 1H), 9.88 (d, *J* = 9.1 Hz, 1H), 8.06 (s, 1H), 8.00 (t, *J* = 2.5 Hz, 1H), 7.71 - 7.55 (m, 2H), 7.40 (m, 1H), 7.32 - 7.09 (m, 2H), 6.97 (s, 2H), 5.05 - 4.89 (m, 2H), 4.19 (q, *J* = 7.1 Hz, 2H), 4.01 (m, 1H), 3.84 (dd, *J* = 14.6, 5.3 Hz, 1H), 3.16 - 3.04 (m, 1H), 1.46 (d, *J* = 6.0 Hz, 3H), 1.29 (t, *J* = 7.1 Hz, 3H).

**Example 72:** The synthetic steps for preparation of ethyl (4-(($1^3E,1^4E$)-$1^7$-amino-$4^5$-fluoro-9-oxo-5-oxo-2,8-diaza-1(5,3)-pyrazolo[1,5-a]pyrimidin-4(3,2)-pyridinacyclononaphan-$1^6$-yl)phenyl)carbam ate (**compound 72**) were shown as follows:

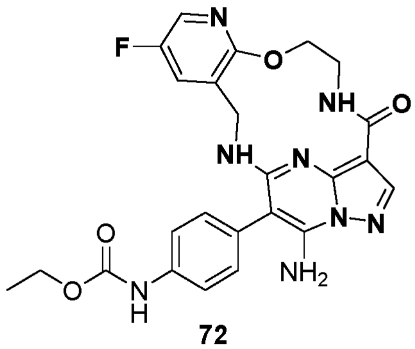

**72**

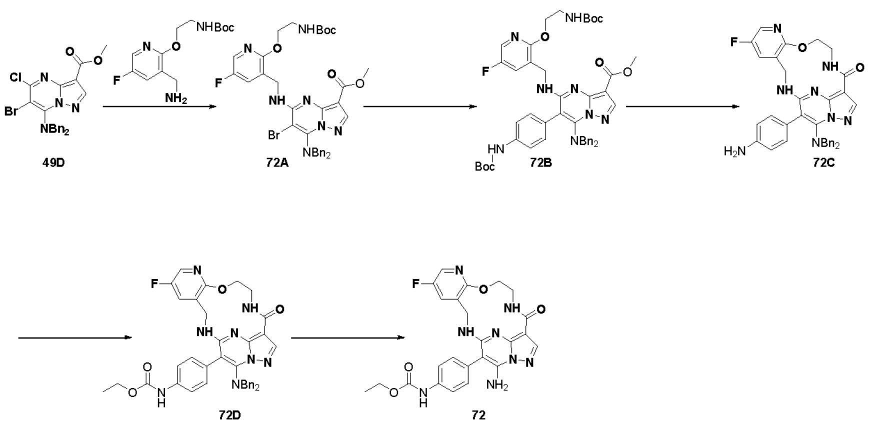

49D 72A 72B 72C 72D 72

**Step 1:** Preparation of methyl 6-bromo-5-((2-(2-((tert-butyloxycarbonyl)amino)ethoxy)-5-fluoropyridin-3-yl)methyl)amino)-7-(dibenzylamino)pyrazolo[1,5-a]pyrimidin-3-carboxylate **(compound 72A)**

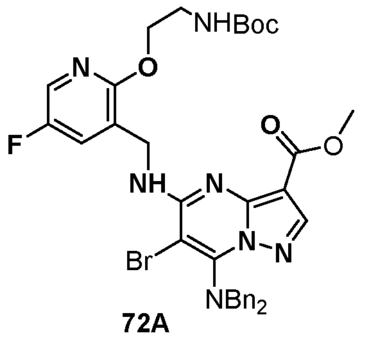

**72A**

Tert-butyl (2-((3-(aminomethyl)-5-fluoropyridin-2-yl)oxy)ethyl)carbamate (0.73 g, 2.55 mmol), methyl 6-bromo-5-chloro-7-(dibenzylamino)pyrazolo[1,5-a]pyrimidin-3-carboxylate (1.10 g, 2.23 mmol), N,N-diisopropylethylamine (0.86 g, 6.70 mmol), and ethanol (20 mL) were added into a reaction flask, and then the system was allowed to react overnight at 60 °C. After completion of the reaction, the reaction mixture was concentrated under reduced pressure. The residue was separated and purified by column chromatography to provide **compound 72A.**
MS (ESI) m/z 734.2 $(M+H)^+$

**Step 2:** methyl 5-((2-(2-((tert-butyloxycarbonyl)amino)ethoxy)-5-fluoropyridin-3-yl)methyl)amino)-6-(4-((tert-butyloxycarbonyl)amino)phenyl)-7-(dibenzylamino)pyrazolo[1,5-a]pyrimidin-3-carboxylate **(compound 72B)**

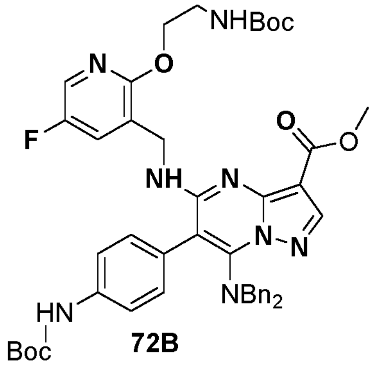

**72B**

Under nitrogen protection, methyl 6-bromo-5-((2-(2-((tert-butyloxycarbonyl)amino)ethoxy)-5-fluoropyridin-3-yl)methyl)amino)-7-(dibenzylamino)pyrazolo[1,5-a]pyrimidin-3-carboxylate (0.85 g, 1.16 mmol), N-Boc-4-aminophenylboronic acid pinacol ester (450 mg, 1.40 mmol), Xphos PdG3 (98 mg, 0.12 mmol), $K_3PO_4$(0.74 g, 3.48 mmol), 1,4-dioxane (16 mL), and water (4 mL) were added into a reaction flask, and then the reaction system was allowed to react overnight at 80 °C. After completion of the reaction, the reaction system was cooled to room temperature, and the reaction was quenched by adding water, followed by extraction with ethyl acetate. The organic phase was dried over sodium sulfate, filtered, and concentrated. The residue was purified by column chromatography to obtain **compound 72B.** MS (ESI) m/z 847.4 $(M+H)^+$

**Step 3:** Preparation of ($1^3$E,$1^4$E)-$1^6$-(4-aminophenyl)-$1^7$-(dibenzylamino)-$4^5$-fluoro-5-oxo-2,8-diaza-1(5,3)-pyrazolo[1,5-a]pyrimidin-4(3,2)-pyridinacyclononaphan-9-one (**compound 72C**)

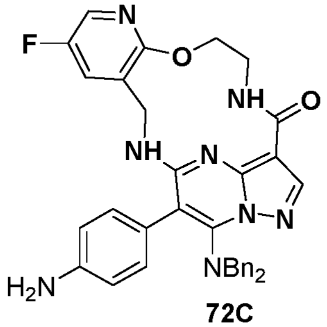

**72C**

Methyl 5-((2-(2-((tert-butyloxycarbonyl)amino)ethoxy)-5-fluoropyridin-3-yl)methyl)amino)-6-(4-((tert-butyloxycarbonyl)amino)phenyl)-7-(dibenzylamino)pyrazolo[1,5-a]pyrimidin-3-carboxylate (800 mg, 0.95 mmol) was dissolved in a mixed solution of methanol (27 mL), THF (9 mL), and water (9 mL), to which was then added LiOH (456 mg, 19.00 mmol). After addition, the reaction system was heated to 60 °C and stirred for 16 h. After completion of the reaction, the reaction system was cooled to 0 °C, and the pH was adjusted to 2-3 with 2N hydrochloric acid. The resultant mixture was extracted with ethyl acetate. The organic phases were combined, dried over anhydrous sodium sulfate, filtered and concentrated. Then, the crude product was dissolved in dichloromethane (10 mL), to which was added a solution of HCl in 1,4-dioxane (10 mL), and the resultant system was allowed to react at room temperature for 4 h. After completion of the reaction, the reaction mixture was concentrated under reduced pressure to obtain the crude product, to which were successively added DMF (150 mL), DCM (300 mL), DIPEA (5.00 g, 38.00 mmol), and FDPP (730 mg, 1.9 mmol). After addition, the reaction system was stirred for 1 h at room temperature. After completion of the reaction, $Na_2CO_3$ solution was added to quench the reaction. Then the resultant mixture was extracted twice with DCM:MeOH=(10:1). The organic phase was dried over anhydrous sodium sulfate, filtered, and concentrated. The residue was purified by column chromatography to obtain **compound 72C.**

MS (ESI) m/z 615.3 $(M+H)^+$

**Step 4:** Preparation of ethyl (4-(($1^3$E,$1^4$E)-$1^7$-(dibenzylamino)-$4^5$-fluoro-9-oxo-5-oxo-2,8-diaza-1(5,3)-pyrazolo[1,5-a]pyrimidin-4(3,2)-pyridinacyclononaphan-$1^6$-yl)phenyl)carbamate (**compound 72D**)

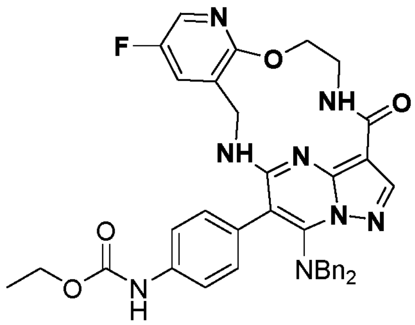

**72D**

($1^3$E,$1^4$E)-$1^6$-(4-aminophenyl)-$1^7$-(dibenzylamino)-$4^5$-fluoro-5-oxo-2,8-diaza-1(5,3)-pyrazolo[1,5-a] pyrimidin-4(3,2)-pyridinacyclononaphan-9-one (500 mg, 0.81 mmol), DIPEA (209 mg, 1.62 mmol), and DCM(20 mL) were added into a reaction flask, to which was added ethyl chloroformate (175 mg, 1.62 mmol) dropwise at 0 °C. After addition, the system was allowed to react at room temperature overnight. After completion of the reaction, the reaction mixture was concentrated under reduced pressure, and the residue was purified by column chromatography to obtain **compound 72D.** MS (ESI) m/z 687.3 $(M+H)^+$

**Step 5:** Preparation of ethyl (4-(($1^3$E,$1^4$E)-$1^7$-amino-$4^5$-fluoro-9-oxo-5-oxo-2,8-diaza-1(5,3)-pyrazolo[1,5-a]pyrimidin-4(3,2)-pyridinacyclononaphan-$1^6$-yl)phenyl)carbamate (**compound 72**)

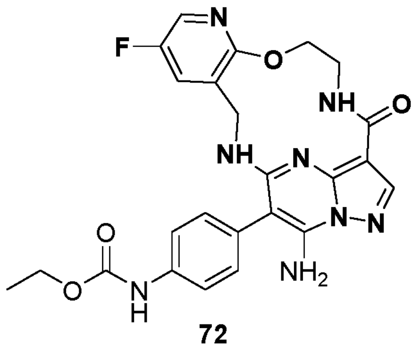

**72**

Ethyl (4-(($1^3$E,$1^4$E)-$1^7$-(dibenzylamino)-$4^5$-fluoro-9-oxo-5-oxo-2,8-diaza-1(5,3)-pyrazolo[1,5-a]pyrimidin-4(3,2)-pyridinacyclononaphan-$1^6$-yl)phenyl)carbamate (232 mg, 0.34 mmol) was dissolved in DCM (10 mL), to which was added trifluoromethanesulfonic acid (2 mL) dropwise at 0 °C. After addition, the reaction system was allowed to react at 0 °C for 1 h. After completion of the reaction, triethylamine was added in an ice bath, and then the reaction mixture was concentrated under reduced pressure. The residue was purified by high pressure preparative separation to provide **compound 72.**

MS (ESI) m/z 507.2 $(M+H)^+$
$^1$H NMR (400 MHz, DMSO-$d_6$) δ 9.85 (s, 1H), 9.75 (dd, *J* = 7.8, 2.5 Hz, 1H), 8.06 (s, 1H), 8.01 (d, *J* = 3.1 Hz, 1H), 7.75 - 7.66 (m, 3H), 7.27 (s, 2H), 6.94 (t, *J* = 6.2 Hz, 1H), 6.69 (s, 2H), 4.98 (m, 1H), 4.58 (m, 1H), 4.36 (m, 1H), 4.18 (q, *J* = 7.1 Hz, 2H), 3.90 (m, 2H), 3.43 (m, 1H), 1.28 (t, *J* = 7.1 Hz, 3H).

**Example 73:** The synthetic steps for preparation of ($1^3E$,$1^4E$)-$1^7$-(amino)-$4^5$-fluoro-$1^6$-phenyl-2,8-aza-1(5,3)-pyrazolo[1,5-a]pyrimidinyl-4(3,2)-pyridinacyclononaphan-9-one (**compound 73**) were shown as follows:

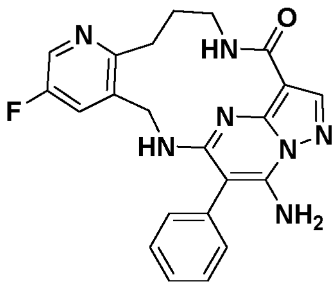

**73**

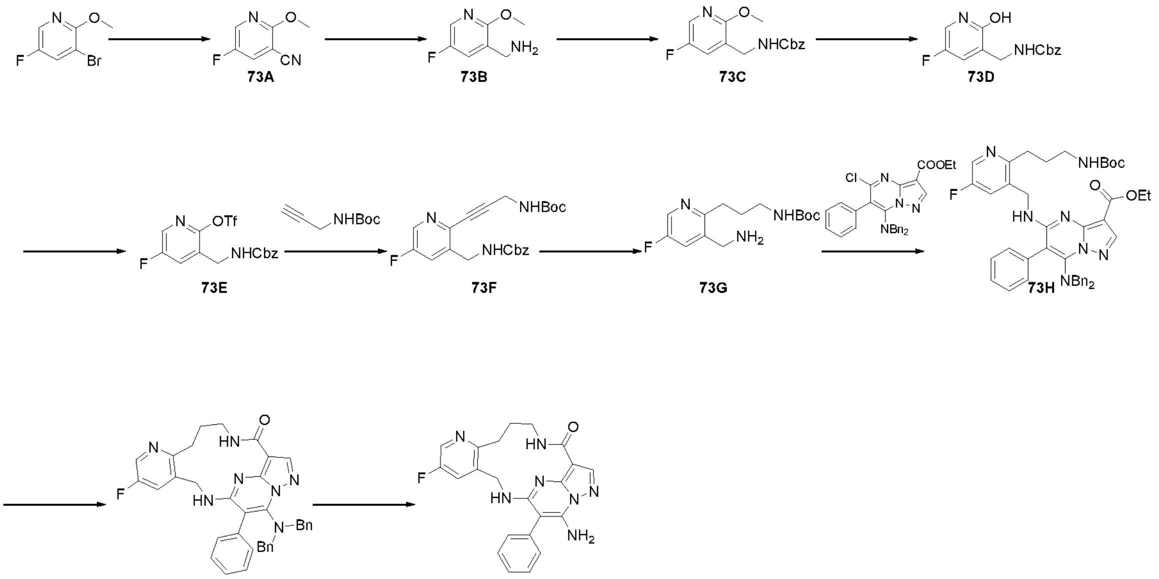


**Step 1:** Preparation of 5-fluoro-3-cyano-2-methoxypyridine (**compound 73A**)

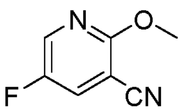

**73A**

Under nitrogen protection, 5-fluoro-3-bromo-2-methoxypyridine (14.2 g, 68.79 mmol), $Pd_2(dba)_3$ (1.89 g, 2.06 mmol), dppf (1.65 g, 2.95 mmol), zinc cyanide (16.1 g, 137.58 mmol), and zinc powder (134 mg, 2.06 mmol) were placed into a flask, to which was added anhydrous DMF (120 mL), and then the reaction system was heated to 100 °C and allowed to react for 13 h. After completion of the reaction, the reaction was quenched by adding water (100 mL), and then extracted with dichloromethane. The organic phases were combined, dried over anhydrous sodium sulfate, filtered, and concentrated. The residue was separated and purified by column chromatography to provide **compound 73A.**
MS (ESI) m/z 153.0 $(M+H)^+$

**Step 2:** Preparation of 5-fluoro-3-methylenamino-2-methoxypyridine (**compound 73B**)

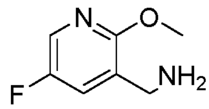

**73B**

5-Fluoro-3-cyano-2-methoxypyridine (8.5 g, 55.92 mmol) and a solution of ammonia in methanol (60 mL) were placed in a dry flask, to which was then added Raney-Ni (850 mg, 10%wt). The reaction system was replaced with hydrogen three times and stirred under hydrogen (2.0 MPa) for 12 h. After completion of the reaction, the reaction mixture was filtered with diatomite, and then the resultant filtrate was concentrated. The residue was separated and purified by column chromatography to provide **compound 73B.**
MS (ESI) m/z 157.1 $(M+H)^+$

**Step 3:** Preparation of (5-fluoro-2-methoxypyridinyl-3-)methylenaminobenzyloxycarbonyl ester (compound **73C**)

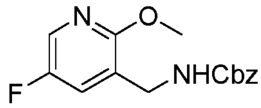

**73C**

5-Fluoro-3-methylenamino-2-methoxypyridine (8.7 g, 55.77 mmol) was dissolved in dichloromethane, to which was then added potassium carbonate (11.5 g, 53.7 mmol). Then, the reaction system was cooled to 0 °C, and benzyl chloroformate (11.8 mL, 83.7 mmol) was slowly added dropwise. After addition, the reaction system was warmed to room temperature and stirred for 2 h. After completion of the reaction, a small amount of water was added to quench the reaction. The resultant mixture was extracted with ethyl acetate, and the organic phase was dried over sodium sulfate, filtered, and concentrated. The residue was purified by column chromatography to obtain **compound 73C.**
MS (ESI) m/z 291.1 $(M+H)^+$

**Step 4:** Preparation of (5-fluoro-2-hydroxypyridinyl-3-)methylenaminobenzyloxycarbonyl ester **(compound 73D)**

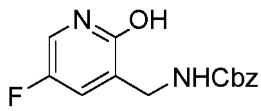

**73D**

(5-Fluoro-2-methoxypyridinyl-3-)methylenaminobenzyloxycarbonyl ester (6.93 g, 23.9 mmol) was dissolved in absolute ethanol (71 mL), to which was then added a solution of HCl in dioxane (4 M, 71 mL). The reaction system was heated to 75 °C and stirred for 3h. After completion of the reaction, sodium bicarbonate solid was added to neutralize the reaction mixture to pH=8, and then the resultant mixture was filtered. The filtrate was concentrated to provide **compound 73D**.
MS (ESI) m/z 277.1 $(M+H)^+$

**Step 5:** Preparation of 3-((benzyloxycarbonylamino)methylene)-5-fluoropyridin-2-yl-trifluoromethanesulfonate (**compound 73E**)

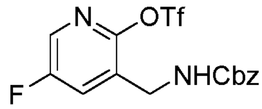

**73E**

(5-Fluoro-2-hydroxypyridinyl-3-)methylenamino benzyloxycarbonyl ester was dissolved in DCM (24 mL), to which was added triethylamine (6.7 mL, 47.8 mmol) in an ice bath, and then trifluoromethanesulfonic anhydride (6.0 mL, 35.8 mmol) was added to the above reaction mixture dropwise. After addition, the reaction system was further stirred at 0 °C for 30 min. After completion of the reaction, saturated sodium bicarbonate solution was added to the reaction mixture, and then the resultant mixture was extracted with dichloromethane. The organic phase was dried over anhydrous sodium sulfate, filtered, and concentrated. The residue was purified by column chromatography to obtain **compound 73E.**
MS (ESI) m/z 409.0 $(M+H)^+$

**Step 6:** Preparation of (3-(3-((benzyloxycarbonylamino)methylene)-5-fluoropyridin-2-yl) propyl-2-yn-1-yl)amino tert-butyloxycarbonyl ester **(compound 73F)**

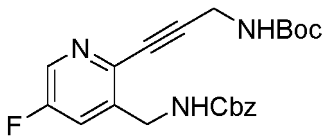

**73F**

3-((benzyloxycarbonylamino)methylene)-5-fluoropyridin-2-yl-trifluoromethanesulfonate (2.38 g, 5.83 mmol), propargylamino tert-butyloxycarbonyl ester (1.17 g, 7.58 mmol), CuI (43.7 mg, 0.23 mmol) and $Pd(PPh_3)_2Cl_2$ (161.5 mg, 0.23 mmol) were weighed and placed in a reaction flask, and after purging with nitrogen, anhydrous tetrahydrofuran (12 mL) and triethylamine (2.1 mL, 14.58 mmol) were added. Then, the reaction system was heated to 70 °C, and further stirred at this temperature for 18 h. After completion of the reaction, the reaction mixture was concentrated, and the residue was purified by column chromatography to obtain **compound 73F.**
MS (ESI) m/z 414.2 $(M+H)^+$

**Step 7:** Preparation of (3-(3-(aminomethyl)-5-fluoropyridin-2-yl)propyl)amino tert-butyloxycarbonyl ester (compound **73G)**

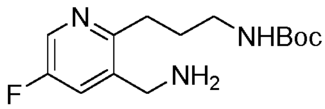

**73G**

(3-(3-((Benzyloxycarbonylamino)methylene)-5-fluoropyridin-2-yl)propyl-2-yn-1-yl)amino tert-butyloxycarbonyl ester (1.25 g, 3.03 mmol) was dissolved in anhydrous methanol (5 mL), to which was then added palladium on carbon (125 mg, 10%wt), after purging three times with hydrogen, the reaction system was heated to 40 °C, and stirred for 24 h at this temperature under 2.0 MPa hydrogen atmosphere. After completion of the reaction, the reaction mixture was filtered through diatomite, and the filtrate was concentrated. The residue was purified by column chromatography to obtain **compound 73G.**
MS (ESI) m/z 284.2 $(M+H)^+$

**Step 8:** Preparation of ethyl 5-(((2-(3-((tert-butyloxycarbonyl)amino)propyl)-5-fluoropyridin-3-yl) methylene)amino)-7-(dibenzylamino)-6-phenylpyrrolo[1,5-a]pyrimidin-3-carboxylate **(compound 73H)**

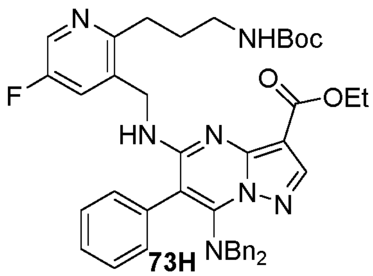

73H

(3-(3-(aminomethyl)-5-fluoropyridin-2-yl)propyl)amino tert-butyloxycarbonyl ester (471 mg, 1.66 mmol) was dissolved in n-butanol (17 mL), to which were then added ethyl 5-chloro-7-(dibenzylamino)-6-phenylpyrrolo[1,5-a]pyrimidin-3-carboxylate (1.24 g, 2.50 mmol) and diisopropylethylamine (0.55 mL, 3.32 mmol). After addition, the reaction system was heated to 120 °C, and further stirred at this temperature for 16 h. After completion of the reaction, the reaction mixture was concentrated, and the residue was purified by column chromatography to obtain **compound 73H.**
MS (ESI) m/z 744.4 $(M+H)^+$

**Step 9:** Preparation of ($1^3E$,$1^4E$)-$1^7$-(dibenzylamino)-$4^5$-fluoro-$1^6$-phenyl-2,8-aza-1(5,3)-pyrazolo[1,5-a]pyrimidin-4(3,2)-pyridinacyclononaphan-9-one **(compound 73I)**

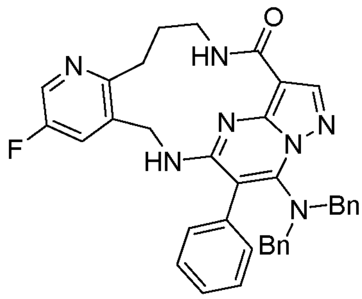

**73I**

Compound **73I** of Example 73 was obtained by the preparation method same as that of step **6** in Example **49.**
MS (ESI) m/z 598.4 $(M+H)^+$

**Step 10:** Preparation of ($1^3E$,$1^4E$)-$1^7$-(amino)-$4^5$-fluoro-$1^6$-phenyl-2,8-aza-1(5,3)-pyrazolo[1,5-a]pyrimidinyl-4(3,2)-pyridinacyclononaphan-9-one **(compound 73)**

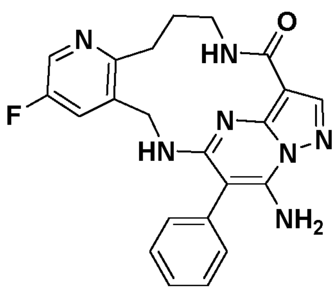

**73**

**Compound 73** of Example 73 was obtained by the preparation method same as that of step **10** in Example **49.**

MS (ESI) m/z 418.2 $(M+H)^+$
$^1$H NMR (400 MHz, DMSO-$d_6$) $\delta$ 8.44 (dd, $J$ = 7.5, 3.8 Hz, 1H), 8.39 (d, $J$ = 2.9 Hz, 1H), 8.03 (s, 1H), 7.66 - 7.57

(m, 2H), 7.52 (m, 2H), 7.42 (d, *J* = 7.4 Hz, 2H), 7.05 - 6.89 (m, 1H), 6.74 (s, 2H), 4.86 - 4.72 (m, 1H), 4.03 (dd, *J* = 15.2, 6.5 Hz, 1H), 3.61 (m, 2H), 3.17 (m, 1H), 2.82 (dd, *J* = 13.5, 8.3 Hz, 1H), 2.41 (m, 1H), 2.05 (m, 1H)

**Example 74:** The synthetic steps for preparation of (*S*,$1^3$*E*,$1^4$*E*)-$1^7$-amino-$4^5$-fluoro-$1^2$,6-dimethyl-$1^6$-phenyl-5-oxa-2,8-diaza-1(5,3)-pyrazolo[1,5-a]pyrimidinyl -4(3,2)-pyridinacyclononaphan-9-one **(compound 74)** were shown as follows:

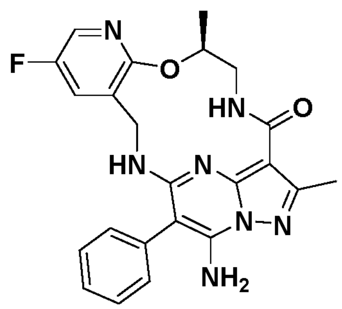

**74**

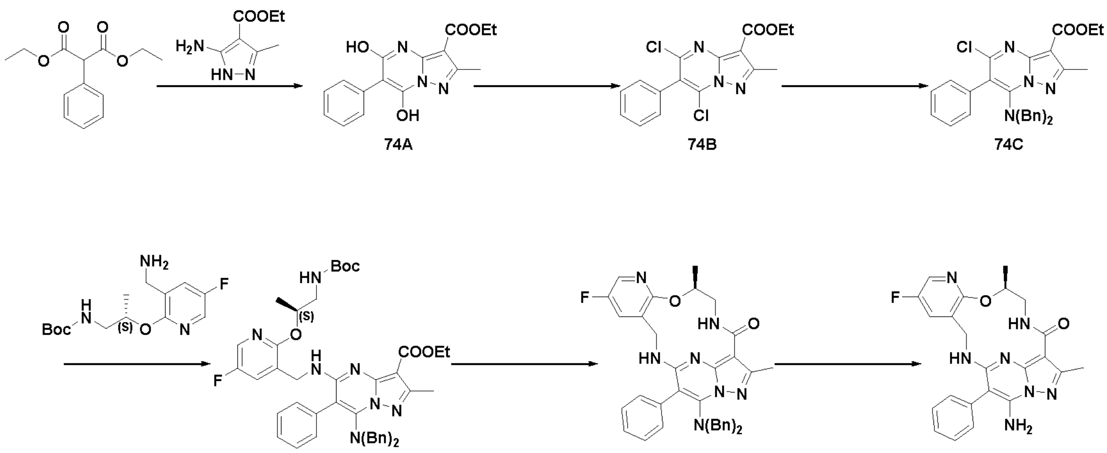

74A

74B

74C

74D

74E

74

**Step 1:** Preparation of ethyl 5,7-dihydroxyl-2-methyl-6-phenylpyrazolo[1,5-a]pyrimidin-3-carboxylate **(compound 74A)**

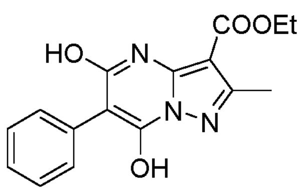

**74A**

Ethyl 5-amino-3-methyl-1H-pyrazol-4-carboxylate (5.2 g, 30.8 mmol) and diethyl 2-phenylmalonate (15.2 g, 61.6 mmol) were dissolved in DMF (31 mL), to which was added sodium ethoxide (4.2 g, 61.6 mmol) at room temperature. Then, the reaction system was heated to 100 °C and stirred for 16 h. After completion of the reaction, the reaction mixture was cooled to room temperature, and then diluted with 30 mL of absolute ethanol. The resultant mixture was filtered, and the filter cake was rinsed with absolute ethanol, to provide **compound 74A.**
MS (ESI) m/z 314.1 $(M+H)^+$

**Step 2:** Preparation of ethyl 5,7-dichloro-2-methyl-6-phenylpyrazolo[1,5-a]pyrimidin-3-carboxylate **(compound 74B)**

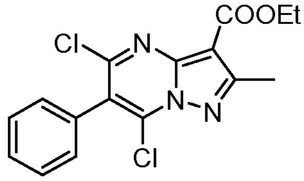

**74B**

Ethyl 5,7-dihydroxyl-2-methyl-6-phenylpyrazolo[1,5-a]pyrimidin-3-carboxylate (6.0 g, 16.9 mmol) and N,N-dimethylphenylamine were placed in a reaction flask, to which was then slowly added phosphorus oxychloride (28 mL) dropwise. After addition, the reaction system was heated to 110 °C, and reacted at this temperature for 15 h. After completion of the reaction, the reaction mixture was concentrated under reduced pressure, and after removing most of phosphorus oxychloride, the residual mixture was poured into ice water, and extracted with dichloromethane. The organic phase was dried over anhydrous sodium sulfate, filtered, and concentrated. The residue was separated and purified by column chromatography to provide **compound 74B.**
MS (ESI) m/z 350.0 $(M+H)^+$

**Step 3:** Preparation of ethyl 5-chloro-7-(dibenzylamino)-2-methyl-6-phenylpyrazolo[1,5-a]pyrimidin-3-carboxylate **(compound 74C)**

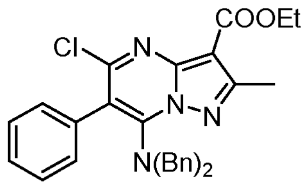

**74C**

Ethyl 5,7-dichloro-2-methyl-6-phenylpyrazolo[1,5-a]pyrimidin-3-carboxylate (2.0 g, 5.7 mmol) was dissolved in anhydrous dichloromethane (11 mL), to which was then added dibenzylamine (1.24 g, 6.3 mmol) and diisopropylethylamine (1.88 mL, 11.4 mmol), and then the reaction system was stirred at room temperature for 24 h. After completion of the reaction, the reaction mixture was concentrated under reduced pressure, and the residue was separated by high pressure preparative separation to provide **compound 74C.**
MS (ESI) m/z 511.2 $(M+H)^+$

**Step 4:** Preparation of ethyl (S)-5-(((2-((1-((tert-butyloxycarbonyl)amino)propan)-2-oxa)-5-fluoropyridin-3-yl)methylene)amino)-7-(dibenzylamino)-2-methyl-6-phenylpyrazolo[1,5-a]pyrimidin-3-carboxylate **(compound 74D)**

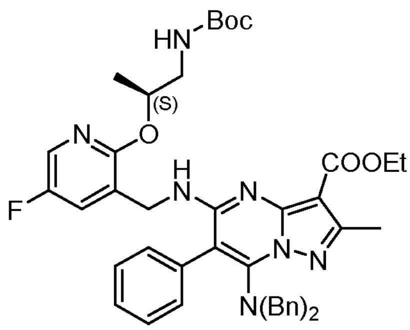

**74D**

Ethyl 5-chloro-7-(dibenzylamino)-2-methyl-6-phenylpyrazolo[1,5-a]pyrimidin-3-carboxylate (1.64 g, 3.2 mmol), (S)-(2-((3-(methylamino)-5-fluoropyridin-2-yl)oxa)propyl)amino tert-butyloxycarbonyl (1.72 g, 5.8 mmol) and DIPEA (1.7 mL, 9.6 mmol) were dissolved in absolute ethanol (10 mL), and then the reaction system was heated to 120 °C and reacted for 12 h. After completion of the reaction, the reaction mixture was diluted by adding water, and the aqueous phase was extracted with dichloromethane. The organic phases were combined, dried over anhydrous sodium sulfate, filtered, and concentrated. The residue was separated and purified by column chromatography to provide **compound 74D.**
MS (ESI) m/z 774.4 $(M+H)^+$

**Step 5:** Preparation of (*S*,$1^3$*E*,$1^4$*E*)-$1^7$-(dibenzylamino)-$4^5$-fluoro-$1^2$,6-dimethyl-$1^6$-pheny1-5-oxa-2,8-diaza-1(5,3)-pyrazolo[1,5-a]pyrimidinyl-4(3,2)-pyridinacyclononaphan-9-one **(compound 74E)**

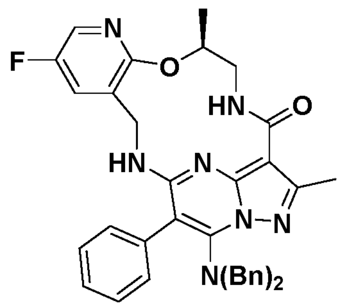

**74E**

**Compound 74E** of Example 74 was obtained by the preparation method same as that of step **6** in Example **49.**
MS (ESI) m/z 628.3 $(M+H)^+$

**Step 6:** Preparation of (*S*,$1^3$*E*,$1^4$*E*)-$1^7$-amino-$4^5$-fluoro-$1^2$,6-dimethyl-$1^6$-phenyl-5-oxa-2,8-diaza-1(5,3)-pyrazolo[1,5-a]pyrimidinyl-4(3,2)-pyridinacyclononaphan-9-one **(compound** 74)

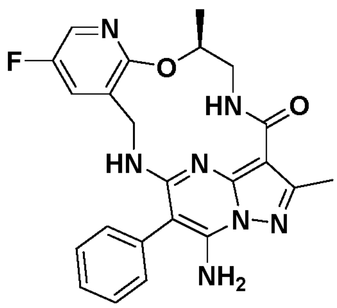

**74**

**Compound 74** of Example 74 was obtained by the preparation method same as that of step **10** in Example **49.**

MS (ESI) m/z 448.2 $(M+H)^+$

$^1$H NMR (400 MHz, DMSO-$d_6$) δ 9.98 - 9.84 (m, 1H), 8.01 (d, *J* = 3.1 Hz, 1H), 7.70 (dd, *J* = 9.1, 3.1 Hz, 1H), 7.60 (t, *J* = 7.5 Hz, 2H), 7.51 (m, 1H), 7.34 (s, 2H), 6.93 (t, *J* = 6.1 Hz, 1H), 6.60 (s, 2H), 5.07 - 4.90 (m, 2H), 4.02 (m, 1H), 3.87 (dd, *J* = 14.6, 5.7 Hz, 1H), 3.10 (m, 1H), 2.48 (s, 3H), 1.47 (d, *J* = 6.1 Hz, 3H).

**Example 75:** The synthetic steps for preparation of (S,$1^3$E,$1^4$E)-$1^7$-amino-$4^5$-fluoro-6,8-dimethyl-$1^6$-phenyl-5-oxo-2,8-diaza-1(5,3)-pyrazolo[1,5-a]pyrimidin-4(3,2)-pyridinacyclononaphan-9-one **(compound 75)** were shown as follows:

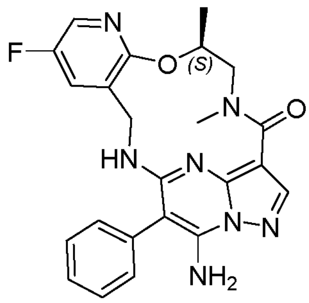

**75**

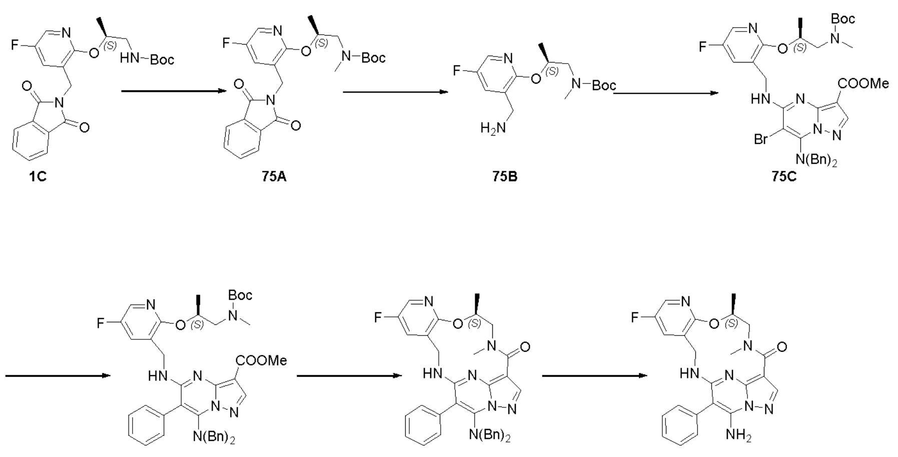

1C

75A

75B

75C

75D

75E

75

**Step 1:** Preparation of tert-butyl (S)-(2-(((3-((1,3-dioxoisoindol-2-yl)methyl)-5-fluoropyridin-2-2-yl)oxy)propyl)(methyl)carbamate **(compound 75A)**

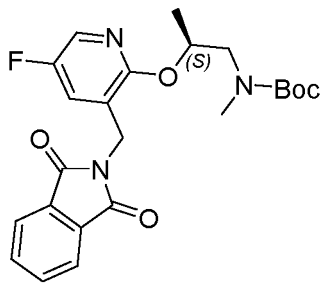

**75A**

Under nitrogen protection, tert-butyl (S)-(2-((3-((1,3-dioxoisoindol-2-yl)methyl)-5-fluoropyridin-2-2-yl)oxy)propyl)carbamate (2.0 g, 4.66 mmol) was dissolved in anhydrous DMF (80 mL), and then the system was cooled to 0°C, to which was added sodium hydride (0.75 g, 9.31 mmol) in portions. After addition, the reaction mixture was stirred at 0 °C for 0.5 h, and then iodomethane (0.99 g, 6.99 mmol) was added. The reaction mixture was futher stirred for 4 h. After completion of the reaction, saturated ammonium chloride solution was slowly added to the reaction system to quench the reaction, and then the resultant mixture was extracted with ethyl acetate. The organic phases were combined, and washed twice with saturated brine. The organic phase was dried over anhydrous sodium sulfate, filtered, and concentrated. The residue was purified by column chromatography to provide **compound 75A.**
MS (ESI) m/z 444.2 $(M+H)^+$

**Step 2:** Preparation of tert-butyl (S)-(2-((3-(aminomethyl)-5-fluoropyridin-2-yloxy)propyl)(methyl) carbamate **(compound 75B)**

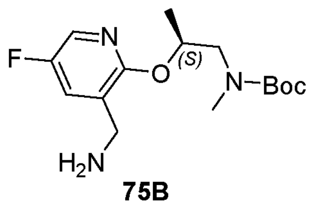

**75B**

tert-Butyl (S)-(2-(((3-((1,3-dioxoisoindol-2-yl)methyl)-5-fluoropyridin-2-2-yl)oxy)propyl) (methyl)carbamate (1.5 g, 3.38 mmol) was dissolved in absolute ethanol (75 mL), to which was added hydrazine hydrate (1.06 g, 16.9 mmol), and then the reaction system was stirred at 80 °C for 5 h. After completion of the reaction, the reaction system was cooled to room temperature, filtered, and concentrated to provide **compound 75B.**
MS (ESI) m/z 314.2 $(M+H)^+$

**Step 3:** Preparation of methyl (S)-6-bromo-5-((2-((1-((tert-butyloxycarbonyl)(methyl)amino)propan-2-yl)oxy)-5-fluoropyridin-3-yl)methyl)amino)-7-(dibenzylamino)pyrazolo[1,5-a]pyrimidin-3-carboxylate **(compound 75C)**

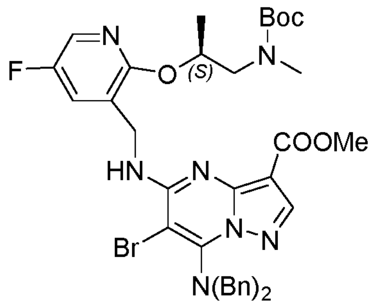

**75C**

Tert-butyl (S)-(2-((3-(aminomethyl)-5-fluoropyridin-2-yloxy)propyl)(methyl)carbamate (0.8 g, 2.55 mmol) and methyl 6-bromo-5-chloro-7-(dibenzylamino)pyrazolo[1,5-a]pyrimidin-3-carboxylate (1.24 g, 2.55 mmol) were dissolved in absolute ethanol (60 mL), to which was then added diisopropylethylamine (0.66 g, 5.10 mmol), and the reaction system was stirred at 60 °C for 18 h. After completion of the reaction, the reaction mixture was concentrated under reduced pressure, and the residue was separated and purified by column chromatography to provide **compound 75C.**
MS (ESI) m/z 762.2 $(M+H)^+$

**Step 4:** Preparation of methyl (S)-5-((2-((1-((tert-butyloxycarbonyl)(methyl)amino)propan-2-yl) oxy)-5-fluoropyridin-3-yl)methyl)amino)-7-(dibenzylamino)-6-phenylpyrazolo[1,5-a]pyrimidin-3-carboxylate **(compound 75D)**

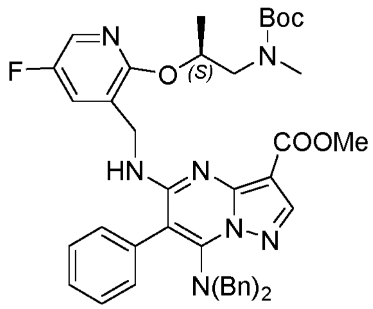

**75D**

Under nitrogen protection, methyl (S)-6-bromo-5-((2-((1-((tert-butyloxycarbonyl)(methyl)amino) propan-2-yl)oxy)-5-fluoropyridin-3-yl)methyl)amino)-7-(dibenzylamino)pyrazolo[1,5-a]pyrimidin-3 -carboxylate (1.4 g, 1.84 mmol), phenylboronic acid (0.34 g, 2.75 mmol), $Pd(dppf)Cl_2$ (132.4 mg, 0.18 mmol), and $K_2CO_3$ (0.51 g, 3.68 mmol) were dissolved in dioxane (40 mL) and water (5 mL), and then the reaction system was stirred at 100 °C for 16 h. After completion of the reaction, the reaction mixture was concentrated, the residue was purified by column chromatography to provide **compound 75D.**
MS (ESI) m/z 760.4 $(M+H)^+$

**Step 5:** Preparation of (S,$1^3$E,$1^4$E)-$1^7$-(dibenzylamino)-$4^5$-fluoro-6,8-dimethyl-$1^6$-phenyl-5-oxo-2,8-diaza-1(5,3)-pyrazolo[1,5-a]pyrimidin-4(3,2)-pyridinacyclononaphan-9-one **(compound 75E)**

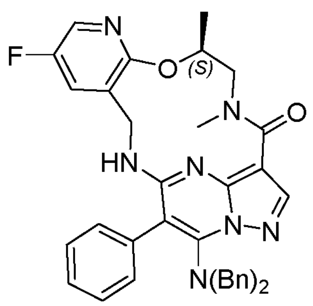

**75E**

**Compound 75E** of Example 75 was obtained by the preparation method same as that of step **6** in Example **49.**
MS (ESI) m/z 628.3 $(M+H)^+$

**Step 6:** Preparation of (S,$1^3$E,$1^4$E)-$1^7$-amino-$4^5$-fluoro-6,8-dimethyl-$1^6$-phenyl-5-oxo-2,8-diaza-1(5,3)-pyrazolo[1,5-a]pyrimidin-4(3,2)-pyridinacyclononaphan-9-one **(compound 75)**

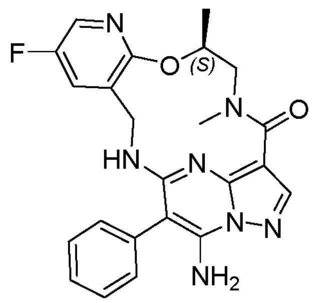

**75**

**Compound 75** of Example 75 was obtained by the preparation method same as that of step **10** in Example **49.**

MS (ESI) m/z 448.2 $(M+H)^+$

$^1$H NMR (400 MHz, DMSO-$d_6$) $\delta$ 7.95 (s, 1H), 7.89-7.85 (d, $J$ = 16.0 Hz, 1H), 7.65-7.62 (m, 1H), 7.58-7.56 (t, $J$ = 8.0 Hz, 2H), 7.49 (m, 1H), 7.32 (s, 2H), 6.77 (s, 1H), 6.45 (s, 2H), 5.21 (s, 1H), 4.83 (m, 1H), 4.27-4.24 (d, $J$ = 12.0 Hz, 1H), 3.79-3.76 (dd, $J$ = 12.0 Hz, 1H), 3.32 (s, 3H), 2.99 (m, 1H), 1.40-1.38 (d, $J$ = 8.0 Hz, 3H).

**Example 76:** The synthetic steps for preparation of ($S$,1$^3E$,1$^4E$)-1$^2$-bromo-1$^6$-phenyl-1$^7$-amino-4$^5$-fluoro-6-methyl-5-oxa-2,8-diaza-1 (5,3)-pyrazolo[1,5-a]pyrimidinyl-4(3,2)-pyridinacyclononaphan-9-one **(compound 76)** were shown as follows:

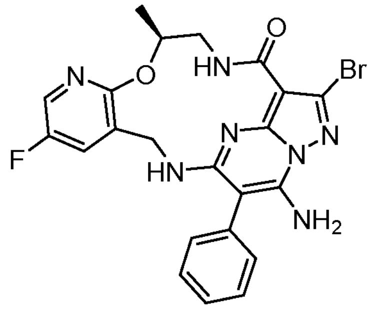

**76**

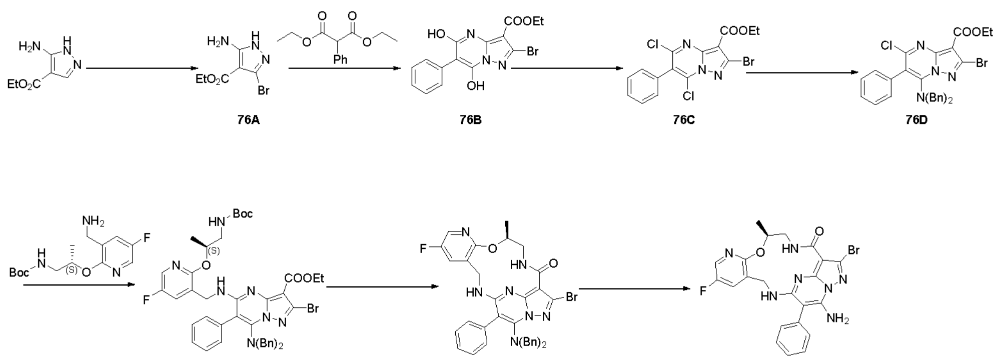

76A 76B 76C 76D

76E 76F 76

**Step 1:** Preparation of ethyl 5-amino-3-bromo-1H-pyrazol-4-carboxylate **(compound 76A)**

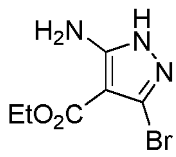

**76A**

Ethyl 5-amino-1H-pyrazol-4-carboxylate (9.3 g, 60.0 mmol) was dissolved in DCM (50.0 mL), to which was then added NBS (16.0g, 90.0 mmol). After addition, the system was further stirred and reacted at room temperature for 16 h. After completion of the reaction, the reaction mixture was quenched with sodium bisulfite solution, and then extracted with DCM. The organic phases were combined, dried over anhydrous sodium sulfate, filtered, and concentrated. The residue was purified by column chromatography to provide **compound 76A.**
MS (ESI) m/z 234.0 $(M+H)^+$

**Step 2:** Preparation of ethyl 2-bromo-5,7-dihydroxyl-6-phenylpyrazolo[1,5-a]pyrimidin-3-carboxylate **(compound 76B)**

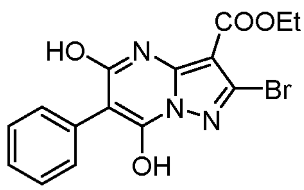

**76B**

Ethyl 5-amino-3-bromo-1H-pyrazol-4-carboxylate (7.5 g, 32.23 mmol) was dissolved in ethanol, to which were successively added diethyl 2-phenylmalonate (15.2 g, 64.45 mmol) and sodium ethoxide (6.6 g, 96.68 mmol) in an ice water bath, and then the reaction system was heated to 90 °C and stirred for 16 h. After completion of the reaction, the reaction system was cooled to 0 °C, and filtered. The filter cake was rinsed with ethanol. The solid was dissolved in water, and then HCl was added to adjust pH to 1. A lot of soild was precipitated and filtered. The filter cake was rinsed with water, and then dried to provide **compound 76B.**
MS (ESI) m/z 378.0 $(M+H)^+$

**Step 3:** Preparation of ethyl 2-bromo-5,7-dichloro-6-phenylpyrazolo[1,5-a]pyrimidin-3-carboxylate **(compound 76C)**

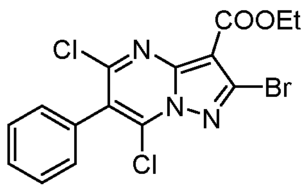

**76C**

At 0 °C, to a reaction flask containing ethyl 2-bromo-5,7-dihydroxcyl-6-phenylpyrazolo[1,5-a]pyrimidin-3-carboxylate (7.3 g, 19.36 mmol), was added $POCl_3$ (100 mL), and then *N,N*-dimethylphenylamine (4.7 g, 38.7 mmol) was slowly added dropwise. After addition, the reaction system was heated to 80°C, and reacted at this temperature for 16 h. After completion of the reaction, the reaction mixture was slowly poured into ice water to quench the reaction, and then extracted with dichloromethane. The organic phases were combined, dried over anhydrous sodium sulfate, filtered, and concentrated. The residue was separated and purified by column chromatography to provide **compound 76C.**
MS (ESI) m/z 413.9 $(M+H)^+$

**Step 4:** Preparation of ethyl 2-bromo-5-chloro-7-(dibenzylamino)-6-phenylpyrazolo[1,5-a]pyrimidin-3-carboxylate **(compound 76D)**

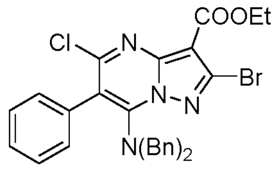

**76D**

Ethyl 2-bromo-5,7-dichloro-6-phenylpyrazolo[1,5-a]pyrimidin-3-carboxylate (4.8 g, 11.6 mmol) was dissolved in a mixed solution of 1,4-dioxane and dichloromethane (dioxane/DCM = 15.0 ml/30.0 ml), and then DIPEA (3.0 g, 23.2 mmol) and dibenzylamine (2.9g, 15.1mmol) were successively added. After addition, the reaction system was stirred at room temperature for 3 h. After completion of the reaction, the reaction mixture was concentrated, and the residue was purified by column chromatography to provide **compound 76D.**
MS (ESI) m/z 575.1 $(M+H)^+$

**Step 5:** ethyl (*S*)-2-bromo-5-(((2-((1-(tert-butyloxycarbonylamino)propan-2-yl)oxy)-5-fluoropyridin-3-ylmethyleneamino)-7-(dibenzylamino)-6-phenylpyrazolo[1,5-a]pyrimidin-3-carboxylate **(compound 76E)**

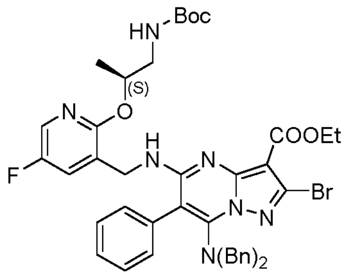

**76E**

Ethyl 2-bromo-5-chloro-7-(dibenzylamino)-6-phenylpyrazolo[1,5-a]pyrimidin-3-carboxylate (1.5 g, 2.61 mmol) was dissolved in n-butanol (26 mL), to which were then added DIPEA (673 mg, 5.22 mmol) and (*S*)-(2-((3-(aminomethylene)-5-fluoropyridin-2-yl)oxy)propyl)amino tert-butyloxycarbonyl ester (1.56 g, 5.22 mmol). The reaction system was heated to 100 °C and then stirred for 16. After completion of the reaction, the reaction mixture was concentrated, and the residue was purified by column chromatography to provide **compound 76E.**
MS (ESI) m/z 838.3 $(M+H)^+$

**Step 6:** Preparation of (*S*,$1^3$*E*,$1^4$*E*)-$1^7$-(dibenzylamino)-$4^5$-fluoro-$1^2$,6-dimethyl-$1^2$-bromo-$1^6$-phenyl-5-oxa-2,8-diaza-1(5,3)-pyrazolo[1,5-a]pyrimidinyl-4(3,2)-pyridinacyclononaphan-9-one **(compound 76F)**

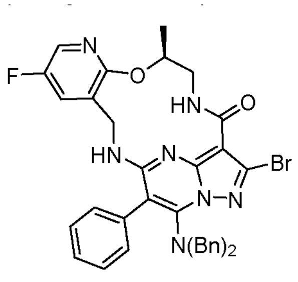

75F

**Compound 75F** of Example 76 was obtained by the preparation method same as that of step **6** in Example **49.** MS (ESI) m/z 628.3 (M+H)+

**Step 7:** Preparation of (*S*,$1^3$*E*,$1^4$*E*)-$1^2$-bromo-$1^6$-phenyl-$1^7$-amino-$4^5$-fluoro-6-methyl-5-oxa-2,8-diaza-1(5,3)-pyrazolo[1,5-a]pyrimidinyl-4(3,2)-pyridinacyclononaphan-9-one **(compound 76)**

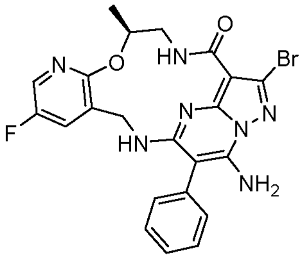

**76**

**Compound 76** of Example 76 was obtained by the preparation method same as that of step 10 in Example **49.**

MS (ESI) m/z 512.1 (M+H)+

$^1$H NMR (400 MHz, DMSO-$d_6$) δ 9.86 (d, *J* = 8.8 Hz, 1H), 8.00 (d, *J* = 3.1 Hz, 1H), 7.67 (dd, *J* = 8.9, 3.1 Hz, 1H), 7.58 (t, *J* = 7.6 Hz, 2H), 7.50 (m, 1H), 7.34 (s, 2H), 7.06 (q, *J* = 6.2, 5.8 Hz, 1H), 6.82 (s, 2H), 5.07 - 4.86 (m, 2H), 4.00 (m, 1H), 3.88 (dd, *J* = 14.7, 5.8 Hz, 1H), 3.10 (dd, *J* = 13.0, 10.3 Hz, 1H), 1.45 (d, *J* = 6.1 Hz, 3H).

**Example 77:** The synthetic steps for preparation of (*S*,$1^3$*E*,$1^4$*E*)-$1^2$-cyano-$1^6$-phenyl-$1^7$-amino-$4^5$-fluoro-6-methyl-5-oxa-2,8-diaza-1(5,3)-pyrazolo[1,5-a]pyrimidinyl-4(3,2)-pyridinacyclononaphan-9-on e (compound 77) were shown as follows:

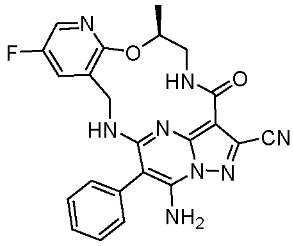

**77**

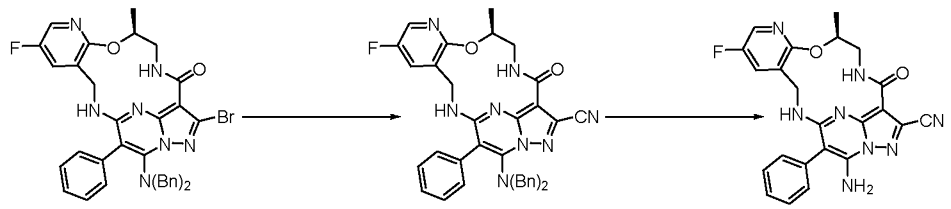

76F 77A 77

**Step 1:** Preparation of ($S$,1$^3E$,1$^4E$)-1$^2$-cyano-1$^6$-phenyl-1$^7$-dibenzylamino-4$^5$-fluoro-6-methyl-5-oxa-2,8-diaza-1(5,3)-pyrazolo[1,5-a]pyrimidinyl-4(3,2)-pyridinacyclononaphan-9-one **(compound 77A)**

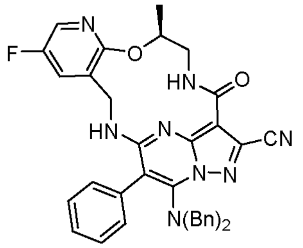

**77A**

Under nitrogen protection, ($S$,1$^3E$,1$^4E$)-1$^2$-bromo-1$^6$-phenyl-1$^7$-(dibenzylamino)-4$^5$-fluoro-6-methyl-5-oxa-2,8-diaza-1(5,3)-pyrazolo[1 ,5-a]pyrimidinyl-4(3,2)-pyridinacyclononaphan-9-one (279 mg, 0.44 mmol) and cuprous cyanide (79.2 mg, 0.88 mmol) were dissolved in DMSO (5.0 mL), and then the reaction system was heated to 150 °C, and stirred at this temperature for 16 h. After completion of the reaction, the reaction was quenched by adding water, and the resultant mixture was extracted with dichloromethane. The organic phases were combined, dried over anhydrous sodium sulfate, filtered, and concentrated. The residue was separated and purified by column chromatography to provide **compound 77A.**
MS (ESI) m/z 639.3 (M+H)$^+$

**Step 2:** Preparation of ($S$,1$^3E$,1$^4E$)-1$^2$-cyano-1$^6$-phenyl-1$^7$-amino-4$^5$-fluoro-6-methyl-5-oxa-2,8-diaza-1(5,3)-pyrazolo[1,5-a]pyrimidinyl-4(3,2)-pyridinacyclononaphan-9-one **(compound 77)**

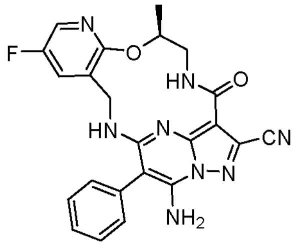

**77**

**Compound 77** of Example 77 was obtained by the preparation method same as that of step **10** in Example **49.**

MS (ESI) m/z 459.2 (M+H)$^+$
$^1$H NMR (400 MHz, DMSO-$d_6$) δ 9.88 (d, $J$ = 8.9 Hz, 1H), 8.01 (d, $J$ = 3.0 Hz, 1H), 7.69 (dd, $J$ = 8.8, 3.1 Hz, 1H), 7.61 (t, $J$ = 7.5 Hz, 2H), 7.53 (t, $J$ = 7.4 Hz, 1H), 7.35 (d, $J$ = 17.4 Hz, 2H), 7.22 (t, $J$ = 6.2 Hz, 1H), 7.06 (s, 2H), 5.04 (m, 1H), 4.92 (m, 1H), 4.02 (m, 1H), 3.92 (dd, $J$ = 14.7, 5.8 Hz, 1H), 3.16 (dd, $J$ = 13.1, 10.3 Hz, 1H), 1.47 (d, $J$ = 6.1 Hz, 3H).

**Example 78:** The synthetic steps for preparation of ($6^3$E,$6^4$E)-$6^7$-amino-$1^5$-fluoro-$6^6$-phenyl-2-oxo-4,7-diaza-6(3,5)-pyrazolo[1,5-a]pyrimidin-1(2,3)-pyridin-3( 1,2)-cyclopentanacyclooctaphan-5-one **(compound 78)** were shown as follows:

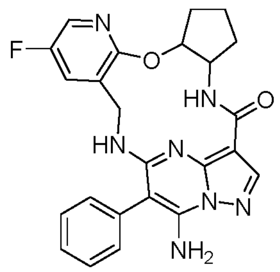

**78**

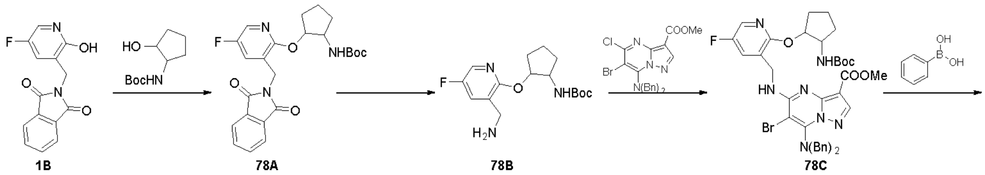

1B 78A 78B 78C

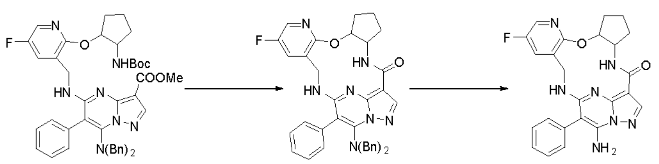

78D 78E 78

**Step 1:** Preparation of tert-butyl (2-((3-((1,3-dioxooctanol-2-yl)methyl)-5-fluoropyridin-2-yl)oxy) cyclopentyl)carbamate **(compound 78A)**

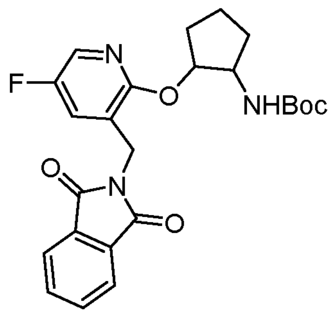

**78A**

Under nitrogen protection, 2-((5-fluoro-2-hydroxypyridin-3-yl)methyl)isoindolin-1,3-dione (2.0 g, 7.35 mmol) and tert-butyl (2-hydroxycyclopentyl)carbamate (1.63 g, 8.08 mmol) were dissolved in anhydrous THF (100 mL), and then the solution was cooled to 0°C, followed by addition of DEAD (2.56 g, 14.7 mmol) and $PPh_3$ (3.84 g, 14.7 mmol). The reaction mixture was stirred at 0 °C for 0.5 h, and then warmed to room temperature and stirred overnight. After completion of the reaction, the reaction was quenched by adding water. The aqueous phase was extracted with ethyl acetate. The organic phases were combined, washed with saturated brine, dried over anhydrous sodium sulfate, filtered, and concentrated. The residue was purified by column chromatography to provide **compound 78A.**
MS (ESI) m/z 456.2 $(M+H)^+$

**Step 2:** Preparation of tert-butyl (2-((3-(aminomethyl)-5-fluoropyridin-2-yl)oxy)cyclopentyl)carbamate **(compound 78B)**

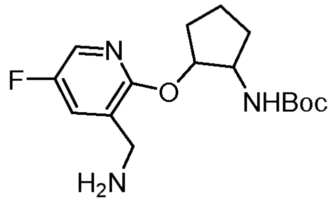

**78B**

(tert-Butyl(2-((3-((1,3-dioxooctanol-2-yl)methyl)-5-fluoropyridin-2-yl)oxy)cyclopentyl)carbamate (2.1 g, 4.61 mmol) was dissolved in absolute ethanol (70 mL), to which was then added hydrazine hydrate (1.36 g, 23.05 mmol), and then the reaction system was stirred at 80 °C for 3 h. After completion of the reaction, the reaction mixture was filtered and concentrated, to provide **compound 78B.**
MS (ESI) m/z 326.2 $(M+H)^+$

**Step 3:** Preparation of methyl 6-bromo-5-((2-((2-((tert-butyloxycarbonyl)amino)cyclopentyl) oxy)-5-fluoropyridin-3-yl)methyl)amino)-7-(dibenzylamino)pyrazolo[1,5-a]pyrimidin-3-carboxylate **(compound 78C)**

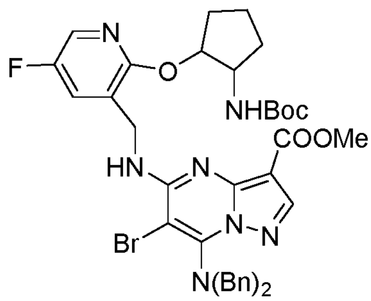

**78C**

tert-Butyl (2-((3-(aminomethyl)-5-fluoropyridin-2-yl)oxy)cyclopentyl)carbamate (1.33 g, 4.07 mmol) and methyl 6-bromo-5-chloro-7-(dibenzylamino)pyrazolo[1,5-a]pyrimidin-3- carboxylate (1.98 g, 4.07 mmol) were dissolved in absolute ethanol (60 mL), to which was then added DIPEA (1.05 g, 8.14 mmol), and the mixture was stirred at 60 °C for 16 h. After completion of the reaction, the reaction mixture was concentrated to provide the crude product, which was separated and purified by column chromatography to provide **compound 78C.**
MS (ESI) m/z 774.2 $(M+H)^+$

**Step 4:** Preparation of methyl 5-((2-((2-((tert-butyloxycarbonyl)amino)cyclopentyl)oxy)-5-fluoropyridin-3-yl)methyl)amino)-7-(dibenzylamino)-6-phenylpyrazolo[1,5-a]pyrimidin-3-carboxylate **(compound 78D)**

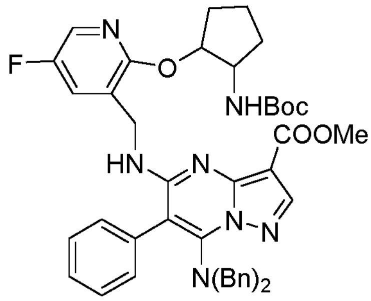

**78D**

Under nitrogen protection, methyl 6-bromo-5-((2-((2-((tert-butyloxycarbonyl)amino)cyclopentyl) oxy)-5-fluoropyridin-3-yl)methyl)amino)-7-(dibenzylamino)pyrazolo[1,5-a]pyrimidin-3-carboxylate (2.1 g, 2.71 mmol), phenylboronic acid (0.66 g, 5.42 mmol), Xphos PdG2 (73.87 mg, 0.1 mmol), and $K_3PO_4$ (1.15 g, 5.42 mmol) were dissolved in dioxane (40 mL) and water (7 mL), and then the reaction system was stirred at 100 °C for 16 h. After completion of the reaction, the reaction mixture was concentrated, and the residue was purified by column chromatography to provide **compound 78D.**
MS (ESI) m/z 772.4 $(M+H)^+$

**Step 5:** Preparation of ($6^3$E,$6^4$E)-$6^7$-(dibenzylamino)-$1^5$-fluoro-$6^6$-phenyl-2-oxo-4,7-diaza-6(3,5)-pyrazolo[1,5-a]pyrimidin-1(2,3)-pyridin-3(1,2)-cyclopentanacyclooctaphan-5-one **(compound 78E)**

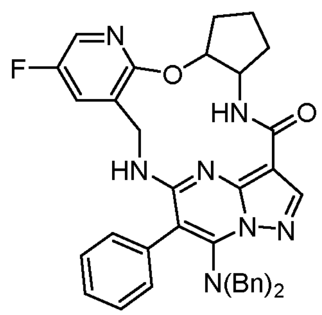

**78E**

**Compound 78E** of Example 78 was obtained by the preparation method same as that of step 6 in Example **49.**
MS (ESI) m/z 640.3 $(M+H)^+$

**Step 6:** Preparation of ($6^3$E,$6^4$E)-6'-amino-$1^5$-fluoro-$6^6$-phenyl-2-oxo-4,7-diazo-6(3,5)-pyrazolo[1,5-a]pyrimidin-1(2,3)-pyridin-3(1,2)-cyclopentanacyclooctaphan-5-one (compound 78)

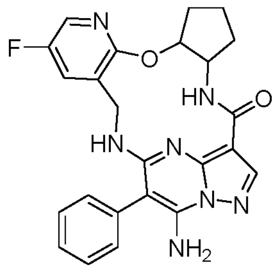

**78**

**Compound 78** of Example 78 was obtained by the preparation method same as that of step 10 in Example **49.**

MS (ESI) m/z 460.2 $(M+H)^+$

$^1H$ NMR (400 MHz, DMSO-$d_6$) δ 9.77-9.75 (d, $J$ = 8.0 Hz, 1H), 8.05 (s, 1H), 7.97-7.96 (d, $J$ = 4.0 Hz, 1H), 7.60-7.49 (m, 4H), 7.36 (s, 2H), 6.97-6.94 (t, $J$ = 12.0 Hz, 1H), 6.69 (s, 2H), 5.66-5.63 (m, 1H), 4.96-4.91 (m, 1H), 4.29 (m, 1H), 3.90 (m, 1H), 2.14 (m, 2H), 1.85 (m, 2H), 1.67 (m, 2H).

**Example 79:** The synthetic steps for preparation of (($1^3$E,$1^4$E)-$1^7$-amino-$6^5$-fluoro-$1^6$-phenyl-5-oxa-8-aza-1(3,5)-pyrazolino[1,5-a]pyrimidin-6(2,3)-pyridin-3(1,2)-pyrrolidinacyclooctaphan-2-one **(compound 79)** were shown as follows:

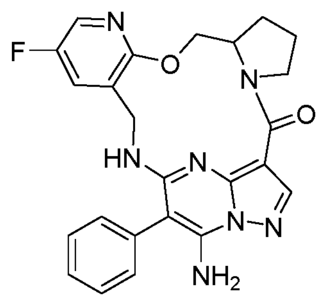

**79**

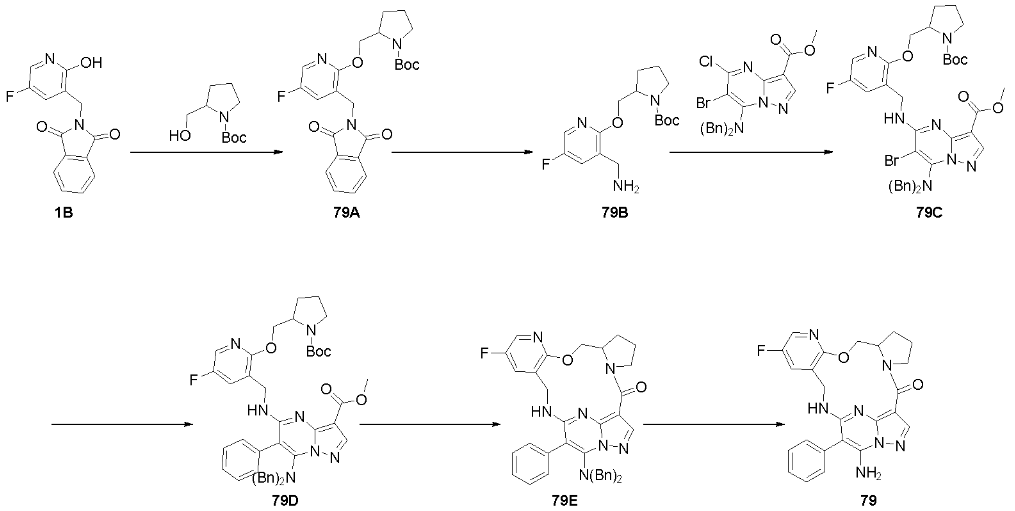

1B

79A

79B

79C

79D

79E

79

**Step 1:** Preparation of tert-butyl 2-((3-((1,3-dioxoisoindol-2-yl)methyl)-5-fluoropyridin-2-yl)oxy) methyl)pyrrolidin-1-carboxylate **(compound 79A)**

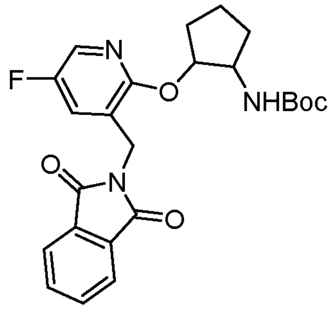

**79A**

Under nitrogen protection, 2-((5-fluoro-2-hydroxypyridin-3-yl)methyl)isoindol-1,3-dione (2.00 g, 7.30 mmol), tert-butyl 2-(hydroxymethyl)pyrrolidin-1-carboxylate (2.20 g, 11 mmol), and $PPh_3$ (2.88 g, 11 mmol) were dissolved in DCM (60 mL), to which was added DEAD (1.90 g, 11 mmol) dropwise at 0 °C. After addition, the reaction system was stirred at room temperature for 3 h. After completion of the reaction, the reaction was quenched by adding water, and the aqueous phase was extracted with ethyl acetate. The organic phases were combined, washed with saturated brine, dried over anhydrous sodium sulfate, filtered, and concentrated. The residue was purified by column chromatography to provide **compound 79A.**
MS (ESI) m/z 456.2 $(M+H)^+$

**Step 2:** Preparation of tert-butyl 2-((3-(aminomethyl)-5-fluoropyridin-2-yl)oxy)methyl) pyrrolidin-1-carboxylate **(compound 79B)**

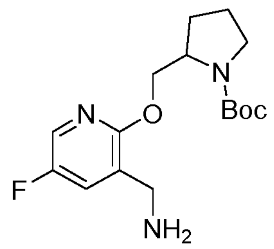

**79B**

tert-Butyl 2-((3-((1,3-dioxoisoindol-2-yl)methyl)-5-fluoropyridin-2-yl)oxy)methyl) pyrrolidin-1-carboxylate (3.00 g, 6.60 mmol) was dissolved in ethanol (30 mL), to which was then added hydrazine hydrate (0.83 g, 13.20 mmol), and then the system was allowed to react overnight at 60 °C. After completion of the reaction, the reaction mixture was filtered and concentrated to provide **compound 79B.**
MS (ESI) m/z 326.2 $(M+H)^+$

**Step 3:** Preparation of methyl 6-bromo-5-((2-((1-(tert-butyloxycarbonyl)pyrrolidin-2-yl)methoxy)-5-fluoropyridin-3-yl)methyl)amino)-7-(dibenzylamino)pyrazolo[1,5-a]pyrimidin-3-carboxylate **(compound 79C)**

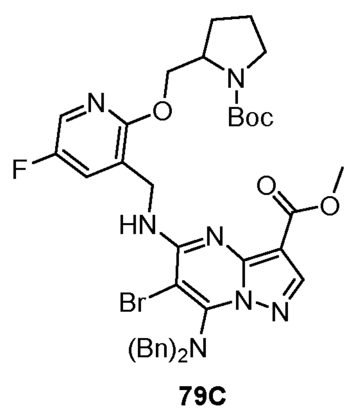

**79C**

tert-Butyl 2-((3-(aminomethyl)-5-fluoropyridin-2-yl)oxy)methyl)pyrrolidin-1-carboxylate (0.80 g, 2.45 mmol), methyl 6-bromo-5-chloro-7-(dibenzylamino)pyrazolo[1,5-a]pyrimidin-3-carboxylate (1.10 g, 2.23 mmol), diisopropylethylamine (0.86 g, 6.7 mmol), and ethanol (20 mL) were added into a reaction flask, and then the system was allowed to react overnight at 60 °C. After completion of the reaction, the reaction mixture was concentrated to provide the crude product, which was separated and purified to obtain **compound 79C.**
MS (ESI) m/z 774.2 $(M+H)^+$

**Step 4:** Preparation of methyl 6-bromo-5-((2-((1-(tert-butyloxycarbonyl)pyrrolidin-2-yl)methoxy)-5-fluoropyridin-3-yl)methyl)amino)-7-(dibenzylamino)pyrazolo[1,5-a]pyrimidin-3-carboxylate **(compound 79D)**

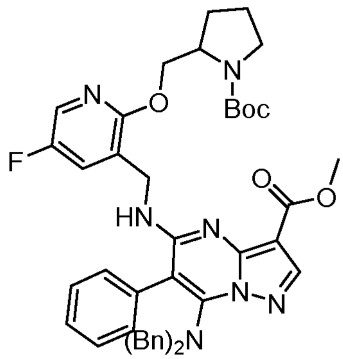

**79D**

Under nitrogen protection, methyl 6-bromo-5-((2-((1-(tert-butyloxycarbonyl)pyrrolidin-2-yl) methoxy)-5-fluoropyridin-3-yl)methyl)amino)-7-(dibenzylamino)pyrazolo[1,5-a]pyrimidin-3-carboxylate (1.40 g, 1.80 mmol), phenylboronic acid (440 mg, 3.60 mmol), XPhos PdG3 (169 mg, 0.20 mmol), XPhos (95 mg, 0.20 mmol), and $K_3PO_4$ (1.14 g, 5.40 mmol) were dissolved in 1,4-dioxane (30 mL) and water (7.5 mL), and then the reaction system was allowed to react overnight at 100 °C. After completion of the reaction, the reaction mixture was concentrated, and the residue was purified by column chromatography to provide **compound 79D.**
MS (ESI) m/z 772.4 $(M+H)^+$

**Step 5:** Preparation of ($1^3$E,$1^4$E)-$1^7$-(dibenzylamino)-$6^5$-fluoro-$1^6$-phenyl-5-oxa-8-aza-1(3,5)-pyrazolo[1,5-a]pyrimidin-6(2,3)-pyridin-3(1,2)-pyrrolidinacyclooctaphan-2-one **(compound 79E)**

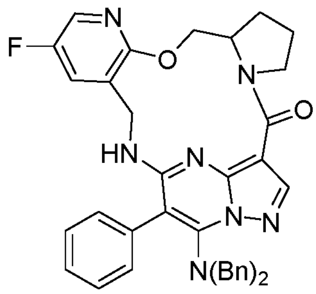

**79E**

**Compound 79E** of Example 79 was obtained by the preparation method same as that of step 6 in Example **49.**
MS (ESI) m/z 640.3 $(M+H)^+$

**Step 6:** Preparation of (($1^3$E,$1^4$E)-$1^7$-amino-$6^5$-fluoro-$1^6$-phenyl-5-oxa-8-aza-1(3,5)-pyrazolino[1,5-a]pyrimidin-6(2,3)-pyridin-3(1,2)-pyrrolidinacyclooctaphan-2-one **(compound 79)**

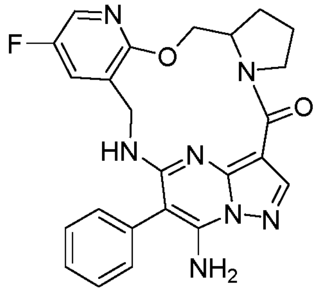

**79**

**Compound 79** of Example 79 was obtained by the preparation method same as that of step **10** in Example **49.**

MS (ESI) m/z 460.2 $(M+H)^+$

$^1$H NMR (400 MHz, DMSO-$d_6$) δ 7.92 (d, *J* = 3.2 Hz, 2H), 7.66 (dd, *J* = 8.9, 3.1 Hz, 1H), 7.58 (t, *J* = 7.6 Hz, 2H), 7.52 - 7.45 (m, 1H), 7.33 (s, 2H), 6.72 (t, *J* = 6.0 Hz, 1H), 6.45 (s, 2H), 4.95 - 4.84 (m, 1H), 4.76 (dd, *J* = 11.1, 2.7 Hz, 1H), 4.52 (s, 1H), 4.26 (m, 1H), 4.02 (d, *J* = 11.0 Hz, 1H), 3.78 (dd, *J* = 13.8, 5.4 Hz, 1H), 2.29 - 2.11 (m, 2H), 2.08 (s, 2H), 1.78 (m, 1H).

**Example 80:** Preparation of 1-(4-((*S*,1$^3$*E*,1$^4$*E*)-1$^7$-amino-4$^5$-fluoro-6-methyl-9-oxo-5-oxa-2,8-diaza-1(5,3)-pyrazolo[1,5-a]pyrimidin-4(3,2)-pyridinacyclononaphan-1$^6$-yl)-phenyl)-3-ethylurea **(compound 80):**

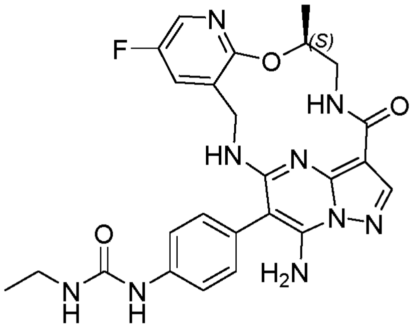

**80**

Using ethylamine to replace cyclopropylmethanol, **compound 80** of Example 80 was obtained by the preparation method same as that of Example **63.**

MS (ESI) m/z 520.2 $(M+H)^+$

$^1$H NMR (400 MHz, DMSO-$d_6$) δ 9.90 (d, *J* = 8.9 Hz, 1H), 8.64 (s, 1H), 8.05 (s, 1H), 8.00 (d, *J* = 3.0 Hz, 1H), 7.70 (dd, *J* = 8.9, 2.9 Hz, 1H), 7.61 (s, 2H), 7.17 (d, *J* = 16.8 Hz, 2H), 6.99 (t, *J* = 6.1 Hz, 1H), 6.65 (s, 2H), 6.15 (t, *J* = 5.6 Hz, 1H), 5.04-4.99 (m, 1H), 4.93 (dd, *J* = 14.3, 5.7 Hz, 1H), 4.04-3.97 (m, 1H), 3.88 (dd, *J* = 14.6, 5.6 Hz, 1H), 3.19 - 3.07 (m, 3H), 1.46 (d, *J* = 6.1 Hz, 3H), 1.08 (t, *J* = 7.1 Hz, 3H).

**Example 81:** Preparation of 3-(4-((*S*,1$^3$*E*,1$^4$*E*)-1$^7$-amino-4$^5$-fluoro-6-methyl-9-oxo-5-oxa-2,8-diaza-1(5,3)-pyrazolo[1,5-*a*]pyrimidin-4(3,2)-pyridinacyclononaphan-1$^6$-yl)-phenyl)-1-ethyl-1-methylurea **(compound 81):**

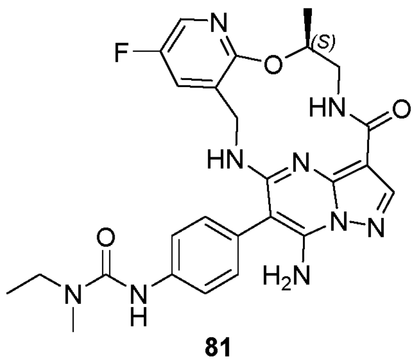

**81**

Using Methyl Ethylamine to replace cyclopropylmethanol, **compound 81** of Example 81 was obtained by the preparation method same as that of Example **63.**

MS (ESI) m/z 534.2 $(M+H)^+$

$^1$H NMR (400 MHz, DMSO-$d_6$) δ 9.90 (d, *J* = 8.9 Hz, 1H), 8.44 (s, 1H), 8.06 (s, 1H), 8.00 (d, *J* = 3.0 Hz, 1H), 7.78 - 7.66 (m, 3H), 7.18 (d, *J* = 16.1 Hz, 2H), 6.99 (t, *J* = 6.1 Hz, 1H), 6.65 (s, 2H), 5.05-4.99 (m, 1H), 4.94 (dd, *J* = 14.4, 5.7 Hz, 1H), 4.04-3.97 (m, 1H), 3.88 (dd, *J* = 14.5, 5.5 Hz, 1H), 3.40 (dd, *J* = 14.2, 7.1 Hz, 2H), 3.12 (dd, *J* = 12.2, 10.7 Hz, 1H), 2.97 (s, 3H), 1.46 (d, *J* = 6.1 Hz, 3H), 1.10 (t, *J* = 7.0 Hz, 3H).

**Example 82:** Preparation of (S,$1^3$E,$1^4$E)-$1^7$-amino-$4^5$-fluoro-6-methyl-$1^6$-(1-methyl-1H-pyrazol-4-yl)-5-oxa-2,8-diaza-1(5,3)-pyrazolo[1,5-a]pyrimidin-4(3,2)-pyridinacyclononaphan-9-one **(compound 82):**

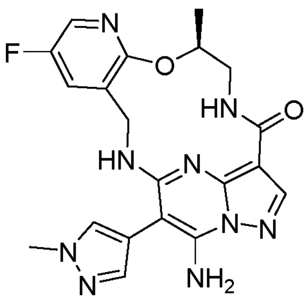

**82**

Using 1-methyl-4-(4,4,5,5-tetramethyl-1,3,2-dioxaborolan-2-yl)-1H-pyrazole to replace p-fluorophenylboronic acid, **compound 82** of Example 82 was obtained by the preparation method same as that of Example **51.**

MS (ESI) m/z 474.2 $(M+H)^+$

$^1$H NMR (400 MHz, DMSO-$d_6$) δ 9.89 (d, *J* = 8.9 Hz, 1H), 8.04 (s, 1H), 8.00 (d, *J* = 3.0 Hz, 1H), 7.83 (s, 1H), 7.75 (dd, *J* = 8.8, 2.9 Hz, 1H), 7.48 (s, 1H), 7.22 (t, *J* = 6.1 Hz, 1H), 6.87 (s, 2H), 5.06 - 4.86 (m, 2H), 4.04 - 3.80 (m, 5H), 3.15 - 3.04 (m, 1H), 1.46 (d, *J* = 6.1 Hz, 3H).

**Example 83:** Preparation of 2-(4-((*S*,$1^3$*E*,$1^4$*E*)-$1^7$-$4^5$-fluoro-6-methyl-9-oxo-5-oxa-2,8-diaza-1(5,3)-pyrazolo[1,5-a]pyrimidin-4(3,2)-pyridinacyclononaphan-$1^6$-yl)phenyl)-N-ethylacetamide **(compound 83):**

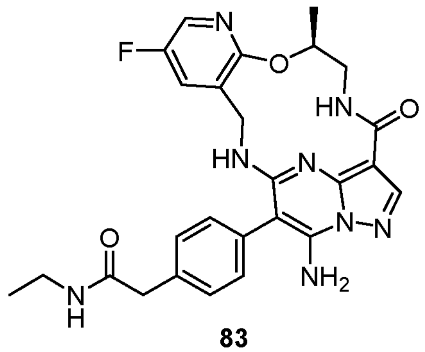

**83**

Using N-ethyl-2-(4-(4,4,5,5-tetramethyl-1,3,2-dioxaborolan-2-yl)phenyl)acetamide to replace 4-chlorophenylboronic acid, **compound 83** of Example 83 was obtained by the preparation method same as that of Example **50.**

MS (ESI) m/z 519.2 $(M+H)^+$
$^1$H NMR (400 MHz, DMSO-$d_6$) δ 9.86 (d, *J* = 8.8 Hz, 1H), 8.30 - 7.93 (m, 3H), 7.71 (dd, *J* = 8.8, 2.6 Hz, 1H), 7.49 (d, *J* = 7.9 Hz, 2H), 7.21 (d, *J* = 64.3 Hz, 2H), 7.00 (t, *J* = 5.9 Hz, 1H), 6.68 (s, 2H), 5.09 - 4.98 (m, 1H), 4.94 (dd, *J* = 14.3, 6.1 Hz, 1H), 4.01 (m, 1H), 3.87 (m, 1H), 3.50 (s, 2H), 3.11 (dd, *J*= 12.8, 6.8 Hz, 3H), 1.46 (d, *J* = 6.0 Hz, 3H), 1.07 (t, *J* = 7.2 Hz, 3H).

**Biological test data:**

Unless otherwise specified, the experimental materials, reagents, operations and methods used in the following activity examples are commercially available or easily known or prepared based on the prior art.

**Experimental Example 1: *In vitro* kinase activity assay of the compound according to the present invention**

**Experimental objectives**

The $IC_{50}$ (half inhibitory concentration) values of the compounds were used as an indicator to evaluate the inhibitory effect of the compound on RET wild-type, RET mutant, TRKa, SRC, BTK and/or mutated BTK kinase.

**Experimental methods**

Using the method of Mobility shift assay, the compounds were tested for inhibitory activity against the following kinases: RET wild type, RET mutant, TRKa, SRC, BTK and/or mutated BTK. The initial concentration of the test compound was 1000 nM, 3-fold dilution, 10 concentrations, detected in a well for each concentration.

**Reagents and materials:**

| Reagent name | Supplier | Article No. | Batch No. |
|---|---|---|---|
| RET | Carna | 08-159 | 13CBS-0134F |
| RETG810R | Proqinase | 1724-0000-1 | 002 |
| RET V804M | signalchem | R02-12GG | Y985-2 |
| BTK | Carna | 08-180 | 14CBS-0619Q |
| BTK C481S | Carna | 08-547 | 14CBS-0633H |
| SRC | Carna | 08-173 | 10CBS-1134K |
| TRKa | Carna | 08-186 | 13CBS-0565G |
| Kinase substrate 2 | GL | 190861 | P200807-YS190861 |

(continued)

| Reagent name | Supplier | Article No. | Batch No. |
|---|---|---|---|
| Kinase substrate 4 | GL | 112395 | P171211-XY112395 |
| Kinase substrate 22 | GL | 112393 | P200403-CL112393 |
| DMSO | Sigma | D8418-1L | SHBG3288V |
| 384-well plate | Corning | 3573 | 12619003 |
| 384-well plate | Corning | 3575BC | 31316039 |
| MgCl2 | Sigma | M1028 | / |
| ATP | Promega | V910B | / |
| DTT | Sigma | D0632 | / |

**Apparatus**

Centrifuge (manufacturer: Eppendorf, model: 5430)
Enzyme microplate reader (manufacturer: Perkin Elmer, model: Caliper EZ Reader)
Echo 550 (manufacturer: Labcyte, model: Echo 550)

**Preparation of kinase reaction buffer:**

20 mM hydroxyethylpiperazineethanesulfonic acid (Hepes) (pH 7.5) buffer, 10 mM $MgCl_2$, 1 mM ethylene glycol bis(aminoethyl ether)tetraacetic acid (EGTA), 0.02% polyoxyethylene lauryl ether (Brij35), 0.02 mg/ml N,O-bis(trimethylsilyl)acetamide (BSA), 0.1 mM $Na_3VO_4$, 2 mM dithiothreitol (DTT), 1% DMSO.

**Compound:**

The test compounds were dissolved in 100% dimethyl sulfoxide (DMSO) system, and prepared as 10 mM for use, and stored in a nitrogen cabinet away from light.

**Reaction conditions:**

| | ATP (μM) | Reaction time |
|---|---|---|
| RET | 16 | 60 min |
| RET G810R | 201 | 4 h |
| RET V804M | 5.4 | 60 min |
| BTK | 71 | 30 min |
| BTK C481S | 90 | 30 min |
| SRC | 19 | 30 min |
| TRKa | 47.8 | 30 min |

**Kinase reaction process:**

(1) Preparing 1 × kinase reaction buffer.
(2) Preparation of compound concentration gradient: the initial concentration of the compounds was 1000 nM, which

was diluted as 100-fold final concentration of solution in 100% dimethyl sulfoxide (DMSO) in a 384-well plate. The compound was accurately 3-fold diluted with the kinase buffer solution, 10 concentrations in total, and the final concentration was 0.0508 nM. The 100-fold final concentration of solution in 100% dimethyl sulfoxide (DMSO) was obtained by dilution in 384 source plate. The liquid separator Echo 550 was used to transfer 250 nl of 100-fold final concentration of compound to the target 384 well plate. 250 nl of DMSO was added to the positive and negative control wells.

(3) 2.5-fold final concentration of kinase solution was prepared by the 1 × kinase buffer.

(4) 10 μL of 2.5-fold final concentration of kinase solution was added to the compound well and the positive control well, respectively; 10 μL of 1× kinase buffer was added to the negative control well.

(5) After centrifuging at 1000 rpm for 30 second, the reaction plate was shaken and mixed, followed by incubation at room temperature for 10 min.

(6) a mixed solution of 25/15-fold final concentration of adenosine triphosphate (ATP) and kinase substrate was prepared by 1× kinase buffer.

(7) The mixed solution (15 μL) of 25/15-fold final concentration of adenosine triphosphate (ATP) and substrate was added to initiate the reaction.

(8) 384-well plate was centrifuged at 1000 rpm for 30 second, shaken and mixed well, and then incubated at room temperature for 30-240 min.

(9) After stopping the kinase reaction, the plate was centrifugated at 1000 rpm for 30 second, shaken and mixed well.

(10)The conversion rate was read with Caliper EZ Reader.

**Data analysis**

**Calculation formula**

$$\% \text{ Inhibition} = \frac{\text{conversion\%}_{\text{max}} - \text{conversion\%_sample}}{\text{conversion\%}_{\text{max}} - \text{conversion\%_min}} * 100$$

wherein Conversion%_sample is the conversion rate reading of the sample; Conversion%_min: the mean value of negative control wells, representing the conversion rate reading of wells without enzymes; Conversion%_max: the mean value of positive control wells, representing the conversion rate reading of wells without compound inhibition.

**Fitting the dose-response curve**

The log value of concentration was taken as the X axis, and the percentage inhibition rate was taken as the Y axis. Graphpad 6.0 analysis software was used to fit the dose-response curve, so as to obtain the $IC_{50}$ value of each compound on the enzyme activity

**The experimental results are shown in Table 1:**

| Compound No. | RET wt ($IC_{50}$, nM) | RET G810R ($IC_{50}$, nM) | RET V804M ($IC_{50}$, nM) | TRKa ($IC_{50}$, nM) | SRC ($IC_{50}$, nM) | BTK ($IC_{50}$, nM) | BTK C481S ($IC_{50}$, nM) |
|---|---|---|---|---|---|---|---|
| 1 | 11 | 42.11 | >10000 | >10000 | 10.8 | 33.5 | 31.31 |
| 2 | 5285 | - | - | 46 | - | - | - |
| 3 | 22 | - | - | >10000 | - | - | 54.75 |
| 4 | 2.2 | 20.96 | 160.5 | 294 | 0.096 | 3.391 | 9.84 |
| 5 | 237 | - | - | >10000 | - | - | - |
| 6 | 5.1 | - | - | 1345 | - | - | - |
| 7 | 10 | - | - | 1853 | - | - | - |
| 8 | 13 | - | - | 1241 | - | - | - |
| 9 | 8.2 | - | - | >10000 | - | 25.9 | 12.95 |
| 10 | 144 | >10000 | >10000 | >10000 | >10000 | >10000 | >10000 |

(continued)

| Compound No. | RET wt ($IC_{50}$, nM) | RET G810R ($IC_{50}$, nM) | RET V804M ($IC_{50}$, nM) | TRKa ($IC_{50}$, nM) | SRC ($IC_{50}$, nM) | BTK ($IC_{50}$, nM) | BTK C481S ($IC_{50}$, nM) |
|---|---|---|---|---|---|---|---|
| 11 | 15 | - | - | 925 | - | - | - |
| 12 | 36 | - | - | >10000 | - | - | - |
| 13 | 5.3 | - | - | >10000 | - | | - |
| 14 | 127 | - | - | >10000 | - | - | - |
| 16 | 543 | - | - | 1367 | - | - | - |
| 17 | >10000 | - | - | >10000 | - | >10000 | >10000 |
| 18 | >10000 | >10000 | >10000 | - | >10000 | >10000 | >10000 |
| 19 | 48.91 | >10000 | >10000 | - | >10000 | >10000 | >10000 |
| 20 | >10000 | - | - | >10000 | - | - | - |
| 21 | 5.9 | - | - | >10000 | - | - | - |
| 22 | 21 | - | - | 304 | - | - | - |
| 23 | 448 | - | - | >10000 | - | - | - |
| 24 | 20 | - | - | >10000 | - | 296 | 397.7 |
| 25 | 366 | - | - | >10000 | - | - | - |
| 26 | 9 | - | - | >10000 | - | - | - |
| 27 | 0.629 | 17.21 | 3.25 | - | 38.41 | 15.4 | 16.93 |
| 28 | 0.359 | 12 | 9.175 | - | 0.4557 | 1.111 | 0.96 |
| 29 | 20 | - | - | >10000 | - | - | - |
| 30 | 5.1 | 15.09 | 76 | >10000 | 3111 | 5177 | - |
| 31 | 4.055 | 12.34 | | >10000 | 843.2 | >10000 | - |
| 32 | 14.25 | 98.17 | 59.02 | - | - | - | - |
| 33 | 407 | - | - | - | 196 | >10000 | |
| 34 | 5.4 | 376.8 | - | - | - | - | - |
| 35 | 1.9 | 196.9 | - | - | - | - | - |
| 36 | 27 | >1000 | - | - | - | - | - |
| 37 | 95 | >1000 | - | - | - | - | - |
| 38 | >1000 | >1000 | - | - | - | >1000 | >1000 - |
| 39 | 3.0 | 375.6 | - | - | - | - | - |
| 40 | 465 | - | - | - | 105 | 176 | - |
| 41 | 13 | >10000 | >10000 | - | - | 2065 | - |
| 42 | >1000 | >1000 | >1000 | - | - | - | - |
| 43 | 68 | >10000 | >10000 | - | - | >10000 | |
| 44 | 286 | >1000 | - | - | - | - | - |
| 45 | 3.9 | 69.96 | - | - | - | 346 | 42.42 |
| 46 | 5.1 | 96.92 | - | - | - | - | - |
| 47 | 26 | >1000 | >1000 | - | - | - | - |

(continued)

| Compound No. | RET wt ($IC_{50}$, nM) | RET G810R ($IC_{50}$, nM) | RET V804M ($IC_{50}$, nM) | TRKa ($IC_{50}$, nM) | SRC ($IC_{50}$, nM) | BTK ($IC_{50}$, nM) | BTK C481S ($IC_{50}$, nM) |
|---|---|---|---|---|---|---|---|
| 48 | >1000 | >1000 | >1000 | - | - | - | - |
| 49 | <0.51 | 9.3 | 1.3 | - | - | 9.2 | 15 |
| 50 | 0.83 | - | - | - | - | 1.8 | 1.48 |
| 51 | 1.6 | - | - | - | - | 1.0 | 1.9 |
| 52 | 5.4 | 588.3 | - | - | - | - | - |
| 53 | >1000 | >1000 | - | - | - | >1000 | >1000 |
| 54 | 105 | >1000 | >1000 | - | 2.1 | 48 | 104 |
| 55 | 1.1 | 84.27 | - | - | - | - | |
| 56 | 1.2 | 49.69 | - | - | 0.13 | 1.5 | 0.96 |
| 57 | 47 | >1000 | - | - | - | - | - |
| 58 | 3.4 | 155.1 | - | - | - | - | - |
| 59 | 2.4 | 130 | 38 | - | - | - | - |
| 60 | 3.5 | 360.9 | - | - | - | 3.6 | - |
| 61 | 1.0 | 21.81 | - | - | - | - | - |
| 62 | 0.26 | 15 | 0.94 | - | - | - | - |
| 63 | 1.2 | 22 | 3.5 | 65 | 2.8 | 33 | - |
| 64 | 128 | - | - | - | 61 | >1000 | |
| 65 | 2.1 | - | - | - | - | 3.5 | 6.6 |
| 66 | 1.3 | 53.73 | - | 101 | - | 1.7 | 1.45 |
| 67 | 1.19 | 35.65 | 3.58 | - | - | - | - |
| 68 | 6.2 | 155 | 15 | - | - | - | - |
| 69 | 0.87 | 16 | 2.8 | - | - | - | - |
| 70 | 15 | >1000 | 551 | - | - | - | - |
| 71 | 1.2 | 23 | 1.6 | - | - | - | - |
| 72 | 0.56 | 14 | 1.4 | - | - | - | - |
| 73 | 4.4 | 238 | 137 | - | - | - | - |
| 74 | 323 | >1000 | >1000 | - | - | >1000 | >1000 |
| 75 | 20 | >1000 | 311 | - | - | 22 | 52 |
| 76 | 922 | >1000 | >1000 | - | - | >1000 | >1000 |
| 77 | >1000 | >1000 | >1000 | - | - | >1000 | >1000 |
| 78 | 2.5 | 337 | 24 | - | - | 1.7 | 1.7 |
| 79 | 104 | >1000 | >1000 | - | - | 45 | 97 |
| 80 | 0.6 | 15 | 0.99 | - | - | - | - |
| 81 | 89 | >1000 | 384 | - | - | - | - |
| 82 | 1.1 | 138 | 9.5 | - | - | - | - |

(continued)

| Compound No. | RET wt ($IC_{50}$, nM) | RET G810R ($IC_{50}$, nM) | RET V804M ($IC_{50}$, nM) | TRKa ($IC_{50}$, nM) | SRC ($IC_{50}$, nM) | BTK ($IC_{50}$, nM) | BTK C481S ($IC_{50}$, nM) |
|---|---|---|---|---|---|---|---|
| 83 | 49 | >1000 | 281 | - | - | - | - |
| "-" represents "not detected". | | | | | | | |

Those skilled in the art will understand that the above description is exemplary and illustrative in nature, and intends to illustrate the present invention and preferred embodiments thereof. Using routine experiments, those skilled in the art will understand that obvious modifications and changes may be made without departing from the spirit of the present invention. All such modifications within the attached scope of the patent application are intended to be included. Therefore, the present invention is intended to be defined by the following claims and equivalents thereof, but not by the above description.
All public publications cited in this specification are incorporated herein by reference.

## Claims

1. A compound of formula (I) or a stereoisomer thereof, a pharmaceutically acceptable salt, a solvate or a tautomer thereof:

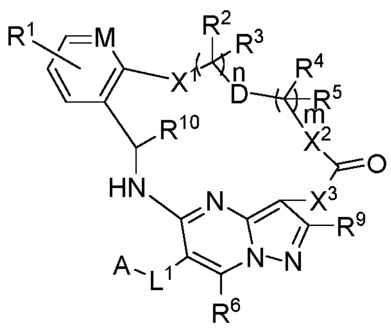

(I)

$L^1$ is selected from absence, O, S, NH, N($C_{1-6}$alkyl), $(CH_2)_n$NH, C(O), hydrogen;
A is selected from absence, substituted or unsubstituted 3- to 10-membered cycloalkyl, substituted or unsubstituted 3- to 10-membered heterocycloalkyl, substituted or unsubstituted 6- to 8-membered monoaryl, substituted or unsubstituted 5- to 10-membered monoheteroaryl and substituted or unsubstituted 8- to 10-membered fused heteroaryl; when said groups are substituted, the substituent(s) is or is selected from oxo, halo, amino, hydroxyl, cyano, $C_{1-6}$alkyl, $C_{3-6}$ cycloalkyl, $C_{3-6}$ heterocycloalkyl, oxo$C_{3-6}$ heterocycloalkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-(CH_2)_qC(O)NHR^a$, $-C(O)NR^aR^b$, halo$C_{1-6}$alkyl, hydroxyl$C_{1-6}$ alkyl and amino$C_{1-6}$alkyl; wherein the heteroatom(s) in said heterocycloalkyl, monoheteroaryl, fused heteroaryl is N, O or S;
when $L^1$ is hydrogen, A is absent;
alternatively, the substituent in said cycloalkyl, heterocycloalkyl, aryl, heteroaryl and fused heteroaryl, and the adjacent atom in the ring are linked to form a ring;
M is selected from N and CH;
$X^1$, $X^2$, and $X^3$ are independently selected from absence, O, S, $S(O)_2$, NH, and N($C_{1-6}$alkyl);
D is selected from absence, substituted or unsubstituted 3- to 10-membered cycloalkyl or 3- to 10-membered heterocycloalkyl, wherein the heteroatom(s) is N, O, S; when said groups are substituted, the substituent(s) is or is selected from oxo, halo, amino, hydroxyl, cyano, $C_{1-6}$alkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-6}$alkyl, hydroxyl$C_{1-6}$alkyl and amino$C_{1-6}$alkyl;
$R^1$, $R^2$, $R^3$, $R^4$, and $R^5$ are each independently selected from hydrogen, halo, amino, hydroxyl, cyano, $C_{1-6}$alkyl,

$C_{1-6}$ alkoxyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-6}$alkyl, hydroxyl$C_{1-6}$ alkyl and amino$C_{1-6}$alkyl;
alternatively, $R^2$ and $R^3$, together with the carbon atom to which they are attached, form a 3- to 7-membered cycloalkyl or 3- to 7-membered heterocycloalkyl, and said cycloalkyl or heterocycloalkyl may be substituted by halo, amino, hydroxyl, cyano, $C_{1-6}$alkyl, $C_{1-6}$ alkoxyl, di($C_{1-6}$alkyl)amino, halo$C_{1-6}$alkyl, hydroxyl$C_{1-6}$alkyl and amino$C_{1-6}$alkyl;

alternatively, $R^4$ and $R^5$, together with the carbon atom to which they are attached, form a 3- to 7-membered cycloalkyl or 3- to 7-membered heterocycloalkyl, and said cycloalkyl or heterocycloalkyl may be substituted by halo, amino, hydroxyl, cyano, $C_{1-6}$alkyl, $C_{1-6}$ alkoxyl, di($C_{1-6}$alkyl)amino, halo$C_{1-6}$alkyl, hydroxyl$C_{1-6}$alkyl and amino$C_{1-6}$alkyl;
alternatively, $R^4$ and $R^5$, together with the carbon atom to which they are attached and $X^2$, form the following structure:

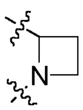 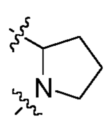 or 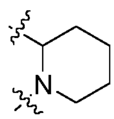 ;

$R^6$ is selected from hydrogen, amino, hydroxyl, halo, cyano, substituted or unsubstituted 3- to 10-membered cycloalkyl, 3- to 10-membered heterocycloalkyl, 6- to 8-membered monoaryl, 5- to 10-membered monoheteroaryl and 8- to 10-membered fused heteroaryl, $-NR^7R^8$, $-NHR^7$, $-(CH_2)_qNR^aR^b$, $-NHC(O)OR^7$, $-NHC(O)NHR^a$, $-NHC(O)R^7$, $-OR^7$, $-OC(O)OR^7$, $-OC(O)R^7$, $-C(O)R^7$, $-C(O)NHR^7$, and $-C(O)NR^7R^8$; when said groups are substituted, the substituent(s) is or is selected from oxo, halo, amino, hydroxyl, cyano, $C_{1-6}$alkyl, 5- to 6-membered aryloxy, 5- to 6-membered heteroaryloxy, $-NH(CH_2)_qR^a$, $-N(CH_2)_qR^aR^b$, $-NC(O)OR^a$, $-NC(O)R^a$, $-OR^a$, $-OC(O)OR^a$, $-OC(O)R^a$, $-C(O)NHR^a$, $-C(O)NR^aR^b$, halo$C_{1-6}$alkyl, hydroxyl$C_{1-6}$ alkyl and amino$C_{1-6}$alkyl; alternatively, the substituent on said cycloalkyl, heterocycloalkyl, aryl, heteroaryl, and fused heteroaryl, and the adjacent atom in the ring are linked to form a ring;
$R^7$ and $R^8$ are selected from $C_{1-4}$ alkyl, halo$C_{1-4}$ alkyl, hydroxyl$C_{1-4}$ alkyl, amino$C_{1-4}$ alkyl, halo$C_{1-4}$ alkyl, substituted or unsubstituted 3- to 10-membered cycloalkyl, 3- to 10-membered heterocycloalkyl, 6- to 8-membered monoaryl, 5- to 10-membered monoheteroaryl, 8- to 10-membered fused heteroaryl; the substituent(s) is oxo, halo, amino, hydroxyl, cyano, $C_{1-6}$alkyl, 5- to 6-membered aryloxy, 5- to 6-membered heteroaryloxy, $-NH(CH_2)_qR^a$, $-N(CH_2)_qR^aR^b$, $-NC(O)OR^a$, $-NC(O)R^a$, $-OR^a$, $-OC(O)OR^a$, $-OC(O)R^a$, $-C(O)NHR^a$, $-C(O)NR^aR^b$, halo$C_{1-4}$ alkyl, hydroxyl$C_{1-4}$ alkyl and amino$C_{1-4}$ alkyl; alternatively, the substituent on said cycloalkyl, heterocycloalkyl, aryl, heteroaryl, and fused heteroaryl, and the adjacent atom in the ring are linked to form a ring;
$R^9$ and $R^{10}$ are independently selected from hydrogen, $C_{1-6}$alkyl, halo$C_{1-6}$alkyl, halogen and cyano; preferably, said $C_{1-6}$alkyl is selected from methyl, ethyl, propyl, isopropyl, n-butyl, and isobutyl;
$R^a$ and $R^b$ are independently selected from $C_{1-4}$ alkyl, $C_{1-4}$ alkyl, $C_{5-6}$ aryl, $C_{5-6}$ heteroaryl, and $C_{1-4}$ alkylsulfonyl, in which said $C_{1-4}$ alkyl, $C_{5-6}$ aryl, and $C_{5-6}$ heteroaryl may be substituted by halo, amino, $C_{3-6}$ cycloalkyl, $C_{3-6}$ heterocycloalkyl, wherein the heteroatom(s) is N, O, or S;
m, n, and q are each independently selected from 0, 1, 2, 3, and 4;
$L^1$ and $R^6$ are not both hydrogen.

**2.** The compound according to claim 1 or a stereoisomer, a pharmaceutically acceptable salt, a solvate or a tautomer thereof, wherein

$L^1$ is selected from absence, O, S, NH, N($C_{1-6}$alkyl), $(CH_2)_nNH$, C(O);
A is selected from substituted or unsubstituted 3- to 6-membered cycloalkyl, substituted or unsubstituted 3- to 6-membered heterocycloalkyl, substituted or unsubstituted 6- to 8-membered monoaryl, substituted or unsubstituted 5- to 7-membered monoheteroaryl and substituted or unsubstituted 8- to 1 0-membered fused heteroaryl; when said groups are substituted, the substituent(s) is selected from oxo, halo, amino, hydroxyl, cyano, $C_{1-6}$alkyl, $C_{3-6}$ cycloalkyl, $C_{3-6}$ heterocycloalkyl, oxo$C_{3-6}$ heterocycloalkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-(CH_2)_qC(O)NHR^a$, $-C(O)NR^aR^b$, halo$C_{1-6}$alkyl, hydroxyl$C_{1-6}$ alkyl and amino$C_{1-6}$alkyl; wherein the heteroatom(s) in said heterocycloalkyl, monoheteroaryl, fused heteroaryl is N, O or S;

$R^a$ and $R^b$ are independently selected from $C_{1-4}$ alkyl, phenyl, $C_{1-4}$ alkylsulfonyl, in which said $C_{1-4}$ alkyl may be substituted by halo, amino, $C_{3-6}$ cycloalkyl;
$X^1$ is selected from absence, O, S, and $S(O)_2$; $X^2$ is selected from NH and N($C_{1-6}$alkyl); and $X^3$ is selected from absence or NH;
D is selected from absence, substituted or unsubstituted 3- to 6-membered cycloalkyl or 3- to 6-membered heterocycloalkyl, and the heteroatom(s) in said cycloalkyl is N, O; when said groups are substituted, the substituent(s) is halo, amino, hydroxyl, $C_{1-4}$ alkyl and halo$C_{1-4}$ alkyl;
$R^1$ is halo, amino, $C_{1-4}$ alkyl or halo$C_{1-4}$ alkyl;
$R^2$, $R^3$, $R^4$, and $R^5$ are independently selected from hydrogen, $C_{1-6}$alkyl or halo$C_{1-6}$alkyl;
$R^6$ is selected from hydrogen, amino, $-NR^7R^8$ or $-NHR^7$;
$R^7$ and $R^8$ are selected from $C_{1-4}$ alkyl and halo$C_{1-4}$ alkyl;
$R^9$ is selected from hydrogen, $C_{1-6}$alkyl, halo$C_{1-6}$alkyl, halogen and cyano; preferably, said $C_{1-6}$alkyl is selected from methyl, ethyl, propyl, isopropyl, n-butyl, and isobutyl;
$R^{10}$ is selected from hydrogen, and $C_{1-6}$alkyl; preferably, said $C_{1-6}$alkyl is selected from methyl, ethyl, propyl, isopropyl, n-butyl, and isobutyl;
m is selected from 0, 1 or 2;
n is selected from 0, 1 or 2.
q is selected from 0, 1 or 2.

**3.** The compound according to any one of claims 1 to 2 or a stereoisomer, a pharmaceutically acceptable salt, a solvate or a tautomer thereof, wherein $L^1$ is absent or NH; more preferably, $L^1$ is absent.

**4.** The compound according to any one of claims 1 to 3 or a stereoisomer, a pharmaceutically acceptable salt, a solvate or a tautomer thereof,

wherein A is selected from substituted or unsubstituted 3- to 6-membered cycloalkyl, substituted or unsubstituted 3- to 6-membered heterocycloalkyl, substituted or unsubstituted phenyl and substituted or unsubstituted 5- to 6-membered monoheteroaryl; when said groups are substituted, the substituent(s) is selected from halo, amino, hydroxyl, cyano, $C_{1-6}$alkyl, $C_{3-6}$ cycloalkyl, $C_{3-6}$ heterocycloalkyl, oxo$C_{3-6}$ heterocycloalkyl, 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-(CH_2)_qC(O)NHR^a$, $-C(O)NR^aR^b$, halo$C_{1-6}$alkyl, hydroxyl$C_{1-6}$ alkyl and amino$C_{1-6}$alkyl; wherein the heteroatom(s) in said heterocycloalkyl, monoheteroaryl, fused heteroaryl is N, O or S;
$R^a$ and $R^b$ are independently selected from $C_{1-4}$ alkyl, phenyl, and $C_{1-4}$ alkylsulfonyl, wherein said $C_{1-4}$ alkyl may be substituted by halo, amino, and $C_{3-6}$ cycloalkyl.

**5.** The compound according to any one of claims 1 to 4 or a stereoisomer, a pharmaceutically acceptable salt, a solvate or a tautomer thereof, wherein A is selected from substituted or unsubstituted following groups: phenyl, pyridyl, cyclopentyl, cyclohexyl, cyclobutyl, cyclopropyl,

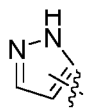 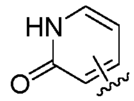 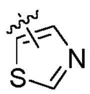 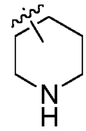 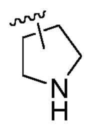 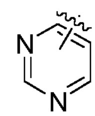

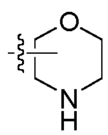 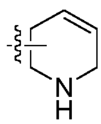 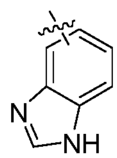 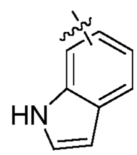 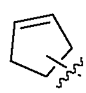 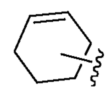 ;

when said groups are substituted, the substituent(s) is selected from oxo, halo, amino, hydroxyl, cyano, $C_{1-4}$ alkyl, substituted or unsubstituted 5- to 6-membered aryloxy, 5- to 6-membered heteroaryloxy, $-NR^aR^b$, $-NHR^a$, $-(CH_2)_qNR^aR^b$, $-NHC(O)OR^a$, $-NHC(O)NHR^a$, $-NHC(O)R^a$, $-OR^a$, $-OC(O)OR^a$, $-OC(O)R^a$, $-C(O)R^a$, $-C(O)NHR^a$, $-C(O)NR^aR^b$, halo$C_{1-4}$ alkyl, hydroxyl$C_{1-4}$ alkyl, and amino$C_{1-4}$ alkyl; in said heteroaryl, the heteroatom(s) is N, O, S;

$R^a$ and $R^b$ are independently selected from $C_{1-4}$ alkyl; preferably, $R^a$ and $R^b$ are independently selected from methyl, ethyl, propyl, isopropyl, n-butyl, isobutyl, and tert-butyl; wherein said $C_{1-4}$ alkyl may be substituted by halo, amino, $C_{3-6}$ cycloalkyl,
q is selected from 0, 1, and 2.

**6.** The compound according to any one of claims 1 to 5 or a stereoisomer, a pharmaceutically acceptable salt, a solvate or a tautomer thereof, wherein A is selected from substituted or unsubstituted following groups: phenyl, cyclopentenyl, cyclohexenyl and pyrazolyl; preferably, phenyl;

when said groups are substituted, the substituent(s) is selected from halo, amino, $C_{1-4}$ alkyl, phenoxy, $-NR^aR^b$, $-NHR^a$, $-NHC(O)OR^a$, $-NHC(O)NHR^a$, $-OR^a$, $-C(O)NHR^a$, and $-(CH_2)_qC(O)NHR^a$;
$R^a$ and $R^b$ are independently selected from $C_{1-4}$ alkyl;
q is selected from 1 and 2.

**7.** The compound according to any one of claims 1 to 6 or a stereoisomer, a pharmaceutically acceptable salt, a solvate or a tautomer thereof, wherein when group A is a substituted group, it is substituted by 1, 2 or 3 substituents; preferably, A is selected from phenyl or cyclohexenyl, which is mono-substituted by the substituents at o-, *m-,* or *p*-position, or disubstituted at *m*- or *p*-position.

**8.** The compound according to any one of claims 1 to 7 or a stereoisomer, a pharmaceutically acceptable salt, a solvate or a tautomer thereof, wherein M is N.

**9.** The compound according to any one of claims 1 to 8 or a stereoisomer, a pharmaceutically acceptable salt, a solvate or a tautomer thereof, wherein
$X^1$ is O; $X^2$ is NH; $X^3$ is absent.

**10.** The compound according to any one of claims 1 to 9 or a stereoisomer, a pharmaceutically acceptable salt, a solvate or a tautomer thereof, wherein D is selected from cyclopropyl, cyclobutyl, cyclopentyl, oxacyclopropyl, oxocyclopropyl, oxocyclobutyl, oxocyclopentyl, aziridinyl, azetidinyl or pyrrolidinyl;
preferably, D is absent.

**11.** The compound according to any one of claims 1 to 10 or a stereoisomer, a pharmaceutically acceptable salt, a solvate or a tautomer thereof, wherein $R^1$ locates at m-position of M, and preferably, $R^1$ is halo, and more preferably, $R^1$ is F or Cl.

**12.** The compound according to any one of claims 1 to 11 or a stereoisomer, a pharmaceutically acceptable salt, a solvate or a tautomer thereof, wherein $R^2$ is selected from hydrogen, $C_{1-6}$alkyl; and $R^3$, $R^4$, and $R^5$ are hydrogen;

**13.** The compound according to claim 1 or a stereoisomer, a pharmaceutically acceptable salt, a solvate or a tautomer thereof, wherein $R^4$, $R^5$, together with the carbon to which they are attached and $X^2$, form the following structure:

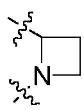 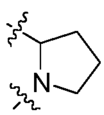 or 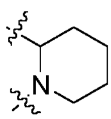 .

**14.** The compound according to any one of claims 1 to 13 or a stereoisomer, a pharmaceutically acceptable salt, a solvate or a tautomer thereof, wherein $R^6$ is selected from hydrogen and amino.

**15.** The compound according to any one of claims 1 to 14 or a stereoisomer, a pharmaceutically acceptable salt, a solvate or a tautomer thereof, wherein
$R^9$ is hydrogen; $R^{10}$ is selected from hydrogen and $C_{1-6}$alkyl; more preferably, $R^9$ and $R^{10}$ are both hydrogen.

**16.** The compound according to any one of claims 1 to 15 or a stereoisomer, a pharmaceutically acceptable salt, a solvate or a tautomer thereof, wherein m is selected from 0, 1 or 2, and preferably, 1; n is selected from 0 or 1, and preferably, 1.

**17.** The compound according to claim 1 or a stereoisomer, a pharmaceutically acceptable salt, a solvate or a tautomer

thereof, wherein

$L^1$ is absent;
A is selected from substituted or unsubstituted following groups: phenyl, cyclopentenyl, cyclohexenyl and pyrazolyl;
when said groups are substituted, they are substituted by 1 or 2 substituents selected from halo, amino, $C_{1-4}$ alkyl, phenoxy, $-NR^aR^b$, $-NHR^a$, $-NHC(O)OR^a$, $-NHC(O)NHR^a$, $-OR^a$, $-C(O)NHR^a$, and $-(CH_2)_qC(O)NHR^a$;
$R^a$ and $R^b$ are independently selected from $C_{1-4}$ alkyl;
q is selected from 1 and 2;
M is N;
$X^1$ is O; $X^2$ is NH; $X^3$ is absent;
D is absent;
$R^1$ is halogen;
$R^2$ is selected from hydrogen, $C_{1-6}$alkyl; and $R^3$, $R^4$ and $R^5$ are hydrogen;
$R^6$ is selected from hydrogen and amino;
$R^9$ is hydrogen;
$R^{10}$ is selected from hydrogen and $C_{1-6}$alkyl;
m is 1, n is 1.

**18.** The compound according to claim 1 or a stereoisomer, a pharmaceutically acceptable salt, a solvate or a tautomer thereof, which has the structure of general formula (Ia)

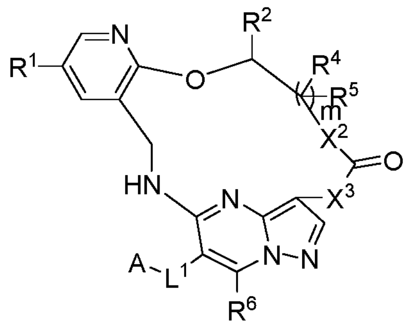

(Ia)

wherein L', A, $X^2$, $X^3$, $R^1$, $R^2$, $R^4$, $R^5$, $R^6$, and m are as defined in any one of claims 1 to 17.

**19.** The compound according to claim 1 or a stereoisomer, a pharmaceutically acceptable salt, a solvate or a tautomer thereof, which has the structure of general formula (Ib)

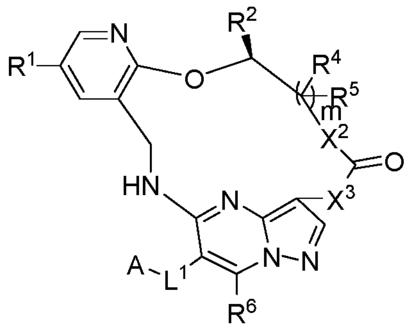

(Ib)

wherein $R^2$ is $C_{1-6}$alkyl, and preferably methyl;
$L^1$, A, $X^2$, $X^3$, $R^1$, $R^4$, $R^5$, $R^6$, m are as defined in any one of claims 1 to 17.

**20.** The compound according to claim 1, which has the following structures

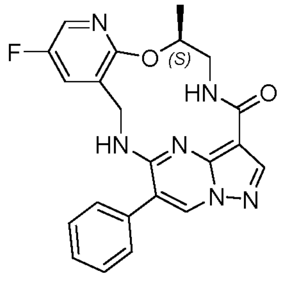

1

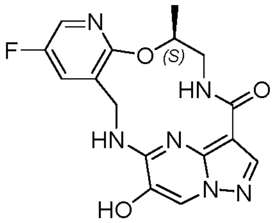

2

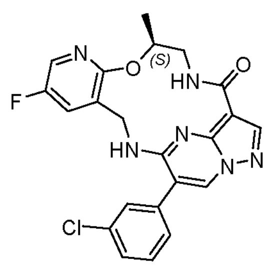

3

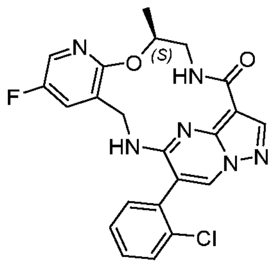

4

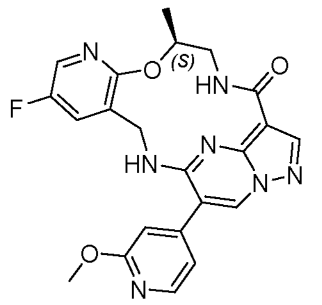

5

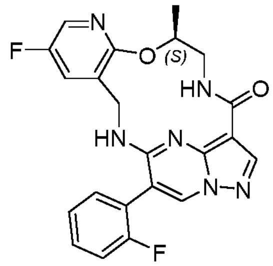

6

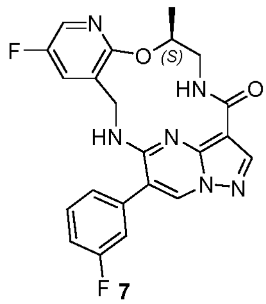

7

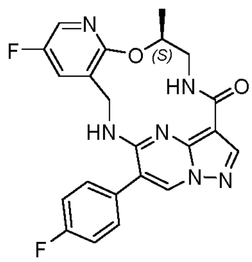

8

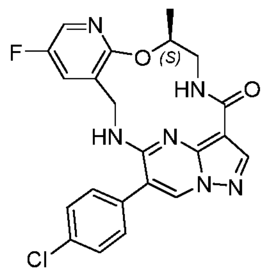

9

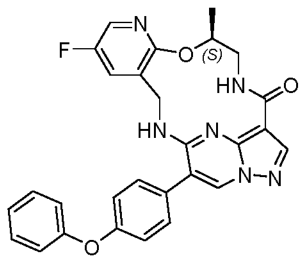

10

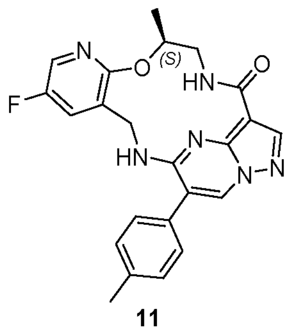

11

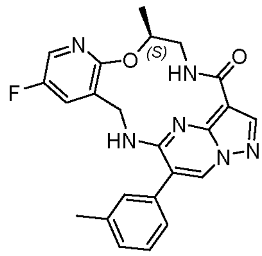

12

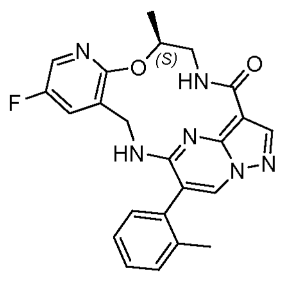

13

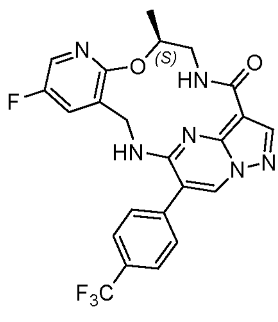

14

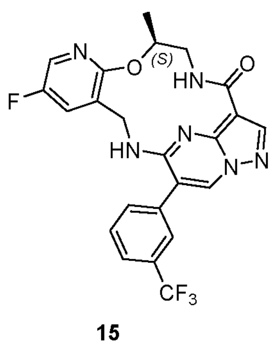

15

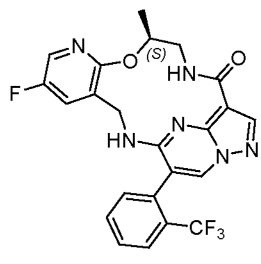

16

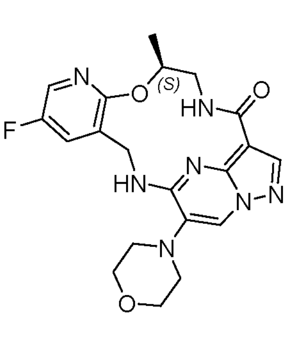

**17**

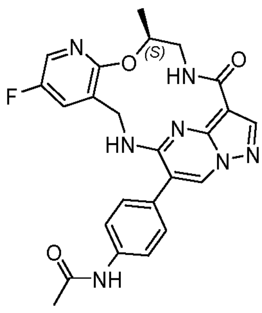

**18**

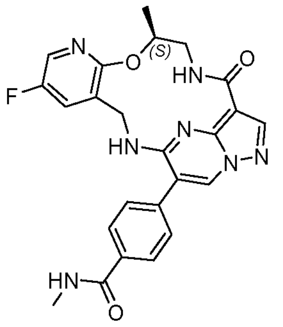

**19**

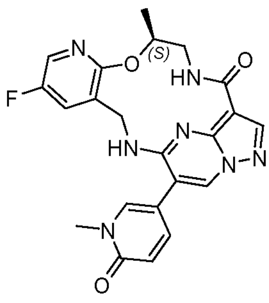

**20**

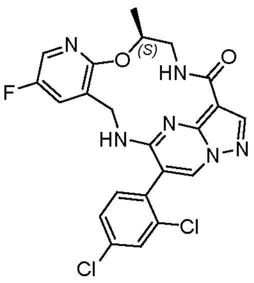

**21**

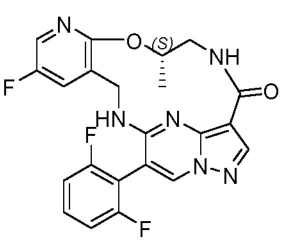

**22**

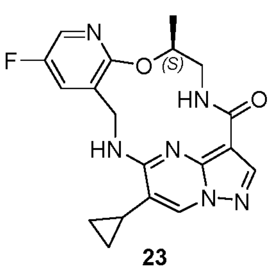

**23**

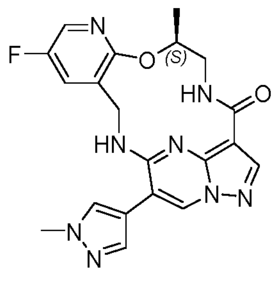

**24**

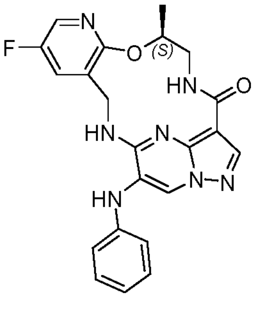

**25**

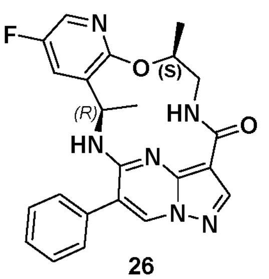

**26**

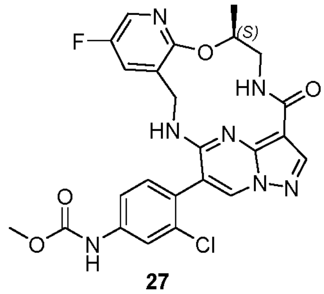

**27**

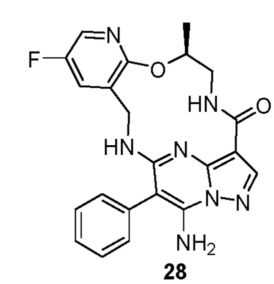

**28**

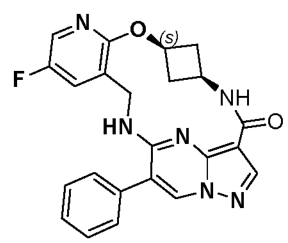

**29**

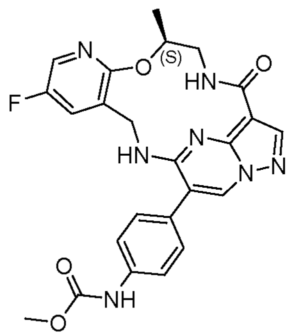

**30**

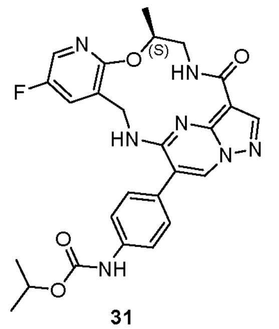

**31**

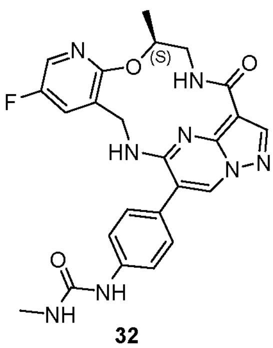

**32**

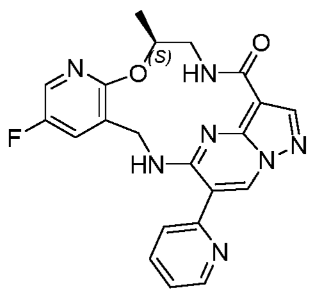
33

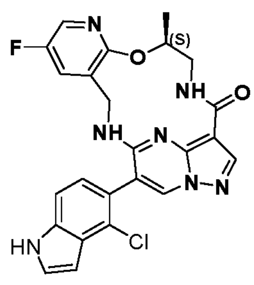
34

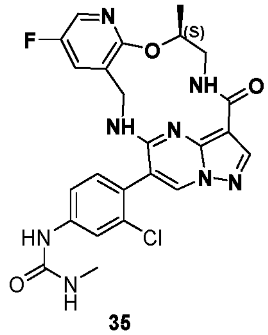
35

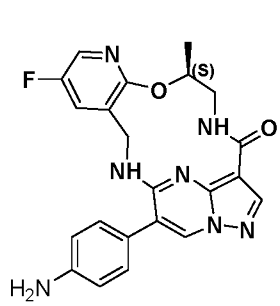
36

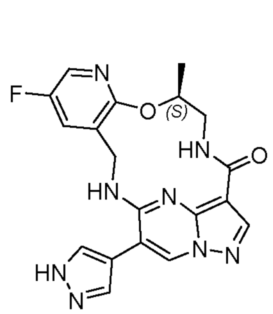
37

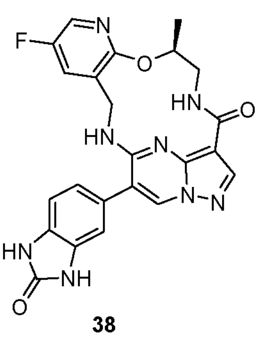
38

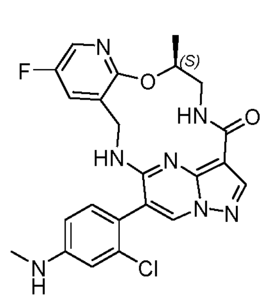
39

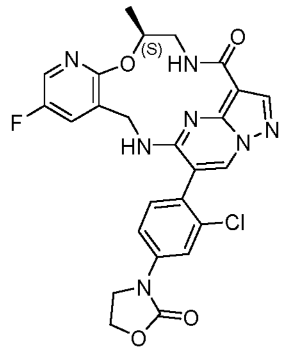
40

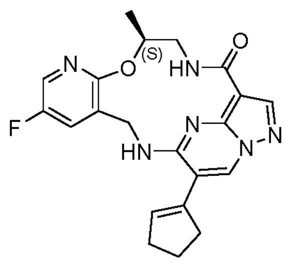
41

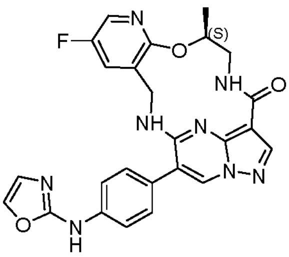
42

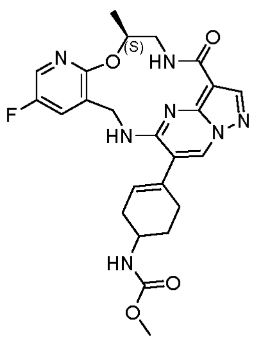
43

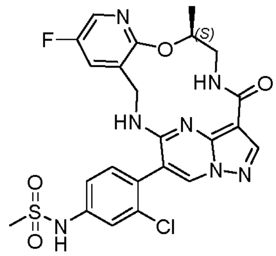
44

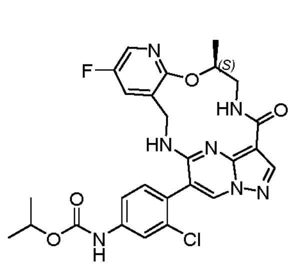
45

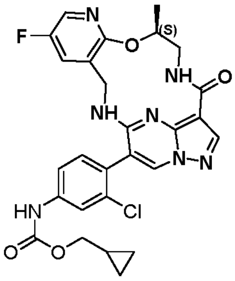
46

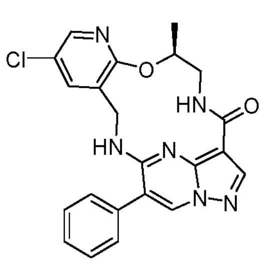
47

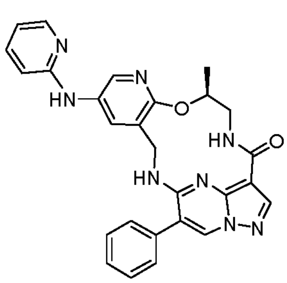
48

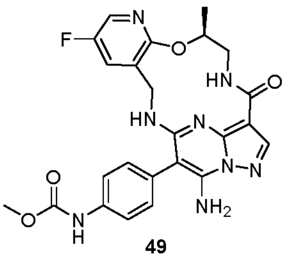
49

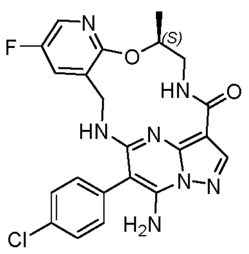
50

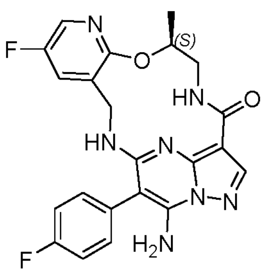
51

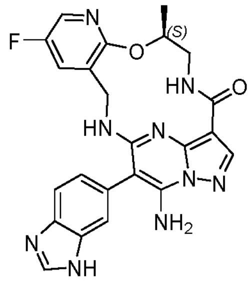
52

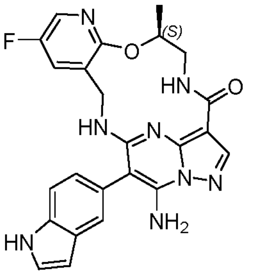
53

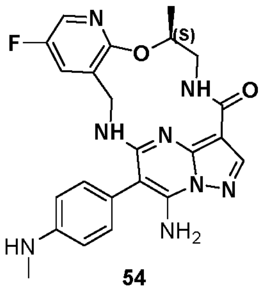
54

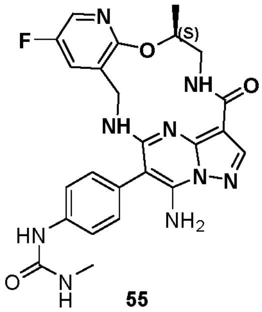
55

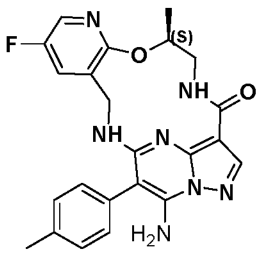
56

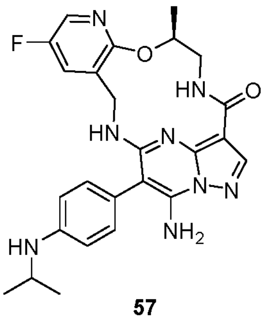
57

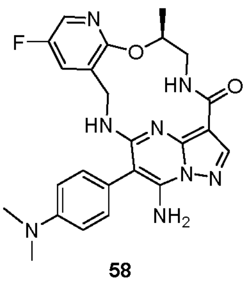
58

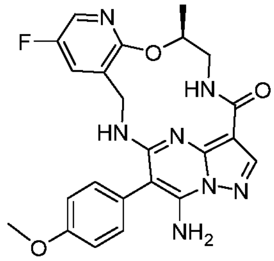
59

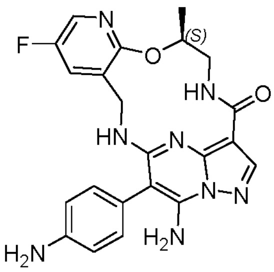
60

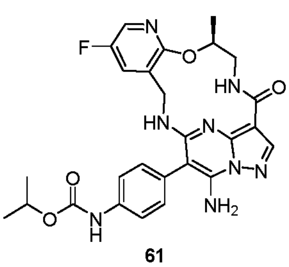
61

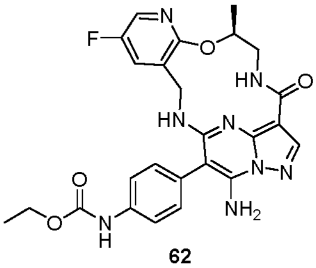
62

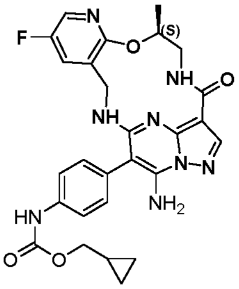
63

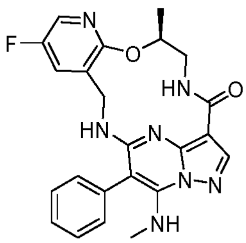
64

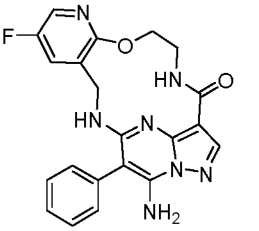
65

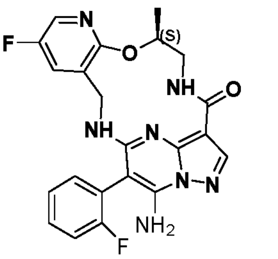
66

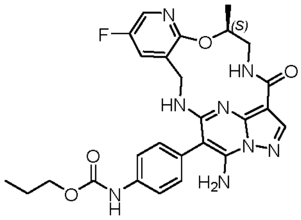
67

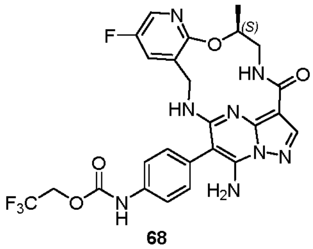
68

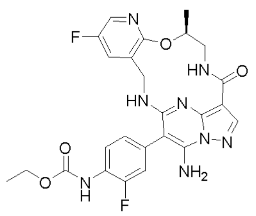
69
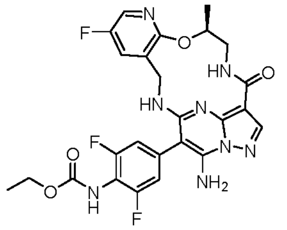
70
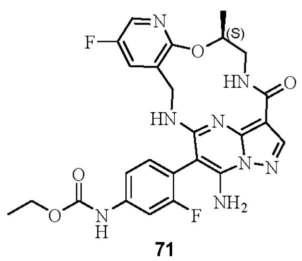
71
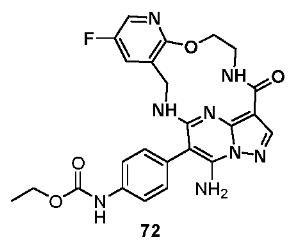
72
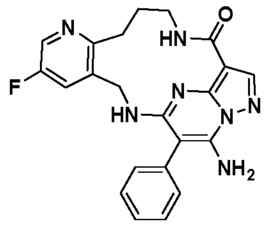
73
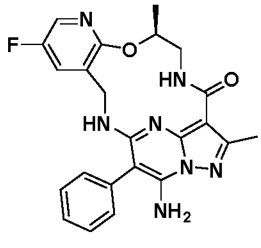
74
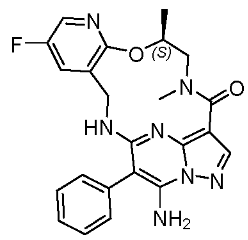
75
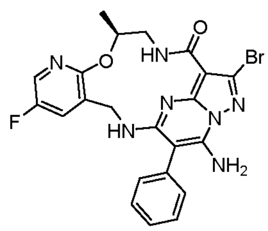
76
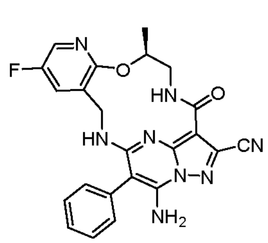
77
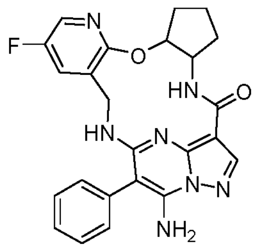
78
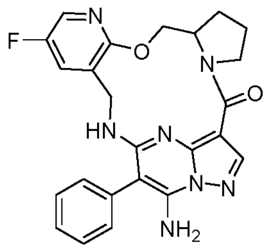
79
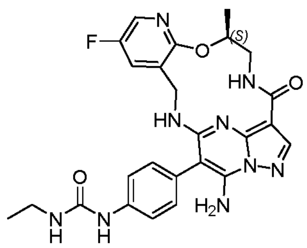
80
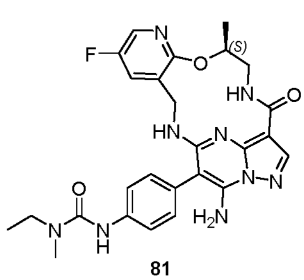
81
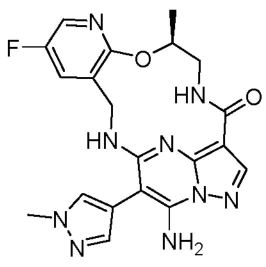
82
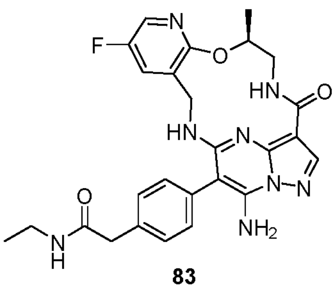
83

or a stereoisomer, a pharmaceutically acceptable salt, a solvate or a tautomer thereof.

**21.** A pharmaceutical composition comprising the compound according to any one of claims 1 to 20 or a stereoisomer, a pharmaceutically acceptable salt, a solvate or a tautomer thereof, as well as pharmaceutically acceptable excipients.

**22.** Use of the compound according to any one of claims 1 to 20 or a stereoisomer, a pharmaceutically acceptable salt, a solvate or a tautomer thereof in the manufacture of a medicament for the treatment of diseases or disorders, wherein said diseases or disorders are selected from cancers.

**23.** The use according to claim 22, wherein said cancers are lung cancer, papillary thyroid cancer, medullary thyroid cancer, differentiated thyroid cancer, recurrent thyroid cancer, refractory differentiated thyroid cancer, type 2A or type 2B multiple endocrine neoplasia (MEN2A or MEN2B respectively), pheochromocytoma, parathyroid hyperplasia, breast cancer, colorectal cancer, papillary renal cell cancer, gastrointestinal mucosal ganglioneuroma, and cervical cancer.

**24.** The use according to claim 23, said cancers are associated with the following dysregulations: RET gene, RET kinase, or caners caused by dysregulated expression or activity or level of either of them.

**25.** The use according to claim 23 or 24, said cancers are medullary thyroid cancer (MTC), non-small cell lung cancer (NSCLC), as well as metastatic solid tumors and advanced solid tumors with RET gene mutations/fusion.

**26.** Use of the compound according to any one of claims 1 to 20 or a stereoisomer, a pharmaceutically acceptable salt, a solvate or a tautomer thereof in the manufacture of a medicament for the treatment of BTK-mediated diseases.

**27.** The use according to claim 26, said BTK-mediated diseases are selected from cancers or autoimmune diseases.

**28.** The use according to claim 27, said cancers are selected from one or more of diffuse large B-cell lymphoma, mantle cell lymphoma, chronic lymphocytic lymphoma, extranodal marginal zone B-cell lymphoma, B-cell chronic lymphocytic leukemia, B-cell prolymphocytic leukemia, mature B-cell acute lymphoblastic leukemia, 17p-deficient chronic lymphoblastic leukemia, Waldenstrom macroglobulinemia, lymphoplasmacytic lymphoma, splenic marginal zone lymphoma, plasma cell myeloma, plasmacytoma, intranodal marginal zone B-cell lymphoma, mantle cell lymphoma, intravascular large B cell lymphoma, and primary exudative lymphoma; said autoimmune diseases are selected from one or more of systemic lupus erythematosus, rheumatoid arthritis, and multiple sclerosis.

**29.** Use of the compound according to any one of claims 1 to 20 or a stereoisomer, a pharmaceutically acceptable salt, a solvate, or a tautomer thereof in the manufacture of a medicament for the treatment of SRC-mediated diseases.

**30.** The use according to claim 29, said SRC-mediated diseases are selected from cancers; preferably, said cancers are selected from one or more of triple-negative breast cancer (TNBC), non-small cell lung cancer, pancreatic cancer, colorectal cancer, and prostate cancer.

**INTERNATIONAL SEARCH REPORT**

International application No. **PCT/CN2021/098124**

**A. CLASSIFICATION OF SUBJECT MATTER**

C07D 487/04(2006.01)i; C07D 519/00(2006.01)i; A61K 31/519(2006.01)i; A61P 35/00(2006.01)i; A61P 35/02(2006.01)i; A61P 29/00(2006.01)i; A61P 37/02(2006.01)i; A61P 37/00(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

WPI, CNPAT, REGISTRY(STN), CAPLUS(STN), CNKI; 大环, 杂环, 稠环, 杂芳基, 嘧啶, 吡唑, 吡啶, 酰胺, 激酶, 抑制剂, 癌, 肿瘤, 淋巴瘤, 白血病, 炎症, 免疫, macrocyclic, derivative? , heterocycle, heteroaromatic, fused, pyrid+, aromatic, RET, rearranged during transfection, BKT, RTK, receptor tyrosine, kinase, inhibitor?, cancer, tumour? , autoimmune, inflammation, immune, structural formula

**C. DOCUMENTS CONSIDERED TO BE RELEVANT**

| Category* | Citation of document, with indication, where appropriate, of the relevant passages | Relevant to claim No. |
|---|---|---|
| X | CN 106170289 A (TP THERAPEUTICS, INC.) 30 November 2016 (2016-11-30) claims 35 and 37, description paragraphs [0300]-[0313], [0328]-[0329], abstract | 1-30 |
| X | CN 109516999 A (ZHENGZHOU TETRANOV MEDICINE CO., LTD.) 26 March 2019 (2019-03-26) claims 1, 15 and 16, description paragraphs [0061], [0071] and [0075] | 1-30 |
| X | CN 107735399 A (TP THERAPEUTICS, INC.) 23 February 2018 (2018-02-23) claims 1, 11, 56-60, description paragraphs [0492]-[0493] | 1-30 |
| X | CN 111171049 A (KBP BIOSCIENCES CO., LTD.) 19 May 2020 (2020-05-19) claims 1, 7, 17-20 | 1-30 |
| X | CN 110950889 A (BEIJING CENTAURUS BIOPHARMA TECHNOLOGY CO., LTD.) 03 April 2020 (2020-04-03) embodiments 1-5, 10-12, 20, 24 | 1, 3, 8-16 |
| A | WO 2019184955 A1 (FOCHON PHARMACEUTICALS, LTD. et al.) 03 October 2019 (2019-10-03) entire description | 1-30 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 August 2021** | **27 August 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/098124**

| Patent document cited in search report | Publication date (day/month/year) | Patent family member(s) | Publication date (day/month/year) |
|---|---|---|---|
| CN 106170289 A | 30 November 2016 | IL 246860 A | 26 September 2019 |
| | | WO 2015112806 A3 | 12 November 2015 |
| | | CN 106170289 B | 23 July 2019 |
| | | US 2017002023 A1 | 05 January 2017 |
| | | WO 2015112806 A2 | 30 July 2015 |
| | | AP 2016009383 A0 | 31 August 2016 |
| | | SG 10202000191 Y A | 30 March 2020 |
| | | JP 2017503867 A | 02 February 2017 |
| | | CL 2016001876 A1 | 03 February 2017 |
| | | EA 031863 B1 | 29 March 2019 |
| | | US 2017334929 A1 | 23 November 2017 |
| | | AP 201609383 A0 | 31 August 2016 |
| | | LT 3097107 T | 25 July 2019 |
| | | US 2019169207 A1 | 06 June 2019 |
| | | EP 3097107 A4 | 09 August 2017 |
| | | SG 11201605929R A | 30 August 2016 |
| | | HR P20191283 T1 | 18 October 2019 |
| | | ZA 201604654 B | 28 October 2020 |
| | | CN 110317214 A | 11 October 2019 |
| | | SI 3097107 T1 | 30 August 2019 |
| | | AU 2019279951 A1 | 16 January 2020 |
| | | DK 3097107 T3 | 08 July 2019 |
| | | PT 3097107 T | 19 July 2019 |
| | | EP 3097107 A2 | 30 November 2016 |
| | | EP 3636649 A1 | 15 April 2020 |
| | | US 2020216465 A1 | 09 July 2020 |
| | | KR 20160111395 A | 26 September 2016 |
| | | AU 2015209239 B2 | 12 September 2019 |
| | | PE 20160931 A1 | 21 September 2016 |
| | | PH 12016501463 A1 | 06 February 2017 |
| | | IL 268820 D0 | 31 October 2019 |
| | | EP 3636648 A1 | 15 April 2020 |
| | | PL 3097107 T3 | 31 January 2020 |
| | | ES 2735729 T3 | 20 December 2019 |
| | | EP 3636650 A1 | 15 April 2020 |
| | | IL 246860 D0 | 31 August 2016 |
| | | HU E045208 T2 | 30 December 2019 |
| | | MX 2016009588 A | 15 May 2017 |
| | | EP 3572416 A1 | 27 November 2019 |
| | | EA 201892241 A1 | 28 February 2019 |
| | | US 10618912 B2 | 14 April 2020 |
| | | US 9714258 B2 | 25 July 2017 |
| | | JP 6490713 B2 | 27 March 2019 |
| | | RS 59059 B1 | 30 August 2019 |
| | | EA 201691492 A1 | 30 June 2017 |
| | | CA 2936079 A1 | 30 July 2015 |
| | | CN 110317213 A | 11 October 2019 |
| | | EP 3097107 B1 | 17 April 2019 |
| | | HK 1231407 A1 | 22 December 2017 |
| CN 109516999 A | 26 March 2019 | None | |

**INTERNATIONAL SEARCH REPORT**
**Information on patent family members**

International application No.
**PCT/CN2021/098124**

| Patent document cited in search report | Publication date (day/month/year) | Patent family member(s) | Publication date (day/month/year) |
|---|---|---|---|
| CN 107735399 A | 23 February 2018 | WO 2017004342 A1 | 05 January 2017 |
| | | EP 3317285 B1 | 27 January 2021 |
| | | US 2018186813 A1 | 05 July 2018 |
| | | RU 2018103907 A3 | 07 October 2019 |
| | | CA 2989327 A1 | 05 January 2017 |
| | | US 10316044 B2 | 11 June 2019 |
| | | AU 2016287568 B2 | 20 August 2020 |
| | | RU 2018103907 A | 05 August 2019 |
| | | EP 3317285 A1 | 09 May 2018 |
| | | RU 2732405 C2 | 16 September 2020 |
| | | IL 256377 D0 | 28 February 2018 |
| | | US 2019284209 A1 | 19 September 2019 |
| | | KR 20180023970 A | 07 March 2018 |
| | | CN 107735399 B | 26 January 2021 |
| | | TW 201716415 A | 16 May 2017 |
| | | AU 2016287568 A1 | 04 January 2018 |
| | | EP 3317285 A4 | 27 February 2019 |
| | | MX 2017017097 A | 23 May 2018 |
| | | AU 2020217336 A1 | 27 August 2020 |
| | | JP 2018519343 A | 19 July 2018 |
| CN 111171049 A | 19 May 2020 | None | |
| CN 110950889 A | 03 April 2020 | None | |
| WO 2019184955 A1 | 03 October 2019 | CA 3093140 A1 | 03 October 2019 |
| | | KR 20200141041 A | 17 December 2020 |
| | | BR 112020019399 A2 | 05 January 2021 |
| | | CN 111971287 A | 20 November 2020 |
| | | EP 3774812 A1 | 17 February 2021 |
| | | AU 2019241260 A1 | 01 October 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019126121 A

### Non-patent literature cited in the description


- **RONEN GABIZON.** *J. Med. Chem.,* 2020, vol. 63, 5100-51011
- **LIAN XU.** *Blood,* 04 May 2017, vol. 129 (18), 2519-2525
- **HOWARD C. et al.** Ansel's Pharmaceutical Dosage Forms and Drug Delivery Systems. Lippincott, Williams & Wilkins, 2004